# EUROPÄISCHE PATENTANMELDUNG

(11) **EP 3 048 155 A1**
(43) Veröffentlichungstag der Anmeldung: **27.07.2016**
(21) Anmeldenummer: 15168307.5
(22) Anmeldetag: 20.05.2015
(51) Int. Cl.: C09K 11/06, C07D 307/00, H01L 51/00, C07D 471/00, C07D 487/00, C08L 23/06

(54) **ORGANISCHE MOLEKÜLE, INSBESONDERE ZUR VERWENDUNG IN OPTOELEKTRONISCHEN BAUELEMENTEN**

(30) Priorität: 20.01.2015 EP 15151870
(71) Anmelder: cynora GmbH, 76646 Bruchsal (DE)
(72) Erfinder: Ambrosek, David, 10437 Berlin (DE); Danz, Michael, 76344 Eggenstein-Leopoldshafen (DE); Flügge, Harald, 76135 Karlsruhe (DE); Friedrichs, Jana, 76137 Karlsruhe (DE); Grab, Tobias, 76133 Karlsruhe (DE); Jacob, Andreas, 76137 Karlsruhe (DE); Seifermann, Stefan, 77815 Bühl (DE); Volz, Daniel, 76137 Karlsruhe (DE)
(74) Vertreter: Hoppe, Georg Johannes

(57) **Zusammenfassung**

Die Erfindung betrifft organische Moleküle, insbesondere zur Verwendung in optoelektronischen Bauelementen, wie OLED. Erfindungsgemäß weist das organische Molekül eine Struktur der Formel 1 auf 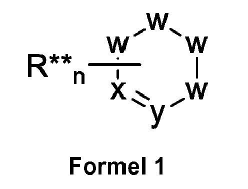 wobei in Formel 1 gilt:
w sind unabhängig voneinander ausgewählt aus sp2-hybridisiertem Kohlenstoff, sp3-hybridisiertem Kohlenstoff, sp2-hybridisiertem Silizium, sp3-hybridisiertem Silizium, sp2-hybridisiertem Stickstoff, sp3-hybridisiertem Stickstoff, oder eine direkte Bindung, wobei in einem erfindungsgemäßen Molekül jeweils nur ein w eine direkte Bindung sein kann und
wobei das zyklische Gerüst aus w, x, y maximal 3 Atome aufweist, die nicht gleich Kohlenstoff sind;
x ist ausgewählt aus sp2-hybridisierten Kohlenstoff, sp2-hybridisierten Silizium oder sp2-hybridisierten Stickstoff;
y ist ausgewählt aus sp2-hybridisierten Kohlenstoff, sp2-hybridisierten Silizium oder sp2-hybridisierten Stickstoff;
n kann alle ganzzahligen Werte von 2 bis q annehmen, wobei q der Gesamtzahl der substituierbaren H-Atome des zyklischen Gerüsts aus w, x, y entspricht;
R** ist entweder ein Rest R* oder eine chemische Einheit AF, wobei in Formel 1 mindestens zwei R** gleich AF sein müssen, wobei mindestens zwei unterschiedliche chemische Einheiten AF1 und AF2 enthalten sind, wobei die Einheiten AF ausgewählt sind aus den in Tabelle 3 aufgeführten Strukturen.

## Beschreibung

Die Erfindung betrifft rein organische Moleküle und deren Verwendung in organischen lichtemittierenden Dioden (OLEDs) und in anderen optoelektronischen Bauelementen.

### Stand der Technik

In den letzten Jahren hat sich die auf OLED (organische lichtemittierende Dioden) basierende Technik im Bereich Bildschirmtechnik etabliert, so dass nun die ersten hierauf aufbauenden kommerziellen Produkte erhältlich werden. Neben der Bildschirmtechnik eignen sich OLEDs auch für die Anwendung in flächiger Beleuchtungstechnik. Aus diesem Grund wird bezüglich der Entwicklung neuer Materialien intensive Forschung betrieben.

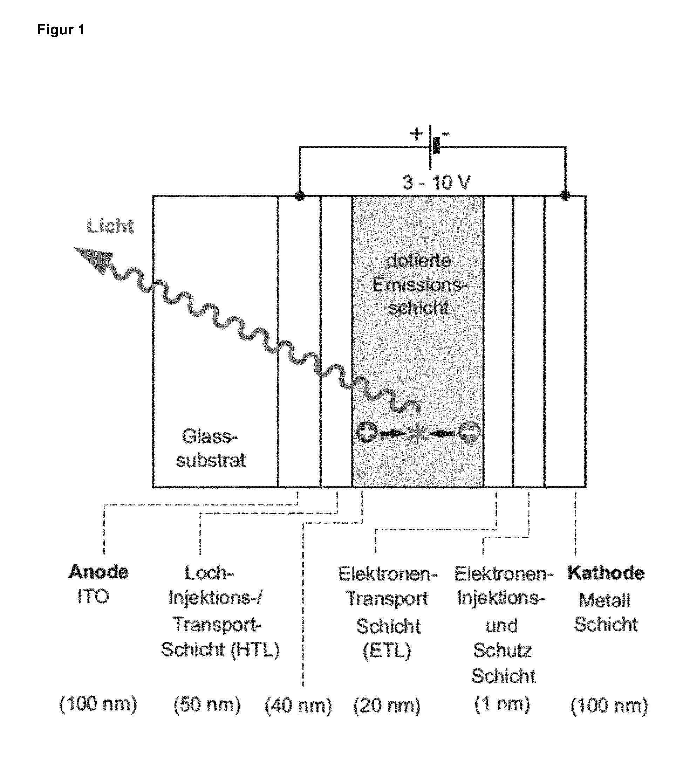

OLEDs sind in der Regel in Schichtenstrukturen realisiert, welche überwiegend aus organischen Materialien bestehen. Zum besseren Verständnis ist in Figur 1 ein vereinfachter Aufbau exemplarisch dargestellt. Herzstück solcher Bauteile ist die Emitterschicht, in der in der Regel emittierende Moleküle in einer Matrix eingebettet sind. In dieser Schicht treffen sich negative Ladungsträger (Elektronen) und positive Ladungsträger (Löcher), die zu sogenannten Exzitonen (= angeregte Zustände) rekombinieren. Die in den Exzitonen enthaltene Energie kann von den entsprechenden Emittern in Form von Licht abgegeben werden, wobei man in diesem Fall von Elektrolumineszenz spricht. Einen Überblick über die Funktion von OLEDs findet sich beispielsweise bei H. Yersin, Top. Curr. Chem. 2004, 241, 1 und H. Yersin, "Highly Efficient OLEDs with Phosphorescent Materials"; Wiley-VCH, Weinheim, Germany, 2008.

Seit den ersten Berichten bezüglich OLEDs (Tang et al. Appl. Phys. Lett. 1987, 51, 913) ist diese Technik besonders auf dem Gebiet der Emittermaterialien immer weiterentwickelt worden. Während die ersten Materialien, die auf rein organischen Molekülen beruhen, aufgrund von Spinstatistik maximal 25 % der Exzitonen in Licht umwandeln konnten, konnte durch die Verwendung von phosphoreszierenden Verbindungen dieses grundsätzliche Problem umgangen werden, so dass zumindest theoretisch alle Exzitonen in Licht umgewandelt werden können. Bei diesen Materialien handelt es sich in der Regel um Übergangsmetall-Komplexverbindungen, in denen das Metall aus der dritten Periode der Übergangsmetalle gewählt wird. Hierbei werden vorwiegend sehr teure Edelmetalle wie Iridium, Platin oder auch Gold eingesetzt. (Siehe dazu auch H. Yersin, Top. Curr. Chem. 2004, 241, 1 und M. A. Baldo, D. F. O'Brien, M. E. Thompson, S. R. Forrest, Phys. Rev. B 1999, 60, 14422). Neben den Kosten ist auch die Stabilität der Materialien zum Teil nachteilig für die Verwendung.

Eine neue Generation von OLEDs basiert auf der Ausnutzung von verzögerter Fluoreszenz (TADF: thermally activated delayed fluorescence oder auch singlet harvesting). Hierbei können beispielsweise Cu(l)-Komplexe verwendet werden, die aufgrund eines geringen Energieabstandes zwischen dem untersten Triplett-Zustand T₁ und dem darüberliegenden Singulett-Zustand S₁ (ΔE(S₁-T₁) Triplett-Exitonen thermisch in einen Singulett-Zustand rückbesetzen können. Neben der Verwendung von Übergangsmetallkomplexen können auch rein organische Moleküle (ohne Metallion) diesen Effekt ausnutzen.

### Beschreibung

Die Erfindung betrifft in einem Aspekt rein organische Moleküle, die in optoelektronischen Bauelementen verwendet werden können.

Derartige organische Moleküle weisen eine Struktur der Formel 1 auf oder haben eine Struktur der Formel 1:

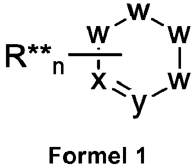

In Formel 1 bedeutet:
w sind unabhängig voneinander ausgewählt aus sp2-hybridisiertem Kohlenstoff, sp3-hybridisiertem Kohlenstoff, sp2-hybridisiertem Silizium, sp3-hybridisiertem Silizium, sp2-hybridisiertem Stickstoff, sp3-hybridisiertem Stickstoff, oder eine direkte Bindung, wobei in einem erfindungsgemäßen Molekül jeweils nur ein w eine direkte Bindung sein kann und wobei das zyklische Gerüst aus w, x, y maximal 3 Atome aufweist, die nicht gleich Kohlenstoff sind;
x ist ausgewählt aus sp2-hybridisierten Kohlenstoff, sp2-hybridisierten Silizium oder sp2-hybridisierten Stickstoff;
y ist ausgewählt aus sp2-hybridisierten Kohlenstoff, sp2-hybridisierten Silizium oder sp2-hybridisierten Stickstoff;
n kann alle ganzzahligen Werte von 2 bis q annehmen, wobei q der Gesamtzahl der substituierbaren H-Atome des zyklischen Gerüsts aus w, x, y entspricht;
R** ist entweder ein Rest R* oder eine chemische Einheit AF, wobei in Formel 1 mindestens zwei R** gleich AF sein müssen;
R* ist bei jedem Auftreten unabhängig voneinander ausgewählt aus der Gruppe bestehend aus H, Deuterium, Phenyl, Naphthyl, N(R²)₂, -CN, -NC, -SCN, -CF₃, -NO₂, C(=O)OH, C(=O)OR , C(=O)N(R³)₂, C(=O)SR³ , C(=S)SR³, Si(R⁴)₃, B(OR⁵)₂, B(N(R⁶)₂)₂, C(=O)R³, P(=O)(R⁷)₂, As(=O)(R⁷)₂, P(=S)(R⁷)₂, As(=S)(R⁷)₂, S(=O)R³, S=NR³, S(=O)NR³, S(=O)₂NR³, S(=O)₂R³, O-S(=O)₂R³, SF₅, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R⁹ substituiert sein kann, wobei eine oder mehrere benachbarte CH₂-Gruppen durch -R⁹C=CR⁹-, -C≡C-, bzw. eine benachbarte CH₂-Gruppe durch -Si(R⁴)₂-, -Ge(R⁴)₂-, -Sn(R⁴)₂, -C(=O)-, -C(=S)-, -C(=Se)-, - C=N-, -C(=O)O-, -C(=O)N(R³)-, -P(=O)(R⁷)-, -As(=O)(R⁷)-, -P(=S)(R⁷), -As(=S)(R⁷)-, -S(=O)-, -S(=O)₂-, -NR²-, -O-, oder -S- ersetzt sein können und wobei ein oder mehrere H-Atome durch Deuterium, F, CI, Br, I, CN, CF₃ oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R⁹ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R⁹ substituiert sein kann, oder eine Kombination dieser Systeme; dabei können zwei oder mehrere dieser Substituenten R* auch miteinander ein monozyklisches aliphatisches Ringsystem mit insgesamt fünf oder sechs Ringgliedern bilden;
R² ist bei jedem Auftreten unabhängig voneinander ausgewählt aus der Gruppe bestehend aus H, Deuterium, Phenyl, Naphthyl, CF₃, C(=O)OR , C(=O)N(R²)₂, Si(R⁴)₃, C(=O)R³, P(=O)(R⁷)₂, As(=O)(R⁷)₂ P(=S)(R⁷)₂, As(=S)(R⁷)₂, S(=O)R³ , S(=O)₂R³, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R⁹ substituiert sein kann, wobei eine oder mehrere benachbarte CH₂-Gruppen durch -R⁹C=CR⁹-, -C≡C-, bzw. eine benachbarte CH₂-Gruppe durch -Si(R⁴)₂-, - Ge(R⁴)₂-, -Sn(R⁴)₂, -C(=O)-, -C(=S)-, -C(=Se)-, -C=N-, -C(=O)O-, -C(=O)N(R³)-, -P(=O)(R⁷)-, - As(=O)(R⁷)-, -P(=S)(R⁷)-, -As(=S)(R⁷)-, -S(=O)-, -S(=O)₂-, -NR²-, -O-, oder -S- ersetzt sein können und wobei ein oder mehrere H-Atome durch Deuterium, F, Cl, Br, I, CN, CF₃ oder NO₂ ersetzt sein können, oder ein aromatisches oder heteroaromatisches Ringsystem mit 5 bis 60 aromatischen Ringatomen, das jeweils durch einen oder mehrere Reste R⁹ substituiert sein kann, oder eine Aryloxy- oder Heteroaryloxygruppe mit 5 bis 60 aromatischen Ringatomen, die durch einen oder mehrere Reste R⁹ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R⁹ substituiert sein kann, oder eine Kombination dieser Systeme; dabei können zwei oder mehrere dieser Substituenten R² auch miteinander ein mono- oder polycyclisches, aliphatisches, aromatisches und/oder benzoannelliertes Ringsystem bilden;
R³ ist bei jedem Auftreten unabhängig voneinander ausgewählt aus der Gruppe bestehend aus H, Deuterium, Phenyl, Naphthyl, CF₃ oder ein aliphatischer, aromatischer und/oder heteroaromatischer Kohlenwasserstoffrest mit 1 bis 20 C-Atomen, in dem auch ein oder mehrere H-Atome durch F oder CF₃ ersetzt sein können; dabei können zwei oder mehrere Substituenten R³ auch miteinander ein mono- oder polycyclisches, aliphatisches Ringsystem bilden;
R⁴ ist bei jedem Auftreten unabhängig voneinander ausgewählt aus der Gruppe bestehend aus H, Deuterium, Phenyl, Naphthyl, N(R²)₂, CN, CF₃, OH, C(=O)OR , C(=O)N(R³)₂, C(=O)R³, P(=O)(R⁷)₂, As(=O)(R⁷)₂, P(=S)(R⁷)₂, As(=S)(R⁷)₂, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R⁹ substituiert sein kann, wobei eine oder mehrere benachbarte CH₂-Gruppen durch - R⁹C=CR⁹-, -C≡C-, bzw. eine benachbarte CH₂-Gruppe durch -Si(R⁴)₂-, -Ge(R⁴)₂-, -Sn(R⁴)₂, - C(=O)-, -C(=S)-, -C(=Se)-, -C=N-, -C(=O)O-, -C(=O)N(R³)-, -P(=O)(R⁷)-, -As(=O)(R⁷)-, - P(=S)(R⁷), -As(=S)(R⁷)-, -S(=O)-, -S(=O)₂-, -NR²-, -O-, oder -S- ersetzt sein können und wobei ein oder mehrere H-Atome durch Deuterium, F, CI, Br, I, CN, CF₃ oder NO₂ ersetzt sein können, oder ein aromatisches oder heteroaromatisches Ringsystem mit 5 bis 60 aromatischen Ringatomen, das jeweils durch einen oder mehrere Reste R⁸ substituiert sein kann, oder eine Aryloxy- oder Heteroaryloxygruppe mit 5 bis 60 aromatischen Ringatomen, die durch einen oder mehrere Reste R⁹ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R⁹ substituiert sein kann, oder eine Kombination dieser Systeme; dabei können zwei oder mehrere dieser Substituenten R⁴ auch miteinander ein mono- oder polycyclisches, aliphatisches, aromatisches und/oder benzoannelliertes Ringsystem bilden;
R⁵ ist bei jedem Auftreten unabhängig voneinander ausgewählt aus der Gruppe bestehend aus Phenyl, Naphthyl, CF₃, C(=0)R , P(=O)(R⁷)₂, As(=O)(R⁷)₂, eine lineare Alkyl-, Alkoxy-oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy-oder Thioalkoxygruppe mit 3 bis 40 C-Atomen, die jeweils mit einem oder mehreren Resten R⁹ substituiert sein kann, wobei eine oder mehrere benachbarte CH₂-Gruppen durch - R⁹C=CR⁹-, -C≡C-, bzw. eine benachbarte CH₂-Gruppe durch -Si(R⁴)₂-, -Ge(R⁴)₂-, -Sn(R⁴)₂, - C(=O)-, -C(=S)-, -C(=Se)-, -C=N-, -C(=O)O-, -C(=O)N(R³)-, -P(=O)(R⁷)-, -As(=O)(R⁷)-, - P(=S)(R⁷)-, -As(=S)(R⁷)-, -S(=O)-, -S(=O)₂-, -NR²-, -O-, oder -S- ersetzt sein können und wobei ein oder mehrere H-Atome durch Deuterium, F, CI, Br, I, CN, CF₃ oder NO₂ ersetzt sein können, oder ein aromatisches oder heteroaromatisches Ringsystem mit 5 bis 60 aromatischen Ringatomen, das jeweils durch einen oder mehrere Reste R⁹ substituiert sein kann, oder eine Aryloxy- oder Heteroaryloxygruppe mit 5 bis 60 aromatischen Ringatomen, die durch einen oder mehrere Reste R⁹ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R⁹ substituiert sein kann, oder eine Kombination dieser Systeme; dabei können zwei oder mehrere dieser Substituenten R⁵ auch miteinander ein mono- oder polycyclisches, aliphatisches, aromatisches und/oder benzoannelliertes Ringsystem bilden;
R⁶ ist bei jedem Auftreten unabhängig voneinander ausgewählt aus der Gruppe bestehend aus Phenyl, Naphthyl, CF₃, Si(R⁴)₃, C(=O)R³, P(=O)(R⁷)₂, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R⁹ substituiert sein kann, wobei eine oder mehrere benachbarte CH₂-Gruppen durch - R⁹C=CR⁹-, -C≡C-, bzw. eine benachbarte CH₂-Gruppe durch-Si(R⁴)₂-, -Ge(R⁴)₂-, -Sn(R⁴)₂, - C(=O)-, -C(=S)-, -C(=Se)-, -C=N-, -C(=O)O-, -C(=O)N(R³)-, -P(=O)(R⁷)-, -As(=O)(R⁷)-, - P(=S)(R⁷), -As(=S)(R⁷)-, -S(=O)-, -S(=O)₂-, -NR²-, -O-, oder -S- ersetzt sein können und wobei ein oder mehrere H-Atome durch Deuterium, F, CI, Br, I, CN, CF₃ oder NO₂ ersetzt sein können, oder ein aromatisches oder heteroaromatisches Ringsystem mit 5 bis 60 aromatischen Ringatomen, das jeweils durch einen oder mehrere Reste R⁹ substituiert sein kann, oder eine Aryloxy- oder Heteroaryloxygruppe mit 5 bis 60 aromatischen Ringatomen, die durch einen oder mehrere Reste R* substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R⁹ substituiert sein kann, oder eine Kombination dieser Systeme; dabei können zwei oder mehrere dieser Substituenten R⁶ auch miteinander ein mono- oder polycyclisches, aliphatisches, aromatisches und/oder benzoannelliertes Ringsystem bilden;
R⁷ ist bei jedem Auftreten unabhängig voneinander ausgewählt aus der Gruppe bestehend aus, Phenyl, Naphthyl, N(R²)₂, CN, CF₃, C(=O)OR , C(=O)N(R³)₂, Si(R⁴)₃, C(=O)R³, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R⁹ substituiert sein kann, wobei eine oder mehrere benachbarte CH₂-Gruppen durch -R⁹C=CR⁹-, -C≡C-, bzw. eine benachbarte CH₂-Gruppe durch -Si(R⁴)₂-, -Ge(R⁴)₂-, -Sn(R⁴)₂, -C(=O)-, -C(=S)-, -C(=Se)-, -C=N-, -C(=O)O-, - C(=O)N(R³)-, -P(=O)(R⁷)-, -As(=O)(R⁷)-, -P(=S)(R⁷), -As(=S)(R⁷)-, -S(=O)-, -S(=O)₂-, -NR²-, - O-, oder -S- ersetzt sein können und wobei ein oder mehrere H-Atome durch Deuterium, F, CI, Br, I, CN, CF₃ oder NO₂ ersetzt sein können, oder ein aromatisches oder heteroaromatisches Ringsystem mit 5 bis 60 aromatischen Ringatomen, das jeweils durch einen oder mehrere Reste R⁹ substituiert sein kann, oder eine Aryloxy- oder Heteroaryloxygruppe mit 5 bis 60 aromatischen Ringatomen, die durch einen oder mehrere Reste R⁹ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R³ substituiert sein kann, oder eine Kombination dieser Systeme; dabei können zwei oder mehrere dieser Substituenten R⁷ auch miteinander ein mono- oder polycyclisches, aliphatisches, aromatisches und/oder benzoannelliertes Ringsystem bilden;
R⁸ ist bei jedem Auftreten unabhängig voneinander ausgewählt aus der Gruppe bestehend aus H, Deuterium, Phenyl, Naphthyl, F, CF₃ oder ein aliphatischer, aromatischer und/oder heteroaromatischer Kohlenwasserstoffrest mit 1 bis 20 C-Atomen, in dem auch ein oder mehrere H-Atome durch F oder CF₃ ersetzt sein können; dabei können zwei oder mehrere Substituenten R⁸ auch miteinander ein mono- oder polycyclisches, aliphatisches Ringsystem bilden;
R⁹ ist bei jedem Auftreten unabhängig voneinander ausgewählt aus der Gruppe bestehend aus H, Deuterium, Phenyl, Naphthyl, N(R²)₂, CN, CF₃, NO₂, OH, COOH, C(=O)OR , C(=O)N(R³)₂, Si(R⁴)₃, B(OR⁵)₂, C(=O)R³, P(=O)(R⁷)₂, P(=S)(R⁷)₂, As(=O)(R⁷)₂, P(=S)(R⁷)₂, S(=O)R³, S(=O)₂R³, OSO₂R³, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R⁸ substituiert sein kann, wobei eine oder mehrere nicht benachbarte CH₂-Gruppen durch -R³C=CR³-, -C≡C-, bzw. eine benachbarte CH₂-Gruppe durch -Si(R⁴)₂-, -Ge(R⁴)₂-, -Sn(R⁴)₂, -C(=O)-, -C(=S)-, -C(=Se)-, - C=N-, -C(=O)O-, -C(=O)N(R³)-, -P(=O)(R⁷)-, -As(=O)(R⁷)-, -P(=S)(R⁷), -As(=S)(R⁷)-, -S(=O)-, -S(=O)₂-, -NR²-, -O-, oder -S- ersetzt sein können und wobei ein oder mehrere H-Atome durch Deuterium, F, CI, Br, I, CN, CF₃ oder NO₂ ersetzt sein können, oder ein aromatisches oder heteroaromatisches Ringsystem mit 5 bis 60 aromatischen Ringatomen, das jeweils durch einen oder mehrere Reste R⁸ substituiert sein kann, oder eine Aryloxy- oder Heteroaryloxygruppe mit 5 bis 60 aromatischen Ringatomen, die durch einen oder mehrere Reste R³ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R⁸ substituiert sein kann, oder eine Kombination dieser Systeme; dabei können zwei oder mehrere dieser Substituenten R⁹ auch miteinander ein mono- oder polycyclisches, aliphatisches, aromatisches und/oder benzoannelliertes Ringsystem bilden;

Eine Arylgruppe im Sinne dieser Erfindung enthält 6 bis 60 aromatische Ringatome; eine Heteroarylgruppe im Sinne dieser Erfindung enthält 5 bis 60 aromatische Ringatome, von denen mindestens eines ein Heteroatom darstellt. Die Heteroatome sind bevorzugt N, O, und S. Dies stellt die grundlegende Definition dar. Werden in der Beschreibung der vorliegenden Erfindung andere Bevorzugungen angegeben, beispielsweise bezüglich der Zahl der aromatischen Ringatome oder der enthaltenen Heteroatome, so gelten diese;

Dabei wird unter einer Arylgruppe bzw. Heteroarylgruppe ein einfacher aromatischer Cyclus, also Benzol, bzw. ein einfacher heteroaromatischer Cyclus, beispielsweise Pyridin, Pyrimidin oder Thiophen, oder ein heteroaromatischer Polycyclus, beispielsweise Napthalin, Phenanthren, Chinolin oder Carbazol verstanden. Ein kondensierter (annelierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen;

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen; Pyrrol, Indol, Isoindol, Carbazol, Pyridin, Chinolin, Isochinolin, Acridin, Phenanthridin, Benzo-5,6-chinolin, Isochinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Napthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benztriazol, 1,2,3-Oxadiazol, 1,2,4-Oxadiazol, 1,2,5-Oxadiazol, 1,2,3,4-Tetrazin, Purin, Pteridin, Indolizin und Benzothiadiazol;

Ein aromatisches Ringsystem im Sinne dieser Erfindung enthält 6 bis 60 C-Atome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind bevorzugt ausgewählt aus, N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10% der verschiedenen Atome), wie z.B. ein sp3-hybridisiertes C-, Si-, oder N-Atom, ein sp2-hybridisiertes C-, N- oder O-Atom oder ein sp-hybridisiertes C-Atom, verbunden sein können. So sollen beispielsweise auch Systeme wie 9,9'-Diarylfluoren, Triarylamin, Diarylether, Stilben etc. als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Aryl- oder Heteroarylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin;

Unter einem aromatischen oder heteroaromatischen Ringsystem mit 5 bis 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Napthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyarzinimidazol, Chinoxalinimidazol, Oxazol, Benzoxazol, Napthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,2,3 Oxadiazol, 1,2,3-Thiadiazol, 1,2,5-Thiadiazol, 1,3,4-Thiadiazol, 1,3,5-Triazin, 1,2,4-Triazin, 1,2,3-Triazin, Tetrazol, 1,2,3,5-Tetrazin, 1,2,3,4-Tetrazin, Purin, Pteridin, Indolizin und Benzothiadiazol oder Kombinationen dieser Gruppen;
AF: Organische chemische Einheit (Rest). Es sind mindestens zwei unterschiedliche chemische Einheiten AF1 und AF2 enthalten,
AF1: eine erste chemische Einheit, aufweisend ein konjugiertes System, insbesondere mindestens sechs in Konjugation stehende π-Elektronen (z. B. in Form mindestens eines aromatischen Systems);
AF2: eine zweite chemische Einheit, aufweisend ein konjugiertes System, insbesondere mindestens sechs in Konjugation stehende π-Elektronen (z. B. in Form mindestens eines aromatischen Systems);
wobei die chemischen Einheiten AF aus der in Tabelle 3 angegebenen Gruppe von chemischen Einheiten ausgewählt ist;
und wobei definitionsgemäß AF2 im Vergleich zu AF1 immer einen betragsmäßig niedrigeren HOMO-Zahlenwert aufweist (und damit auch entsprechend einen betragsmäßig niedrigeren LUMO-Zahlenwert) als AF2 (| E_{HOMO}(AF2)| < |E_{HOMO}(AF1)| und |E_{LUMO}(AF2)| < | E_{LUMO}(AF1) |).

Die erfindungsgemäßen Moleküle zeichnen sich insbesondere dadurch aus, dass
- die Differenz der Energie des HOMO der zweiten chemischen Einheit AF2 und der Energie des HOMO der ersten chemischen Einheit AF1 > 0,8 eV ist (Δ HOMO = HOMO(AF2) - HOMO(AF1) > 0,8 eV);
- die Differenz der Energie des LUMO der zweiten chemischen Einheit AF2 und der Energie des LUMO der ersten chemischen Einheit AF1 > 0,8 eV ist (Δ LUMO = LUMO(AF2) - LUMO(AF1) > 0,8 eV); und/oder
- die Differenz der Energie des LUMO der ersten chemischen Einheit AF1 und der Energie des HOMO der zweiten chemischen Einheit AF2 > 0,9 eV ist (Δ Gap = LUMO (AF1) - HOMO(AF2)> 0,9 eV).

Dabei werden die Energiewerte HOMO(AF1), HOMO(AF2), LUMO(AF1), LUMO(AF2) mithilfe der Dichtefunktionaltheorie (DFT) berechnet, wobei die Anknüpfungspositionen der ambifunktionalen Einheiten und der Separatoren mit einem Wasserstoffatom entsprechend ihrer chemischen Valenzen abgesättigt werden. Die angegeben Grenzen beziehen sich auf Orbitalenergien in eV, die mit dem BP86-Funktional berechnet werden (Becke, A. D. Phys. Rev. A1988, 38, 3098-3100; Perdew, J. P. Phys. Rev. B1986, 33, 8822-8827).

In einer Ausführungsform weisen die organischen Moleküle eine Struktur der Formel 2a oder 2b auf oder haben eine Struktur der Formel 2a oder 2b, wobei die bei Formel 1 angegebenen Definitionen für w, x, y gelten und das organische Molekül mindestens zwei AF aufweist;
wobei gilt: n ist 1 bis q-1, m ist 1 bis q-1, wobei q die Gesamtzahl der substituierbaren H-Atome des zyklischen Gerüsts aus w, x, y ist;
und wobei jedes substituierbare H-Atom der erfindungsgemäßen Moleküle optional durch einen Rest R* substituiert ist;
und wobei z ein sp3-hybridisiertes Kohlenstoffatom (-CR¹⁰₂-), sp3-hybridisiertes Stickstoffatom (-NR¹⁰-), sp3-hybridisiertes Sauerstoffatom oder sp3-hybridisiertes Schwefelatom sein kann;
und wobei im Falle z = (-NR¹⁰-) oder z = (-CR¹⁰₂-) gilt: R¹⁰ ist bei jedem Auftreten unabhängig voneinander ausgewählt aus der Gruppe bestehend aus H, Deuterium, Phenyl, Naphthyl, F, CF₃ oder ein aliphatischer, aromatischer und/oder heteroaromatischer Kohlenwasserstoffrest mit 1 bis 20 C-Atomen, in dem auch ein oder mehrere H-Atome durch F oder CF₃ ersetzt sein können; dabei können zwei oder mehrere Substituenten R auch miteinander ein mono-oder polycyclisches, aliphatisches Ringsystem bilden, außerdem kann R sein: eine lineare Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen;

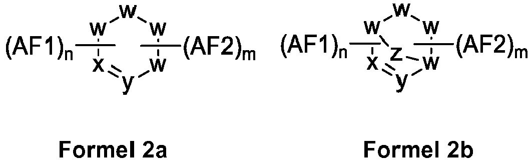

In einer Ausführungsform weisen die organischen Moleküle eine Struktur der Formel 1 oder Formel 2b auf oder haben eine Struktur der Formel 1 oder Formel 2b, wobei die zentrale zyklische Einheit aus w, x, y durch eine [4+2]-Cycloaddition mit normalen Elektronenbedarf (Reaktion zwischen einem elektronenreichen Dien mit einem elektronenarmem Dienophil) gemäß Schema 1 aufgebaut wurde. Es gelten die Definitionen für Formel 2b, wobei optional z nicht enthalten ist.

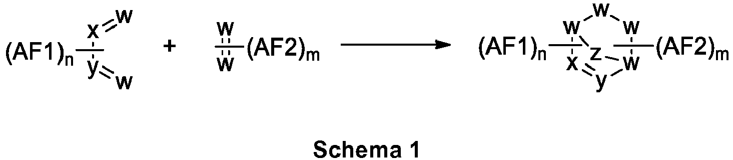

In einer weiteren Ausführungsform weisen die organischen Moleküle eine Struktur der Formel 1 oder Formel 2b auf oder haben eine Struktur der Formel 1 oder Formel 2b, wobei die zentrale zyklische Einheit durch eine [4+2]-Cycloaddition mit inversen Elektronenbedarf (Reaktion eines elektronenarmen Diens mit einem elektronenreichen Dien) gemäß Schema 2 aufgebaut wurde. Es gelten die Definitionen für Formel 2b, wobei optional z nicht enthalten ist.

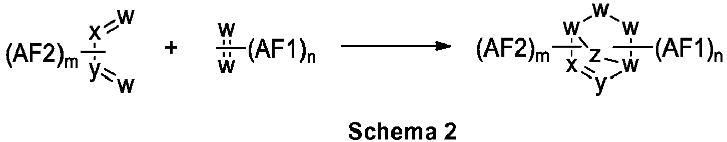

In einer weiteren Ausführungsform weisen die organischen Moleküle eine Struktur der Formel 1 oder Formel 2b auf oder haben eine Struktur der Formel 1 oder Formel 2b, wobei die zentrale zyklische Einheit durch eine [4+2]-Cycloaddition gemäß Schema 2 aufgebaut wurde. Es gelten die Definitionen für Formel 2b, wobei optional z nicht enthalten ist.

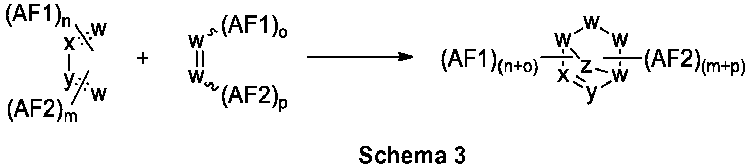

Die zyklische Einheit des organischen Moleküls, enthaltend w, x, y, gemäß Formel 1, Formel 2a oder Formel 2b, hat in den erfindungsgemäßen Molekülen insbesondere die Aufgabe, eine unerwünschte elektronische Kommunikation der einzelnen chemischen Einheiten AF untereinander, welche über konjugierte π-Bindungen stattfindet, zu unterbinden. Daher wird die zyklische Einheit auch als Separator S bezeichnet. Einen Separator im erfindungsgemäßen Sinne stellt die Struktur 2-0 dar. Diese ergibt sich nach Schema 4 aus der Diels-Alder-Reaktion eines Diens mit einem Dienophil.

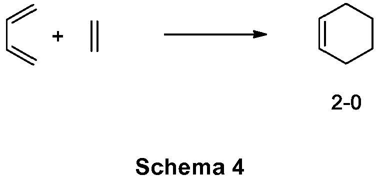

Für den Aufbau eines erfindungsgemäßen Moleküles müssen Dien und/oder Dienophil - wie in Schema 2 und 3 gezeigt - mit den chemischen Einheiten AF1 und AF2 substituiert sein, wobei sämtliche substituierbaren H-Atome des Diens und/oder des Dienophils durch chemische Einheiten AF1 und AF2 ersetzt sein können; wobei mindestens eine Einheit AF1 und mindestens eine von AF1 verschiedene Einheit AF2 im resultierenden Molekül enthalten sein müssen. In Tabelle 1 sind besonders bevorzugte erfindungsgemäße Moleküle, die den Separator 2-0 enthalten und gemäß Schema 1 und Schema 2 darstellbar sind, aufgeführt; sie ergeben sich durch Diels-Alder-Reaktionen unterschiedlich substituierter Diene und Dienophile. Optional kann jedes substituierbare H-Atom der resultierenden Moleküle durch einen Rest R* ersetzt sein.

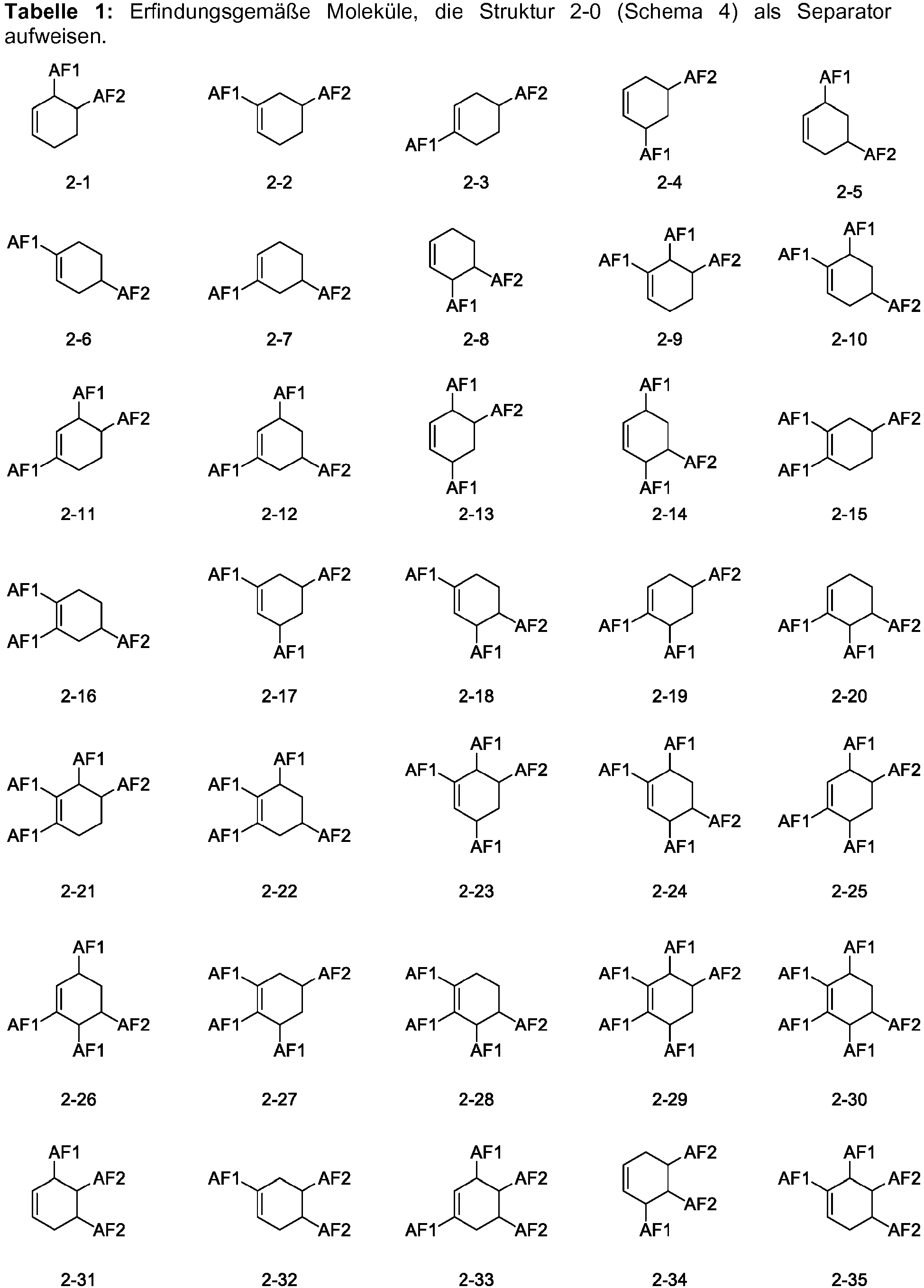

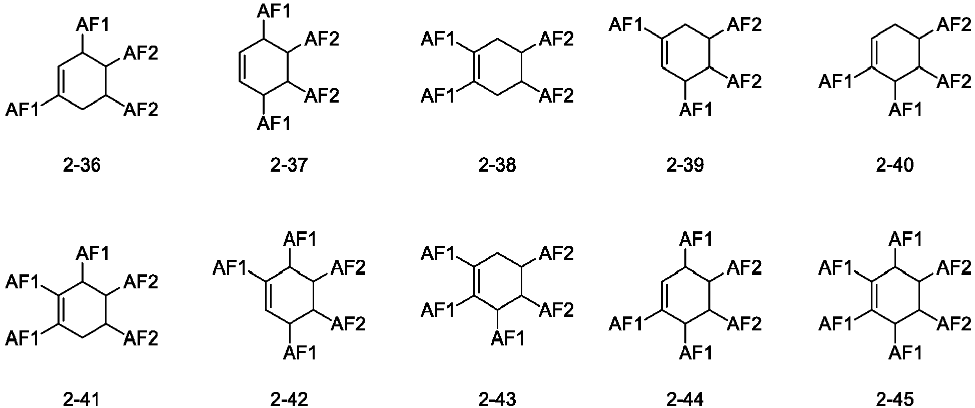

Erfindungsgemäße Moleküle, die nach Schema 3 darstellbar sind, sind in Tabelle 2 aufgeführt. Dabei sind AF1 und AF2 bevorzugt entweder beide an das Dien oder an das Dienophil gebunden, während der jeweils andere Reaktionspartner keine AF trägt; wobei sämtliche substituierbaren H-Atome des Diens oder des Dienophils durch chemische Einheiten AF1 und AF2 ersetzt sein können, wobei mindestens eine Einheit AF1 und mindestens eine von AF1 verschiedene Einheit AF2 im resultierenden Molekül enthalten sein müssen. Optional kann dabei jedes substituierbare H-Atom durch einen Rest R* ersetzt sein.

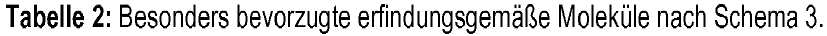

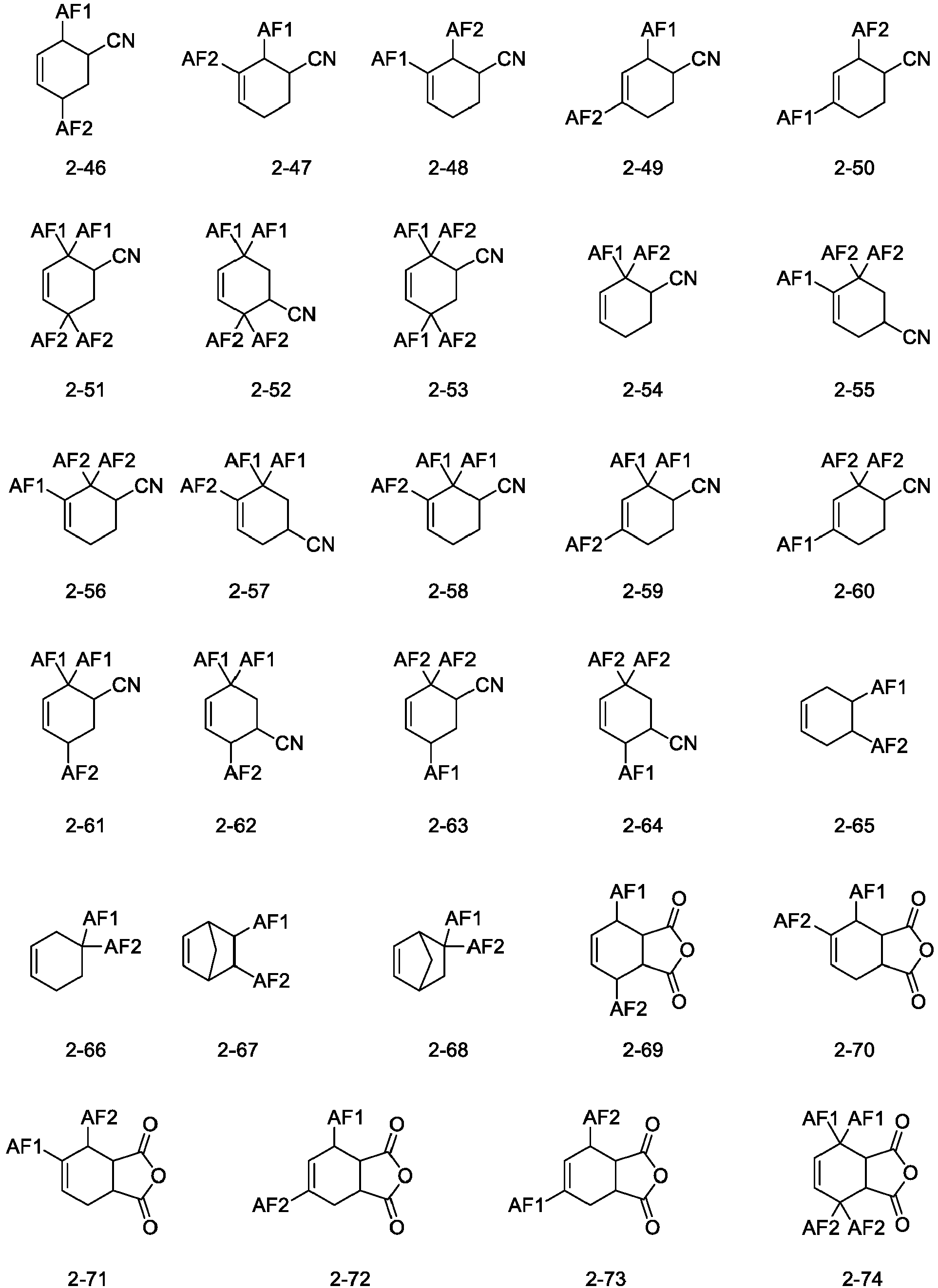

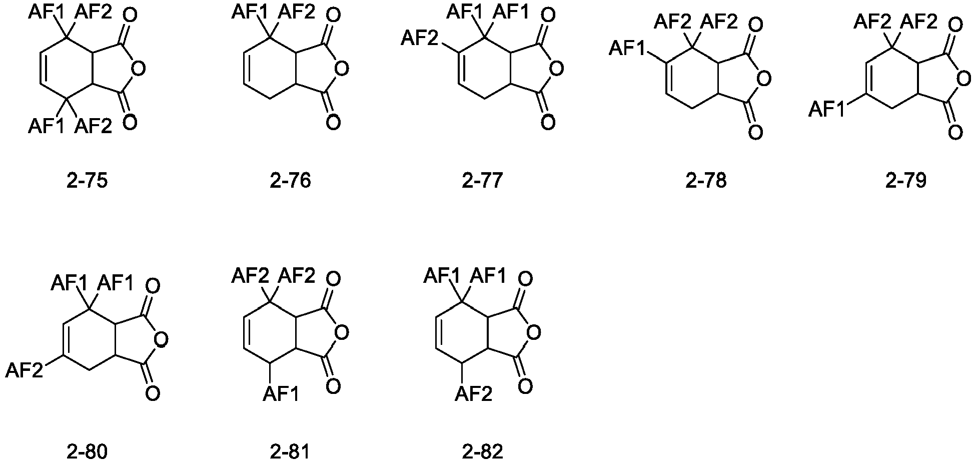

Die chemischen Einheiten AF1 und AF2 der erfindungsgemäßen Moleküle, insbesondere der in Tabelle 1 und Tabelle 2 genannten, sind ausgewählt aus den in Tabelle 3 aufgelisteten Strukturen, wobei jede der chemischen Einheiten in einen Molekül als AF1, in einem anderen Molekül als AF2 auftreten kann. Unter der Voraussetzung, dass in einem Molekül immer zwei unterschiedliche AFs auftreten, können weitere AFs identisch mit ersteren sein. Mögliche Anknüpfungspunkte der chemischen Einheit AF an einen Separator S sind mit Kleinbuchstaben bezeichnet.

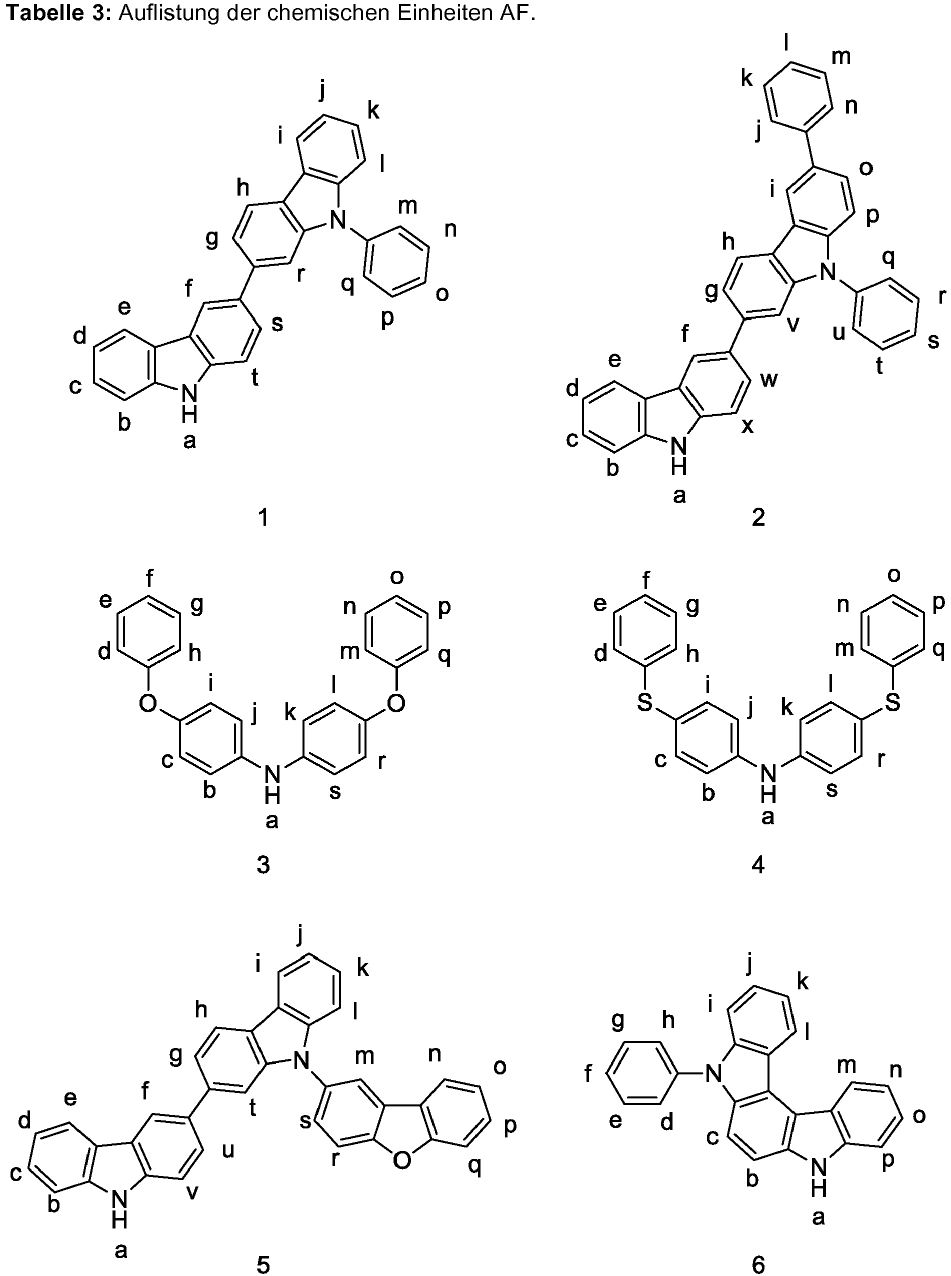

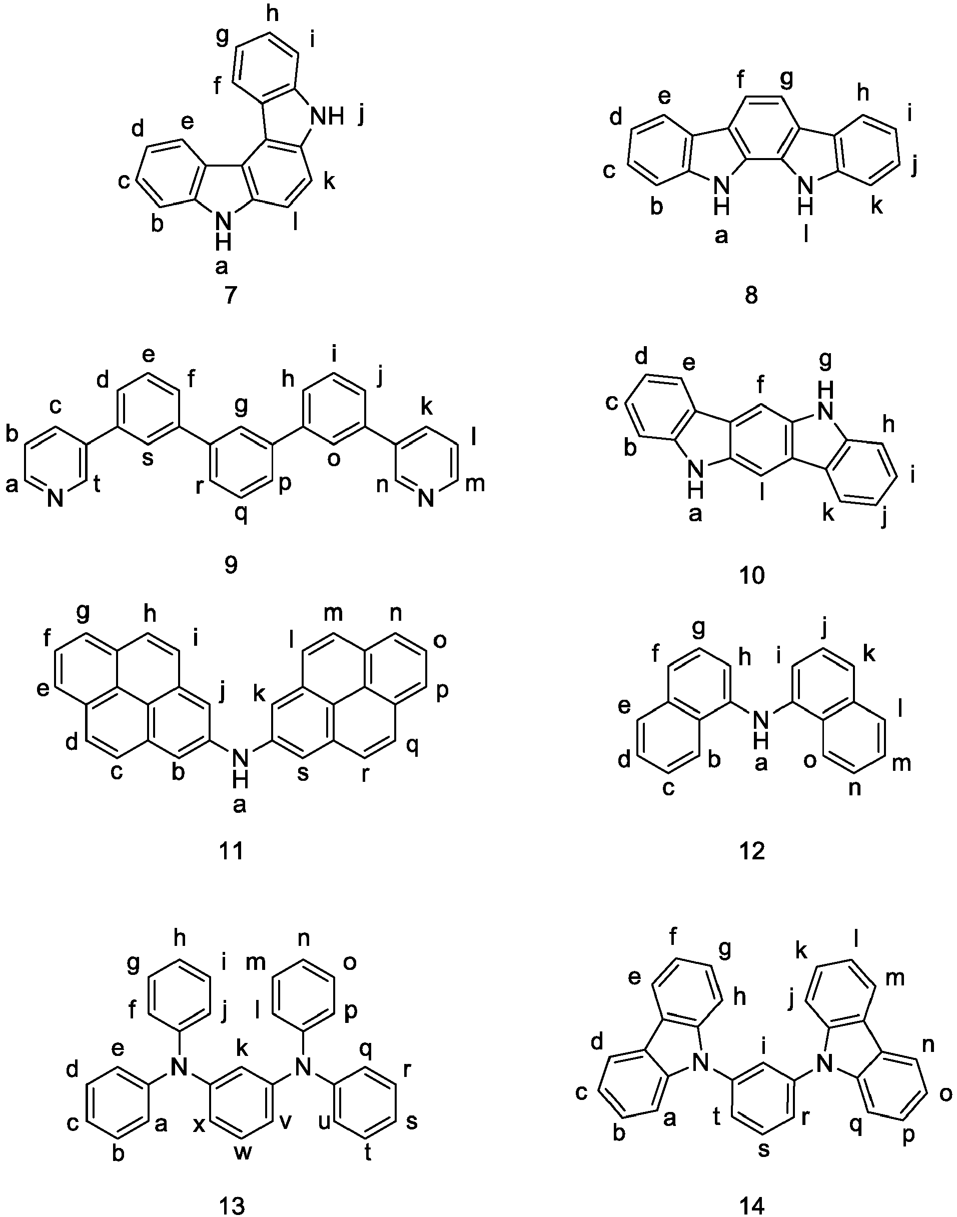

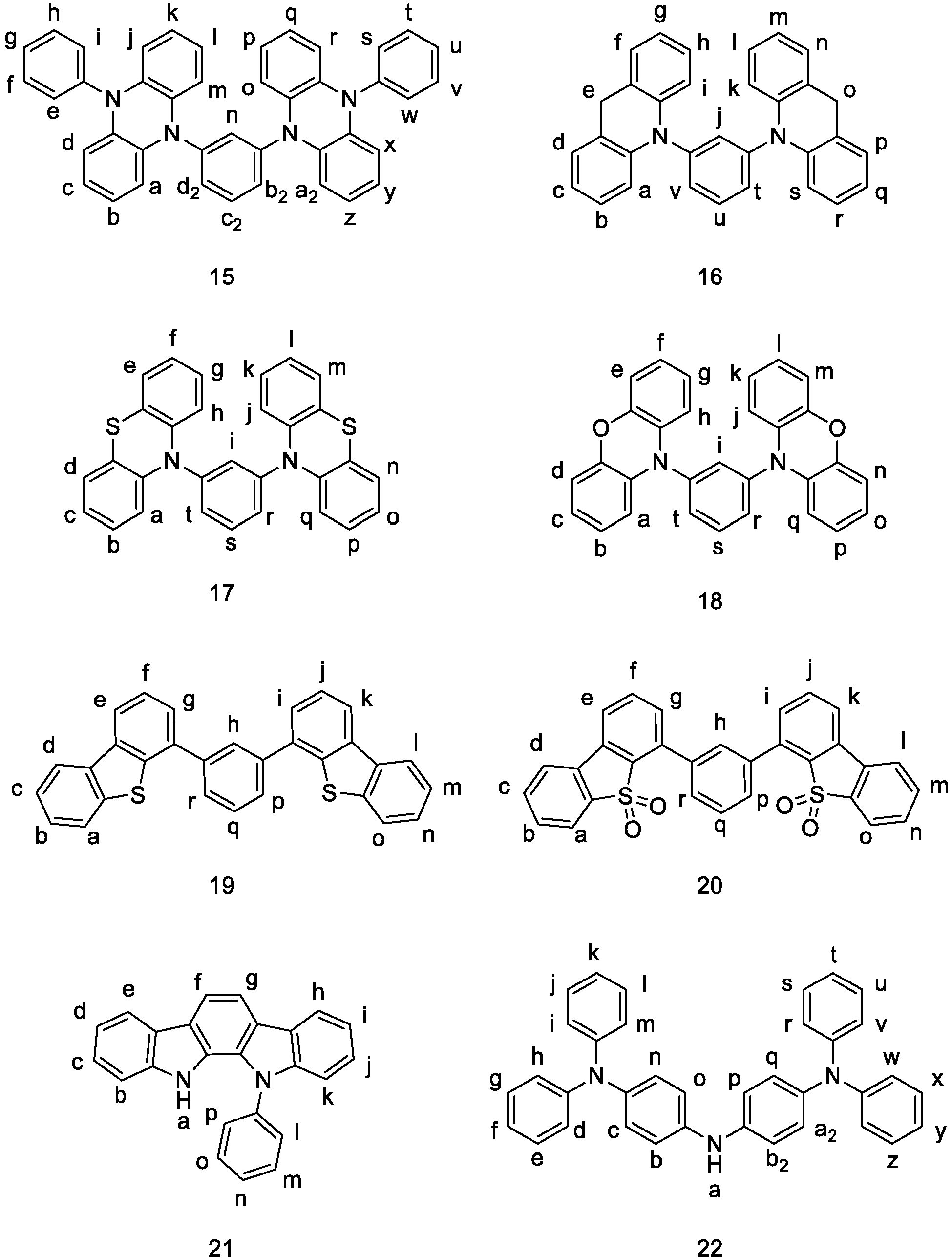

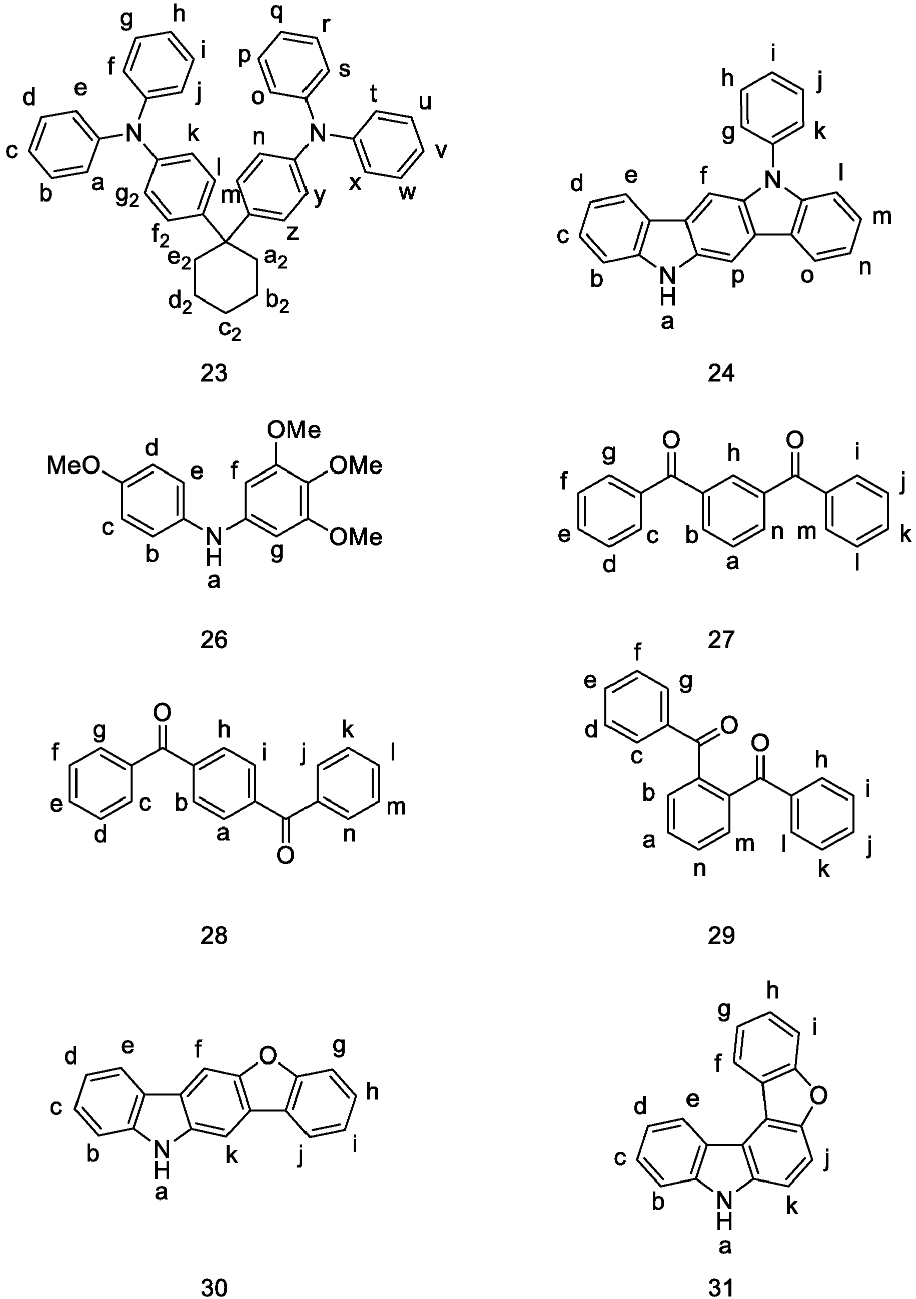

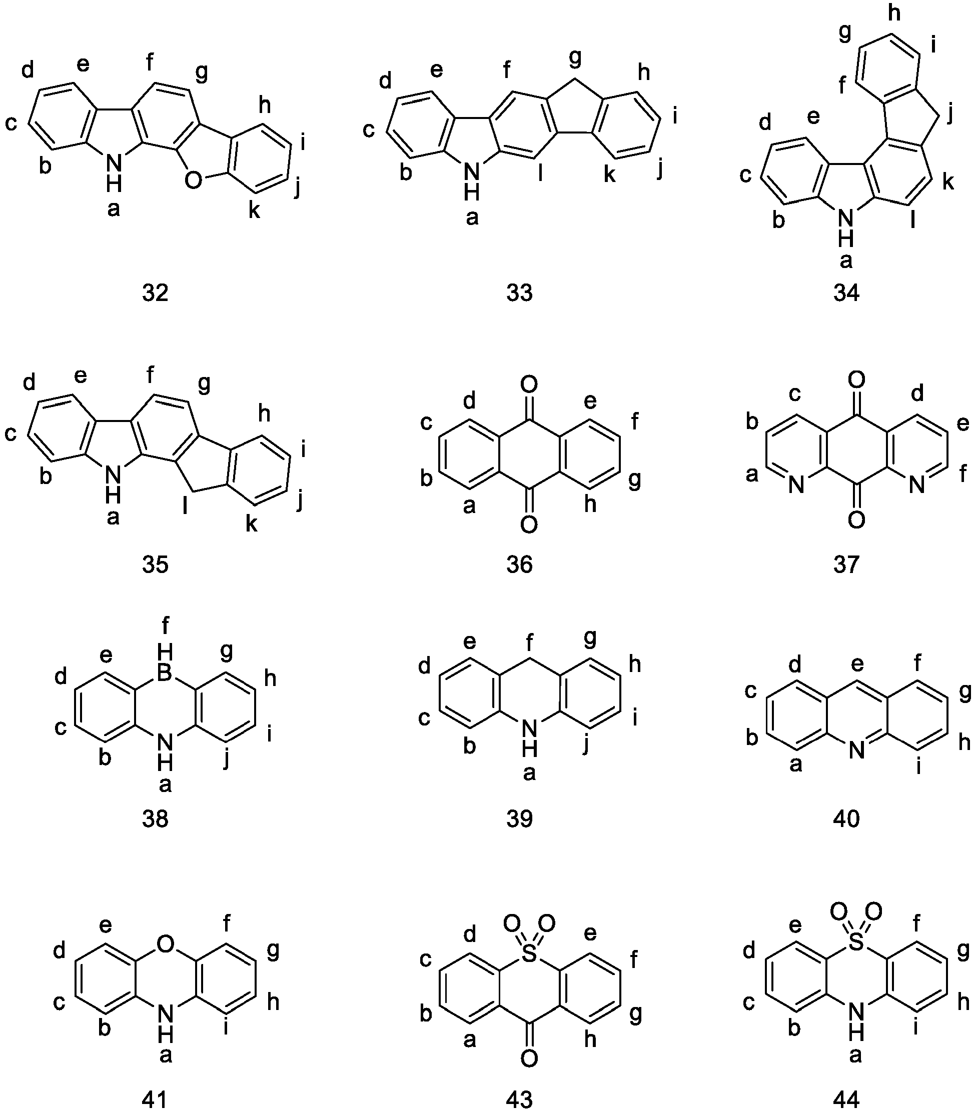

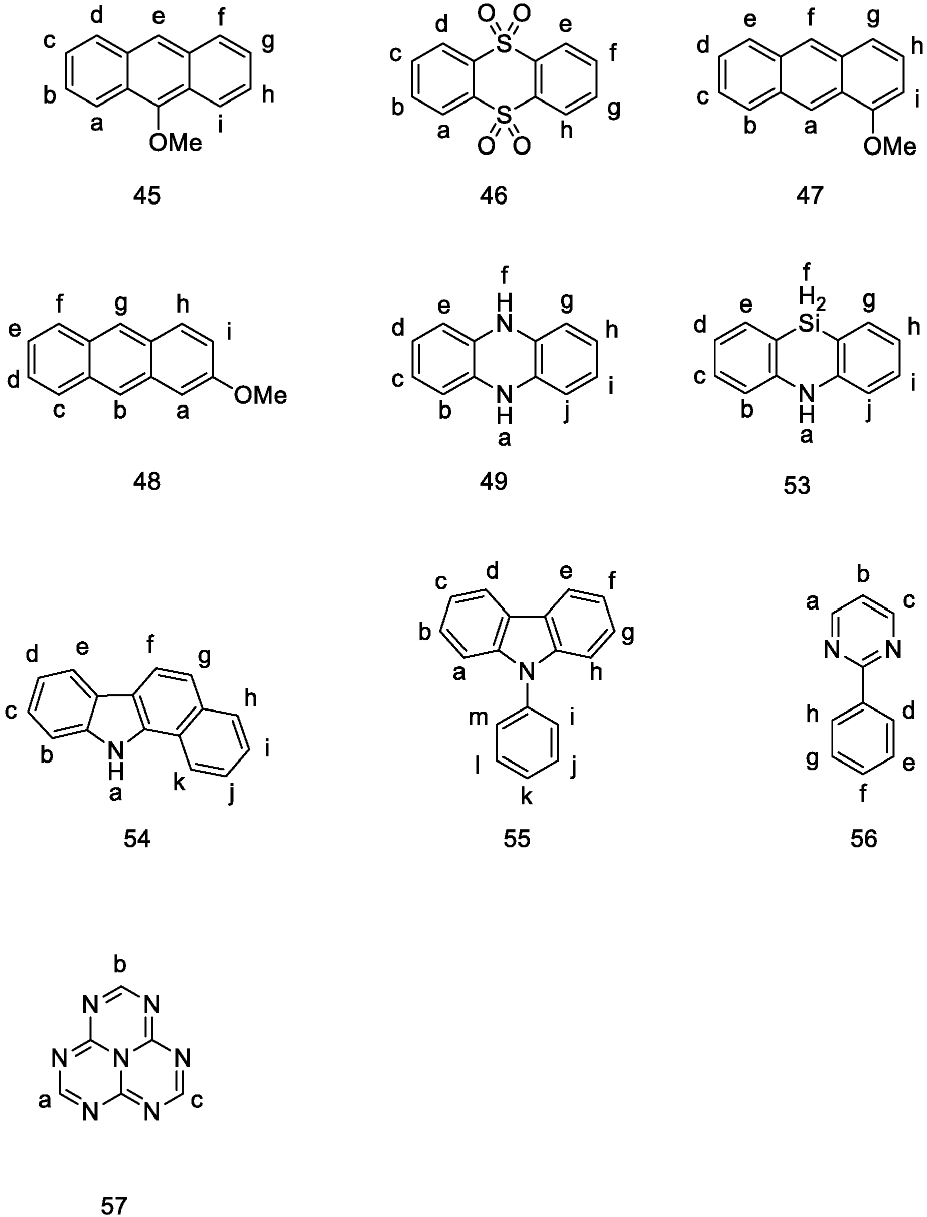

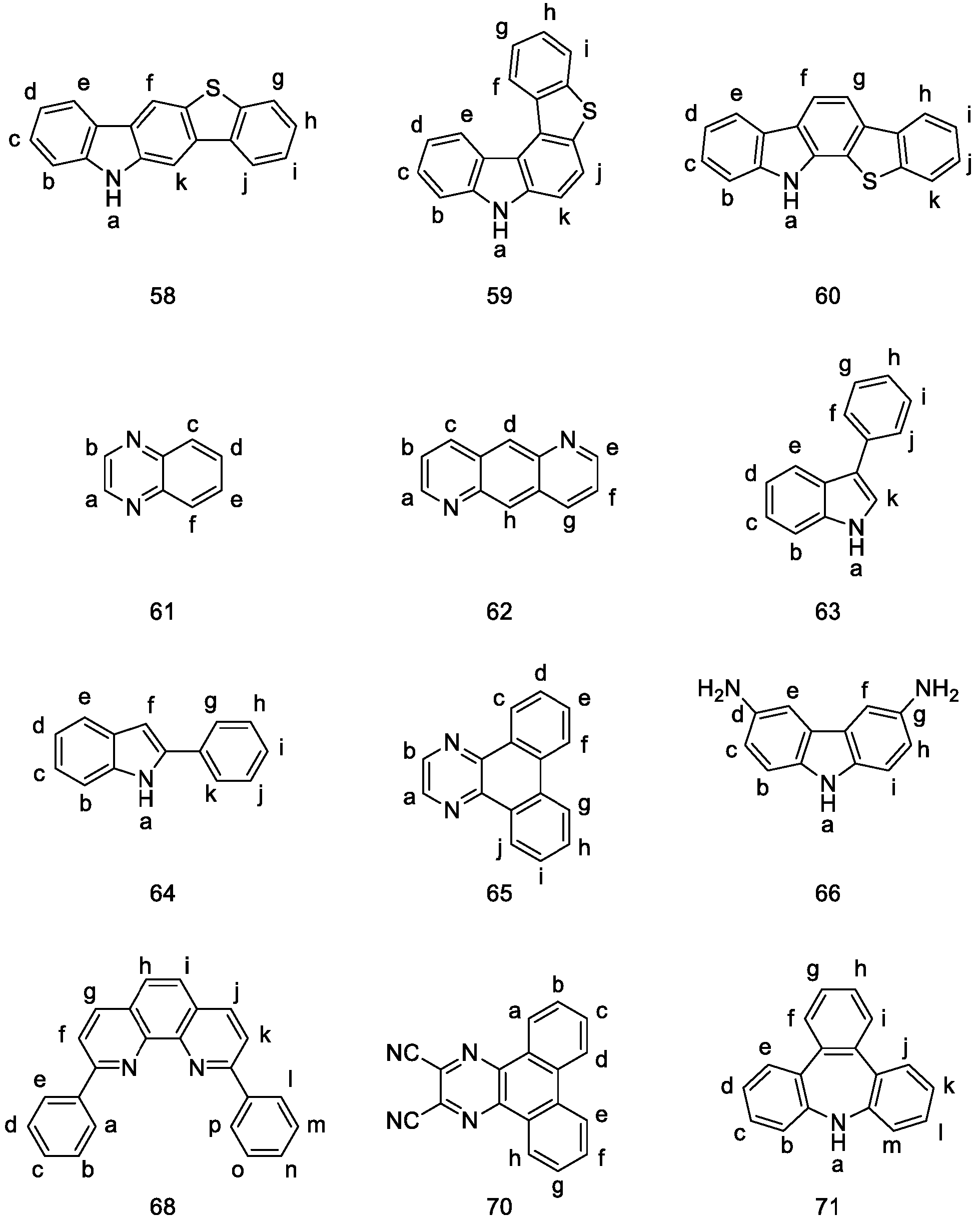

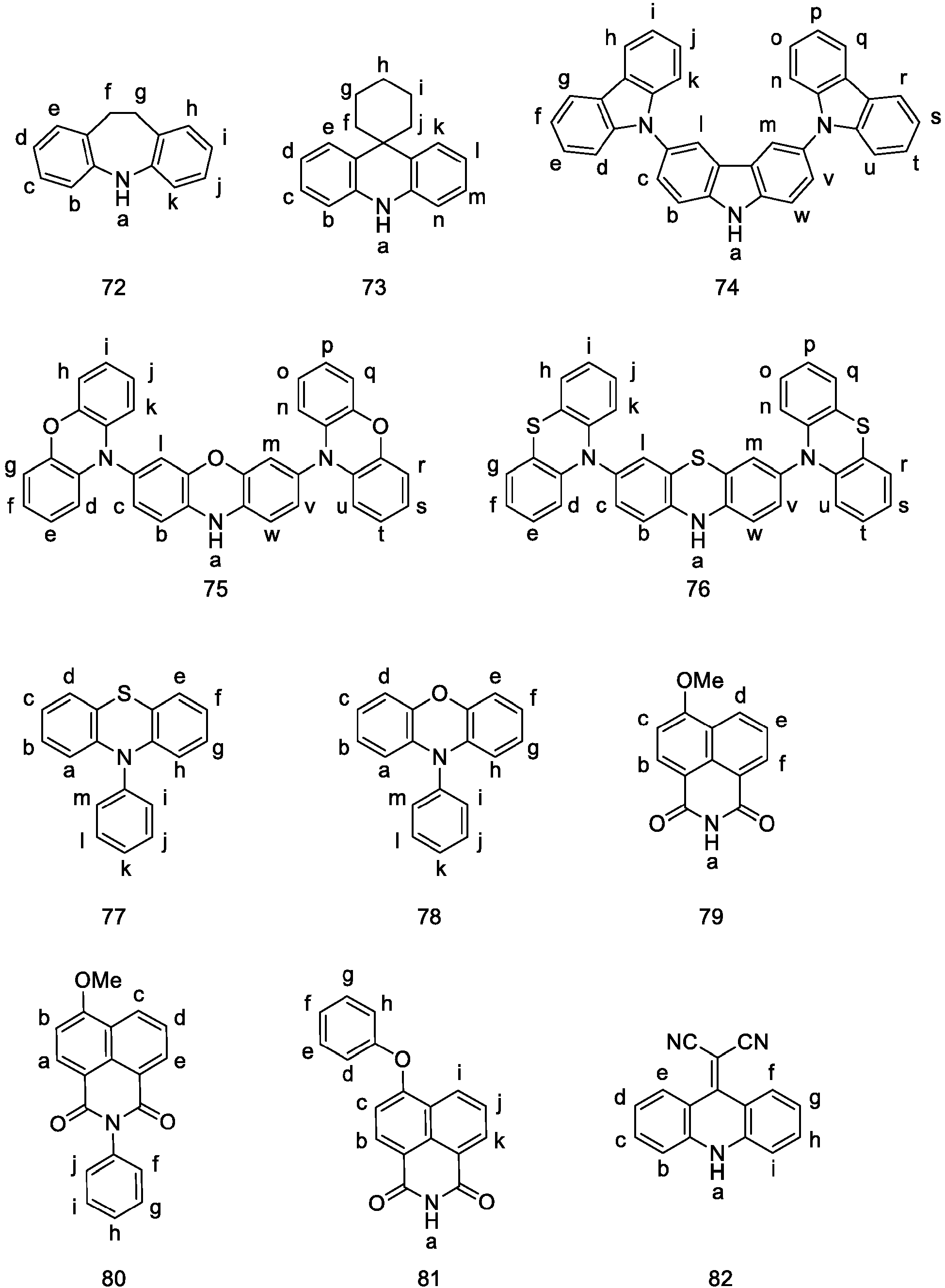

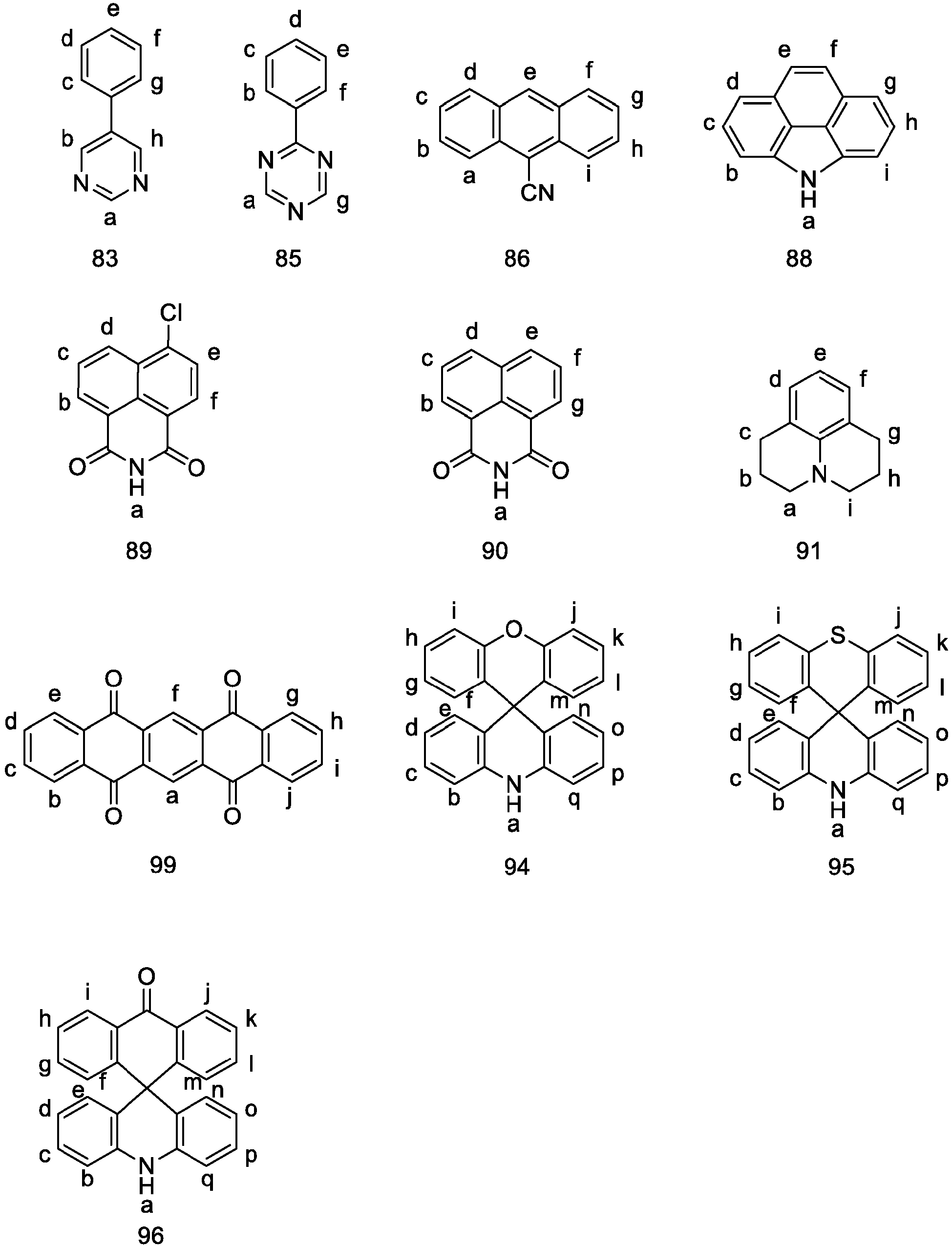

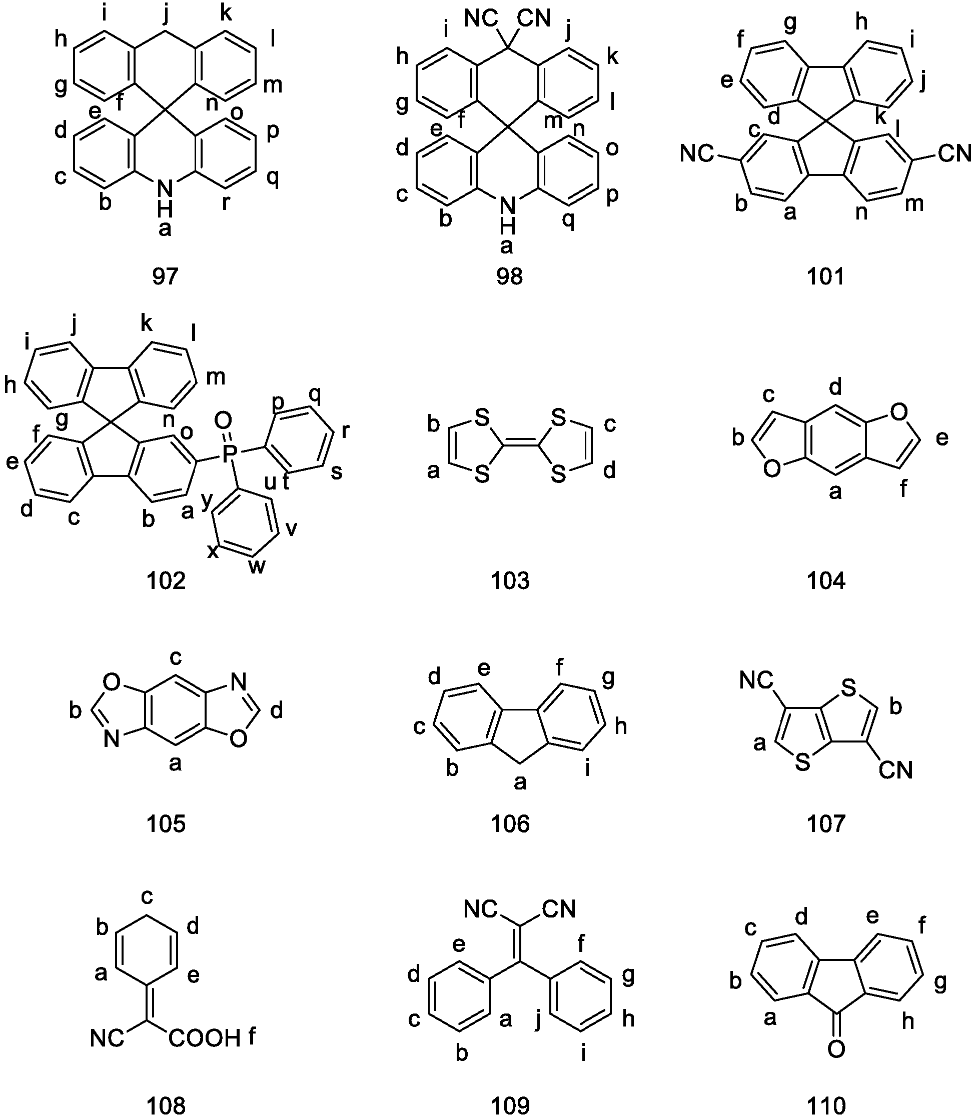

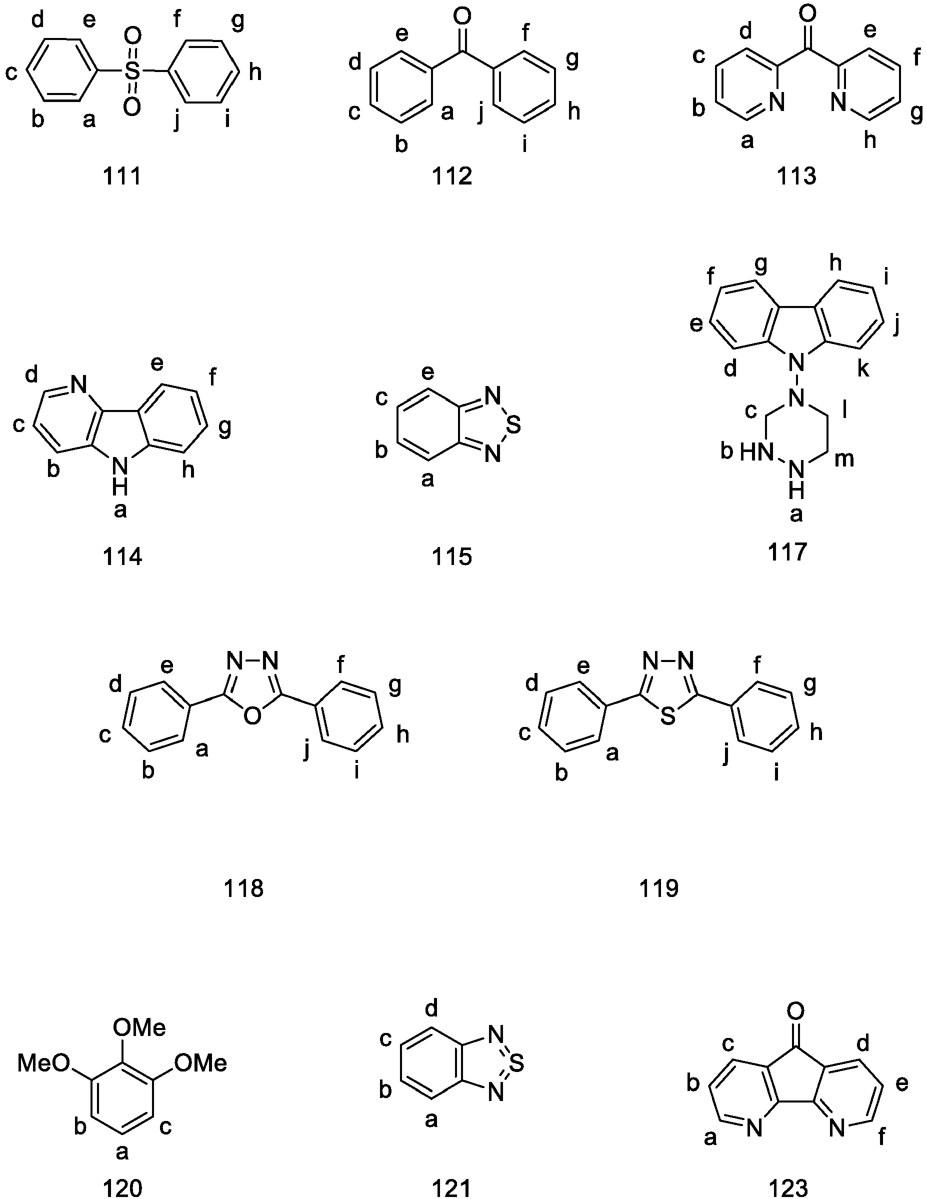

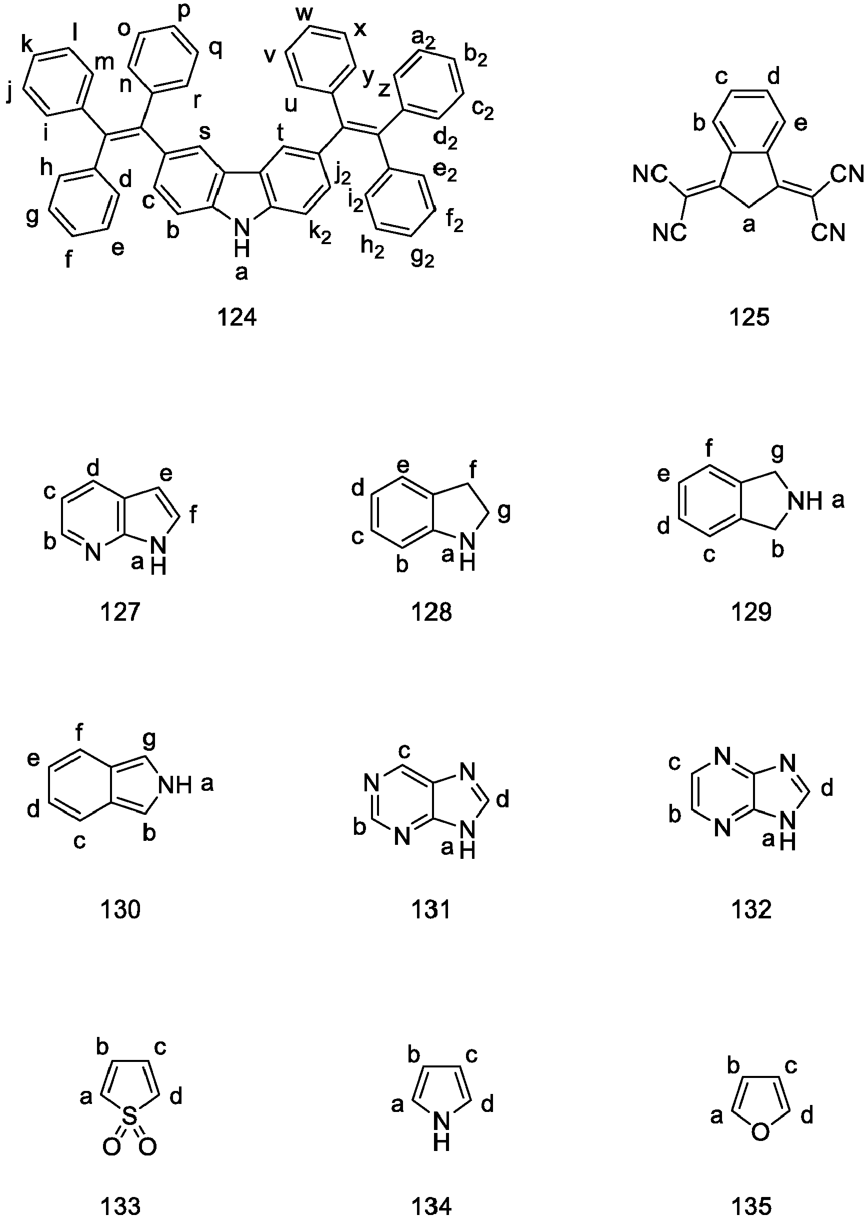

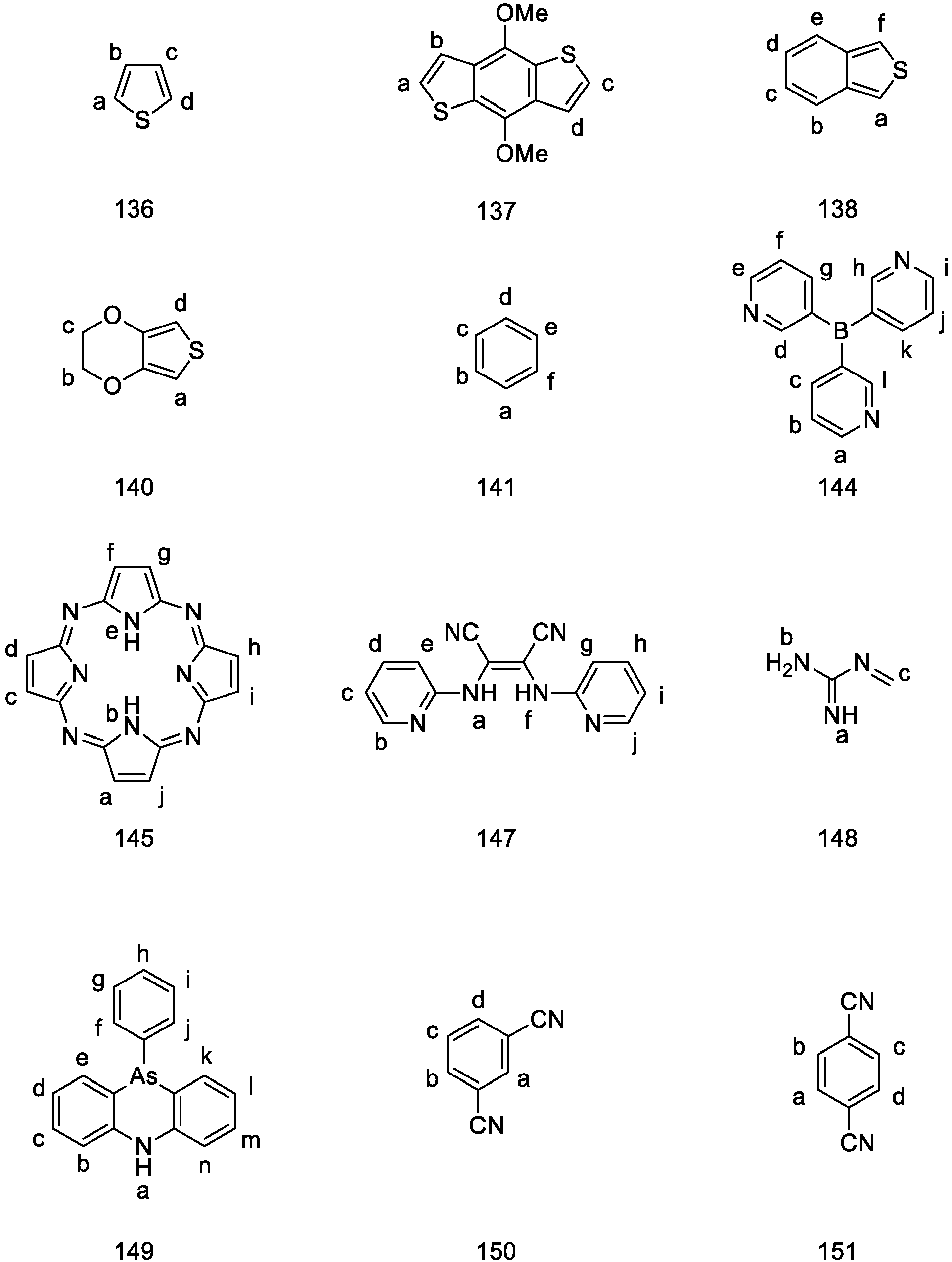

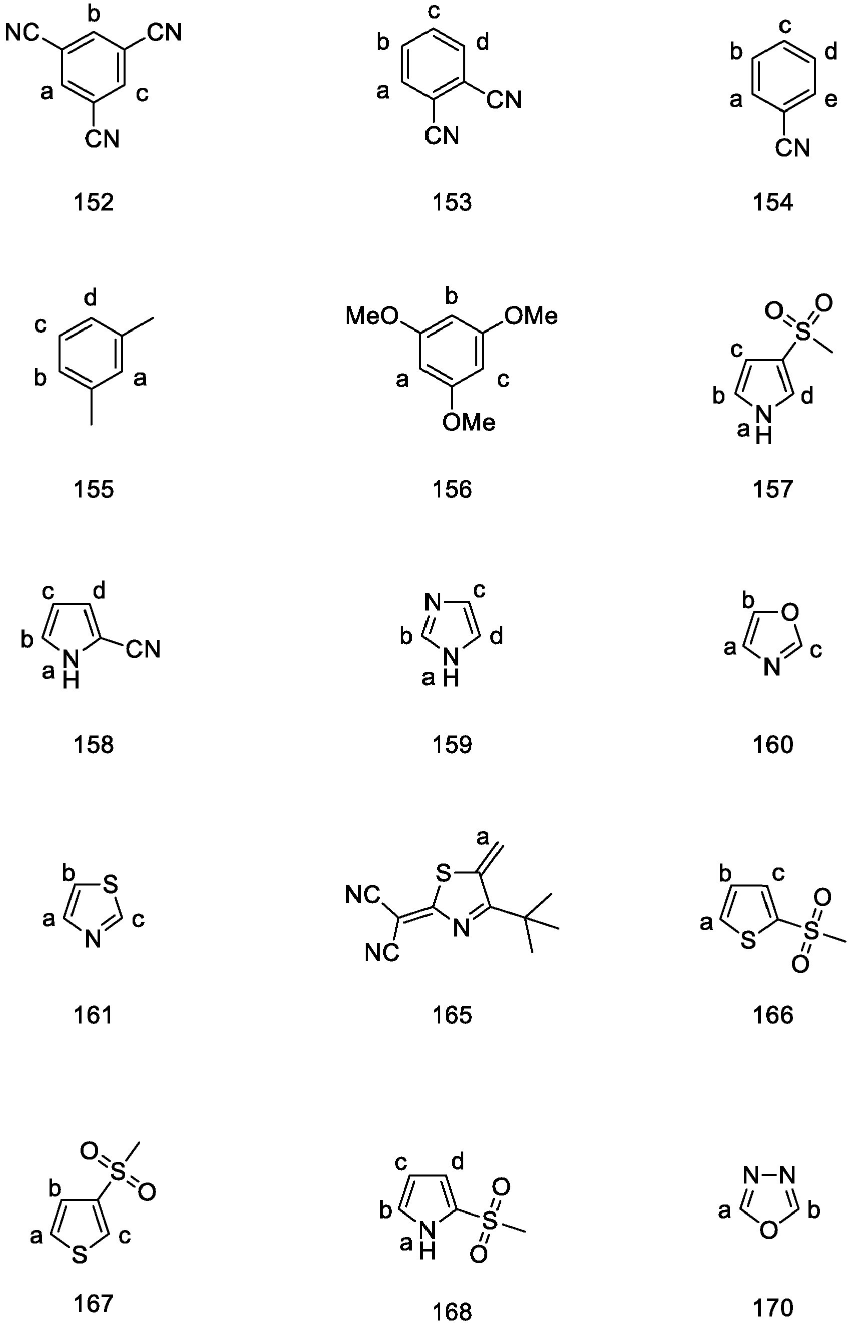

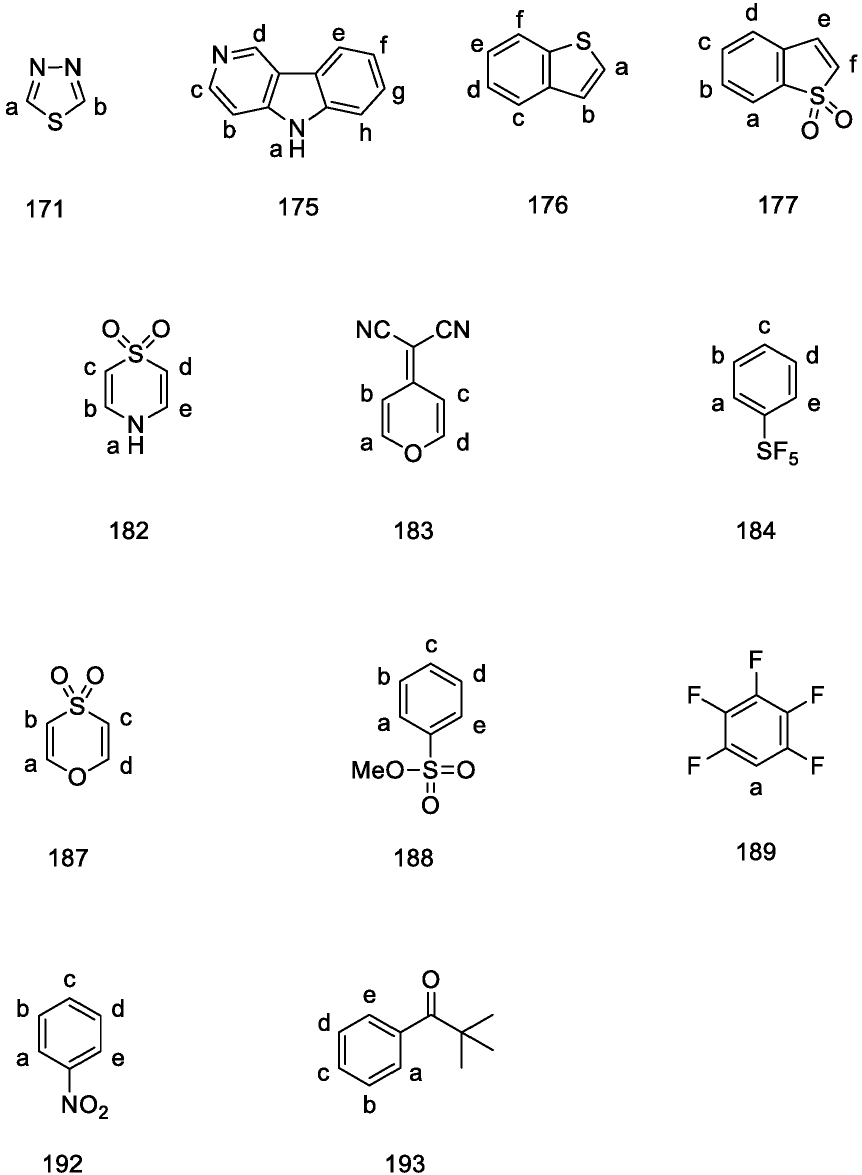

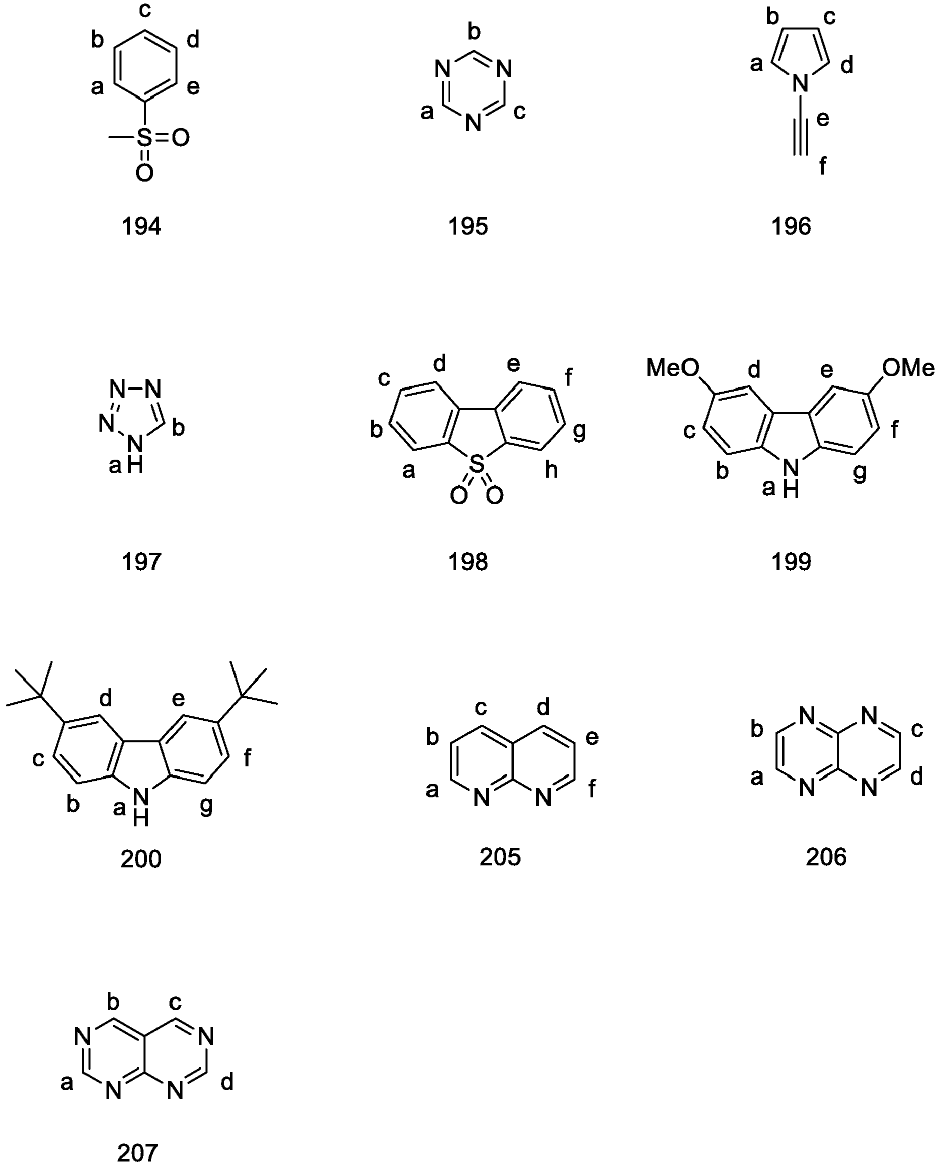

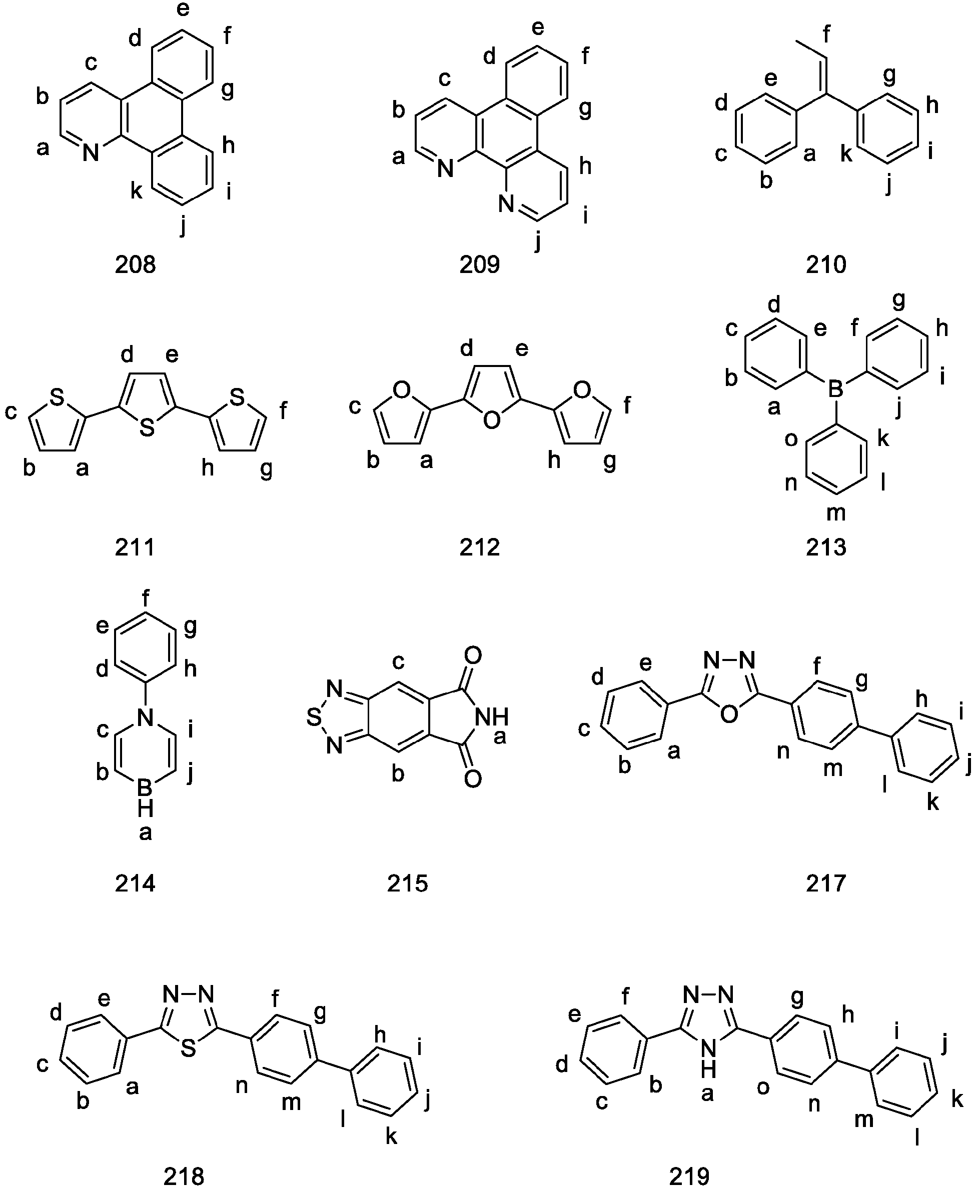

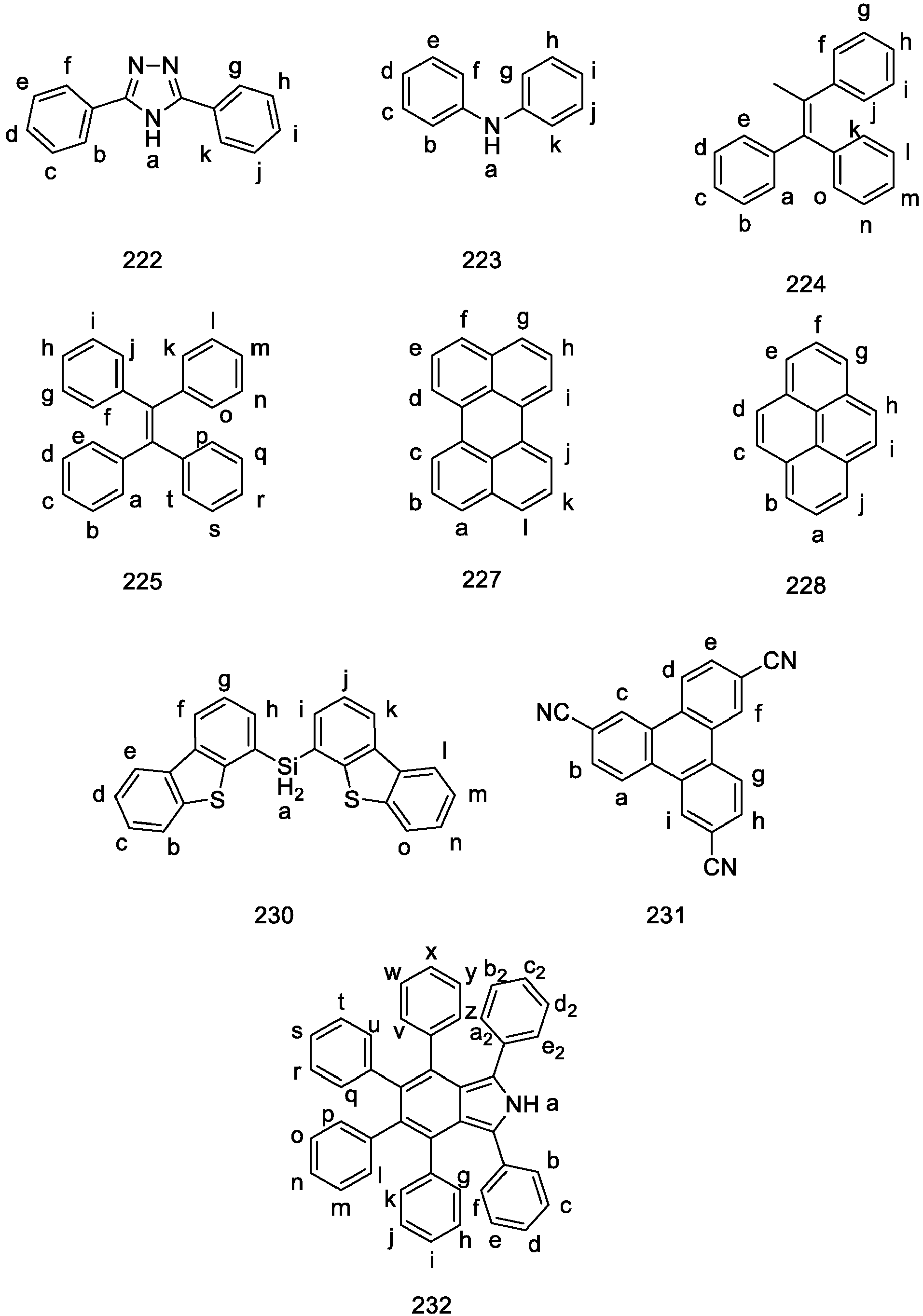

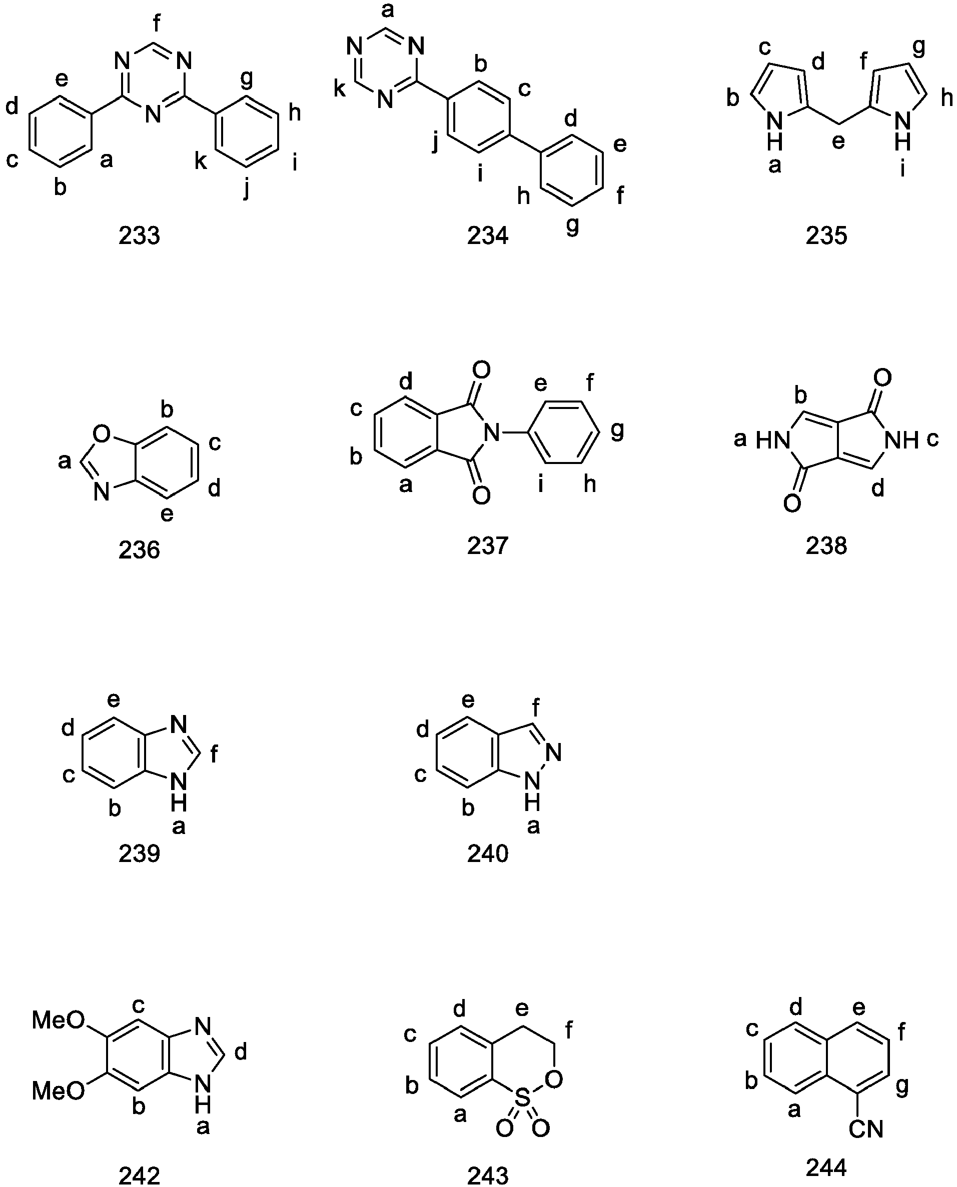

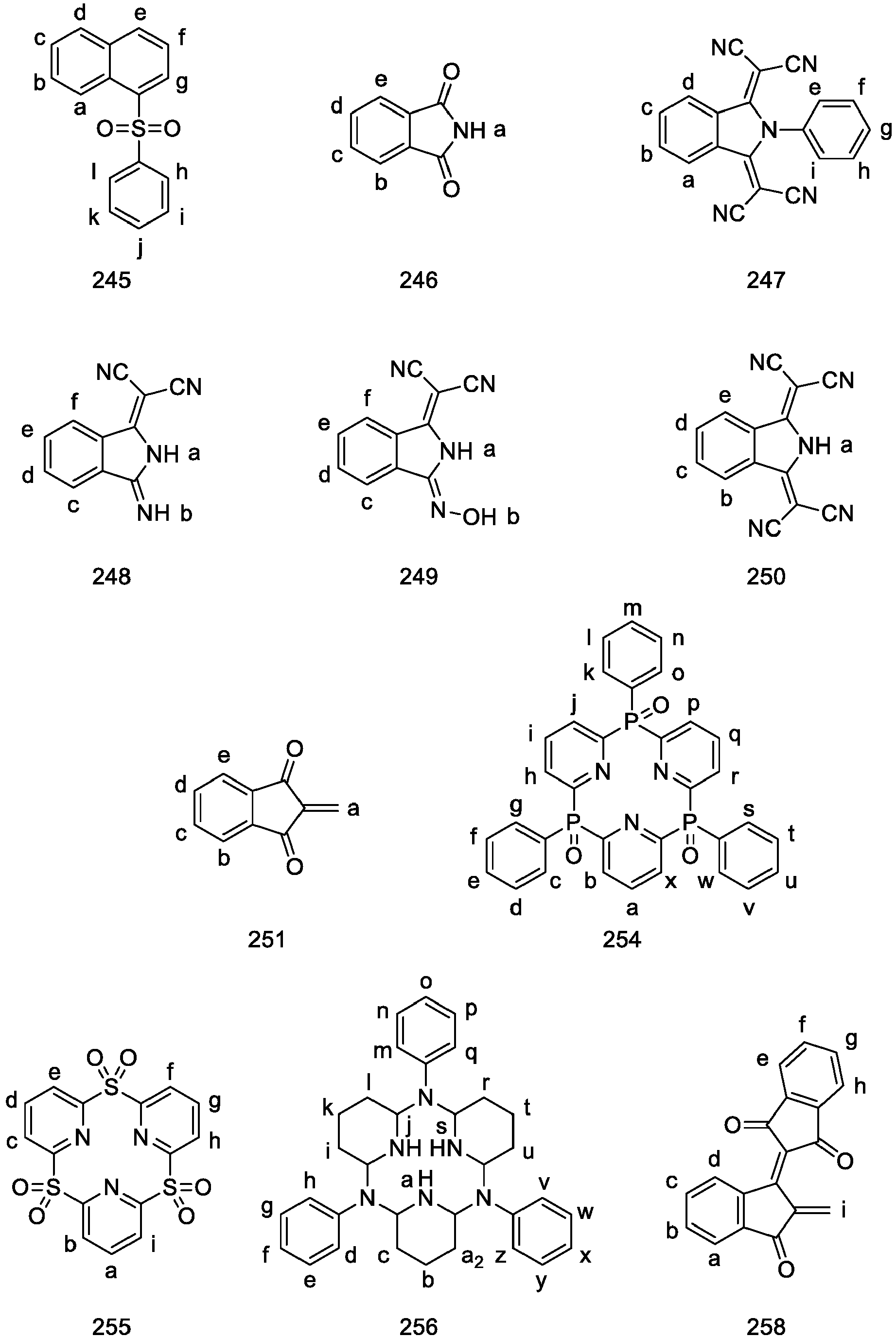

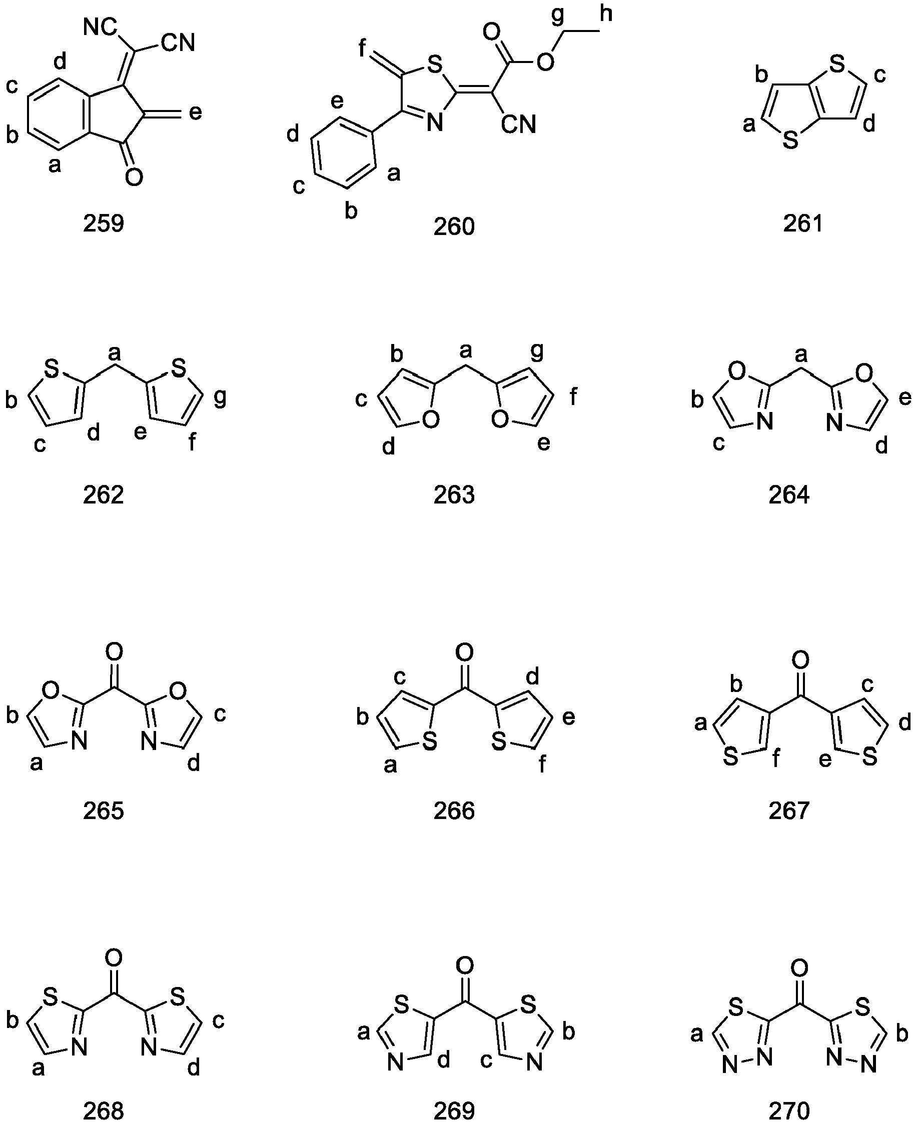

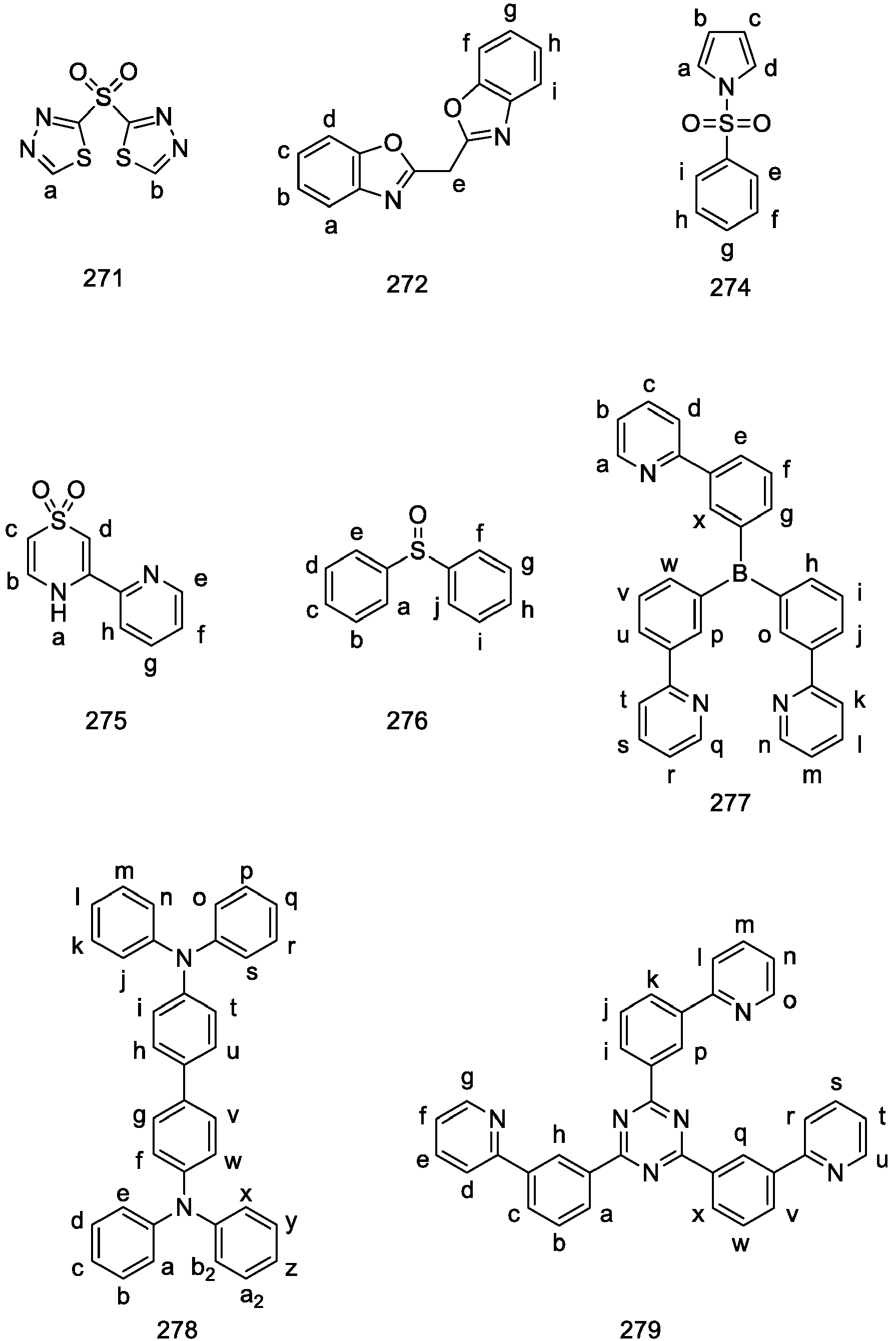

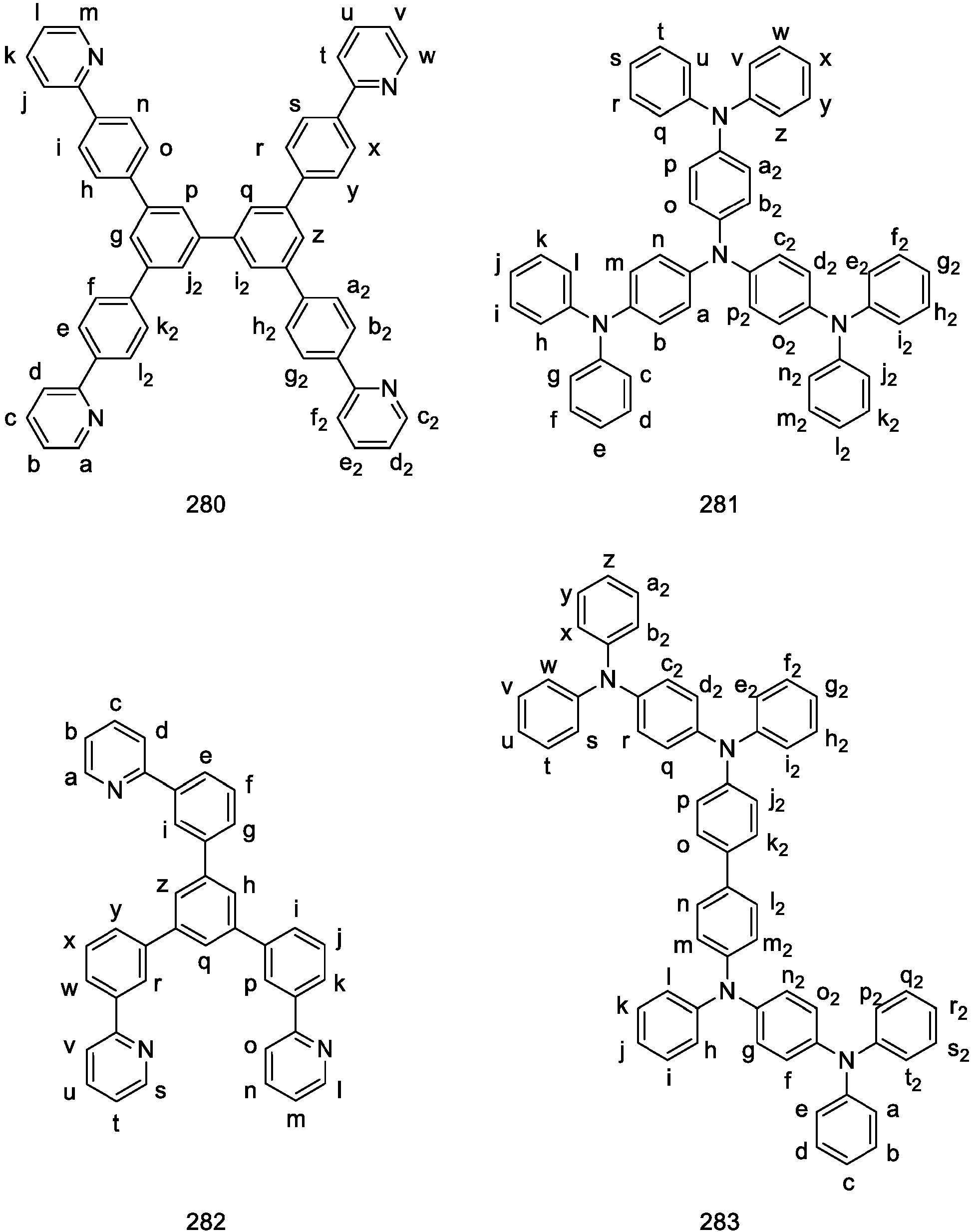

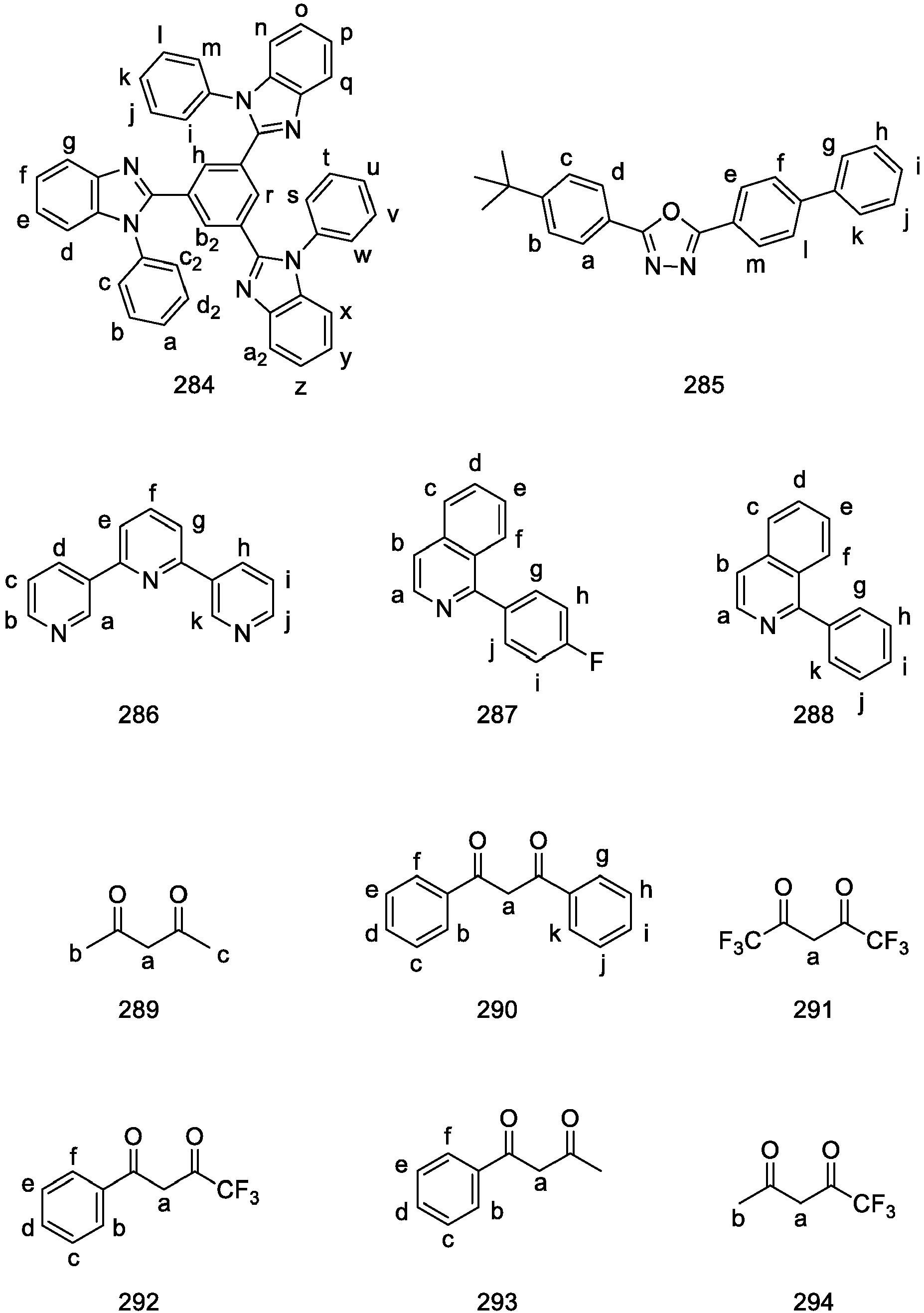

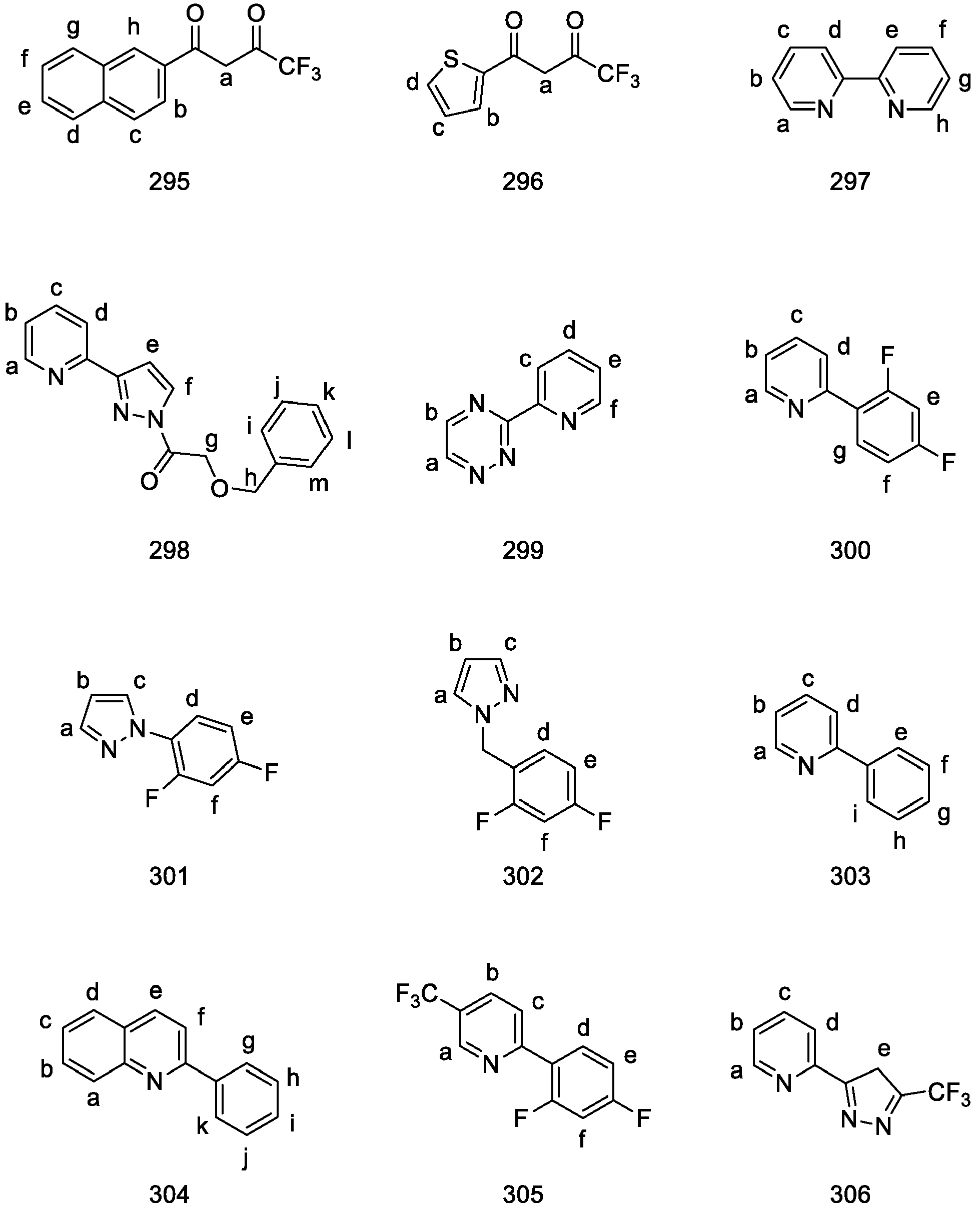

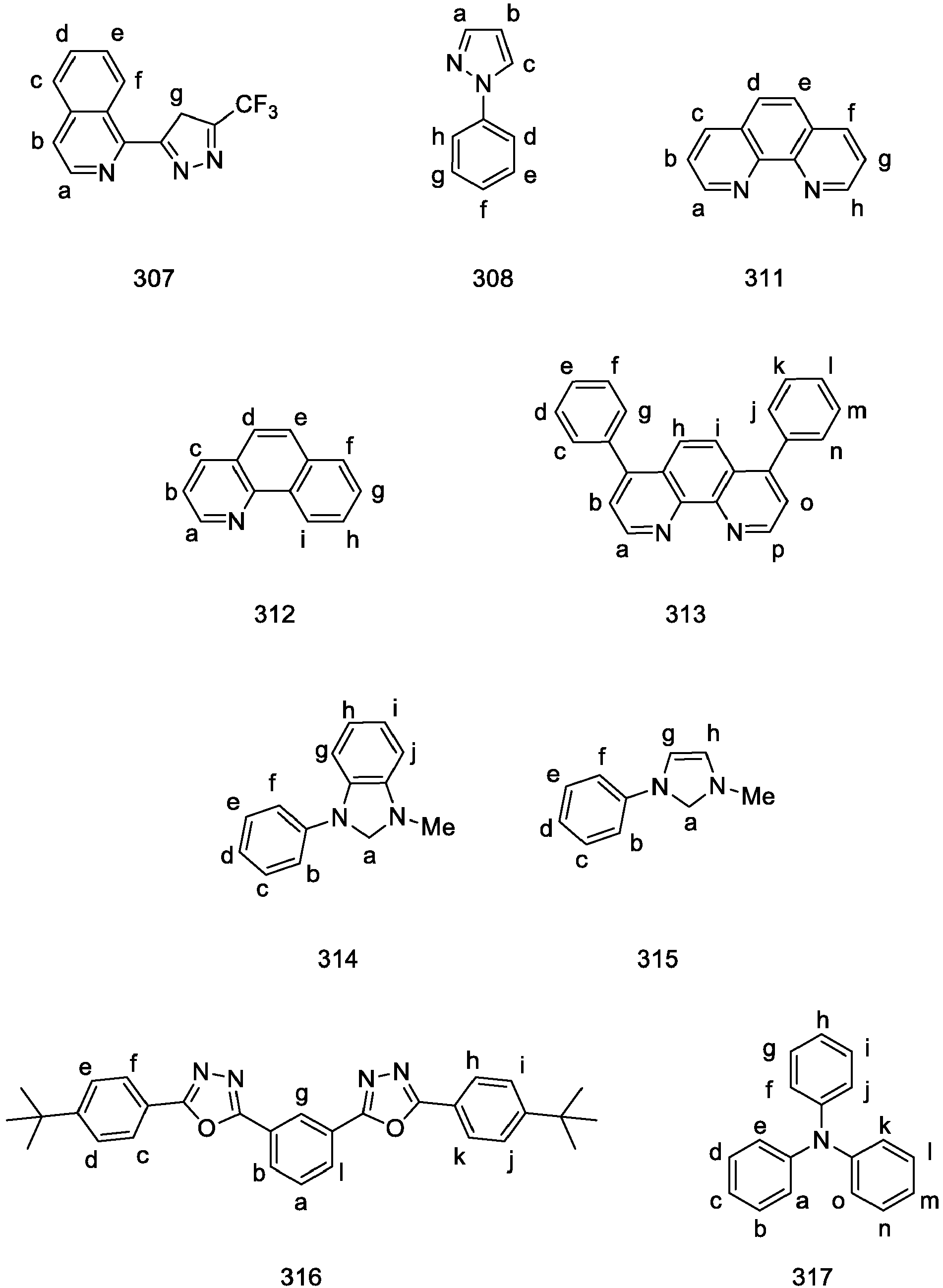

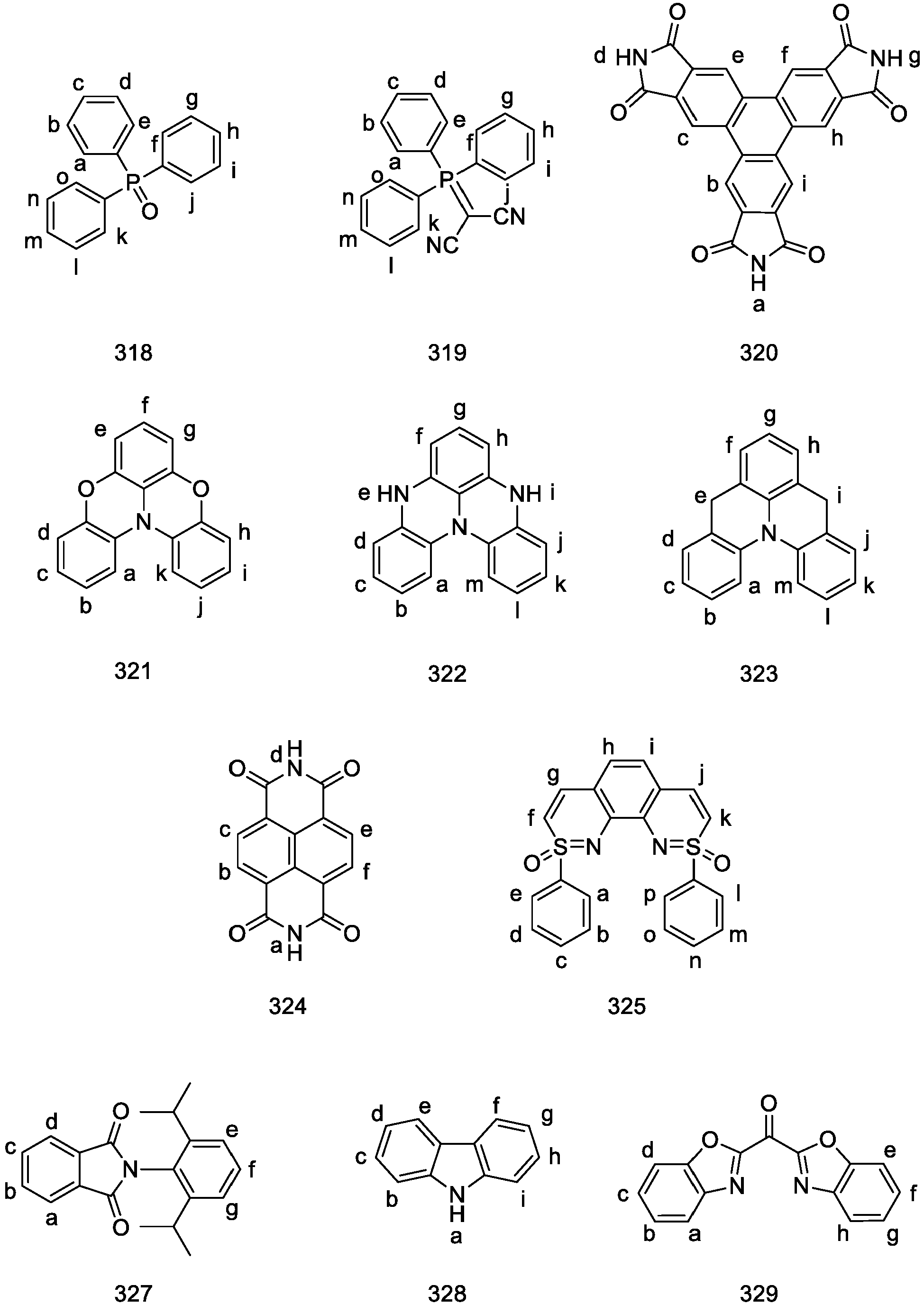

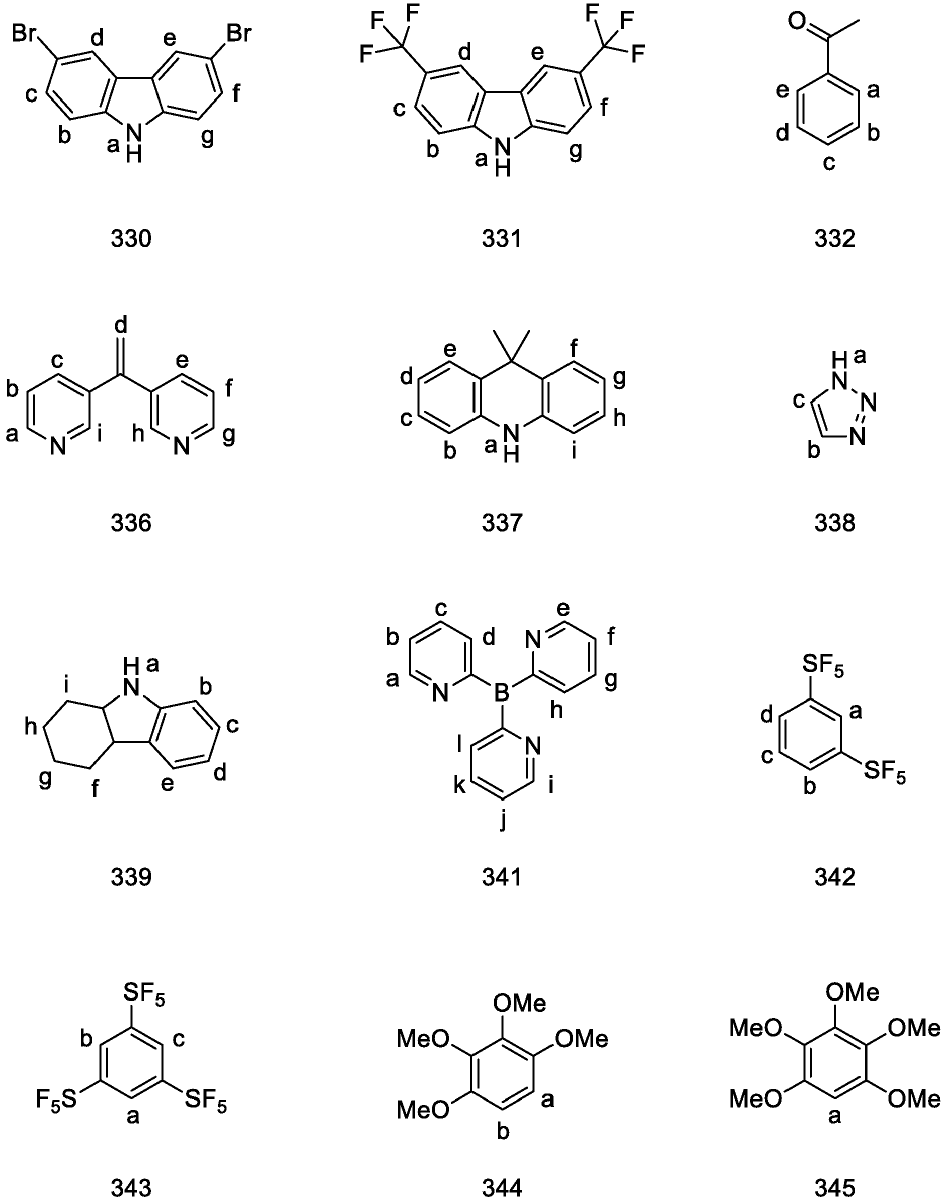

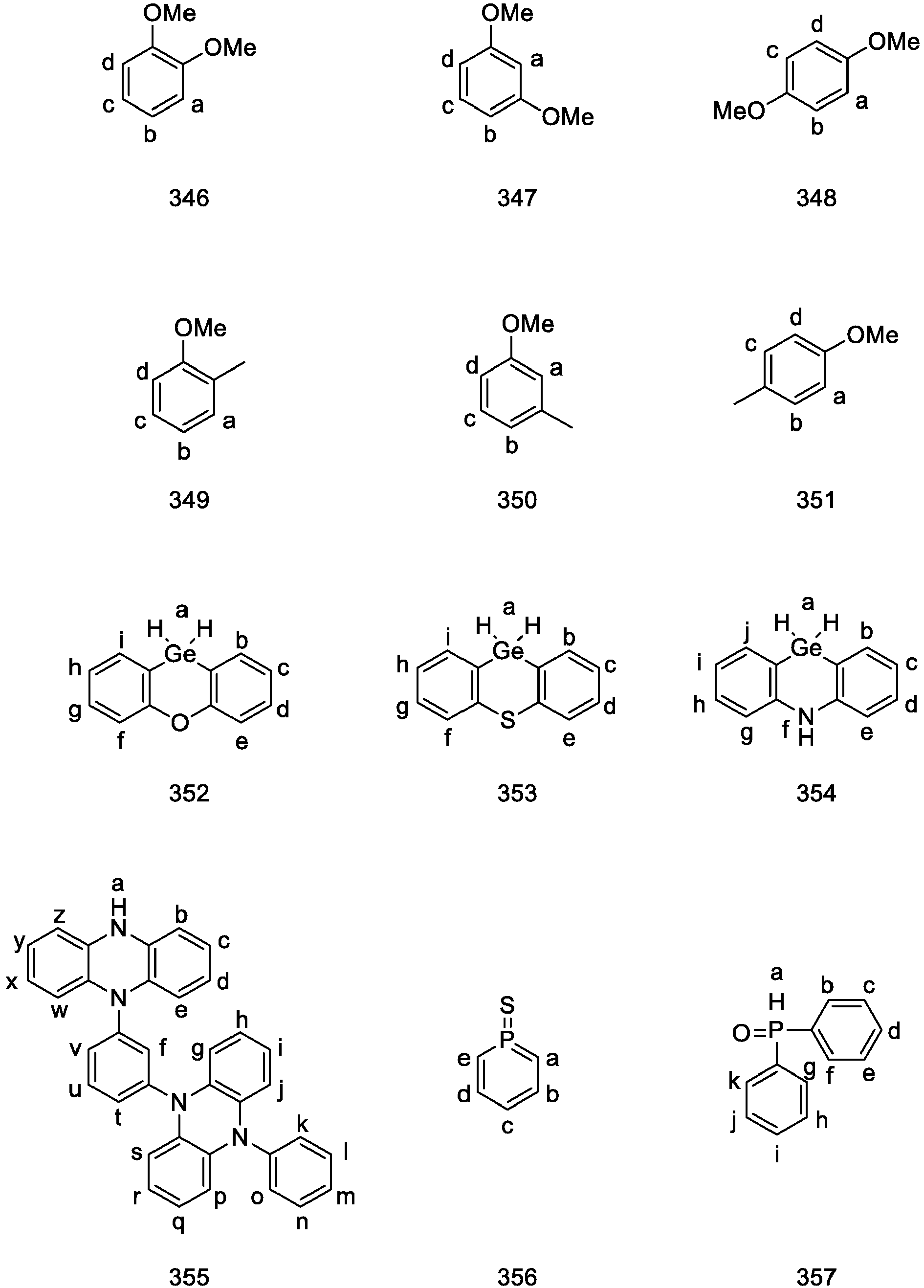

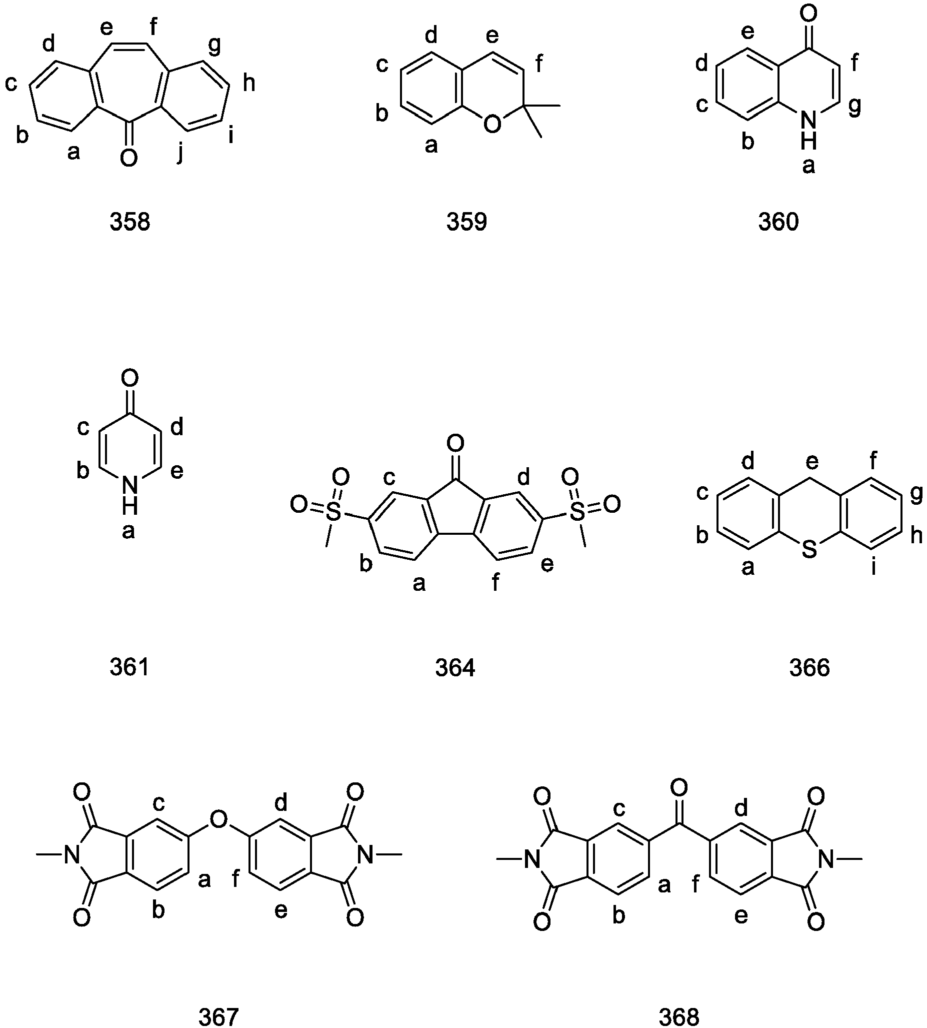

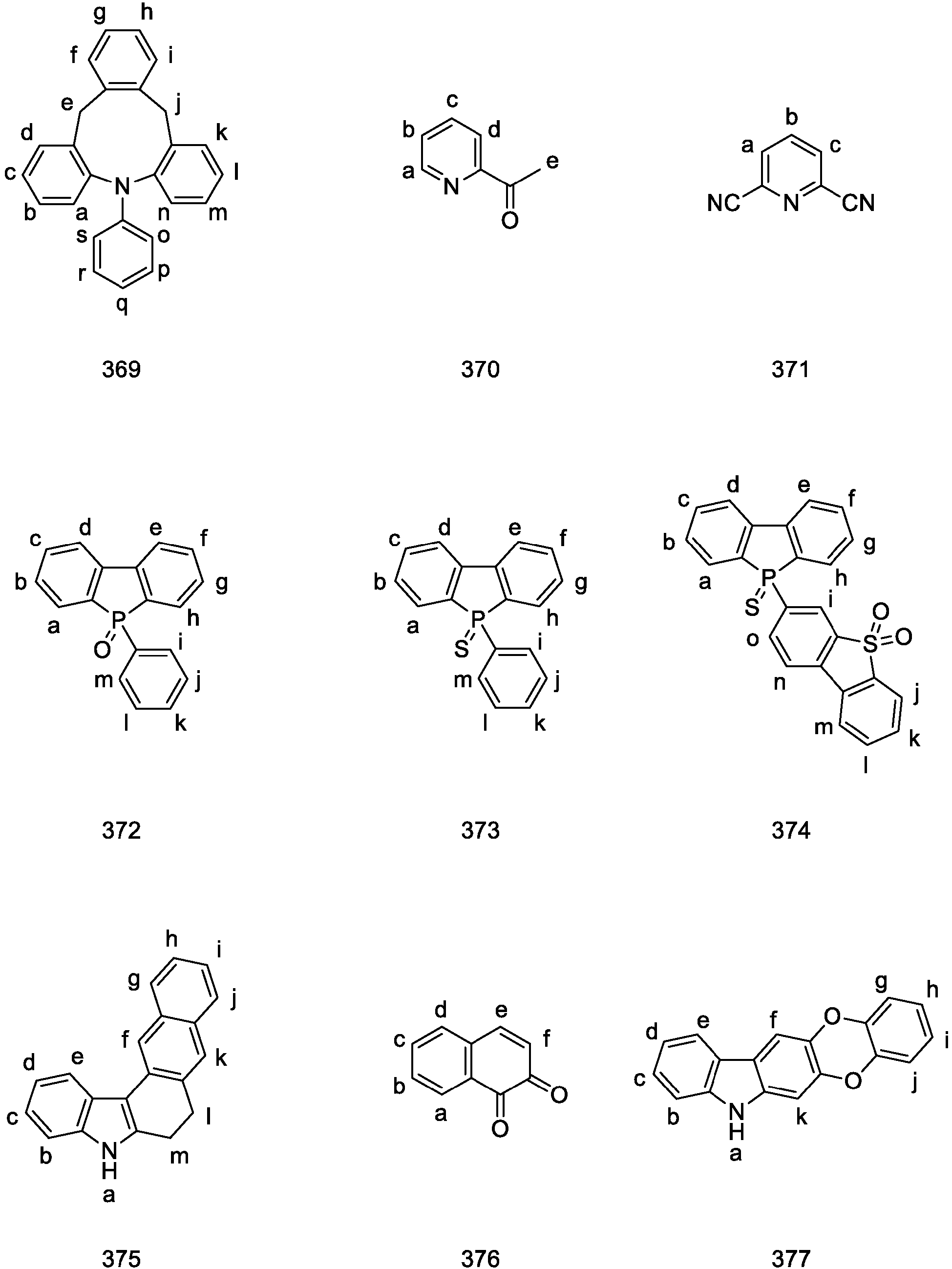

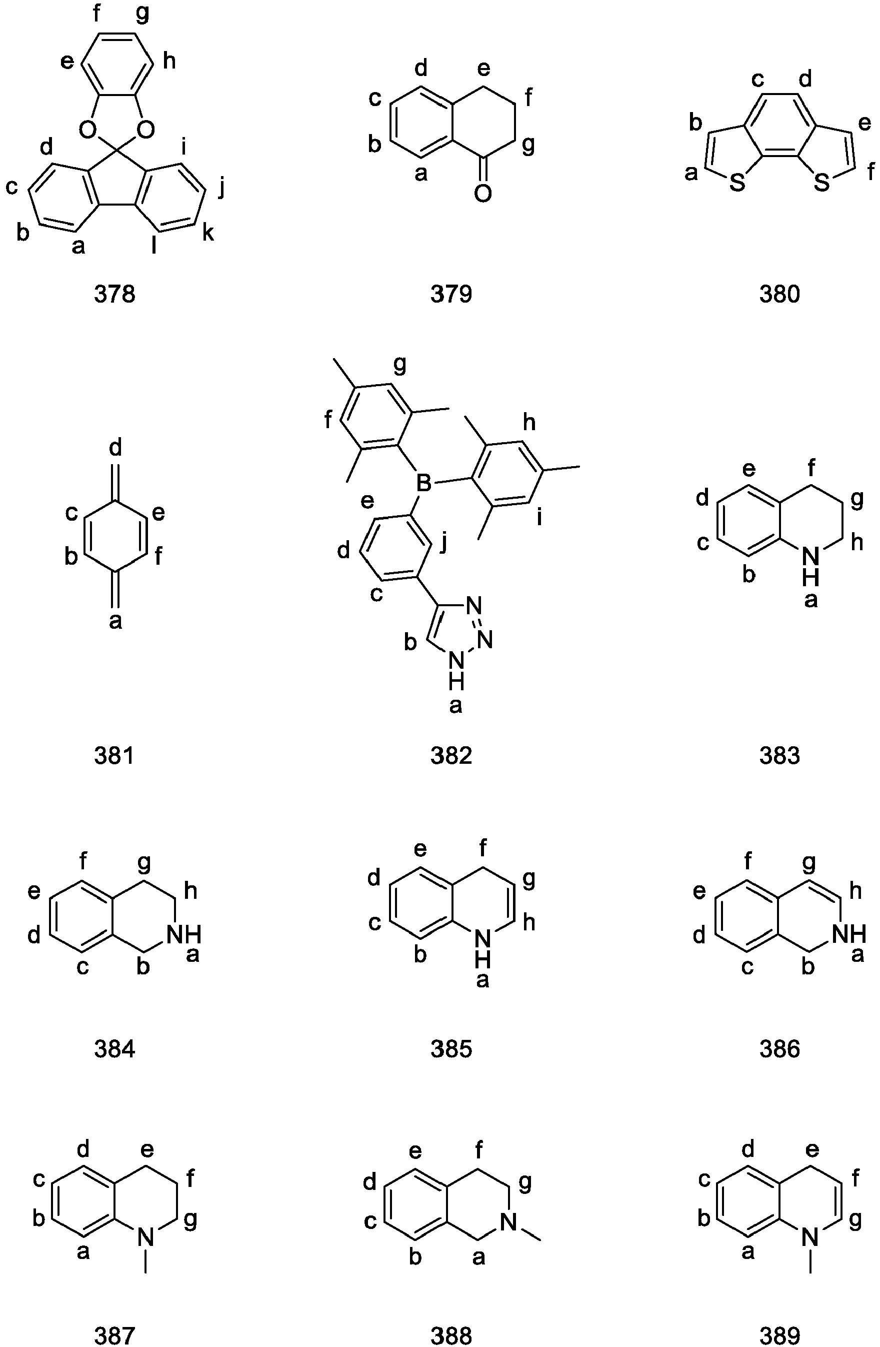

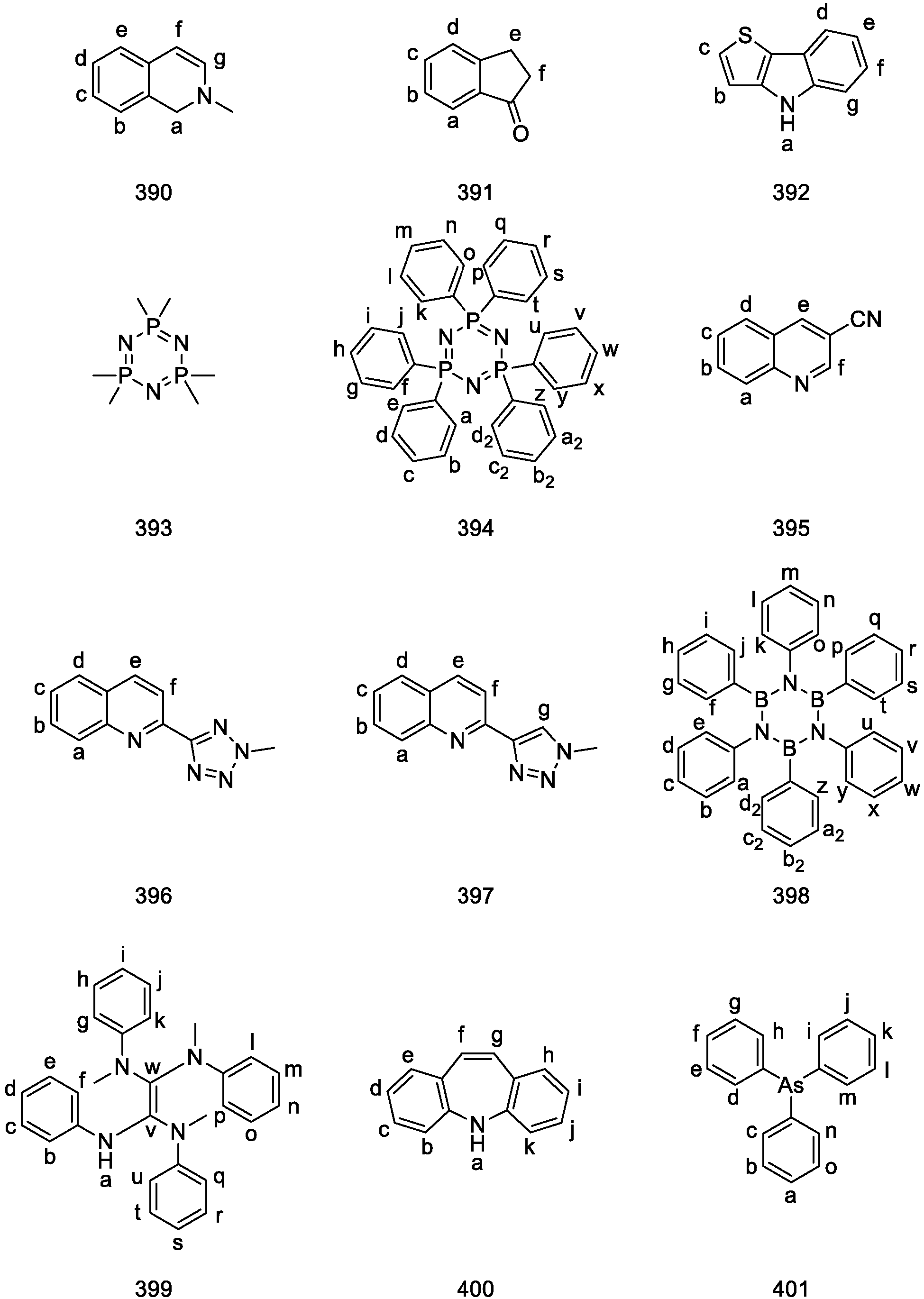

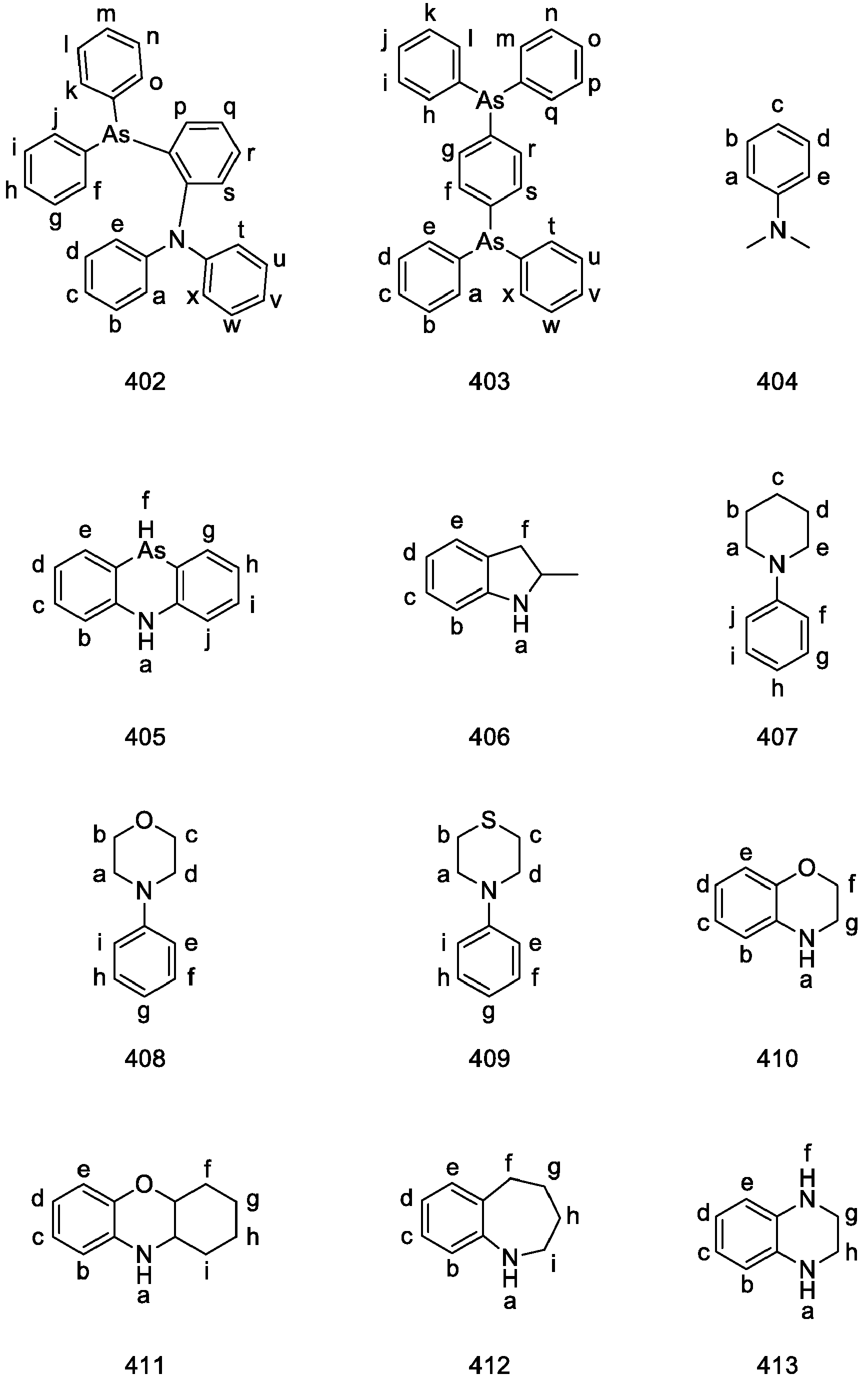

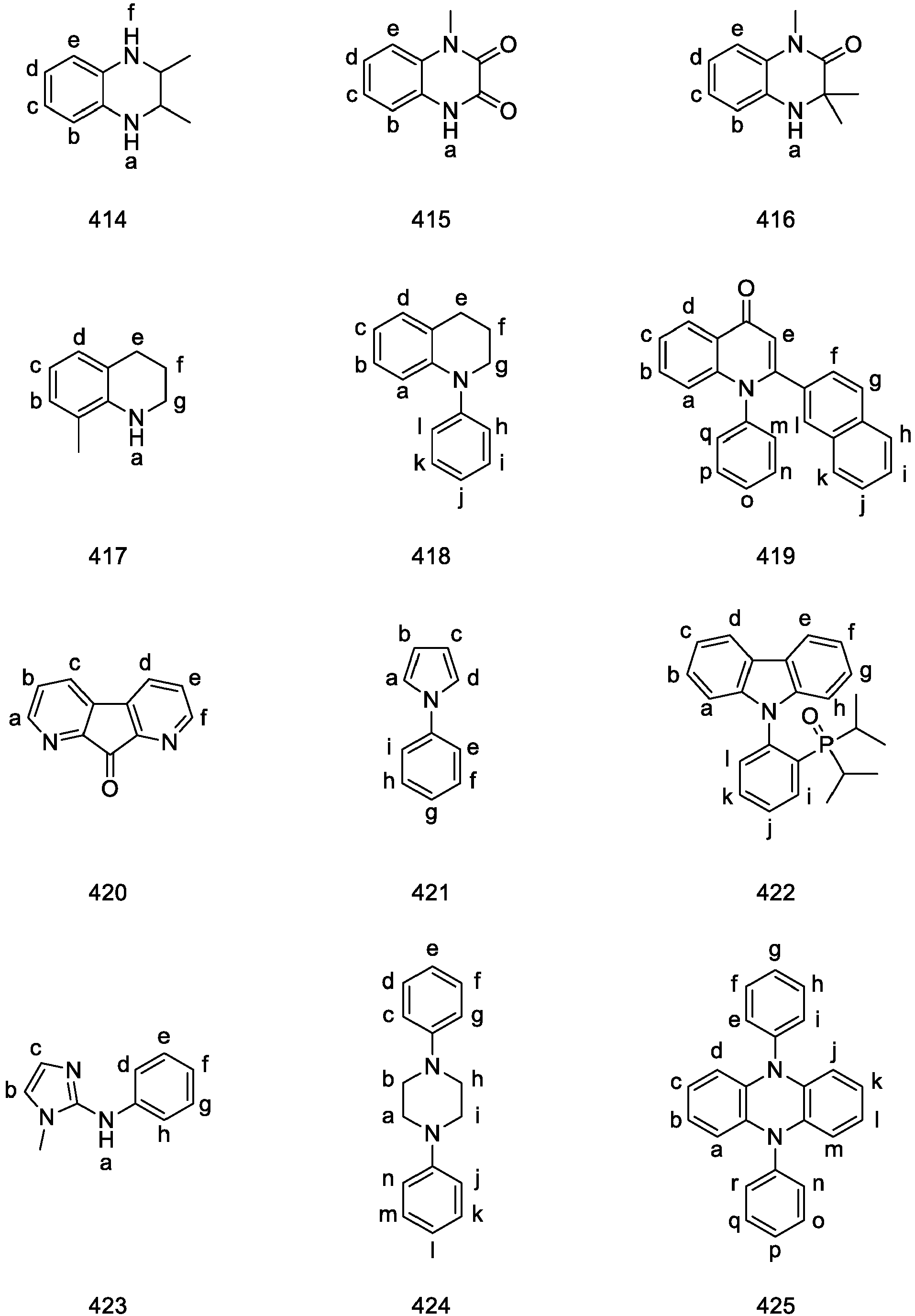

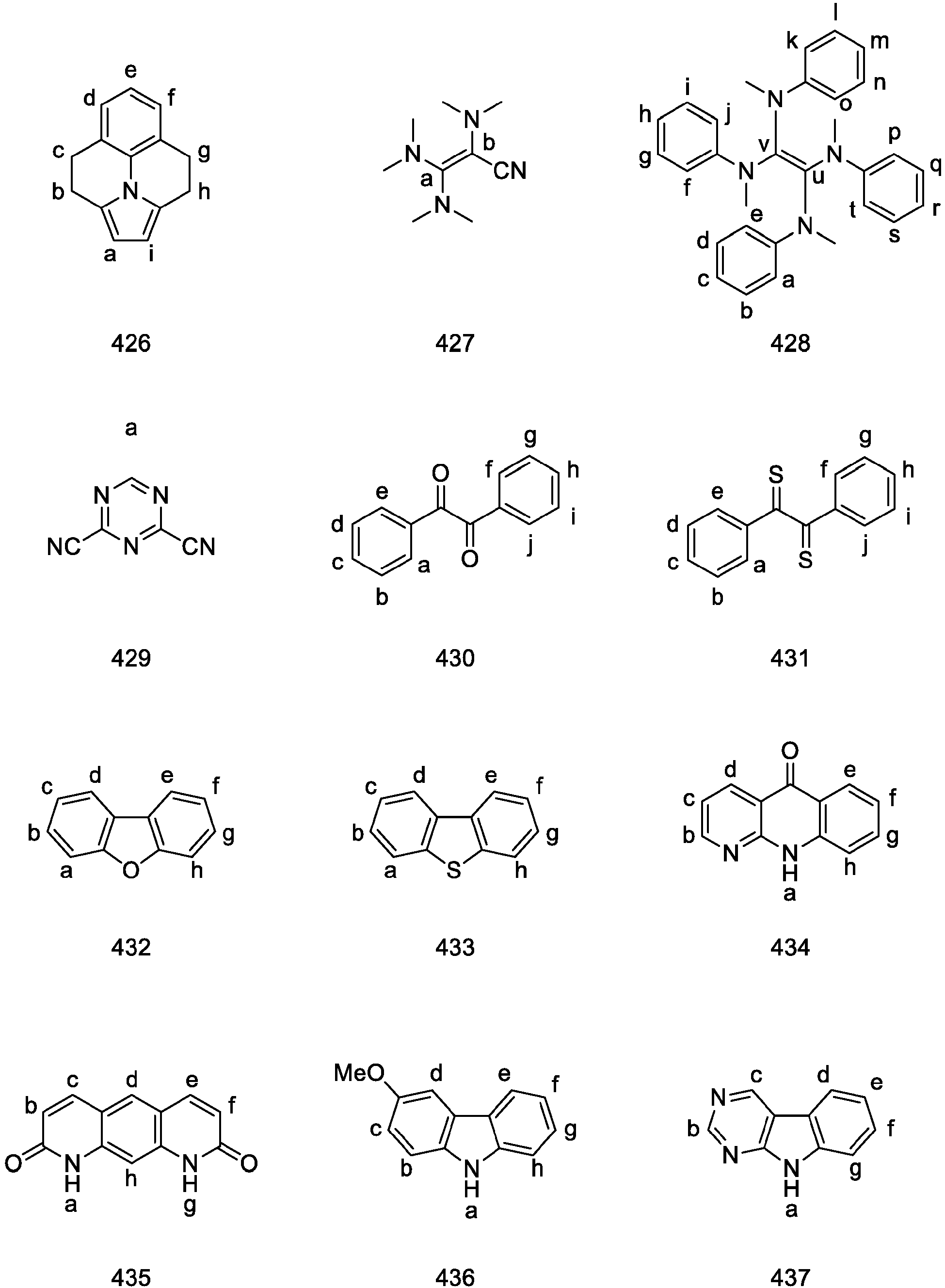

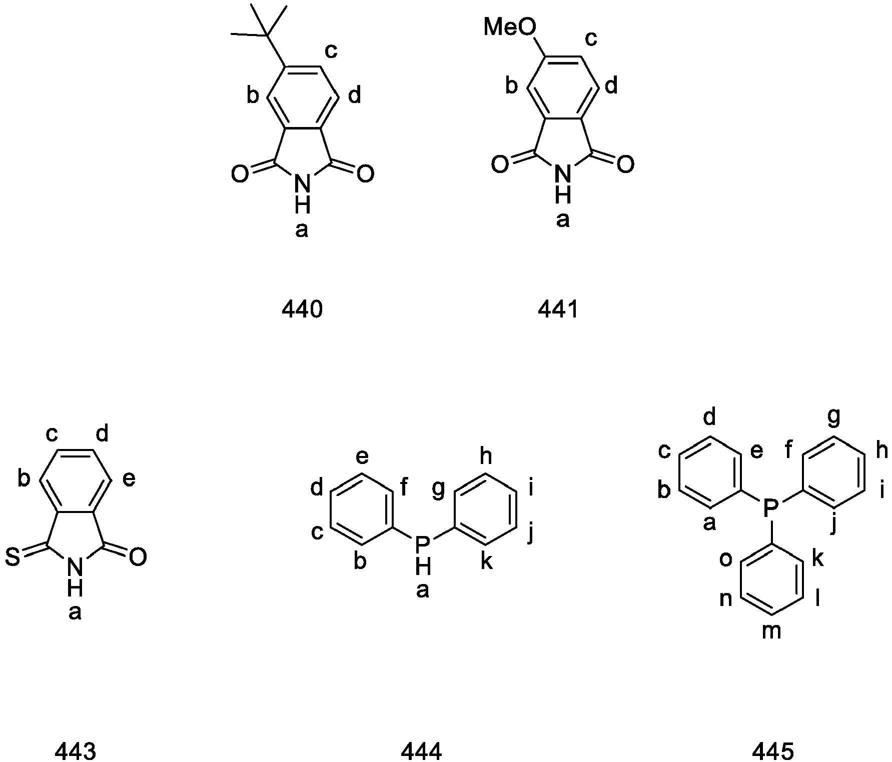

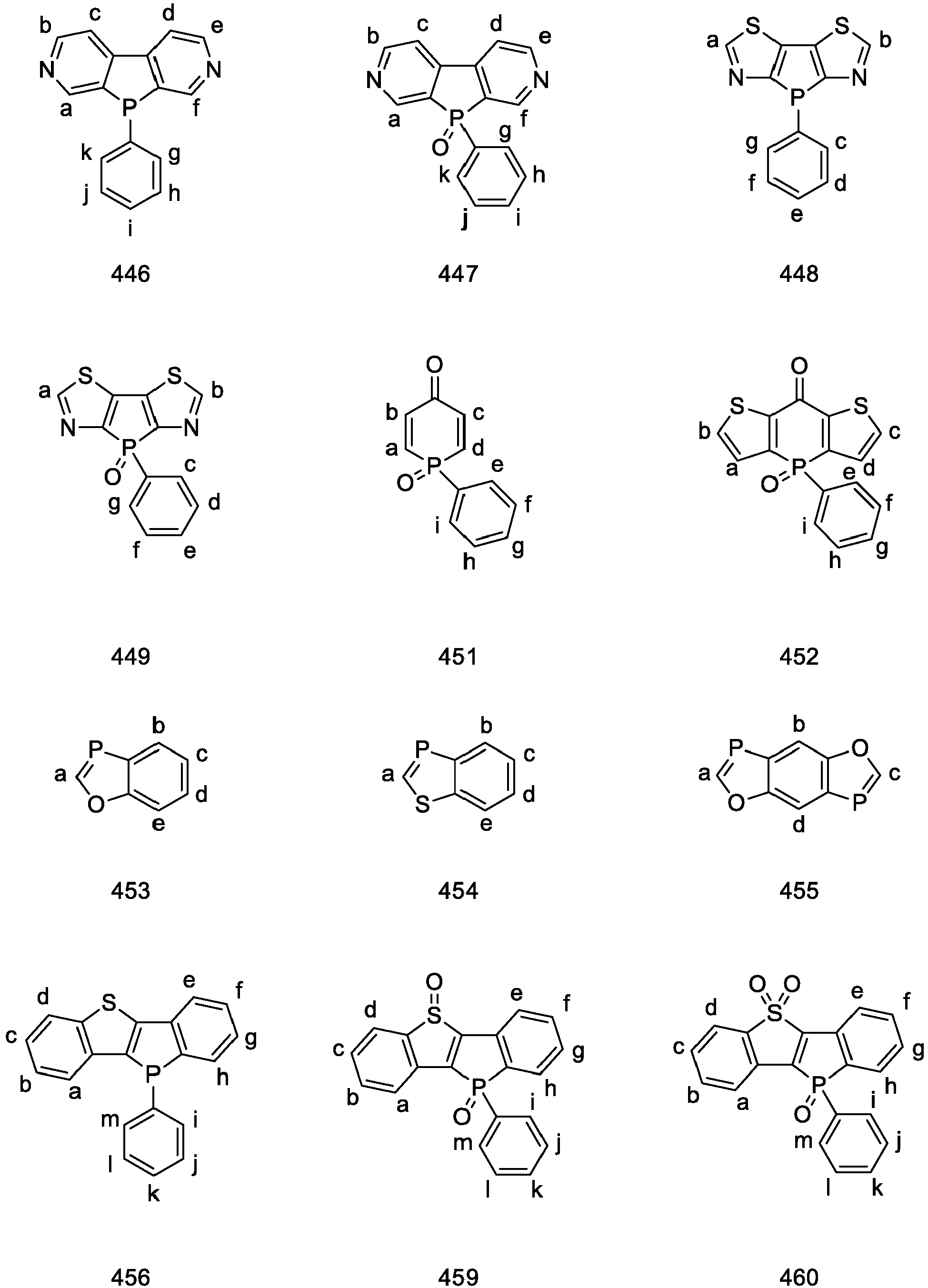

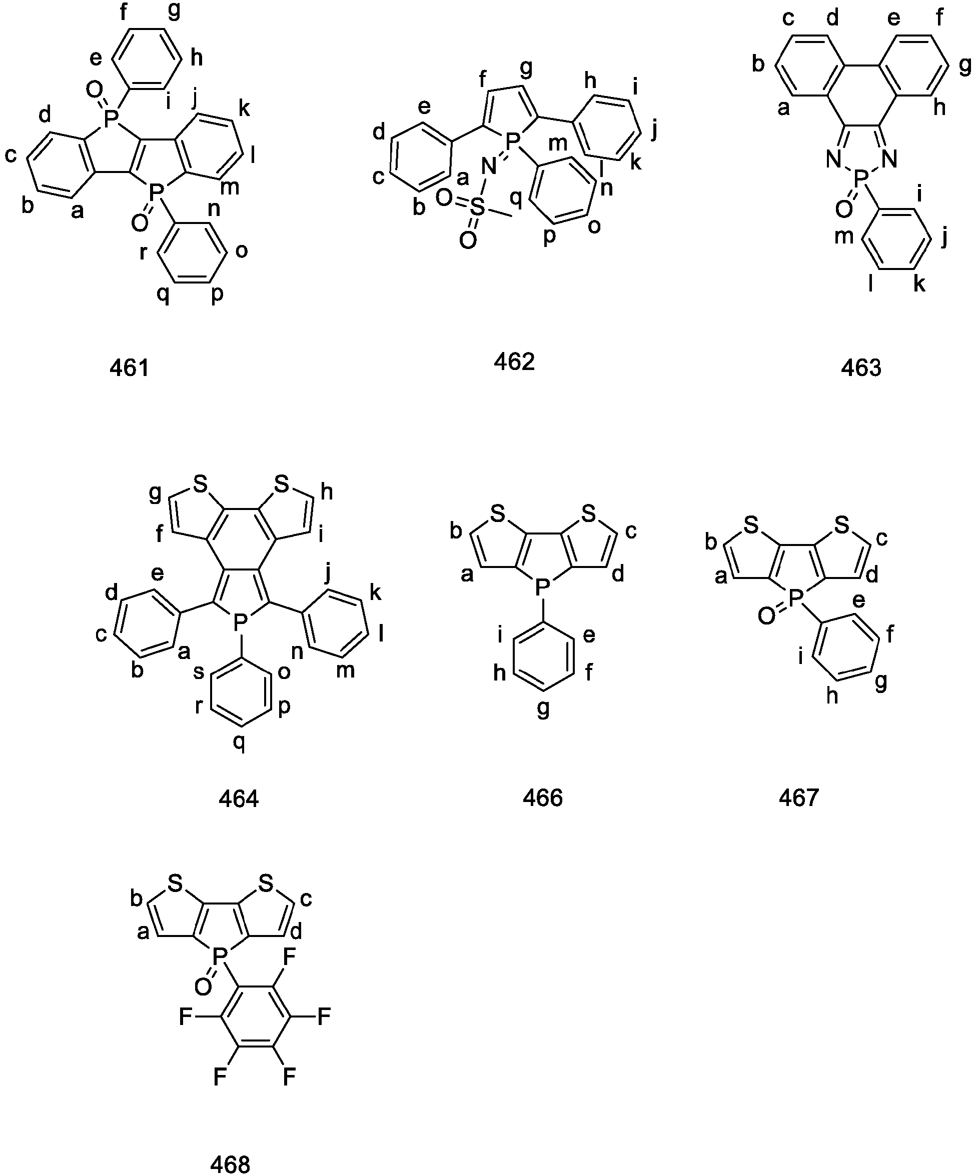

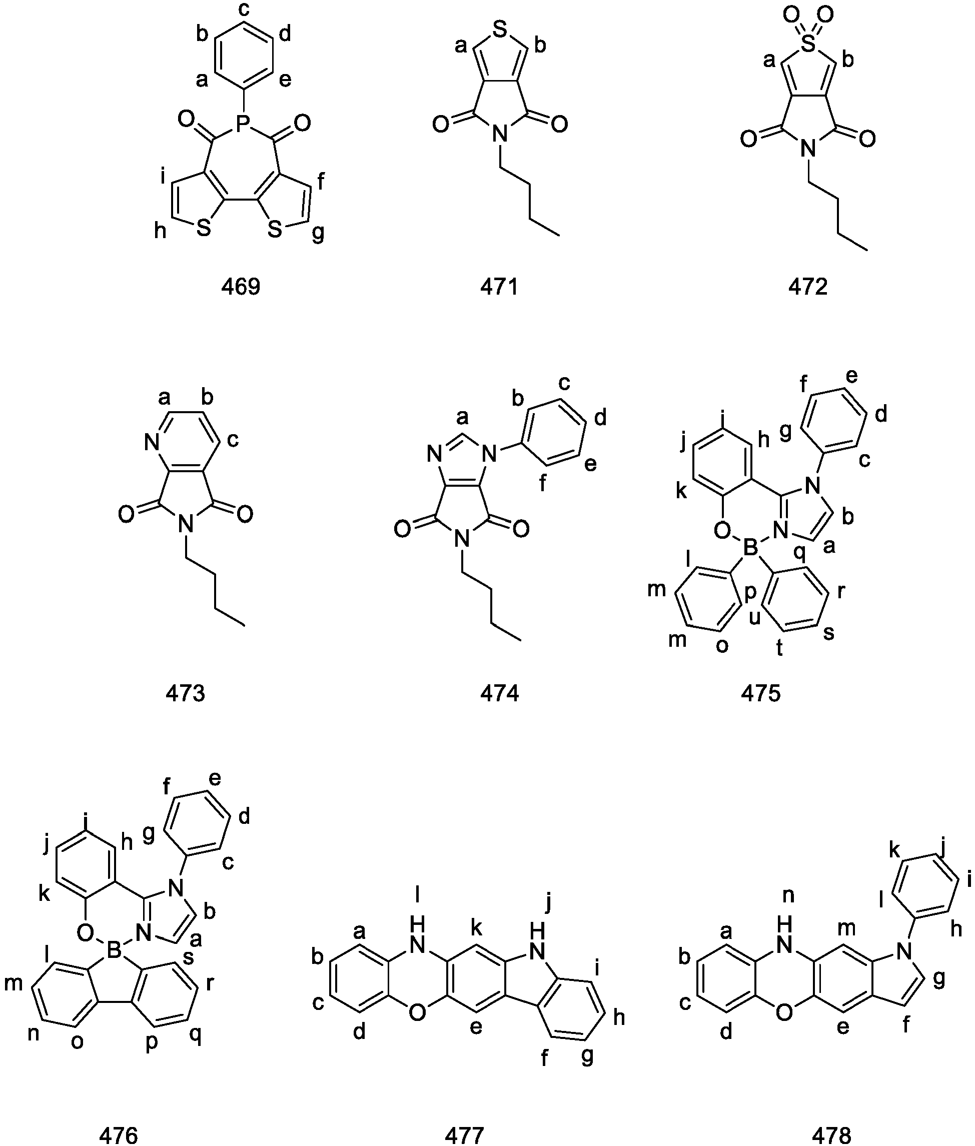

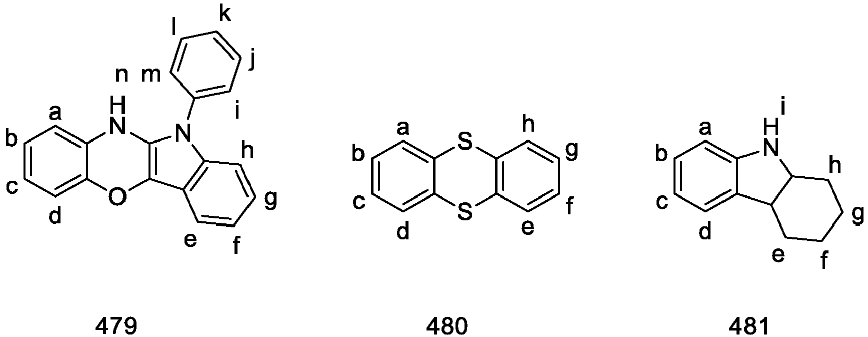

In einer bevorzugten Ausführungsform haben erfindungsgemäße Moleküle die Form AF1-S-AF2, d.h. sie enthalten je eine chemische Einheit AF1 und je eine chemische Einheit AF2; wobei gemäß Formel 1 die beiden Einheiten AF über die in Tabelle 3 definierten Positionen (mit Kleinbuchstaben gekennzeichnet) an eine Separator-Einheit S (aufweisend w, x, y) angebunden sind. Beispiele für solche Moleküle sind in Tabelle 4 genannt. Die Benennung der Moleküle erfolgte dabei folgendermaßen: Die Nummern stehen für die chemischen Einheiten AF1 und AF2 aus Tabelle 3; "S" steht für einen Separator, der bevorzugt aus den Strukturen der Tabelle 5 ausgewählt ist; wobei in den erfindungsgemäßen Molekülen der Form AF1-S-AF2 optional jedes der substituierbaren Protonen durch einen Rest R* ersetzt sein kann. Dies betrifft entsprechend die Substitution von CH₃ zu CH_{2'}AF, CHAF₂, oder CAF₃, von CH₂ zu CH₂AF und CF₂, von CH zu CAF, wobei AF sowohl AF1 als auch AF2 bedeuten kann und auch unterschiedliche AF (AF1 und AF2) an dasselbe Glied angebunden sein können (Fall wie bspw CH₂ zu C(AF1)(AF2)).

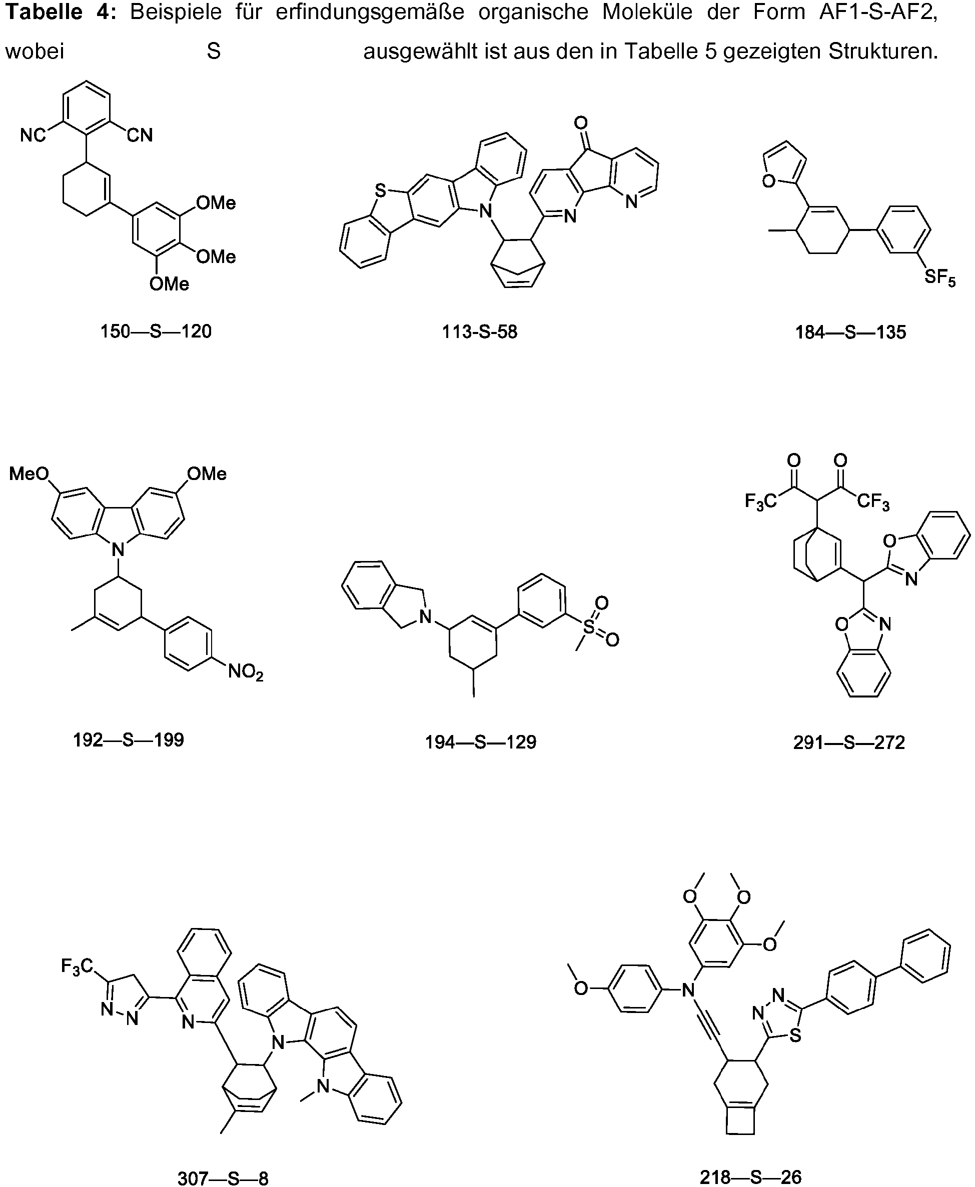

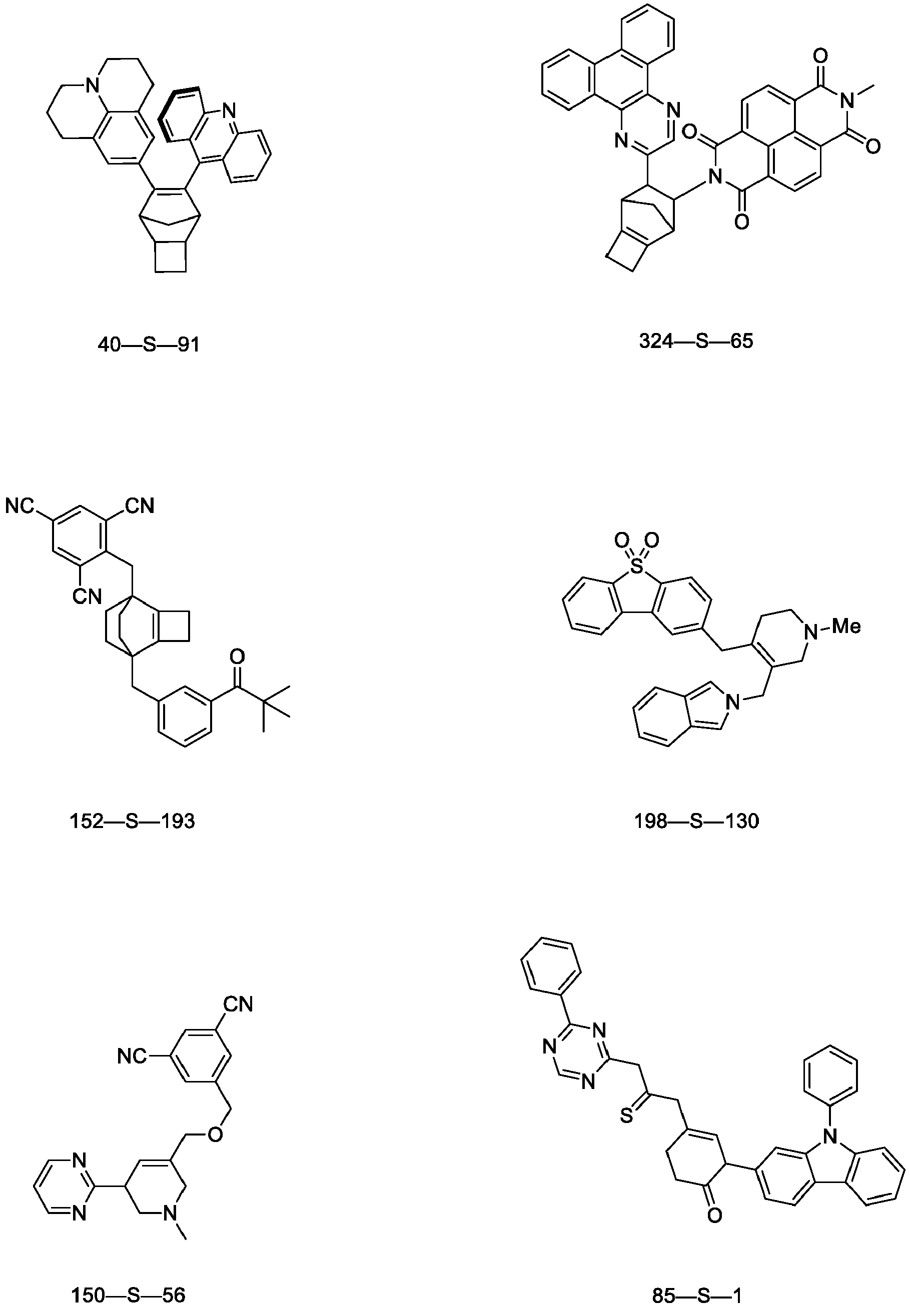

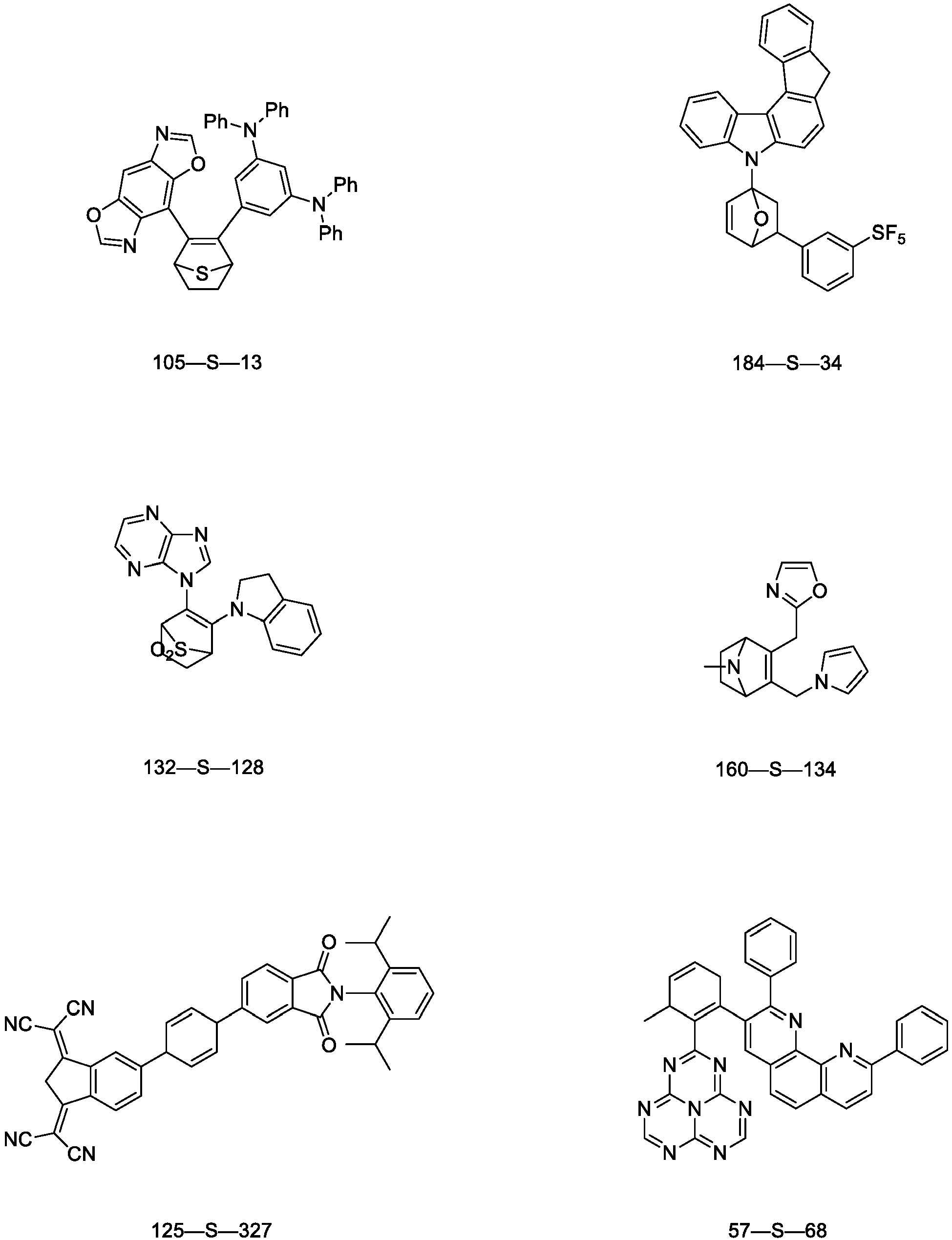

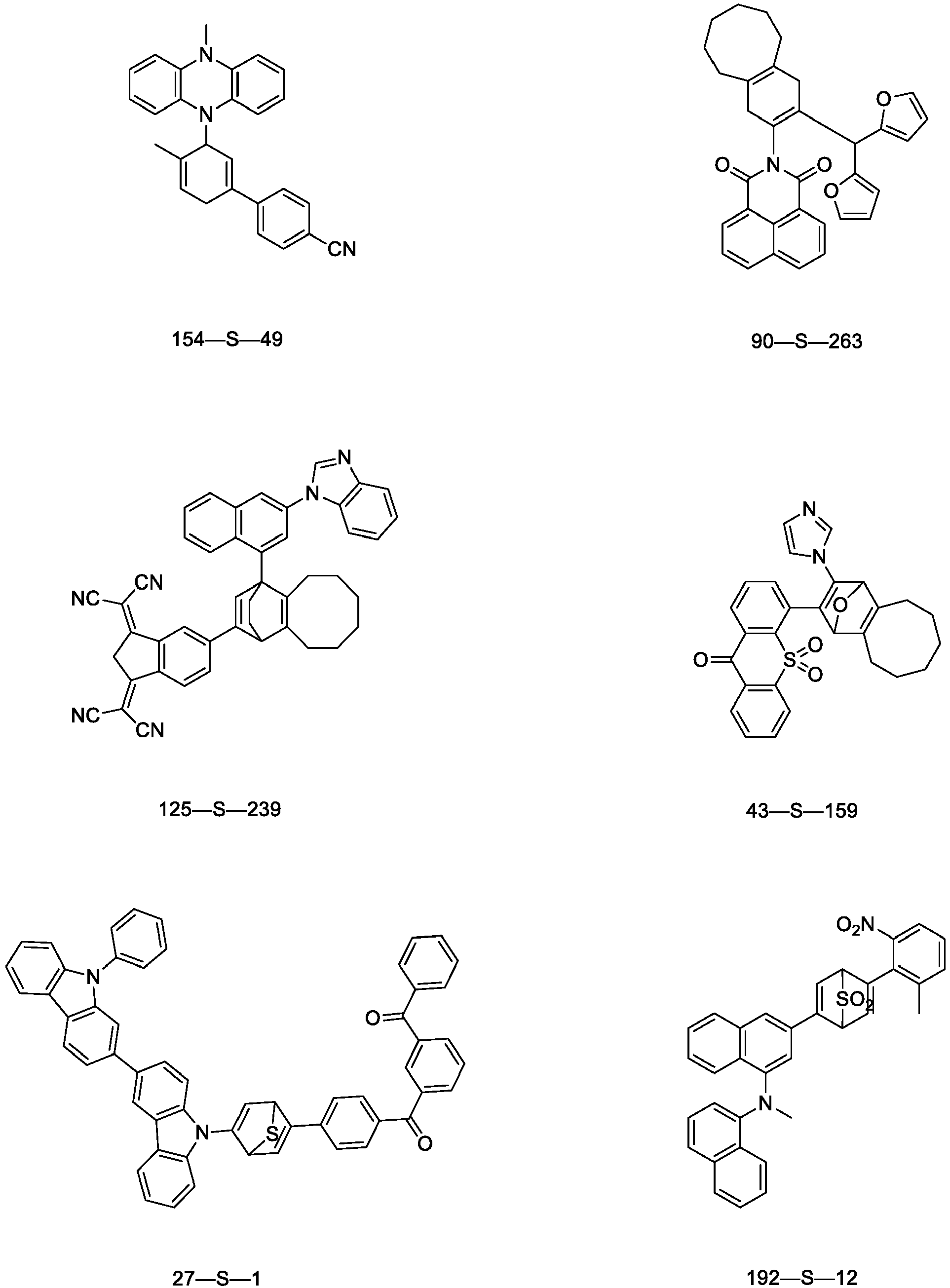

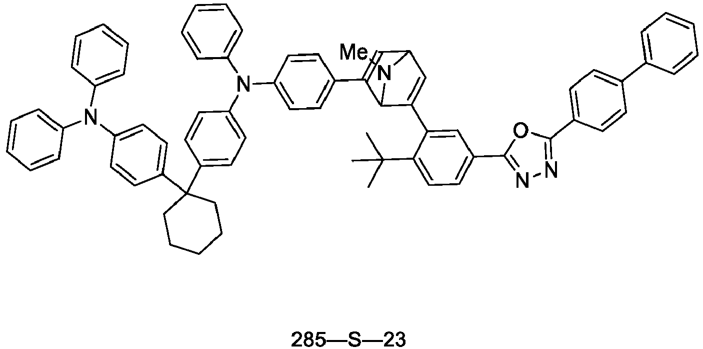

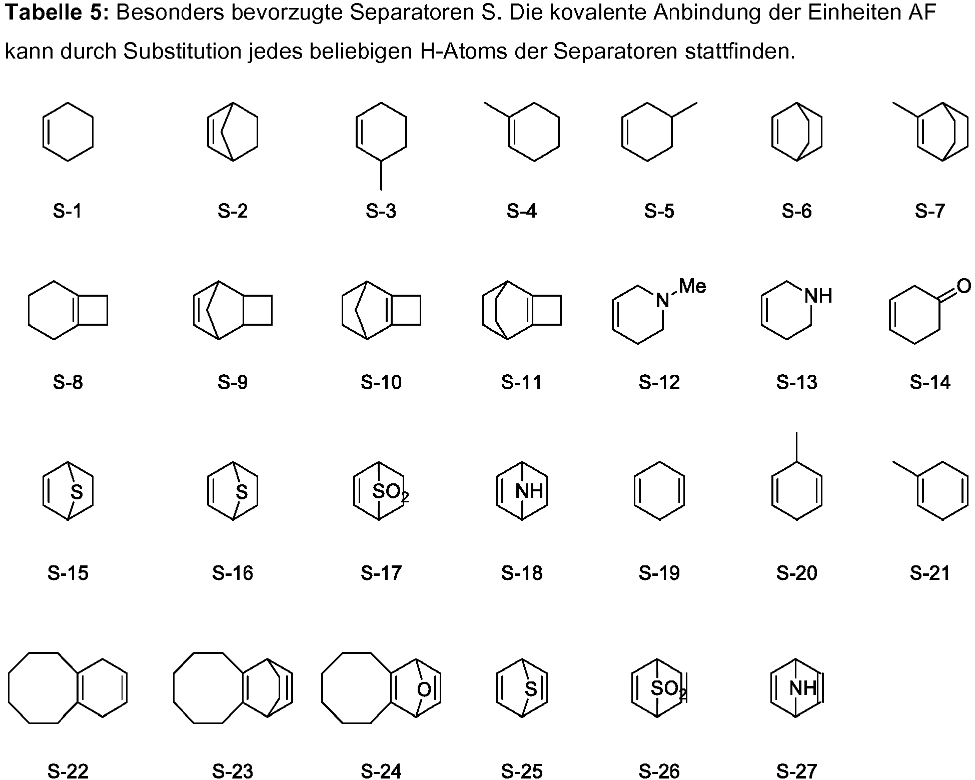

Insbesondere der Separator S unterscheidet die organischen Moleküle funktionell von Molekülen gemäß dem Stand der Technik, da die hier gezeigte Art der Trennung von AFs (oder Donoren und Akzeptoren) bisher noch nicht bekannt ist. Bekannte organische Emitter bestehen in der Regel aus direkt verknüpften chemischen Einheiten. Eine Trennung der konjugierten aromatischen Systeme fand bisher nicht statt, insbesondere in Verbindung mit der Lokalisierung von HOMO und LUMO auf separaten Molekülteilen. Separatoren im Sinne dieser Erfindung verändern die Lage des HOMO bzw. LUMO der in Tabelle 3 gezeigten AFs nicht signifikant. Als nicht signifikant gilt im Rahmen dieser Erfindung eine Veränderung von nicht mehr als +/- 0,4 eV. Die Berechnung derartiger Energien ist bekannt und funktioniert nach der oben beschriebenen Weise per DFT-Rechnung.

Anhand spektroskopischer Auswahlregeln (symmetrische Moleküle) oder durch Messung des Extinktionskoeffizienten (UV/VIS-Spektroskopie) oder quantenchemische Berechnung der Oszillatorstärke kann vorhergesagt werden, ob ein quantenmechanischer Übergang erlaubt ist. Je größer die Oszillatorstärke, desto eher ist ein Übergang erlaubt und desto schneller ist der damit verbundene Prozess (Abklingdauer). Angestrebt sind Abklingdauern von < 200 µs, bevorzugt < 100 µs, besonders bevorzugt < 50 µs. Bei einer langen Abklingdauer des (organischen) Emitters kommt es bei hohen Stromstärken schnell zu Sättigungseffekten, was die Bauteillebensdauer negativ beeinflusst und die Erreichung hoher Helligkeiten verhindert.
Ein Maß für die Abklingdauer ist der ΔE(S₁T₁)-Abstand. Dieser wird durch die Überlappung von HOMO und LUMO beeinflusst. Die Größe des quantenmechanischen Überlappungsintegrals, welche nach oben genannter DFT-Methode berechenbar ist, kann durch Wahl des Separators gezielt gesteuert werden. Kommt es zur völligen Trennung von HOMO und LUMO hat dieses einen Wert von O. Die Wahrscheinlichkeit einer effizienten Emission des organischen Moleküls sinkt drastisch. Bei einem Wert von 1 liegt nicht mehr verzögerte Fluoreszenz (TADF) sondern spontane Emission vor. Die gewünschte Überlappung wird durch die geeignete Wahl eines erfindungsgemäßen Separators S erreicht.

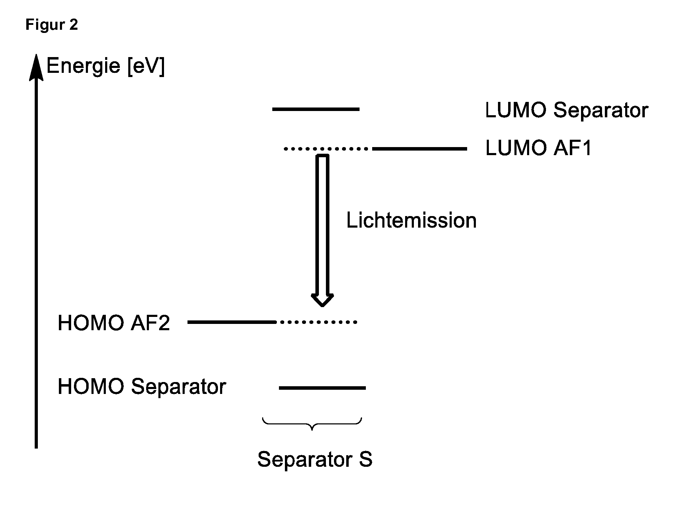

Die gezeigten Separatoren S erfüllen dabei insbesondere zwei wesentliche funktionelle Merkmale (s. Figur 2):
- die HOMO-Energie ist niedriger als die HOMO-Energie der als Donor fungierenden chemischen Einheit AF und
- die LUMO-Energie ist höher als die LUMO-Energie der als Akzeptor fungierenden chemischen Einheit AF.

Weitere erfindungsgemäße Moleküle die Form AF1-S-AF2, die je eine chemische Einheit AF1 und je eine chemische Einheit AF2 enthalten, wobei beide Einheiten ausgewählt sind aus den Strukturen der Tabelle 3 und wobei gemäß Formel 1 die beiden Einheiten AF über die in Tabelle 3 definierten Positionen (mit Kleinbuchstaben gekennzeichnet) an eine Separator-Einheit S (aufweisend w, x, y) angebunden sind, werden in Tabelle 6 genannt. Die Benennung der Moleküle erfolgte dabei folgendermaßen: Die Nummern stehen für die chemischen Einheiten AF1 und AF2 aus Tabelle 3; "S" steht für einen Separator, nach Formel eins bestehend aus w, x, y, der bevorzugt aus den Strukturen der Tabelle 5 ausgewählt ist; wobei in den erfindungsgemäßen Molekülen der Form AF1-S-AF2 optional jedes der substituierbaren Protonen durch einen Rest R* ersetzt sein kann.

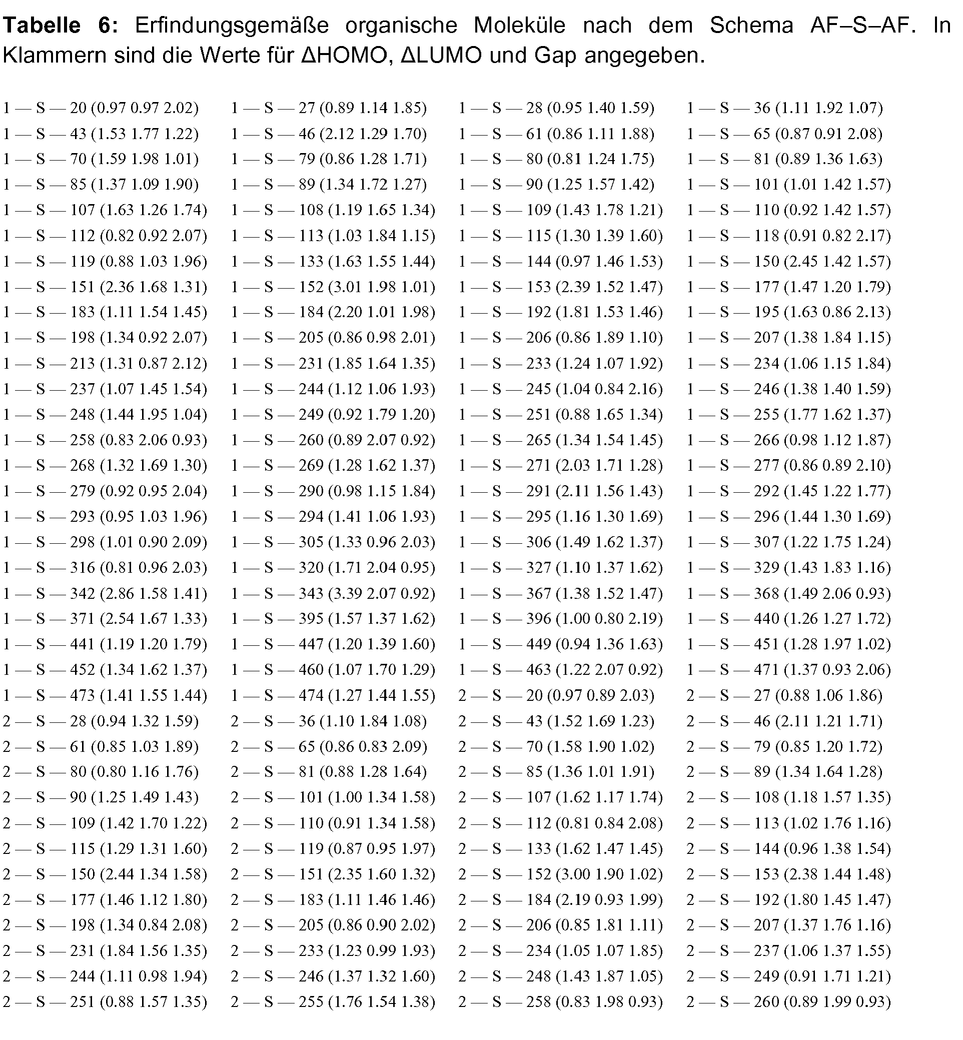

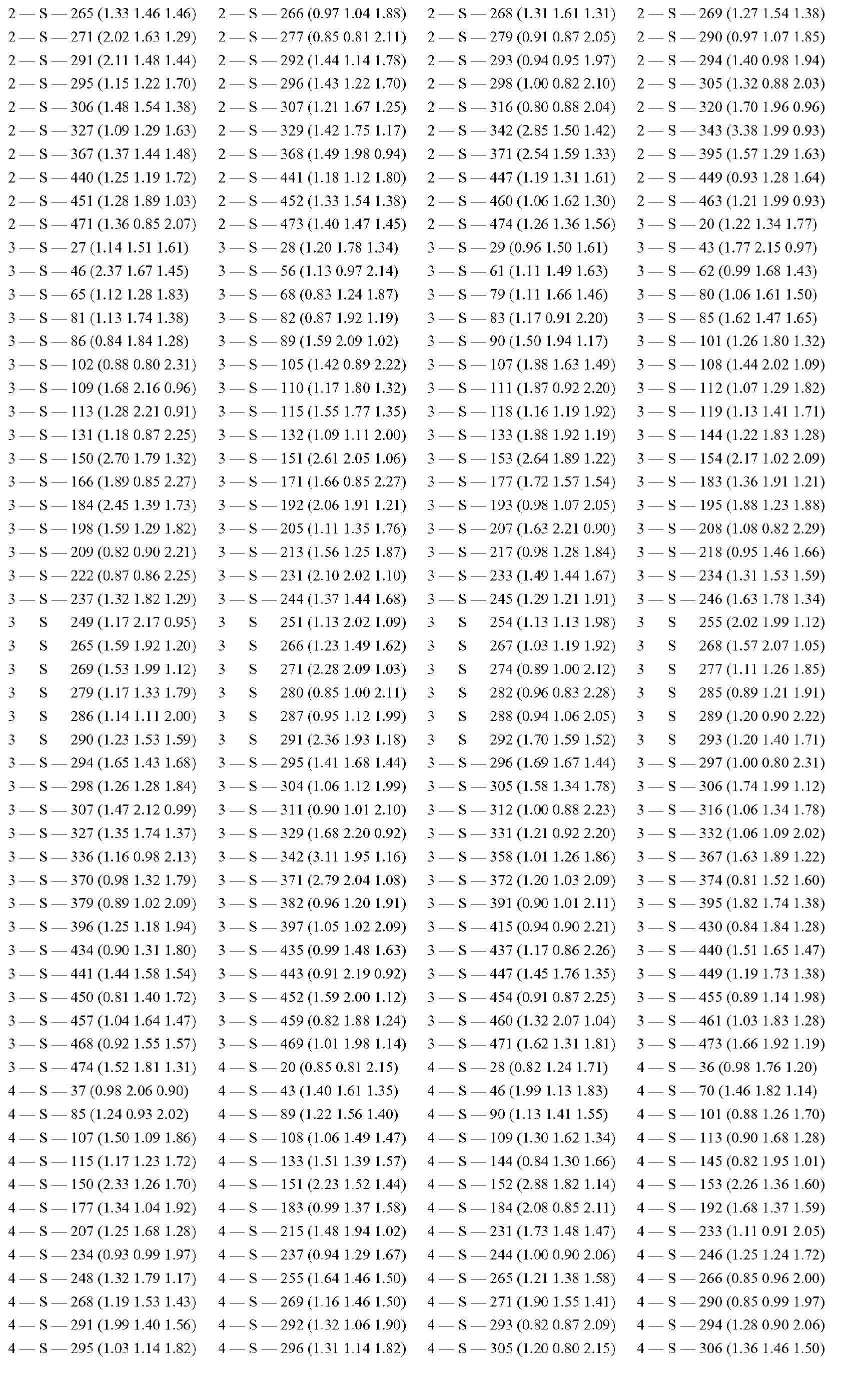

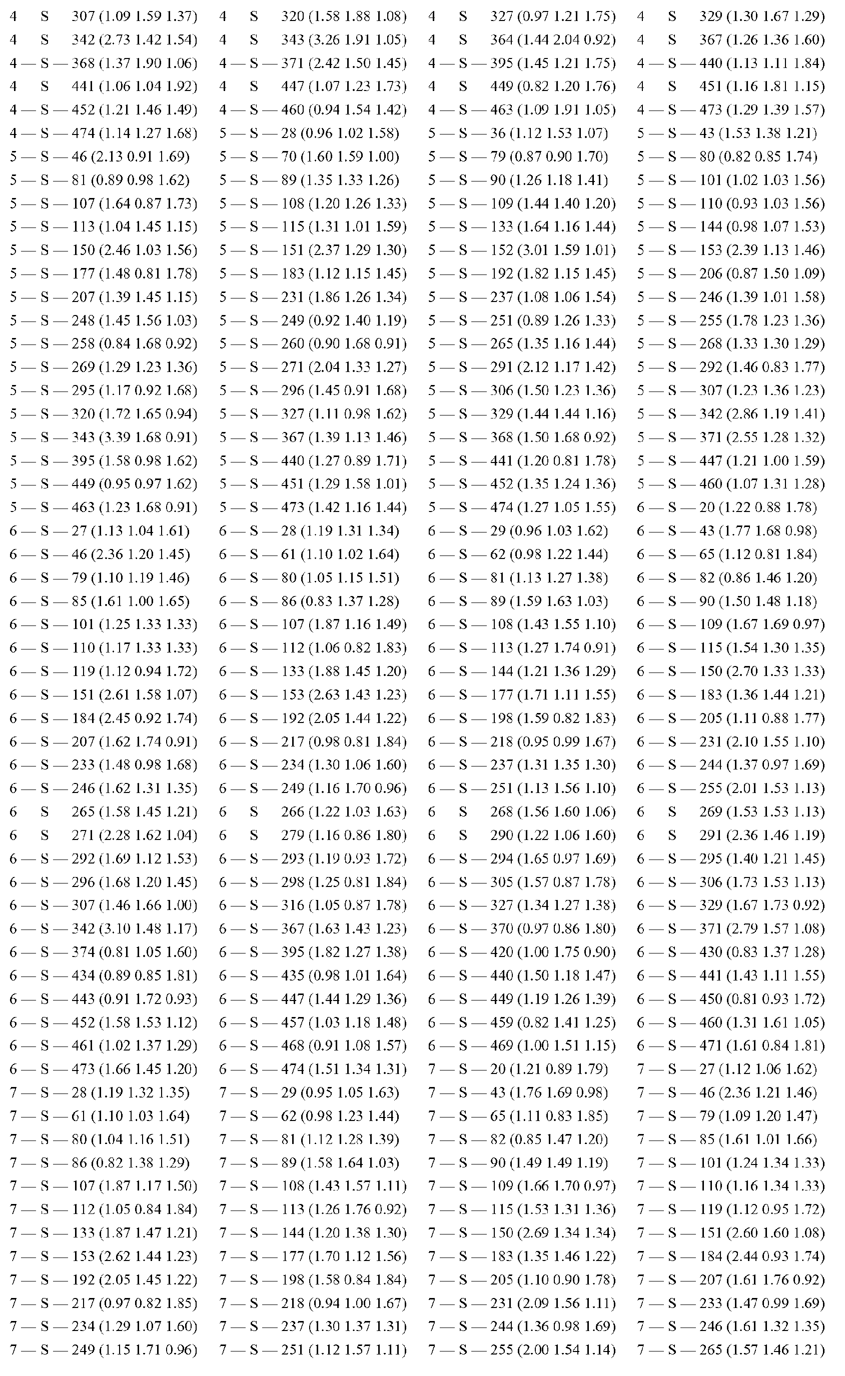

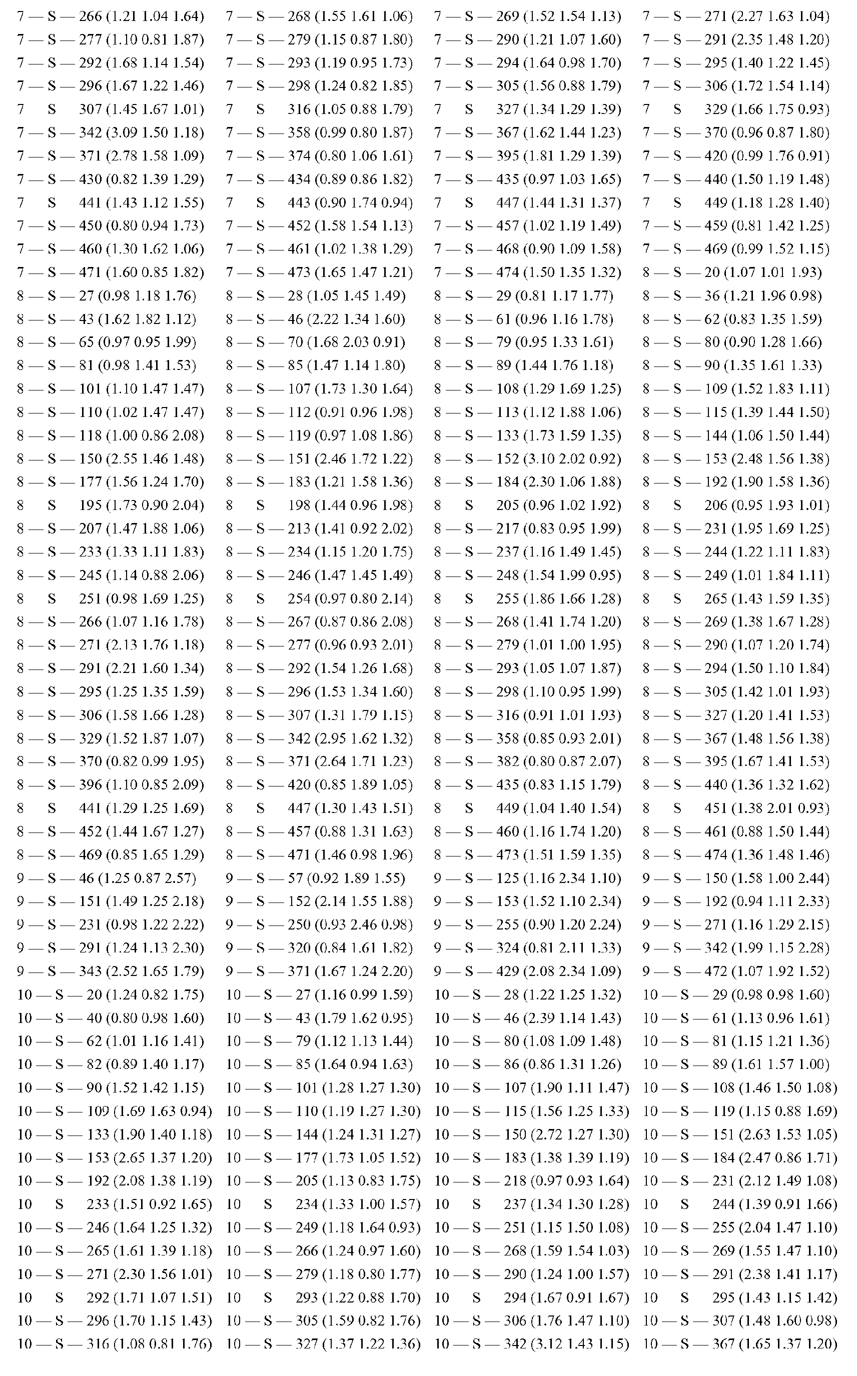

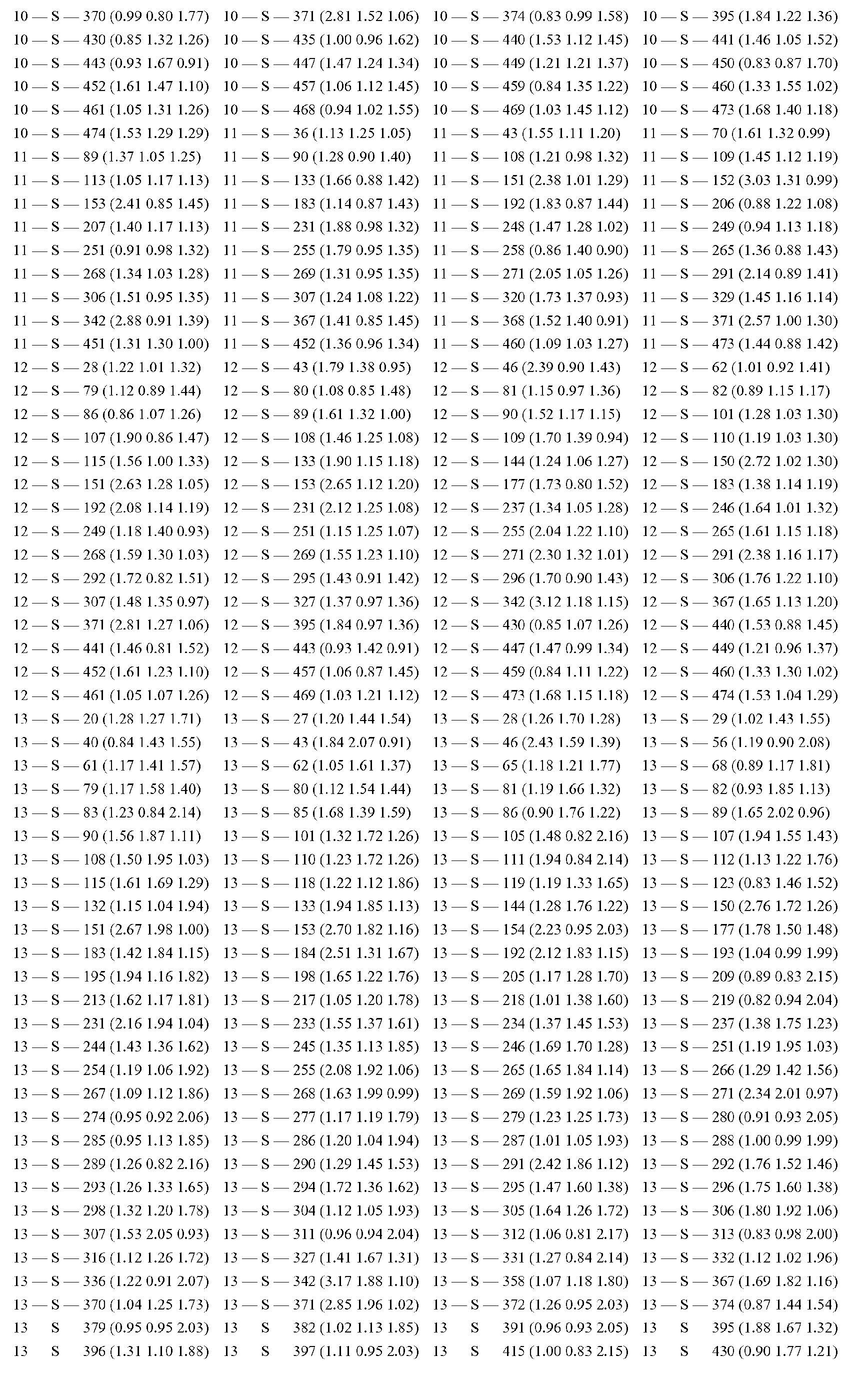

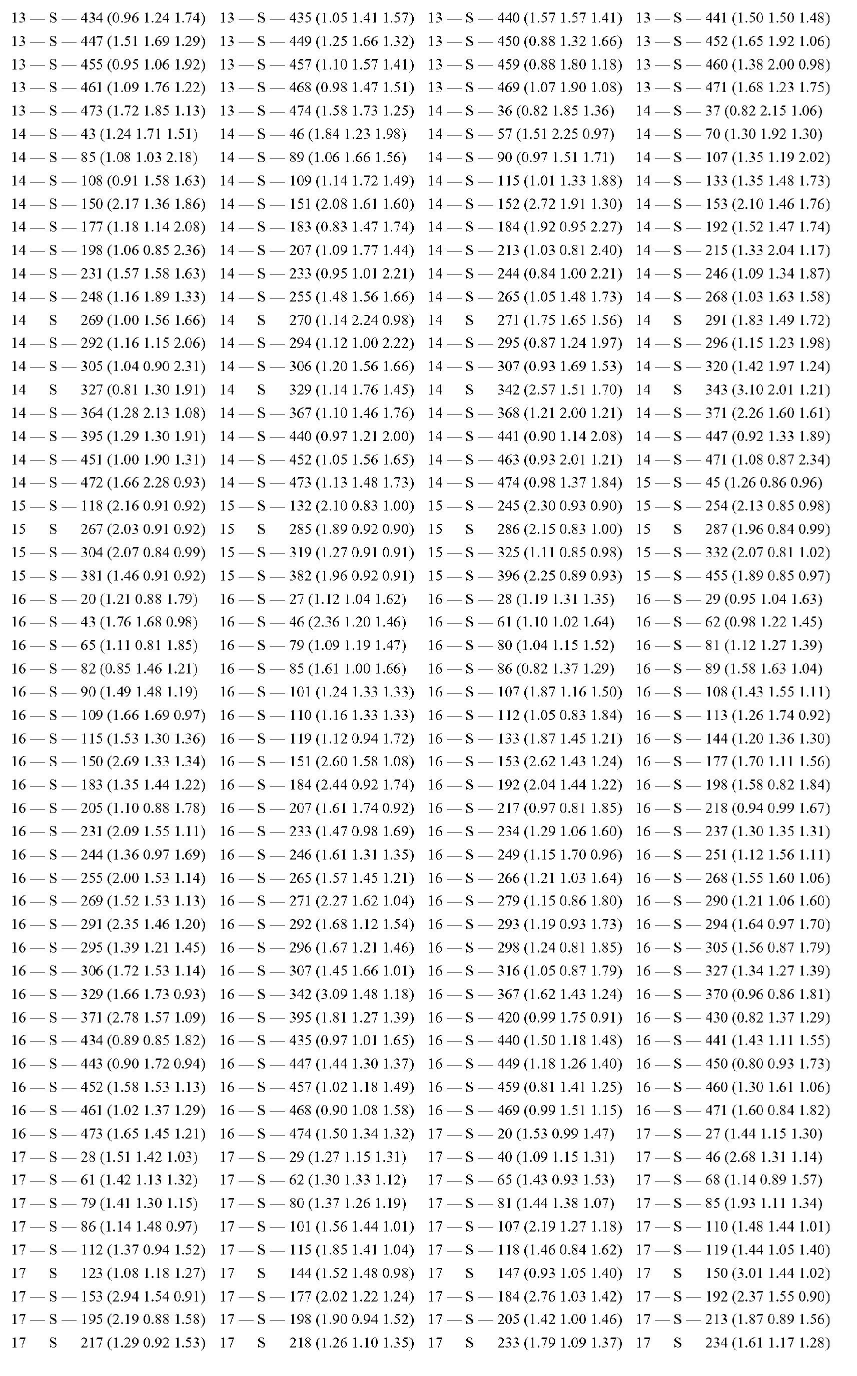

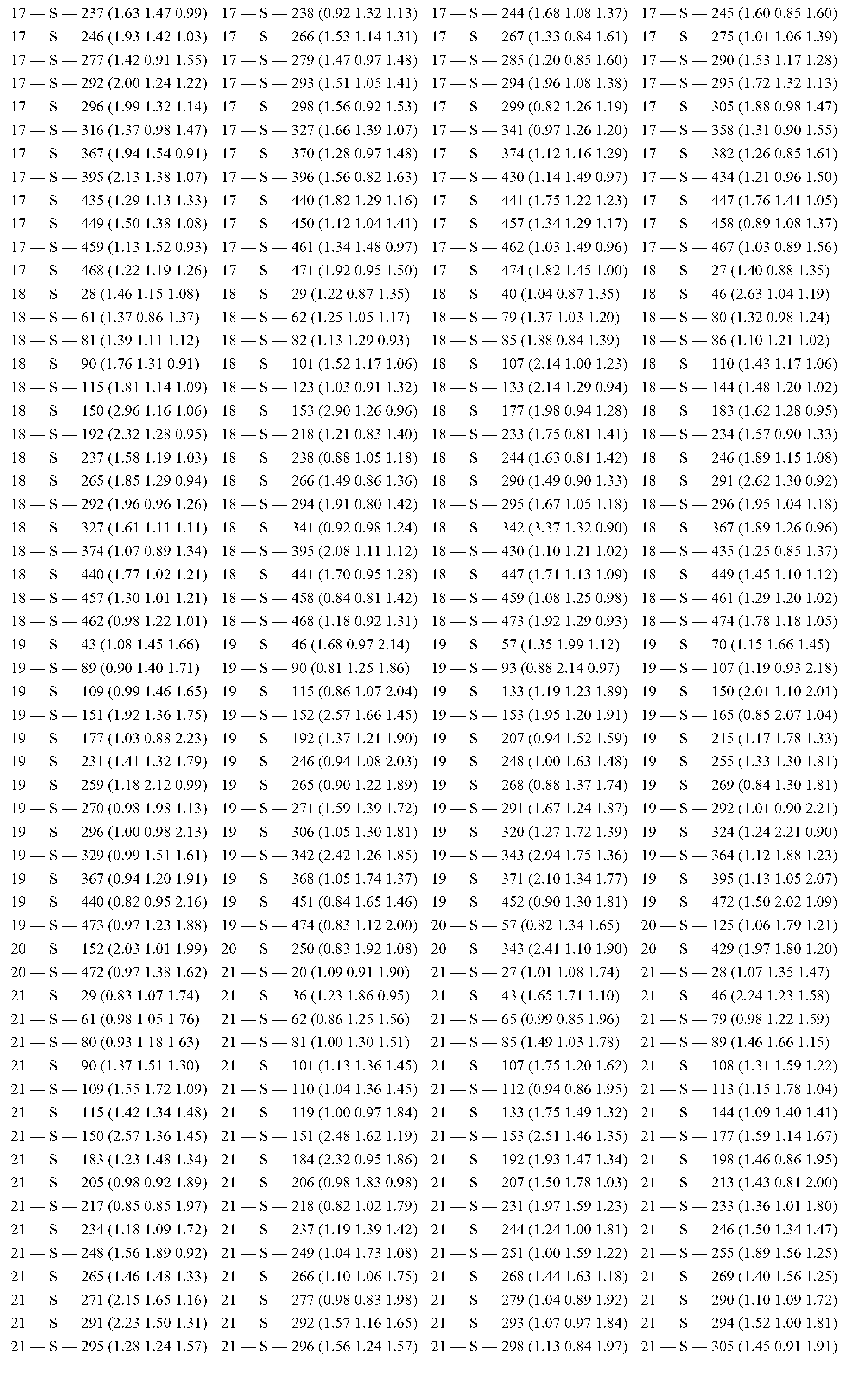

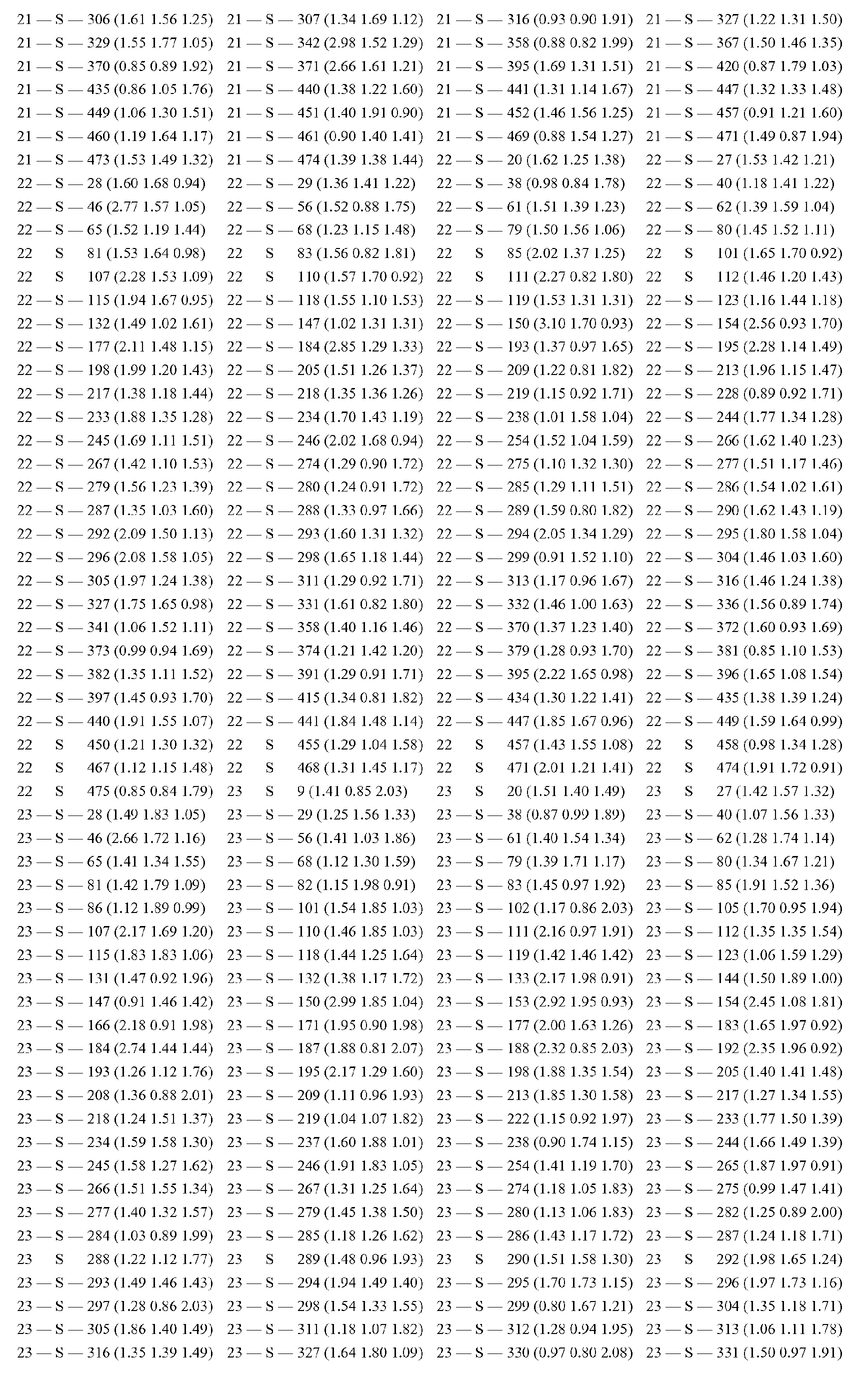

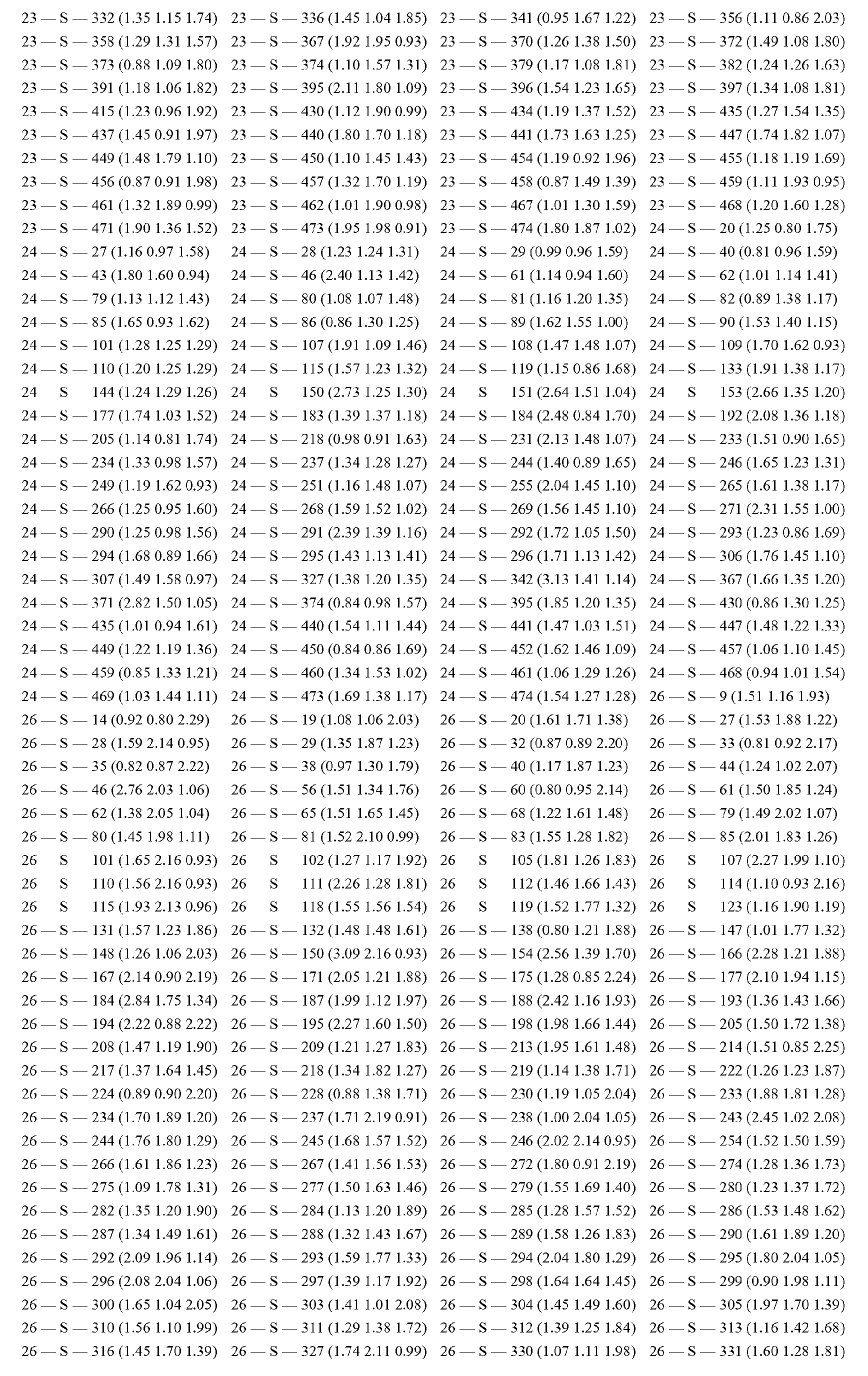

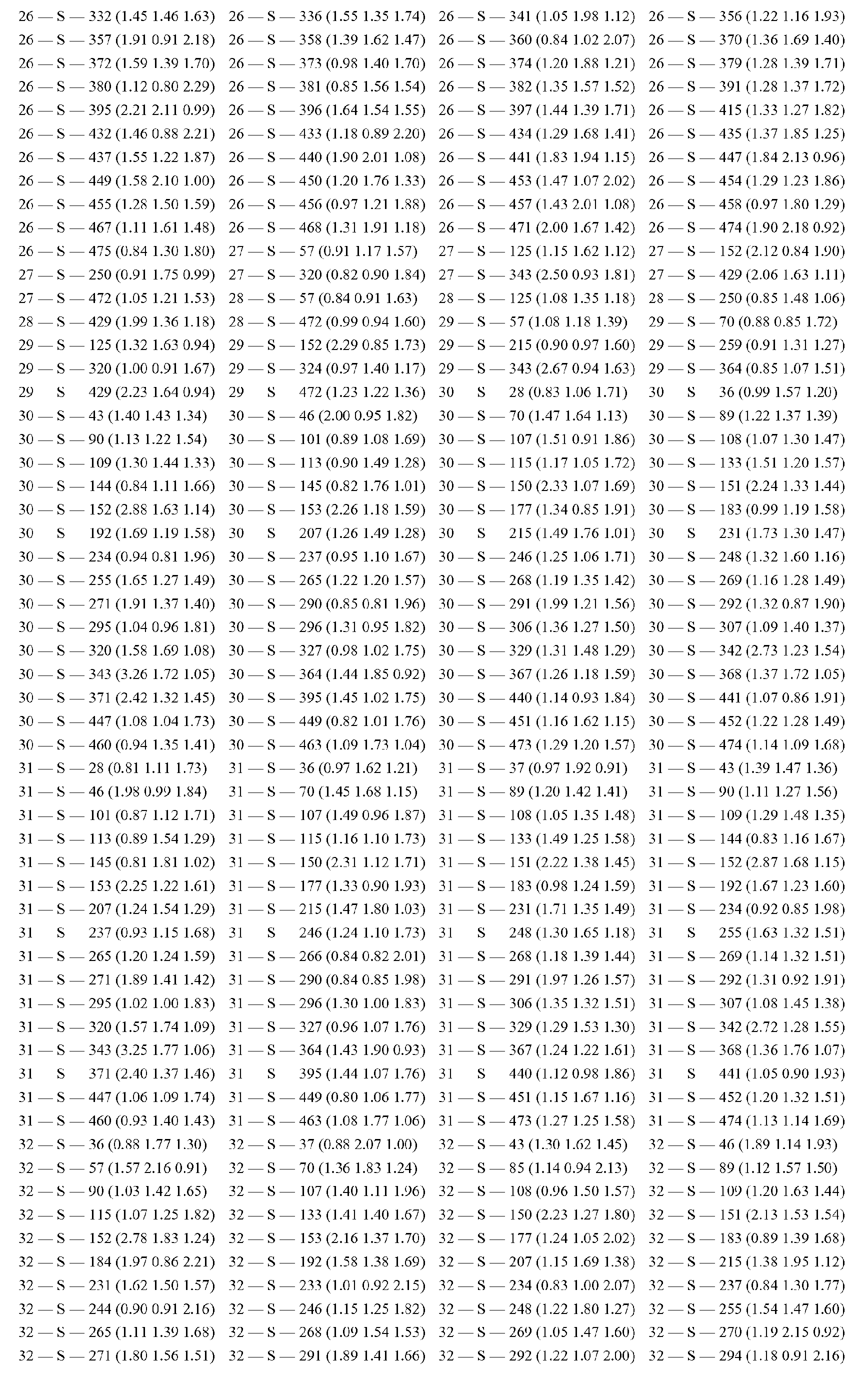

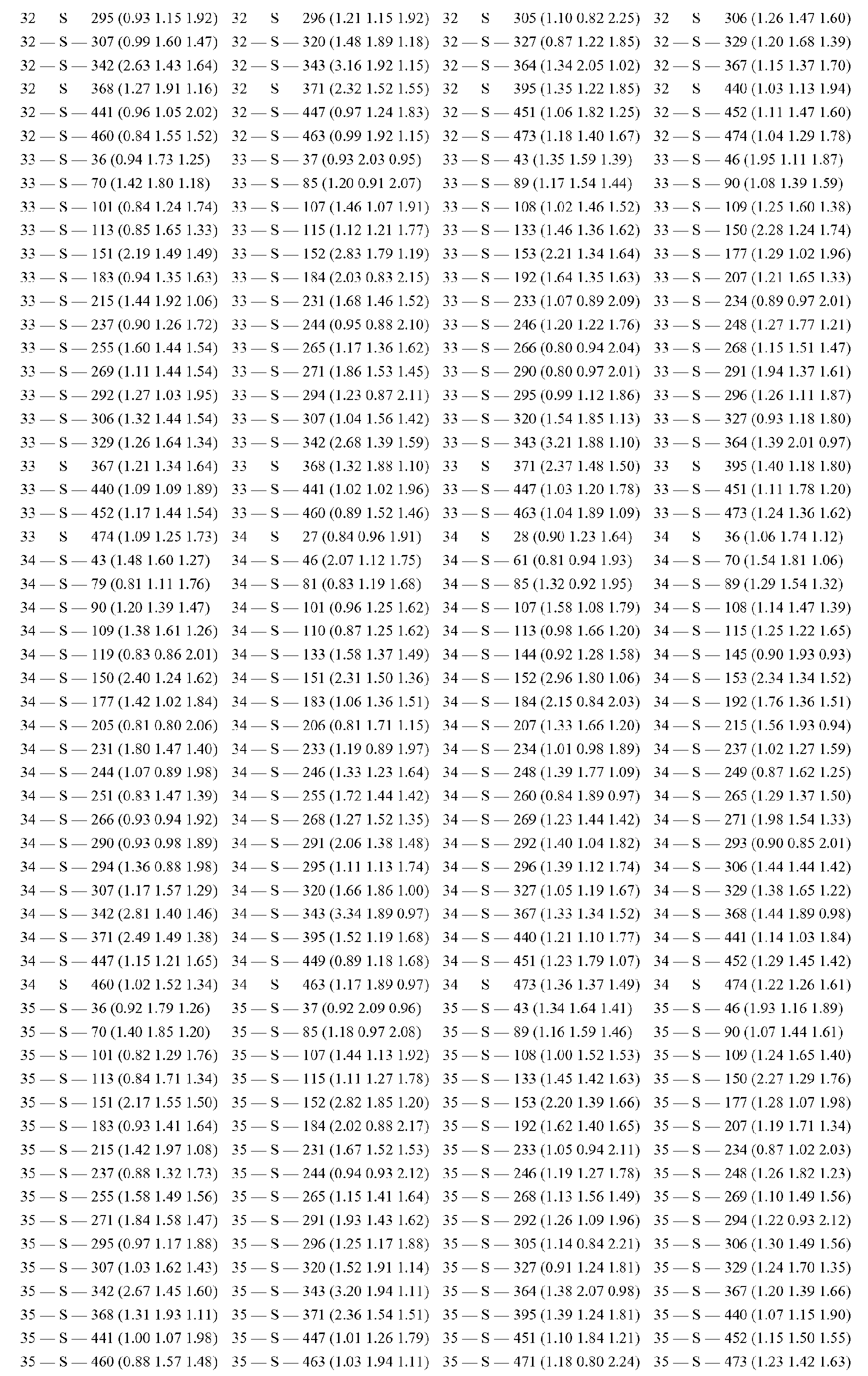

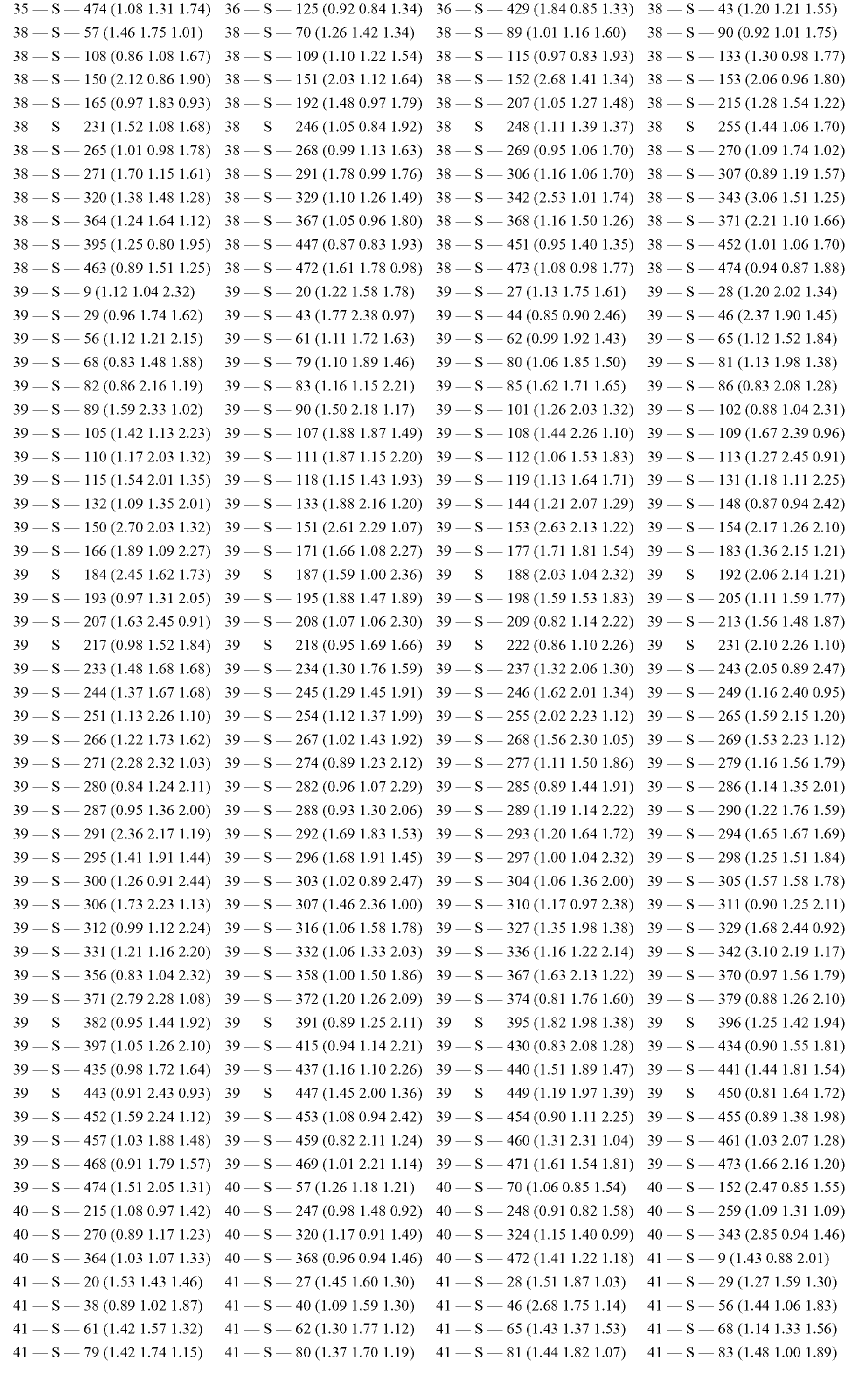

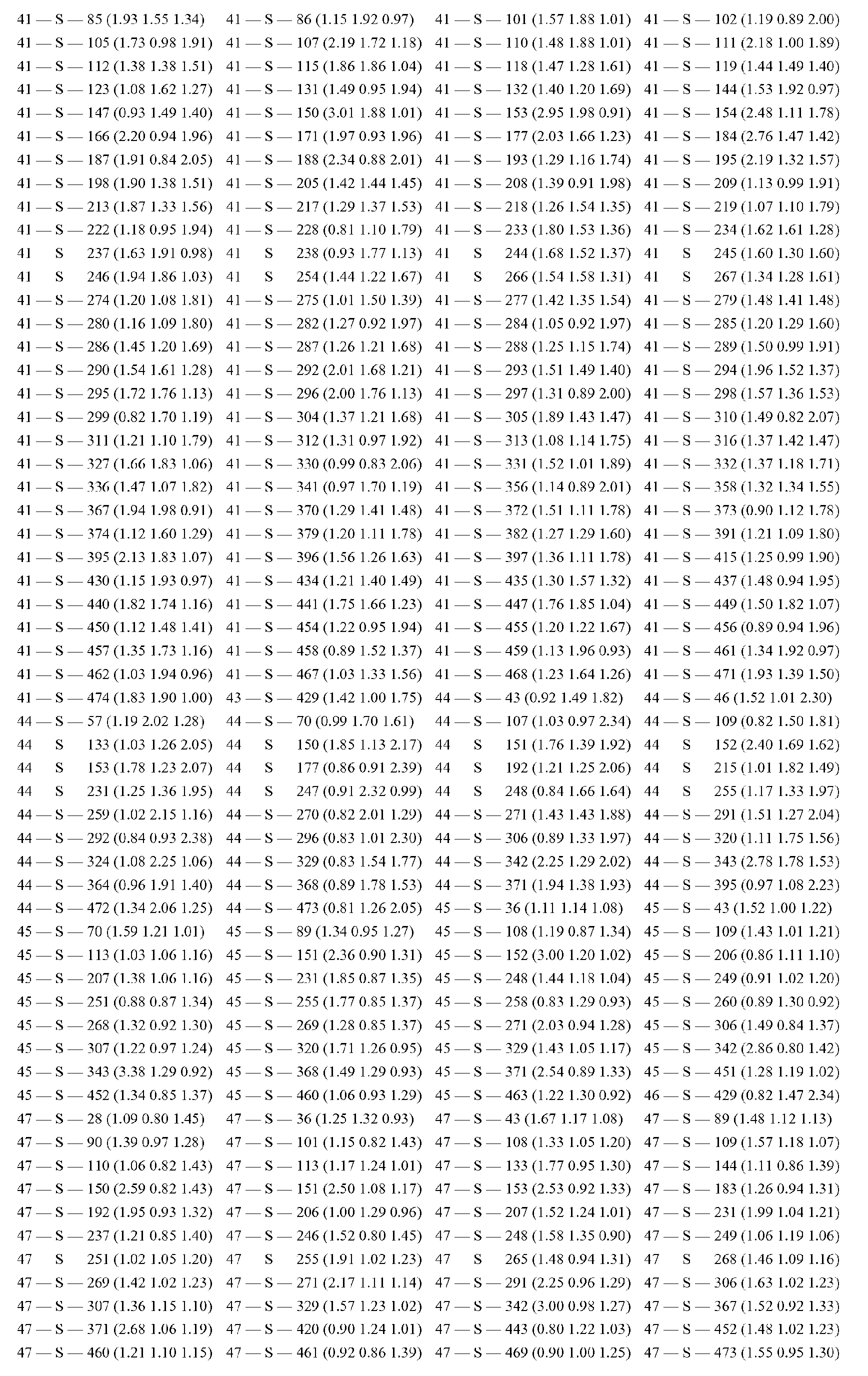

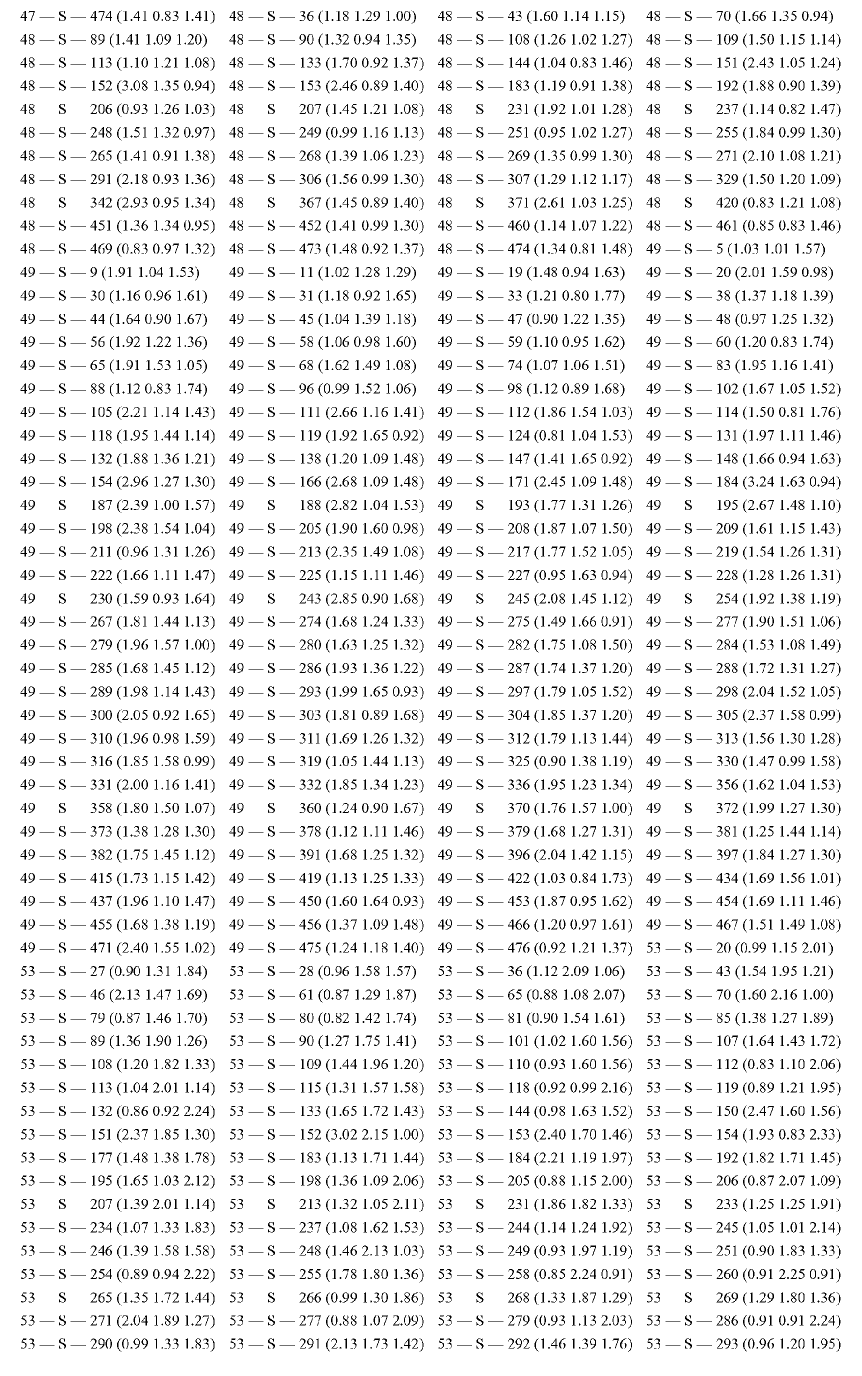

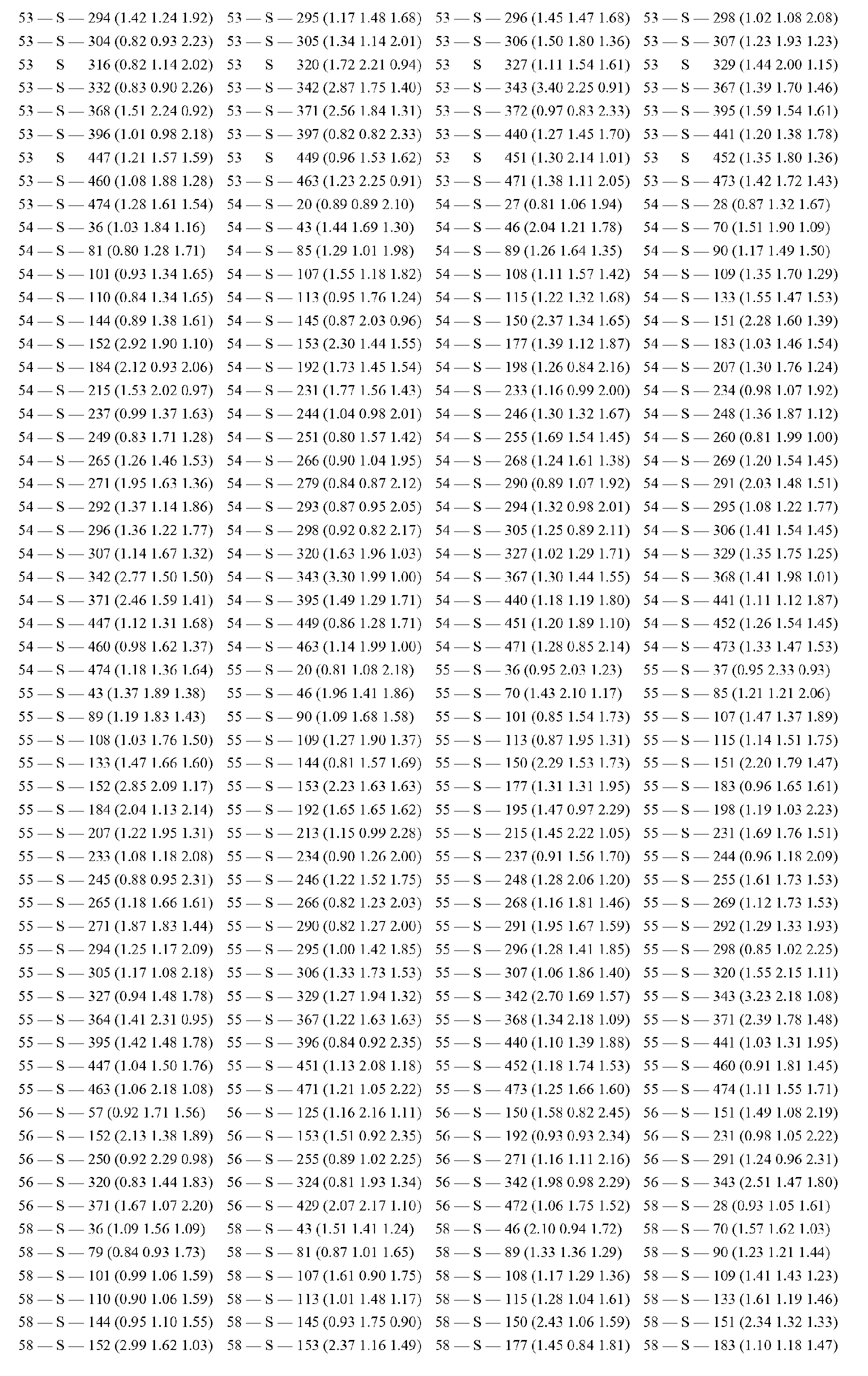

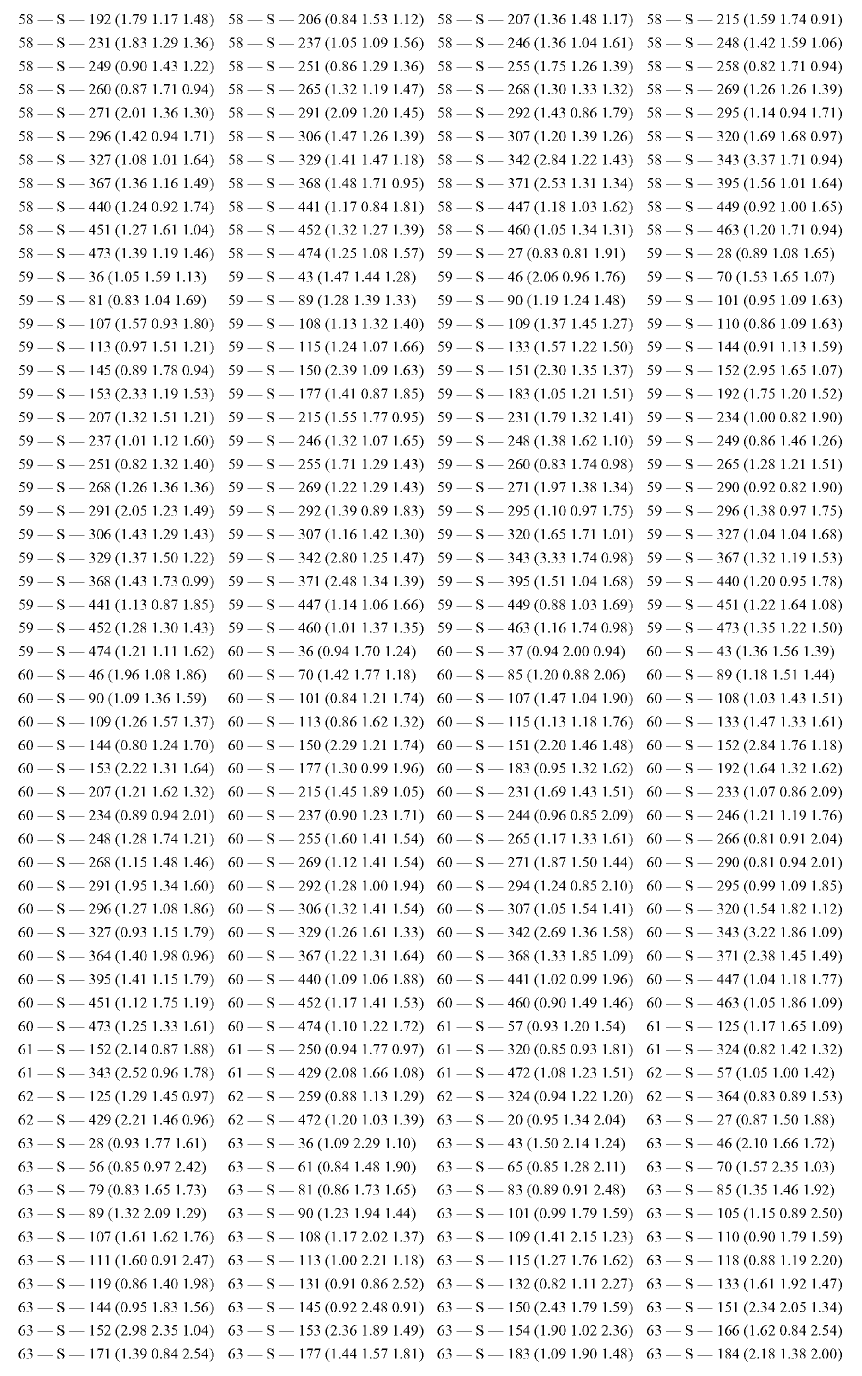

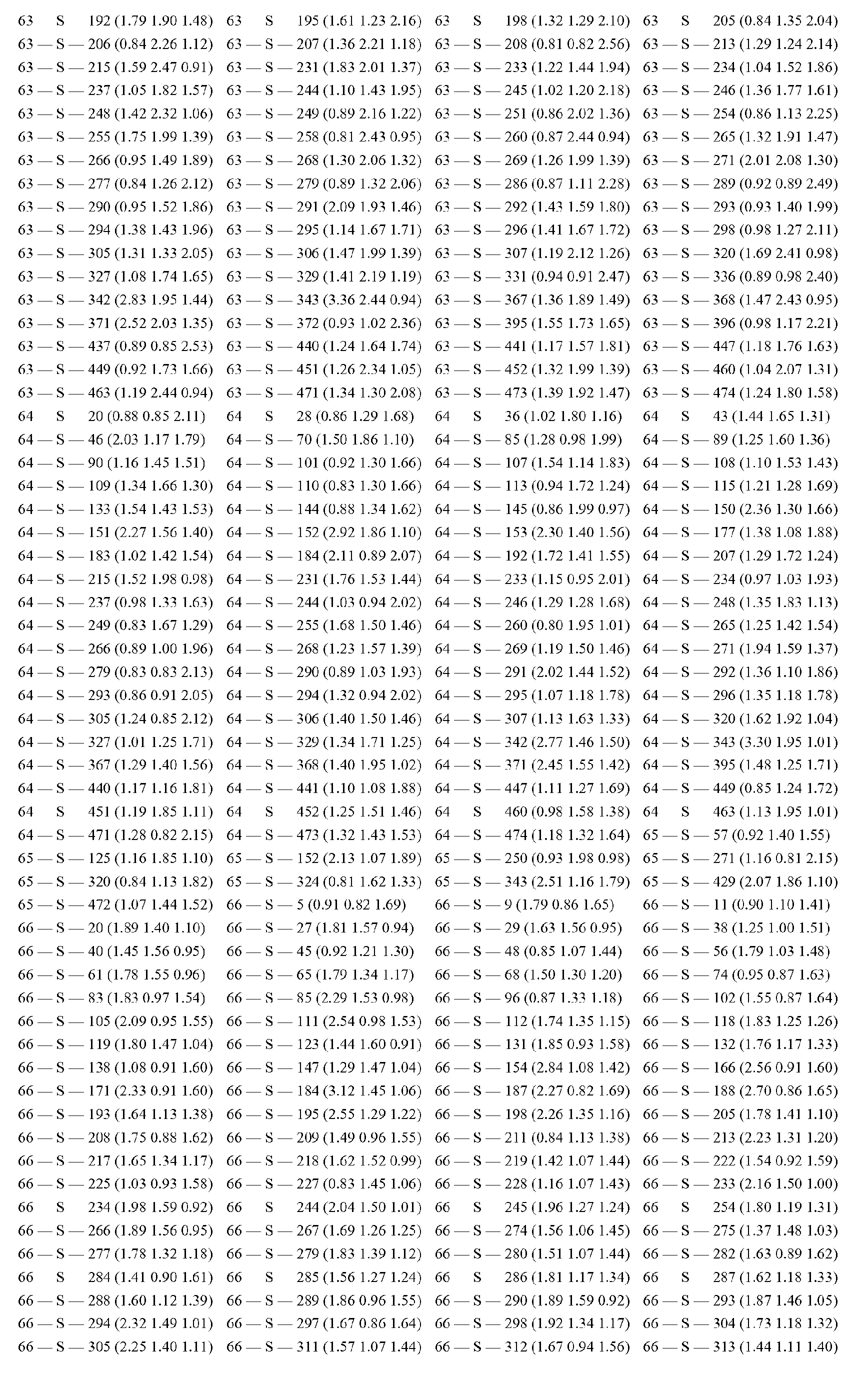

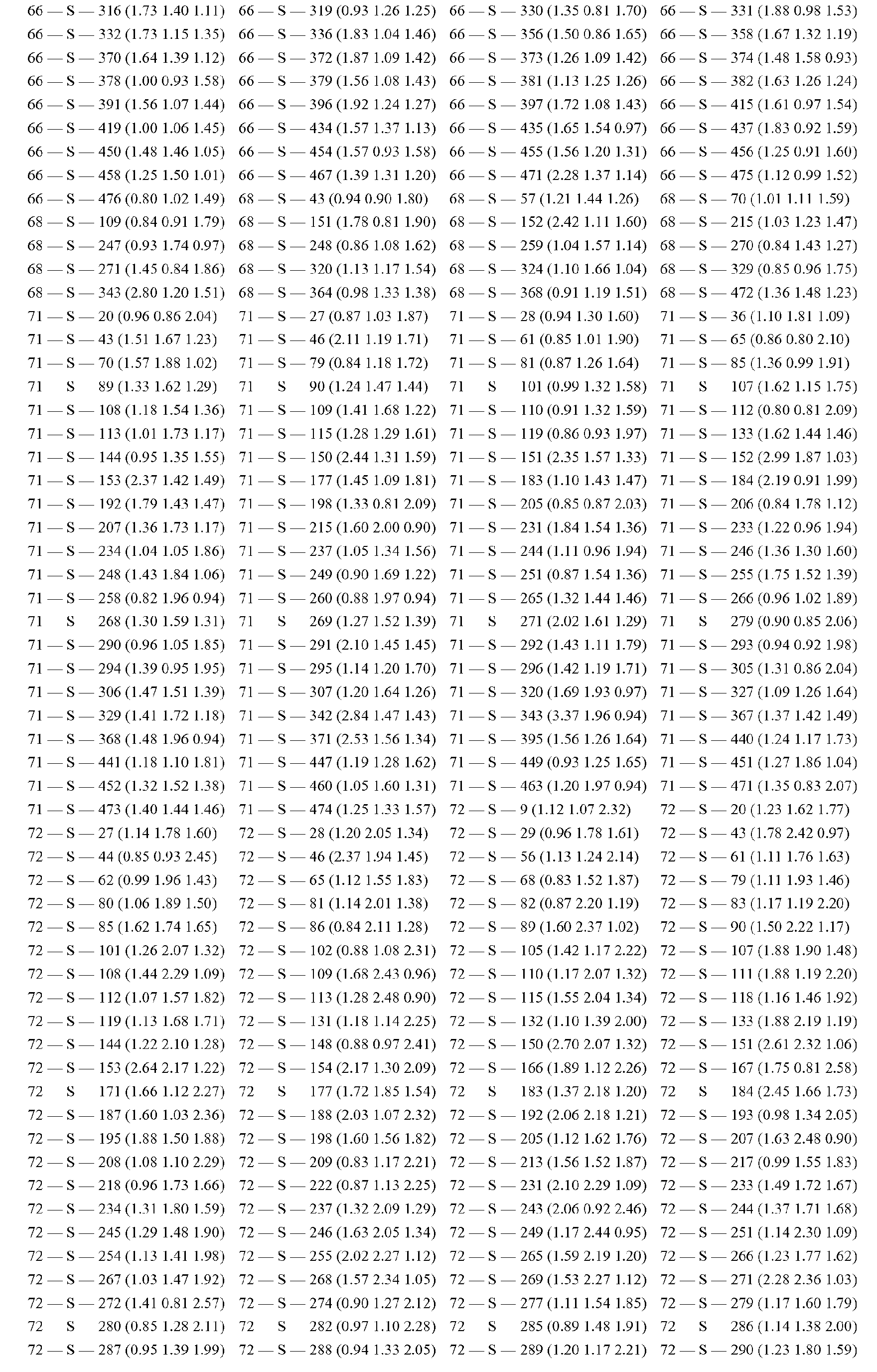

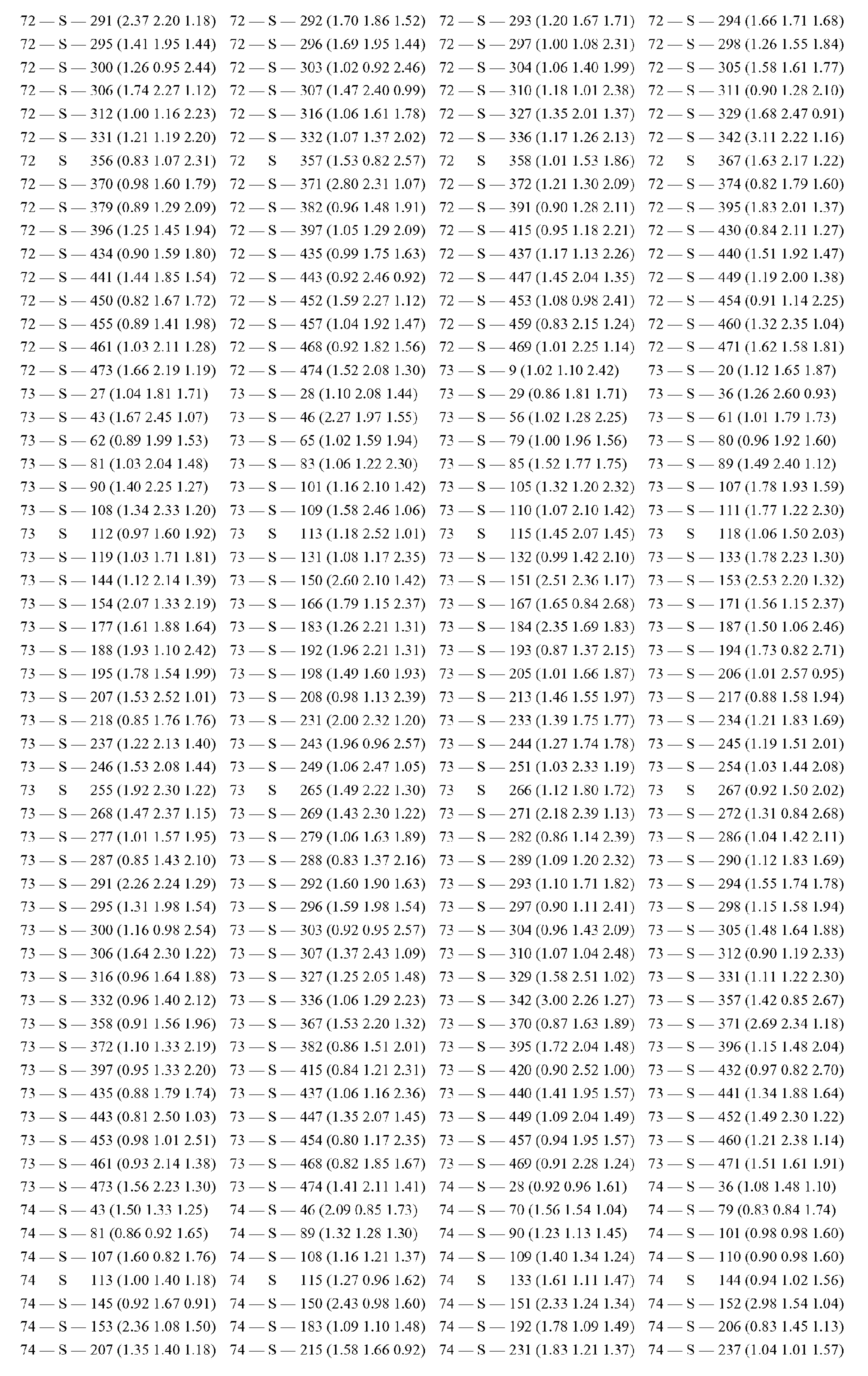

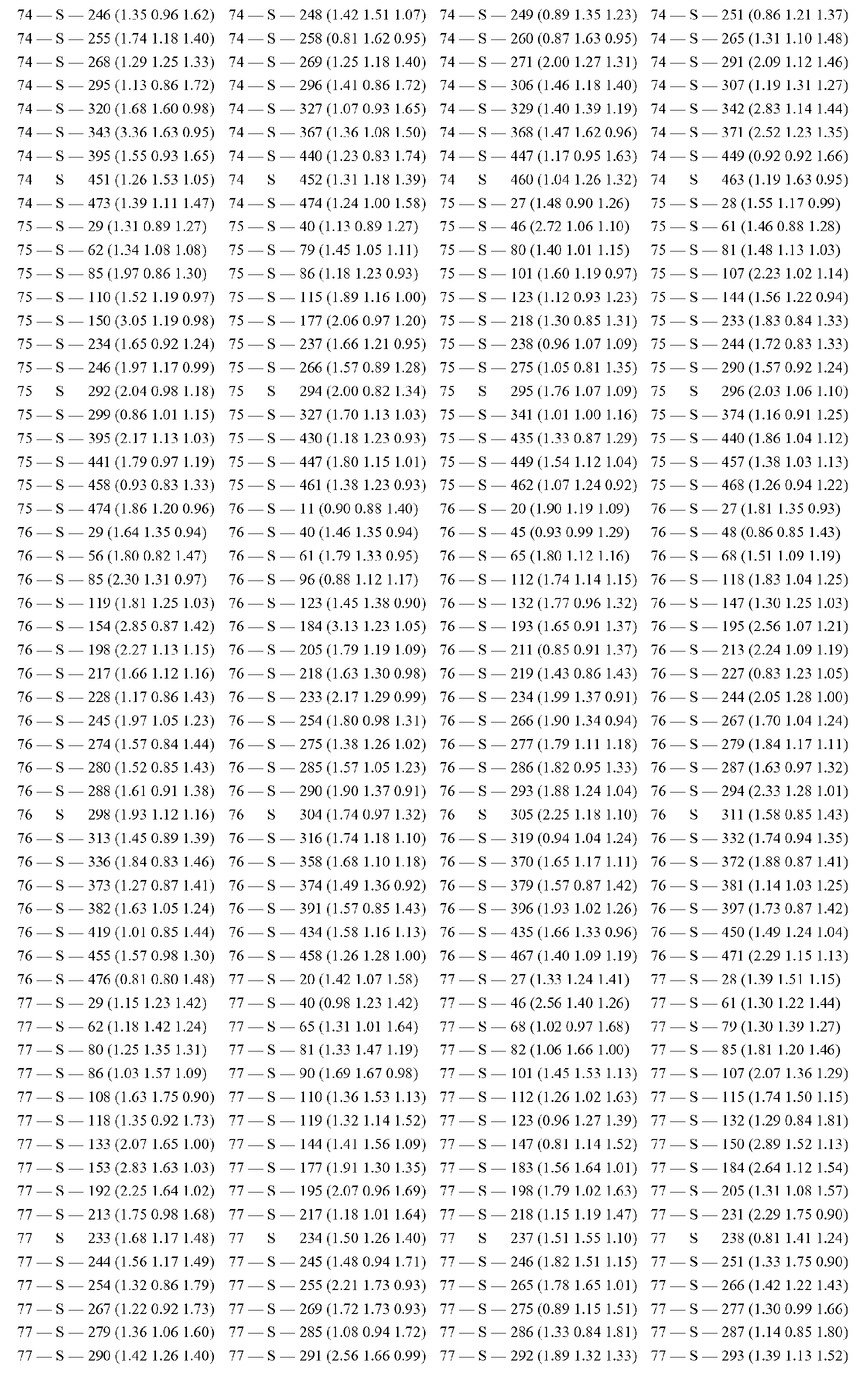

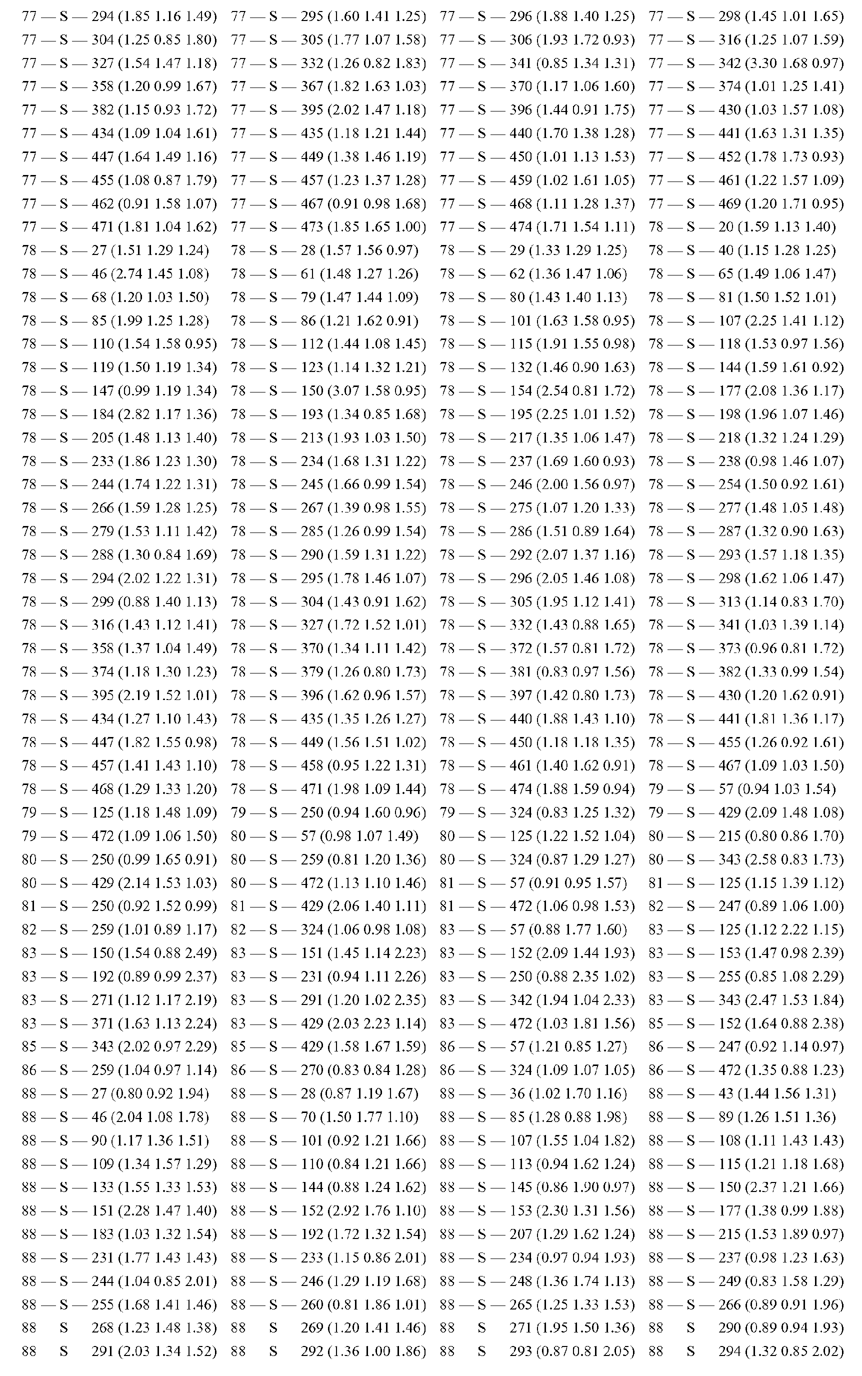

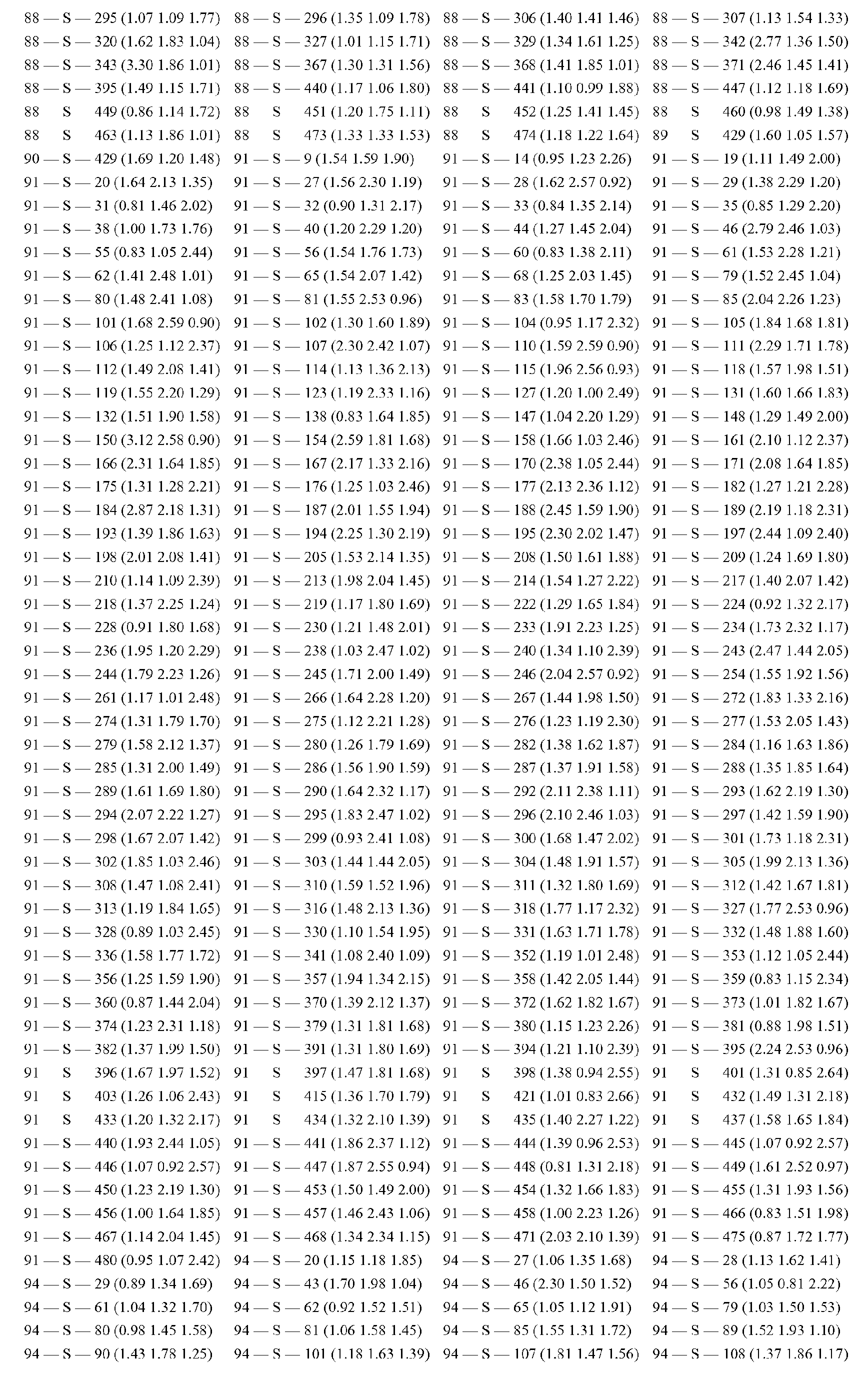

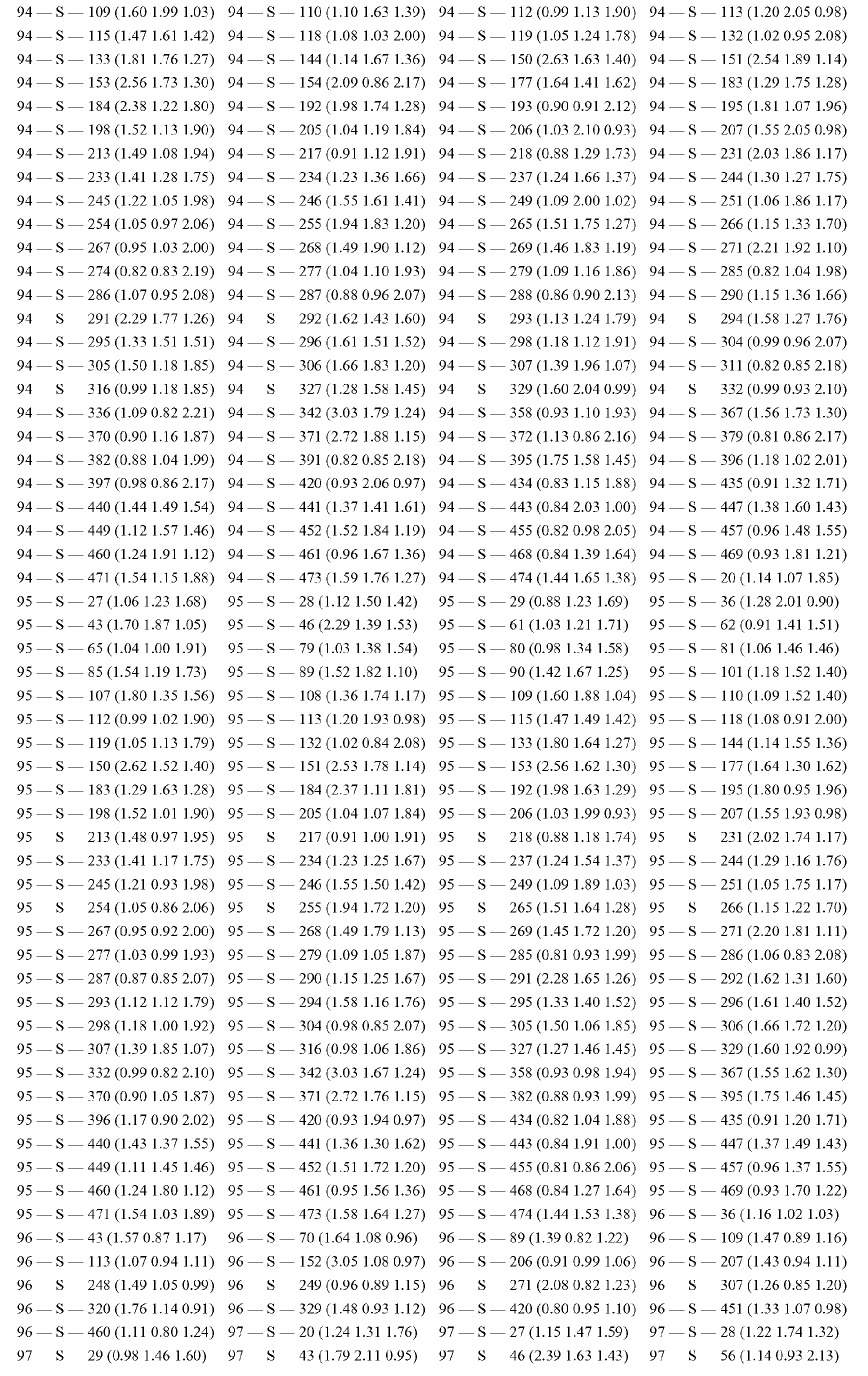

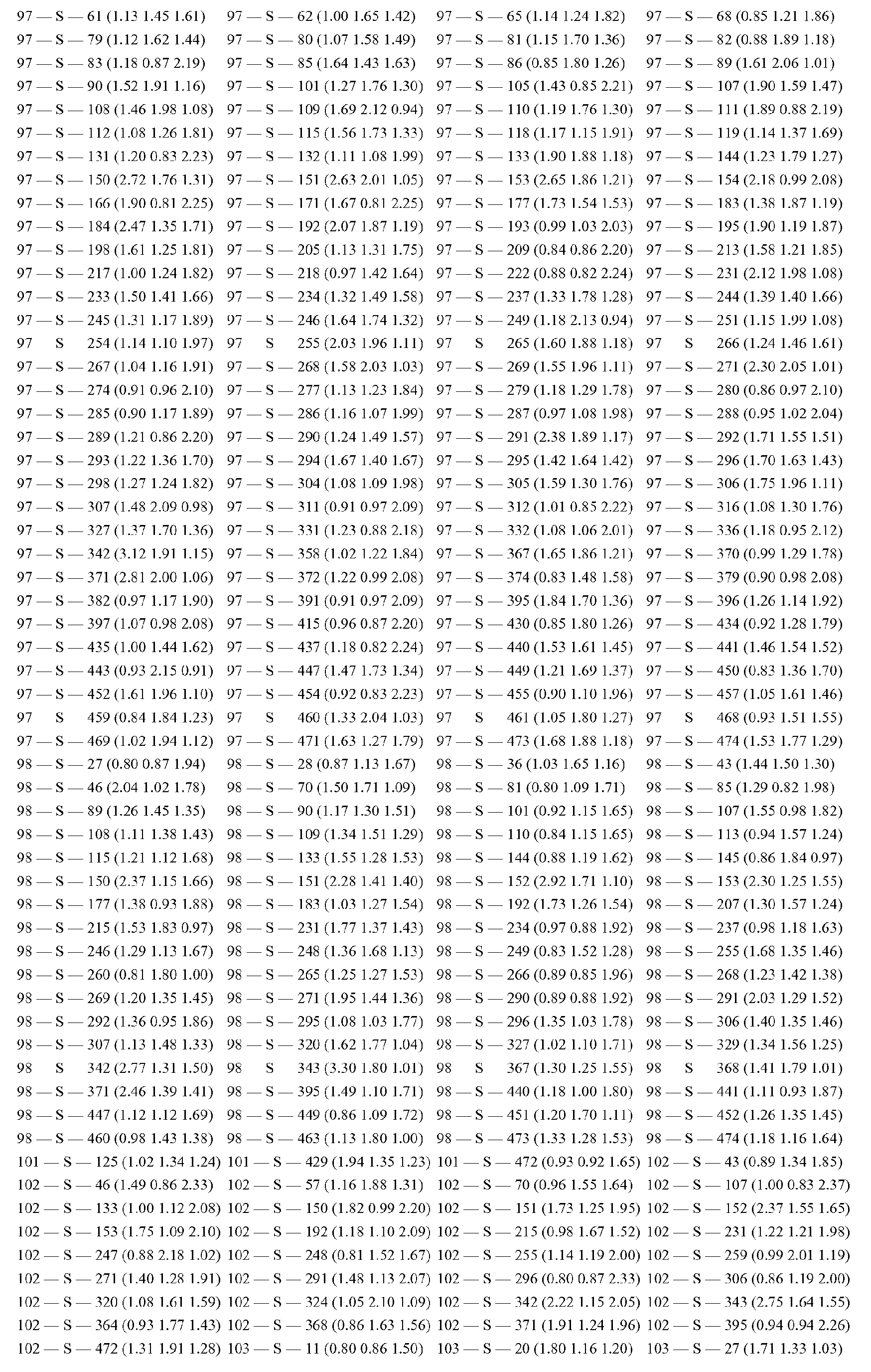

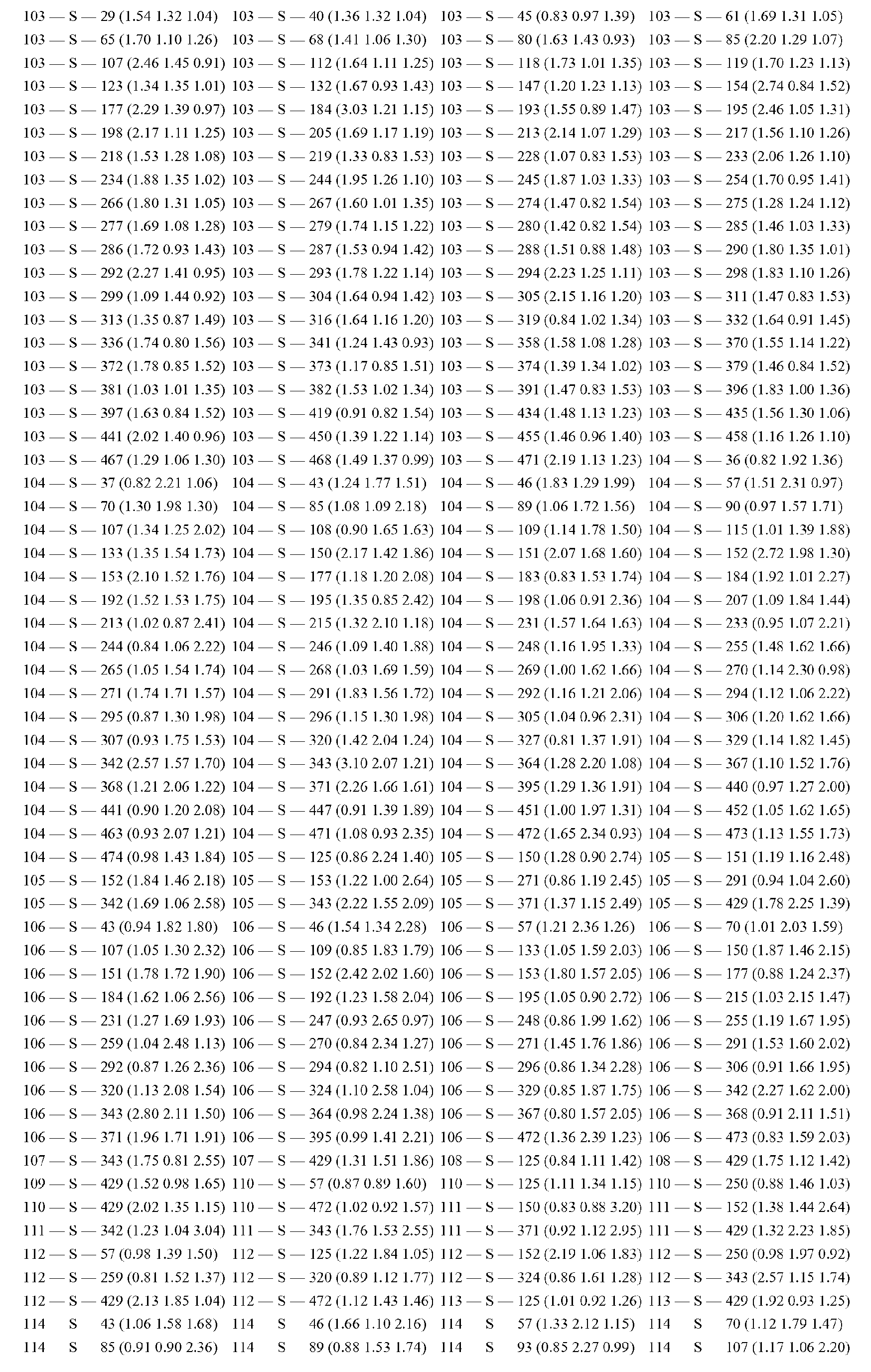

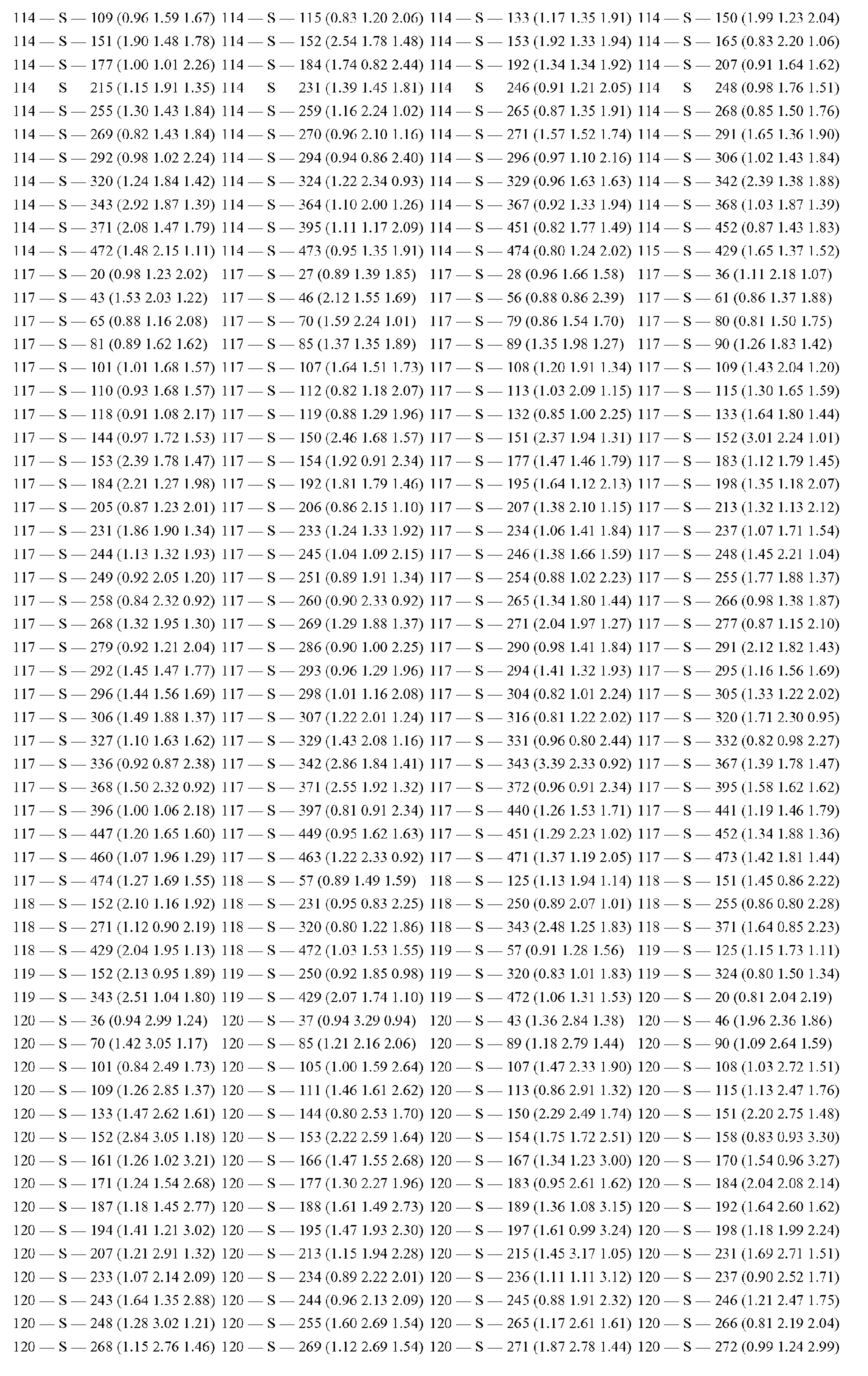

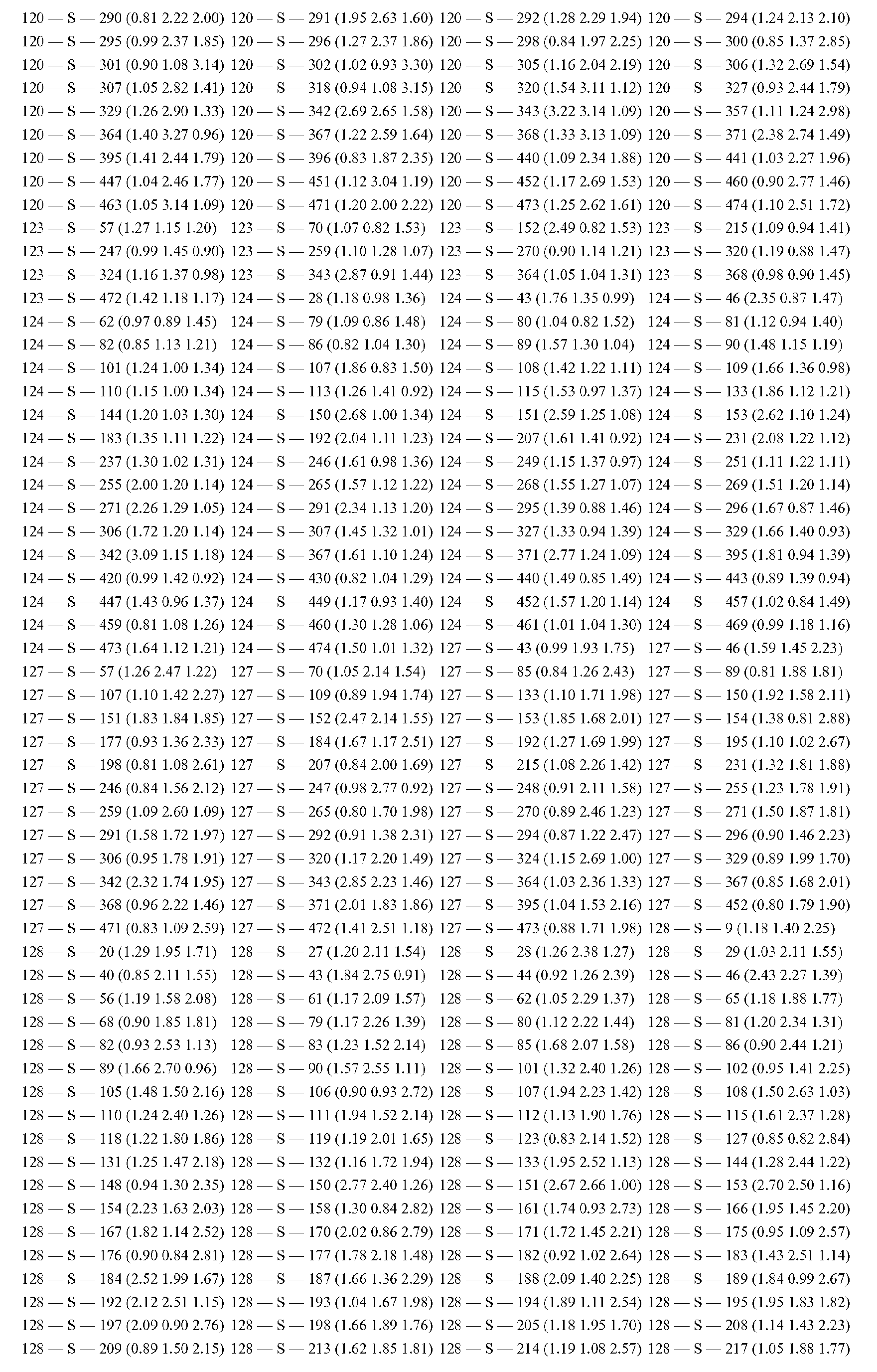

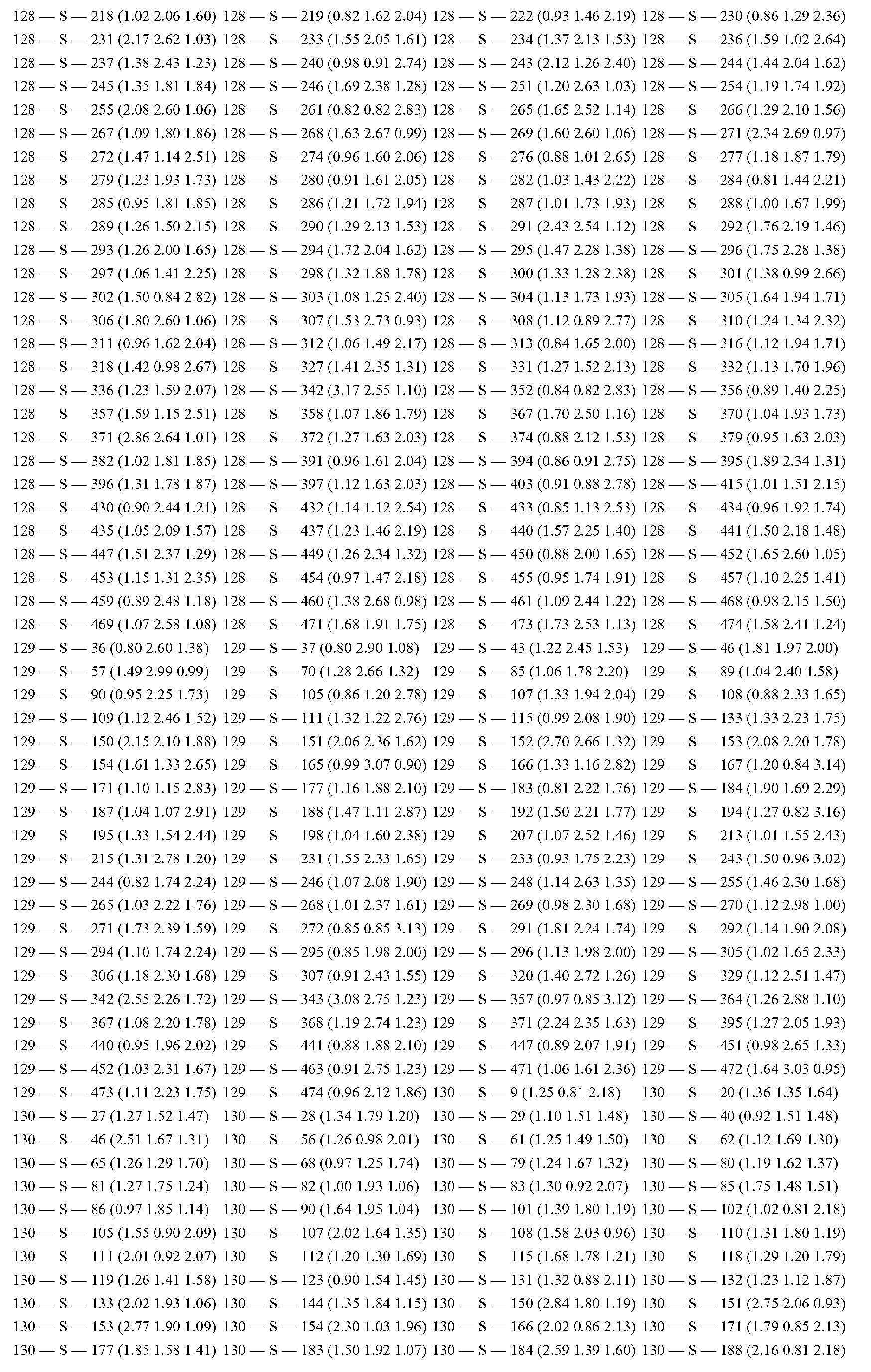

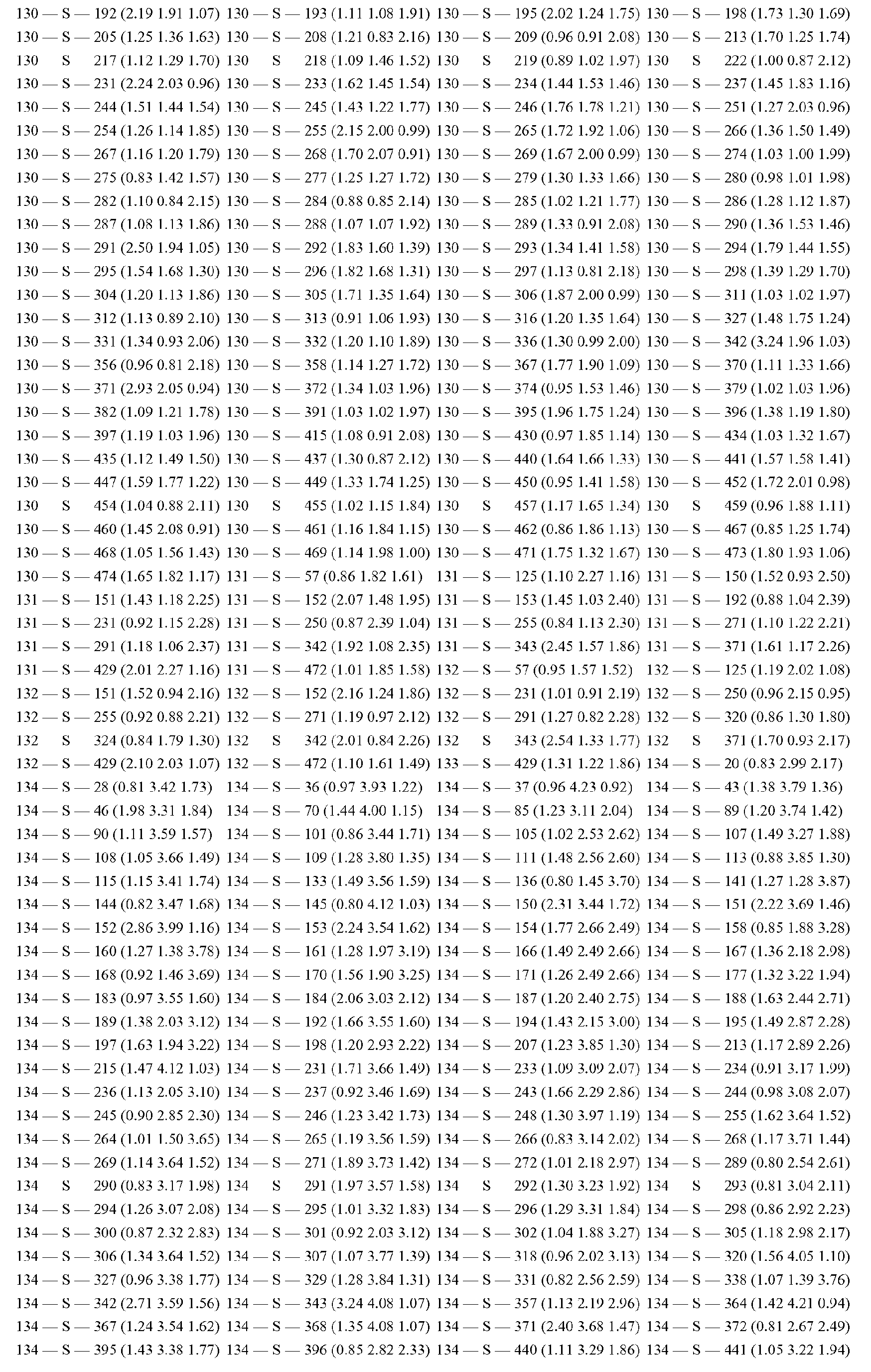

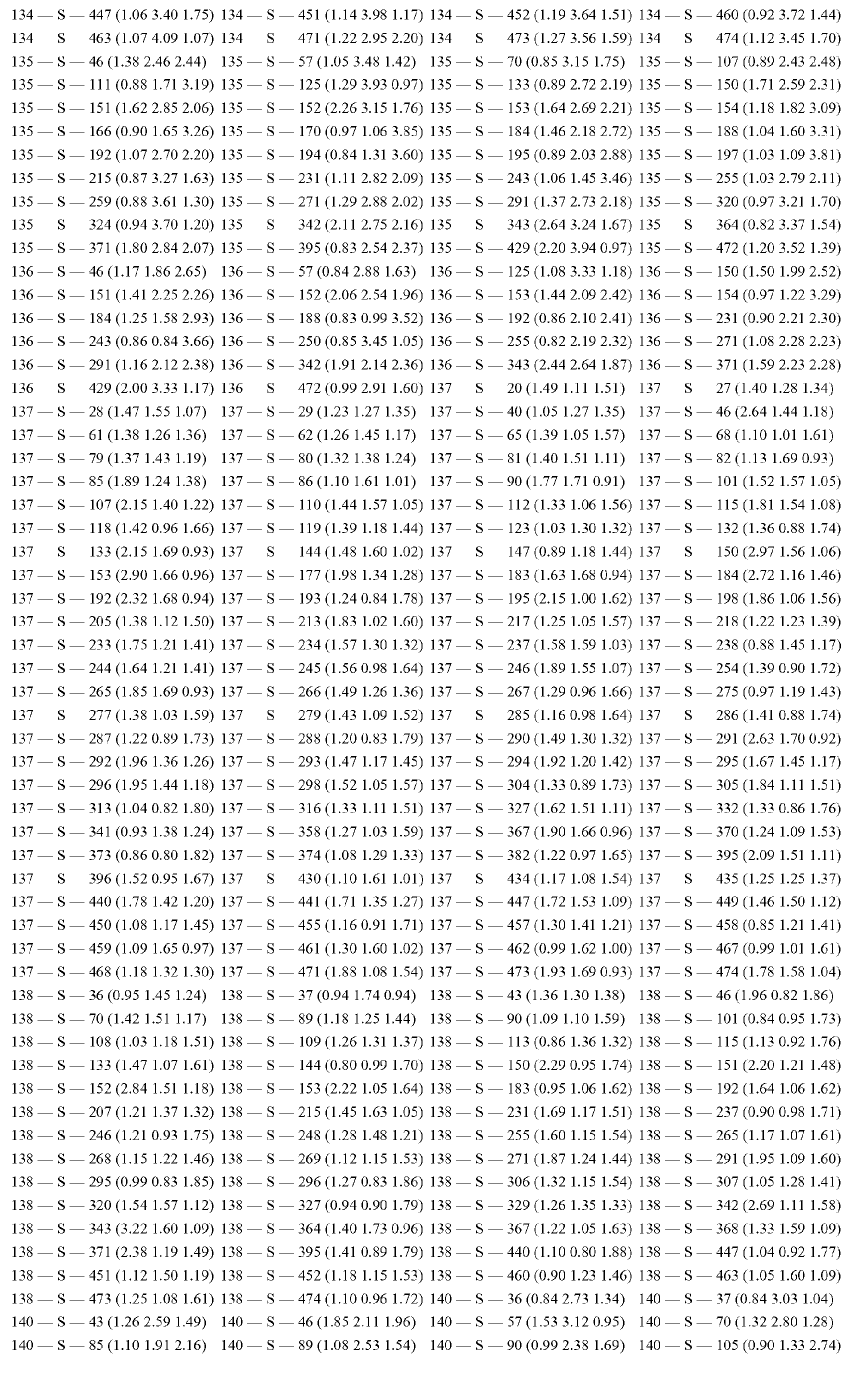

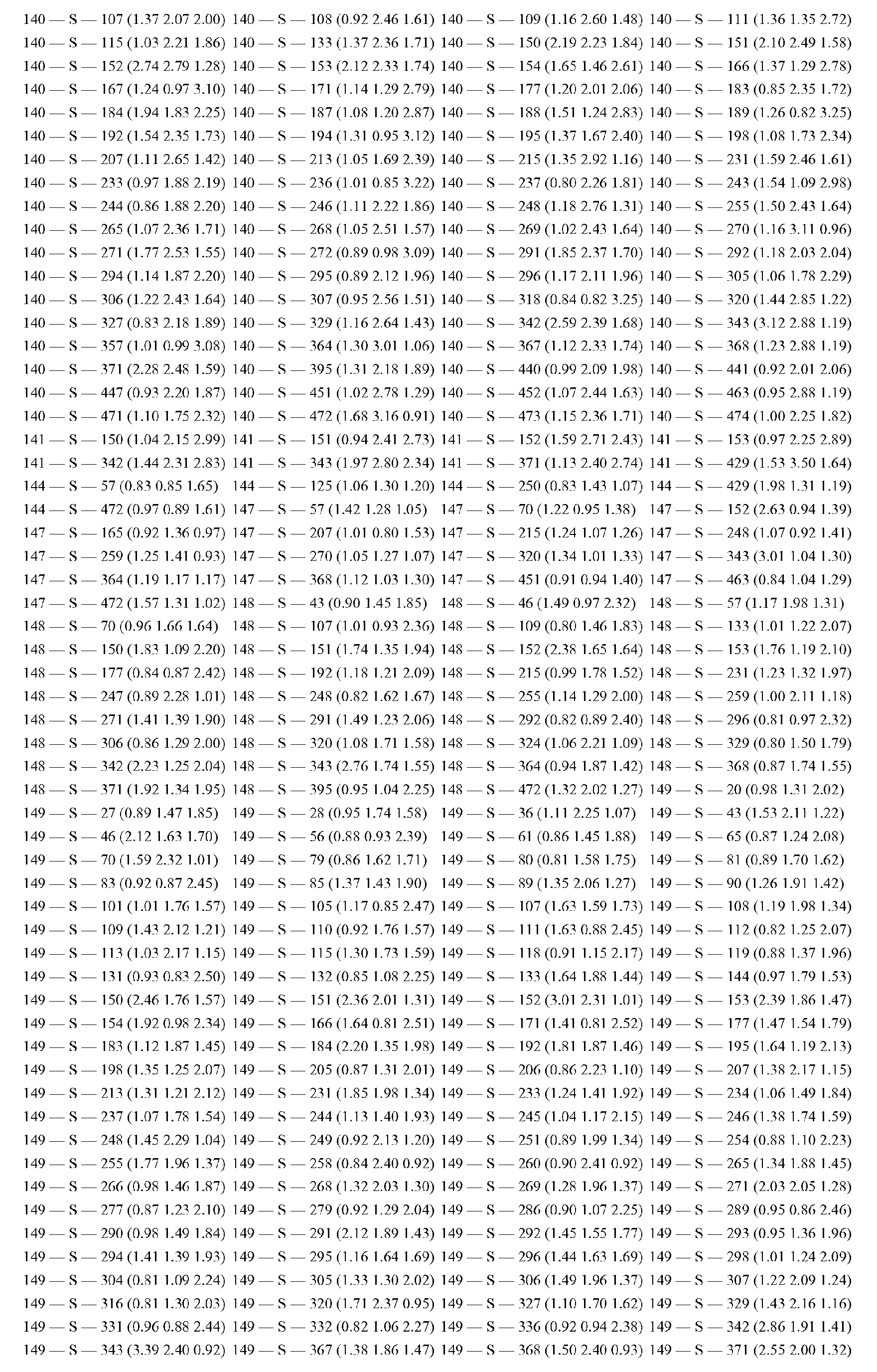

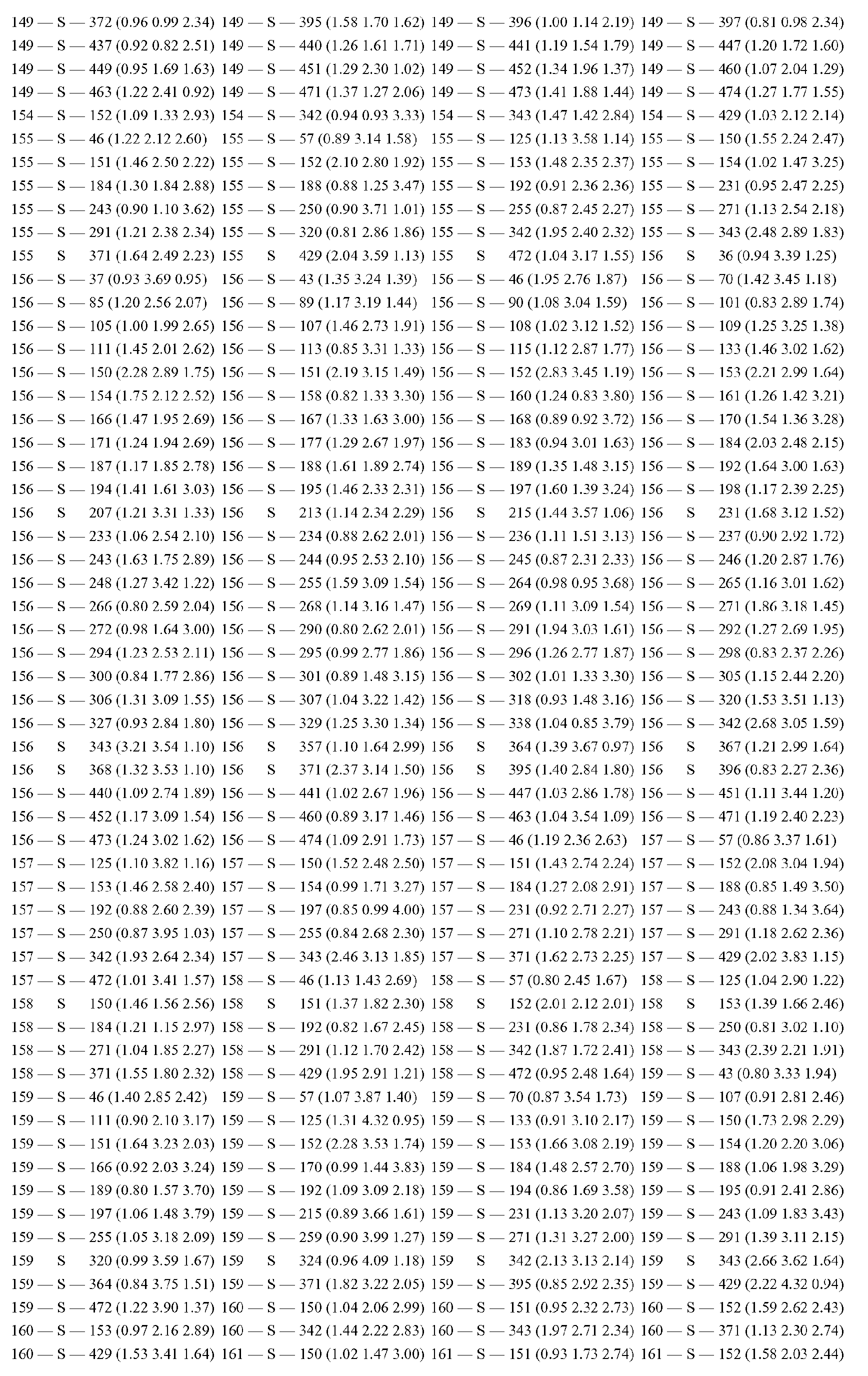

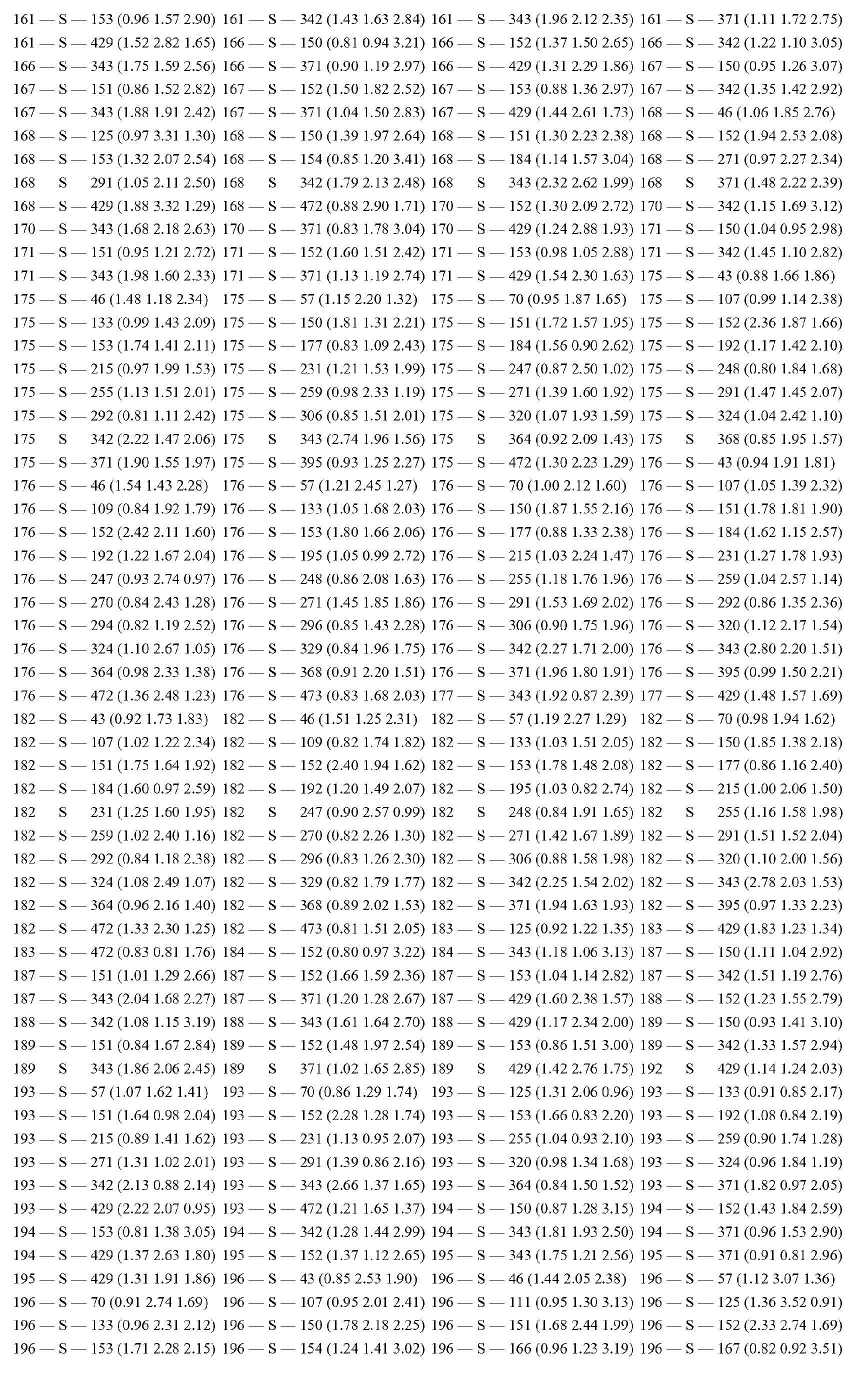

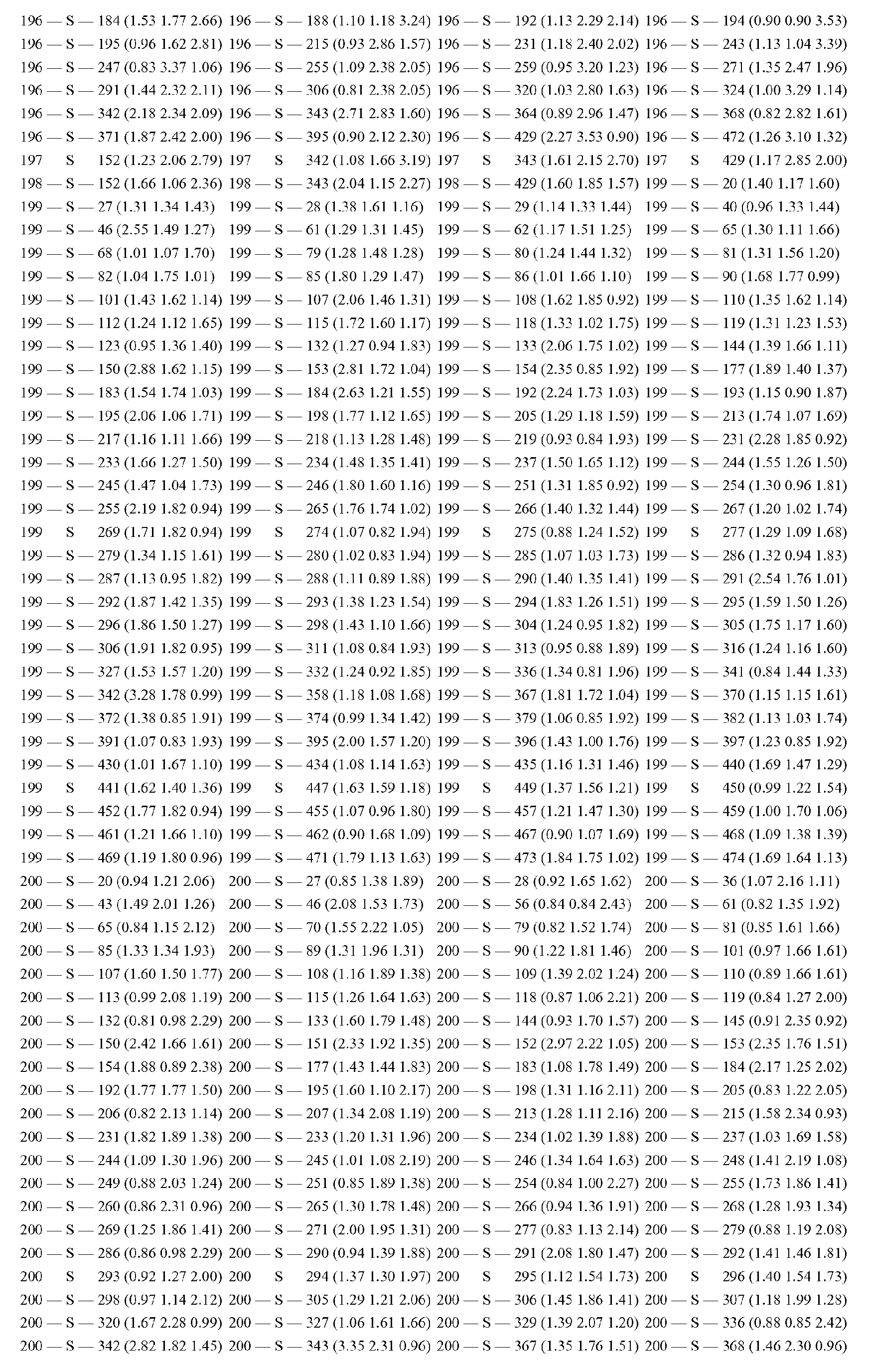

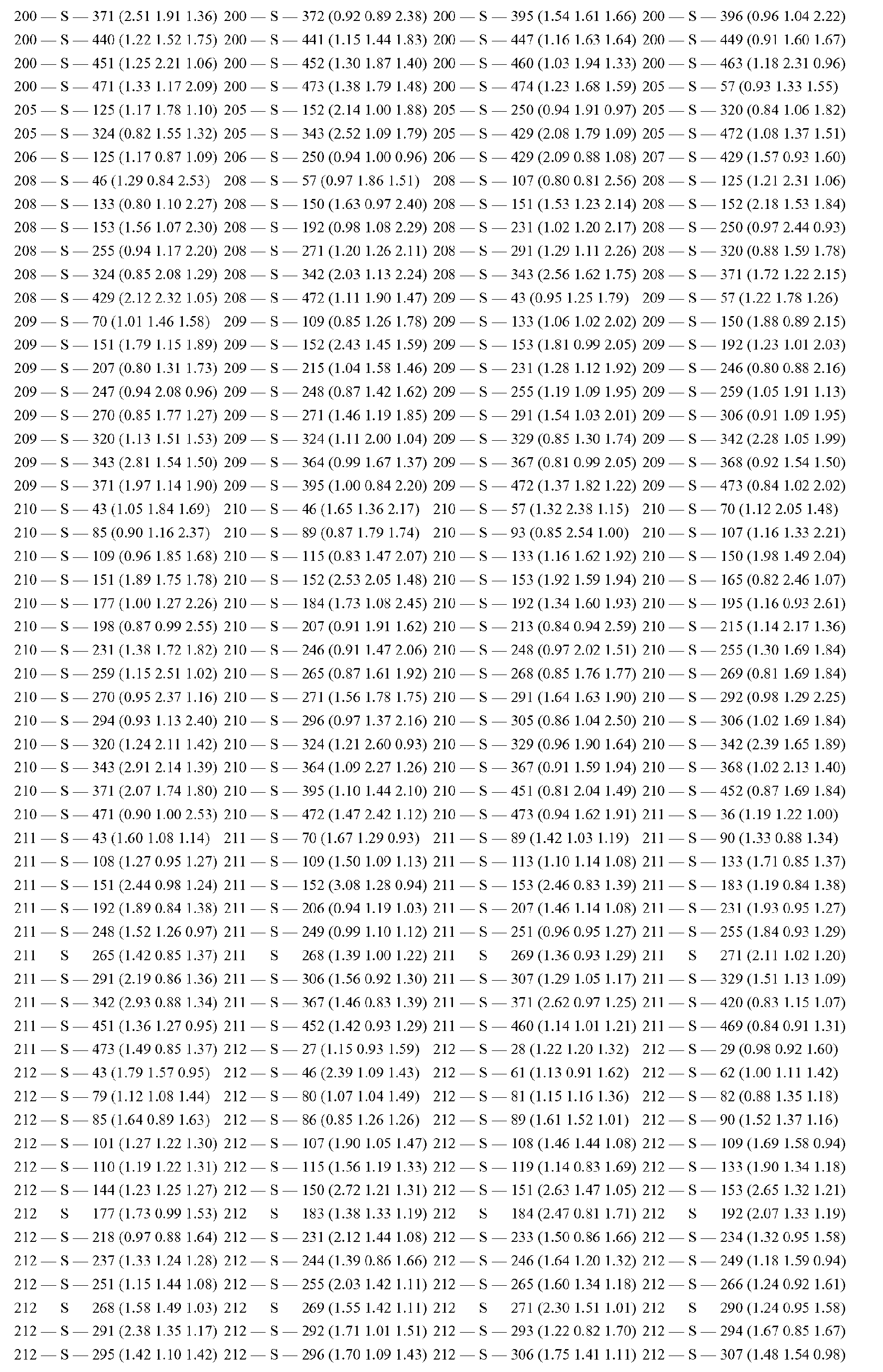

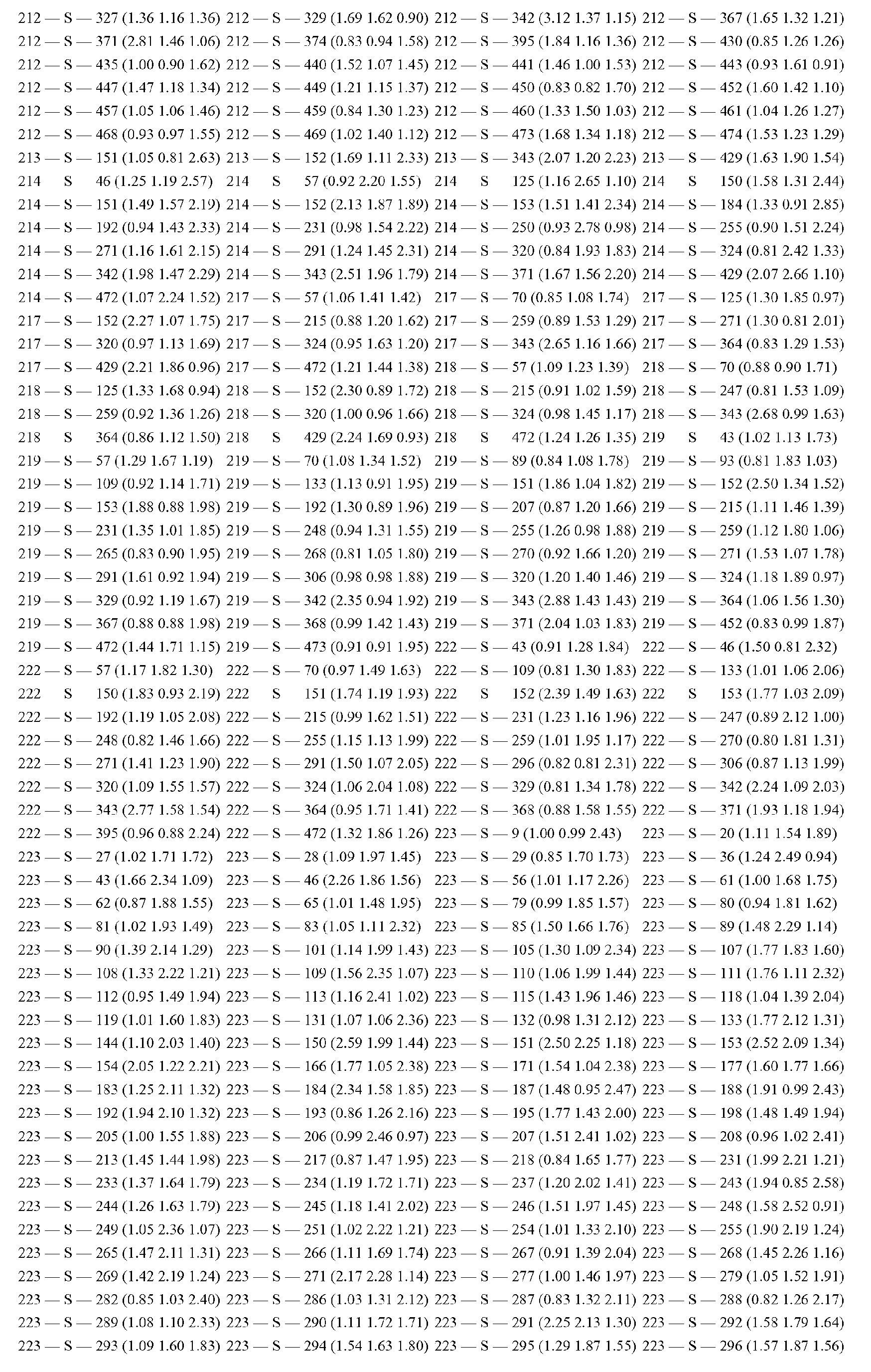

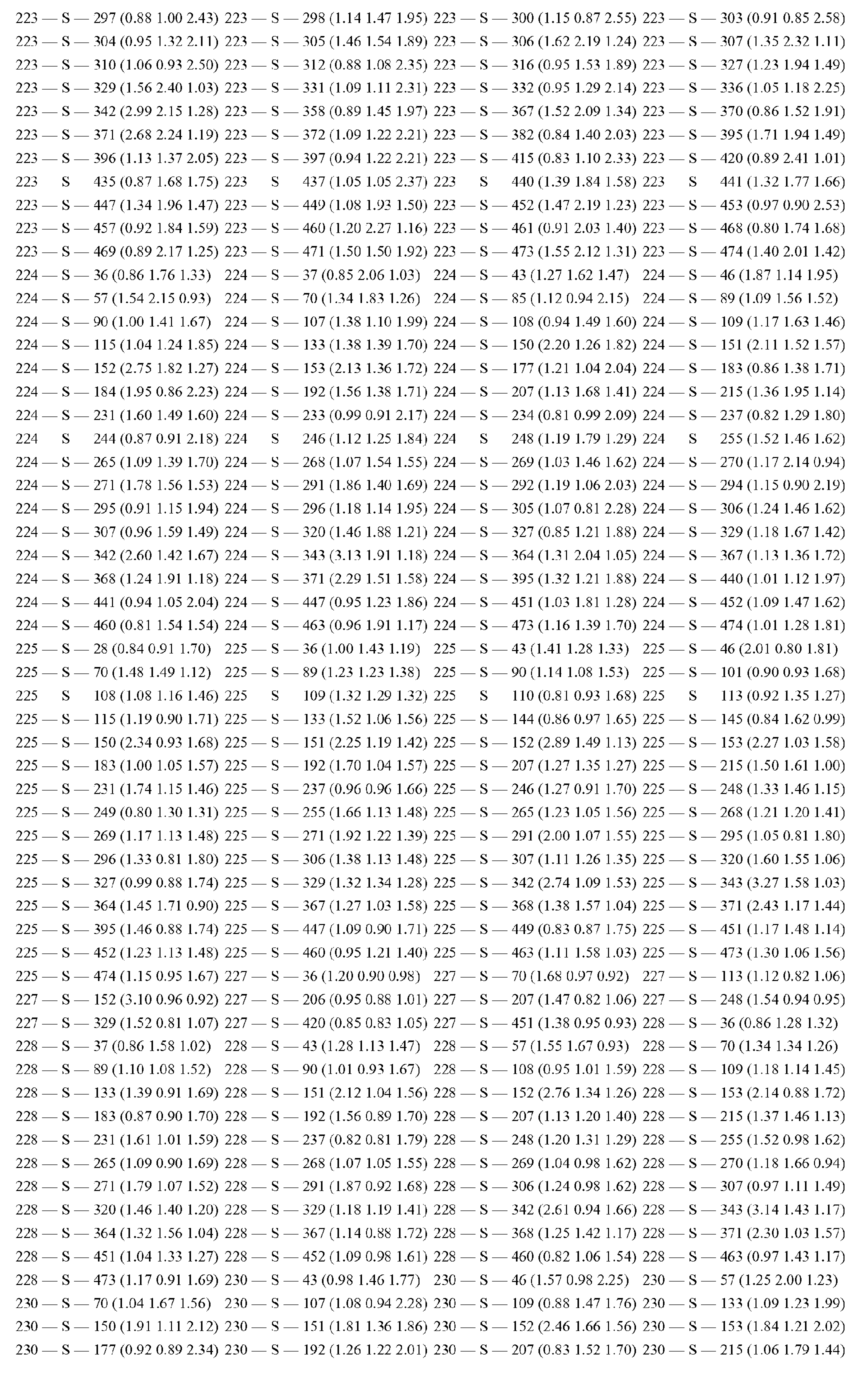

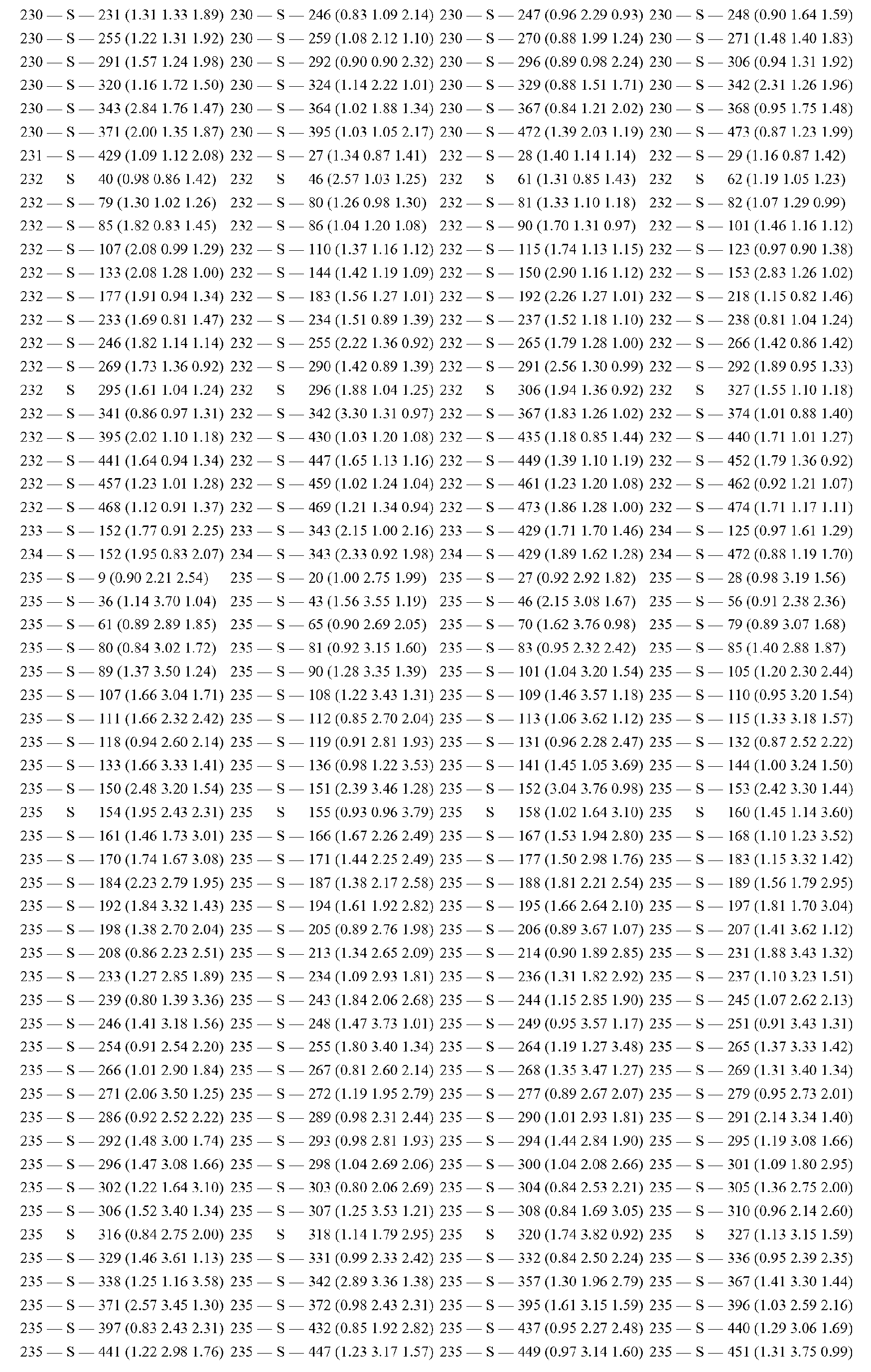

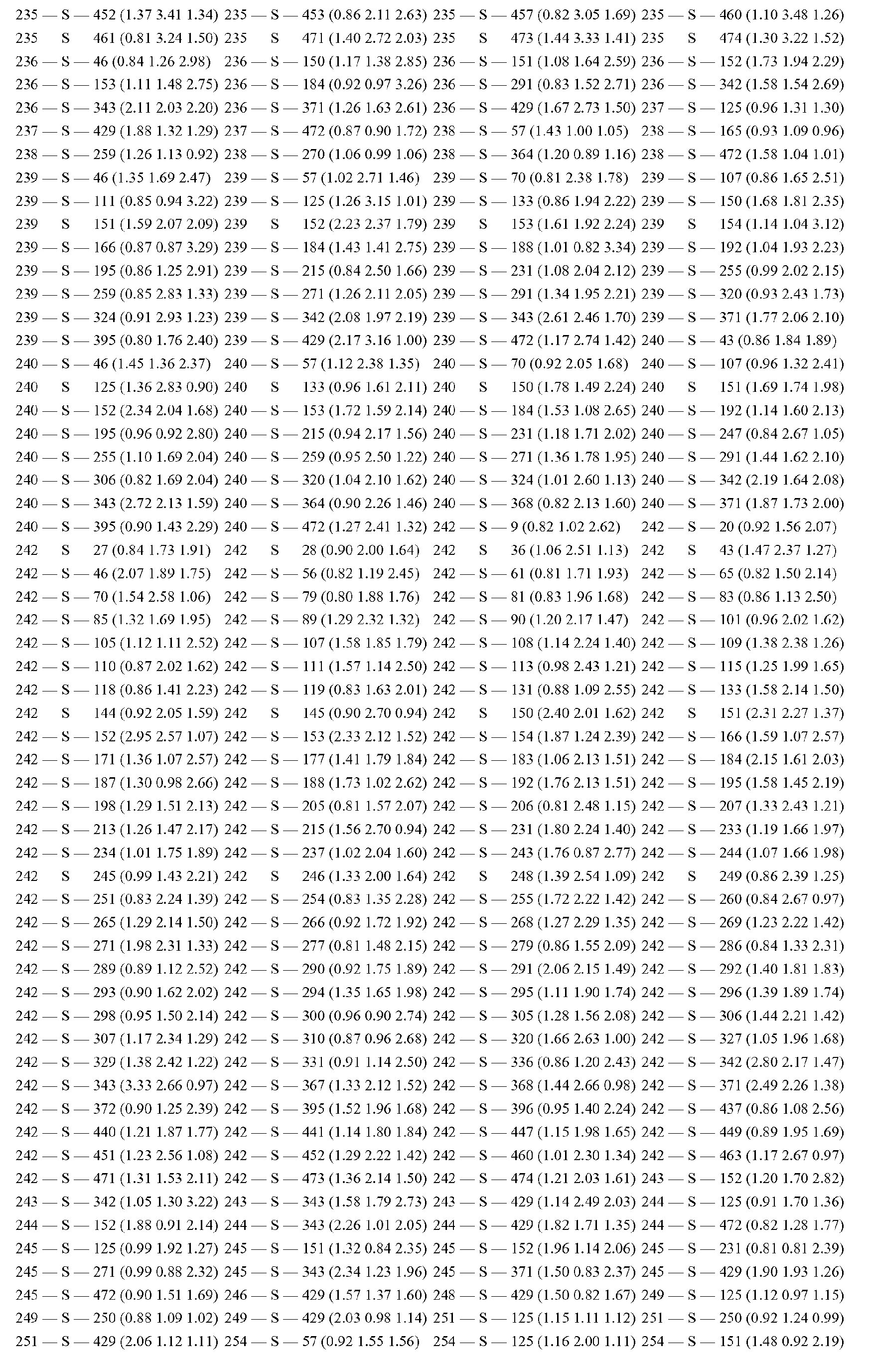

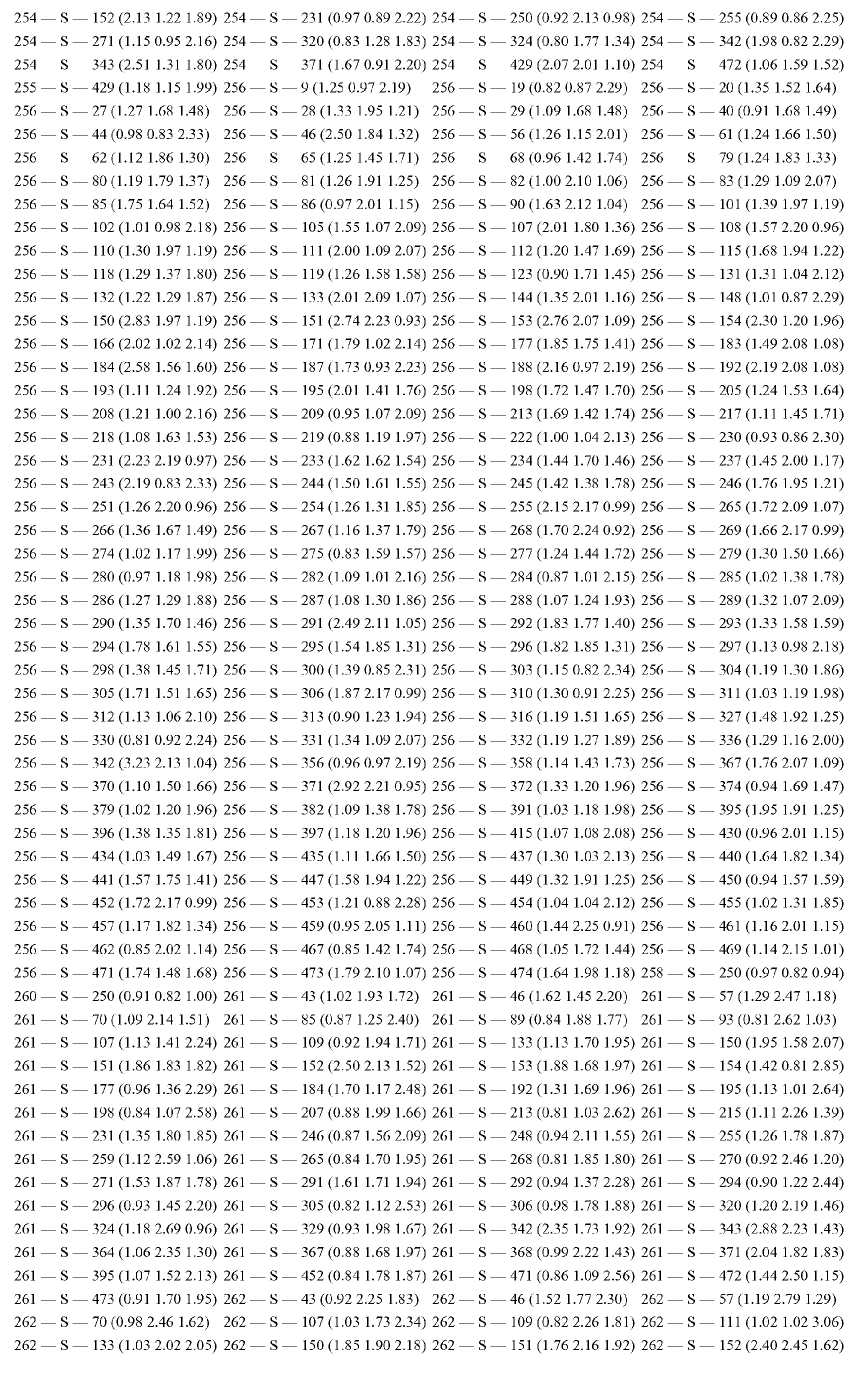

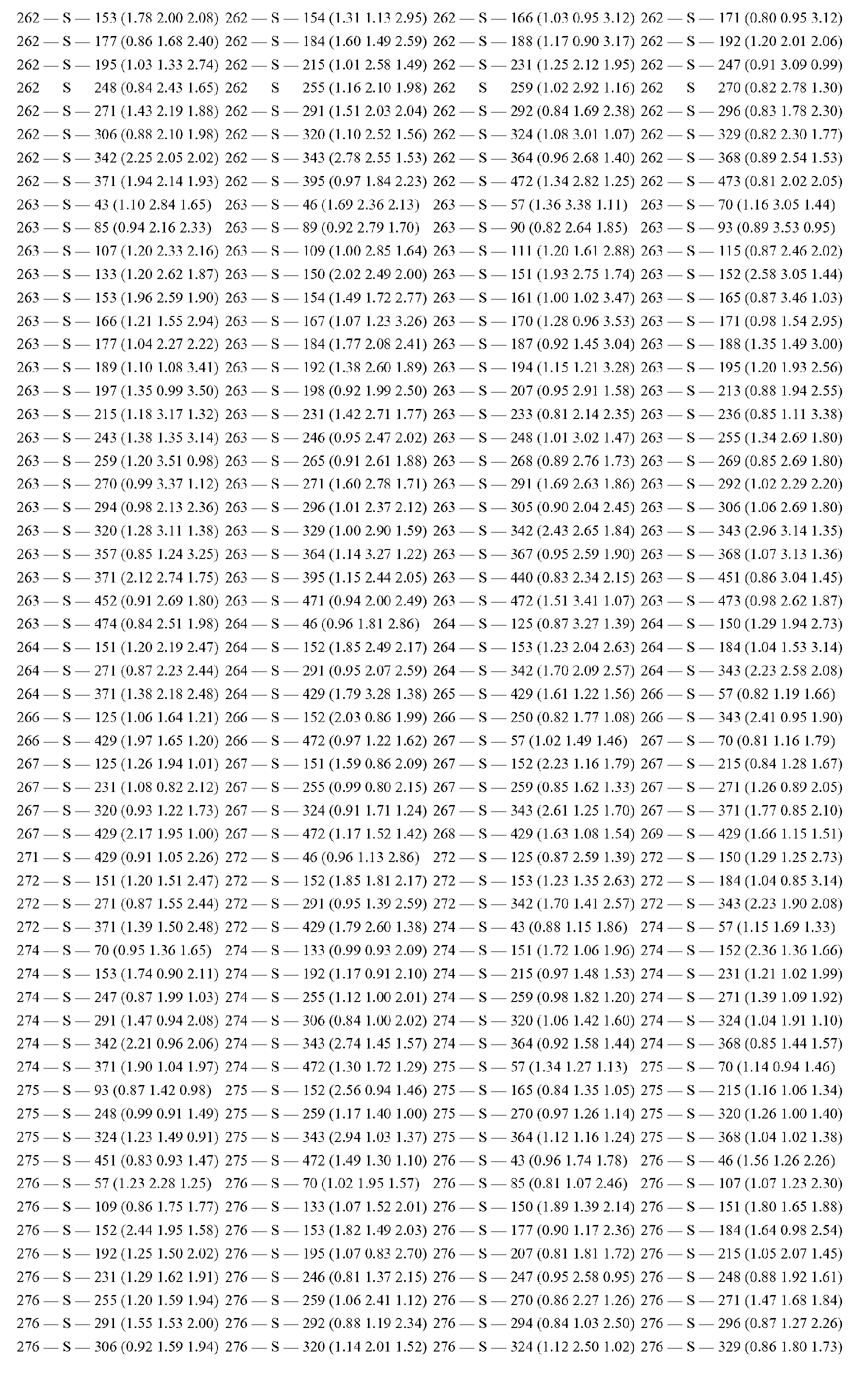

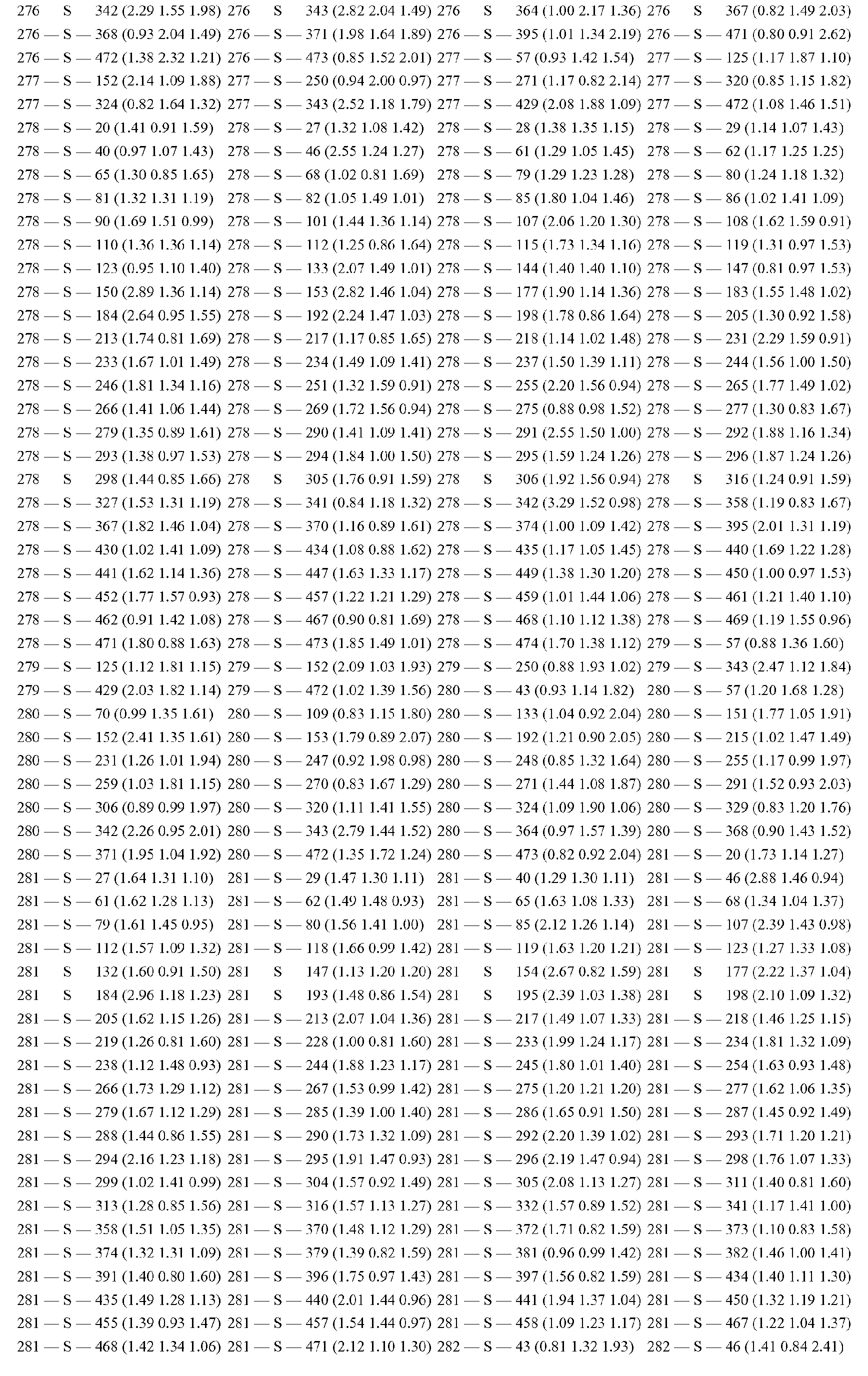

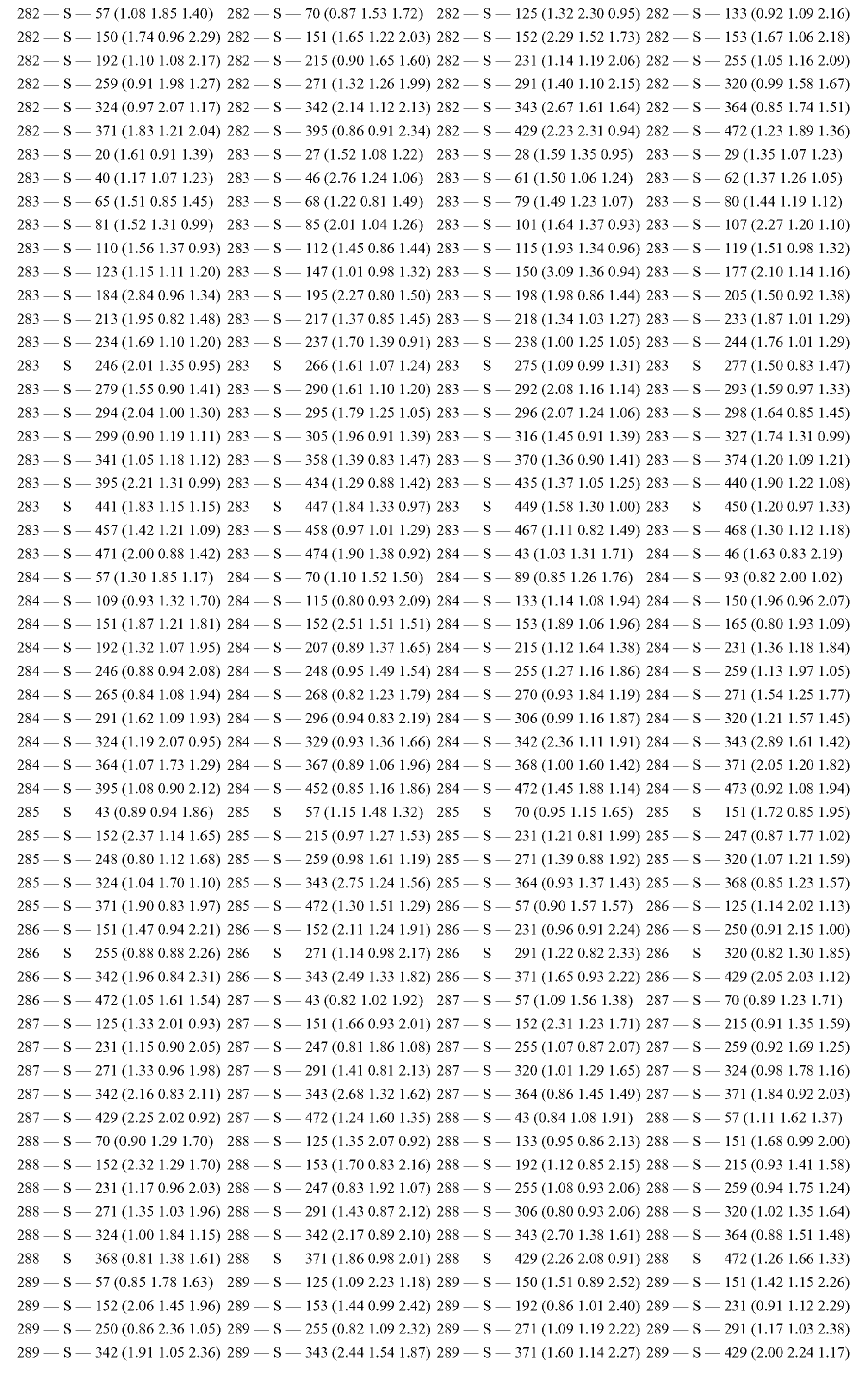

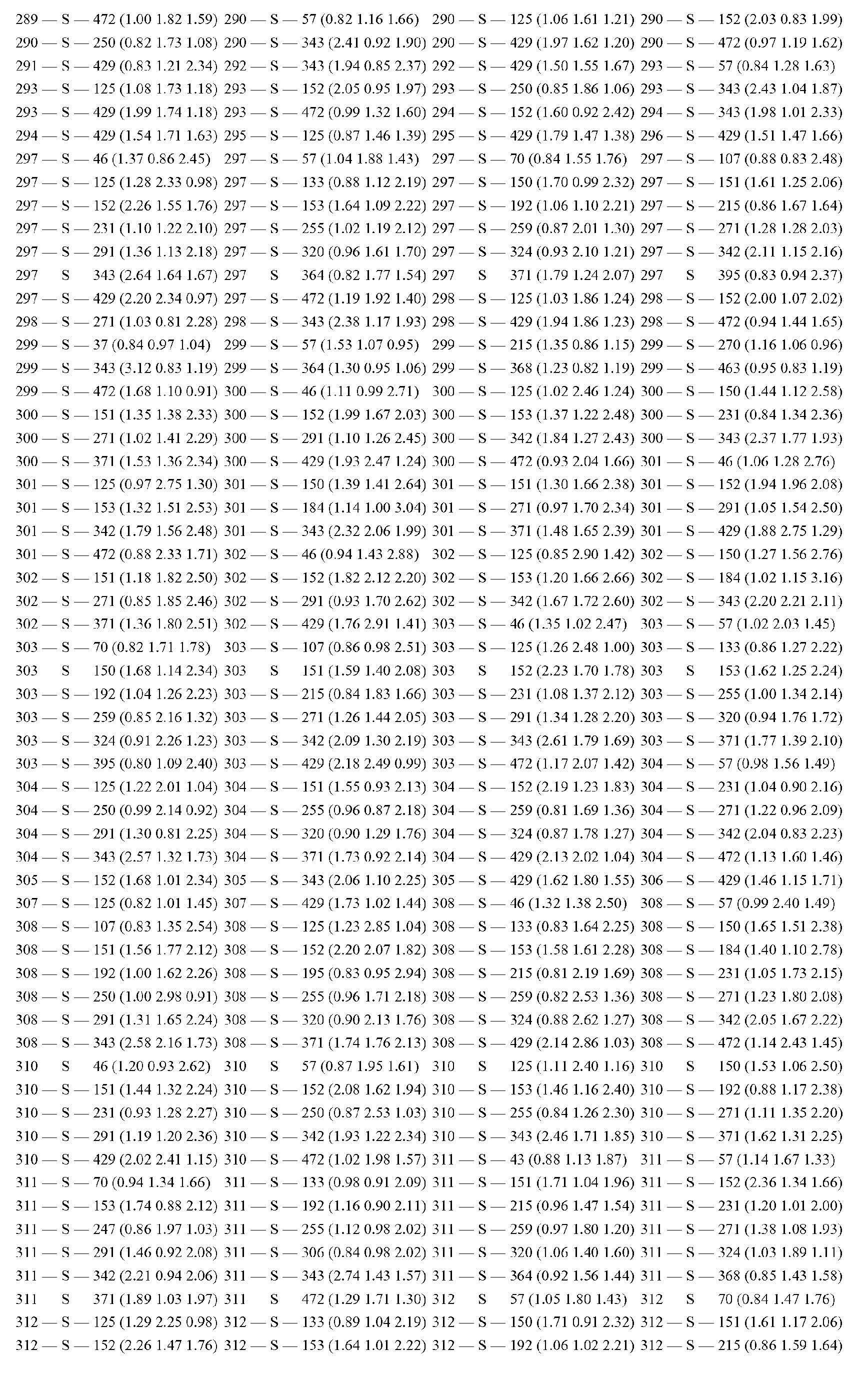

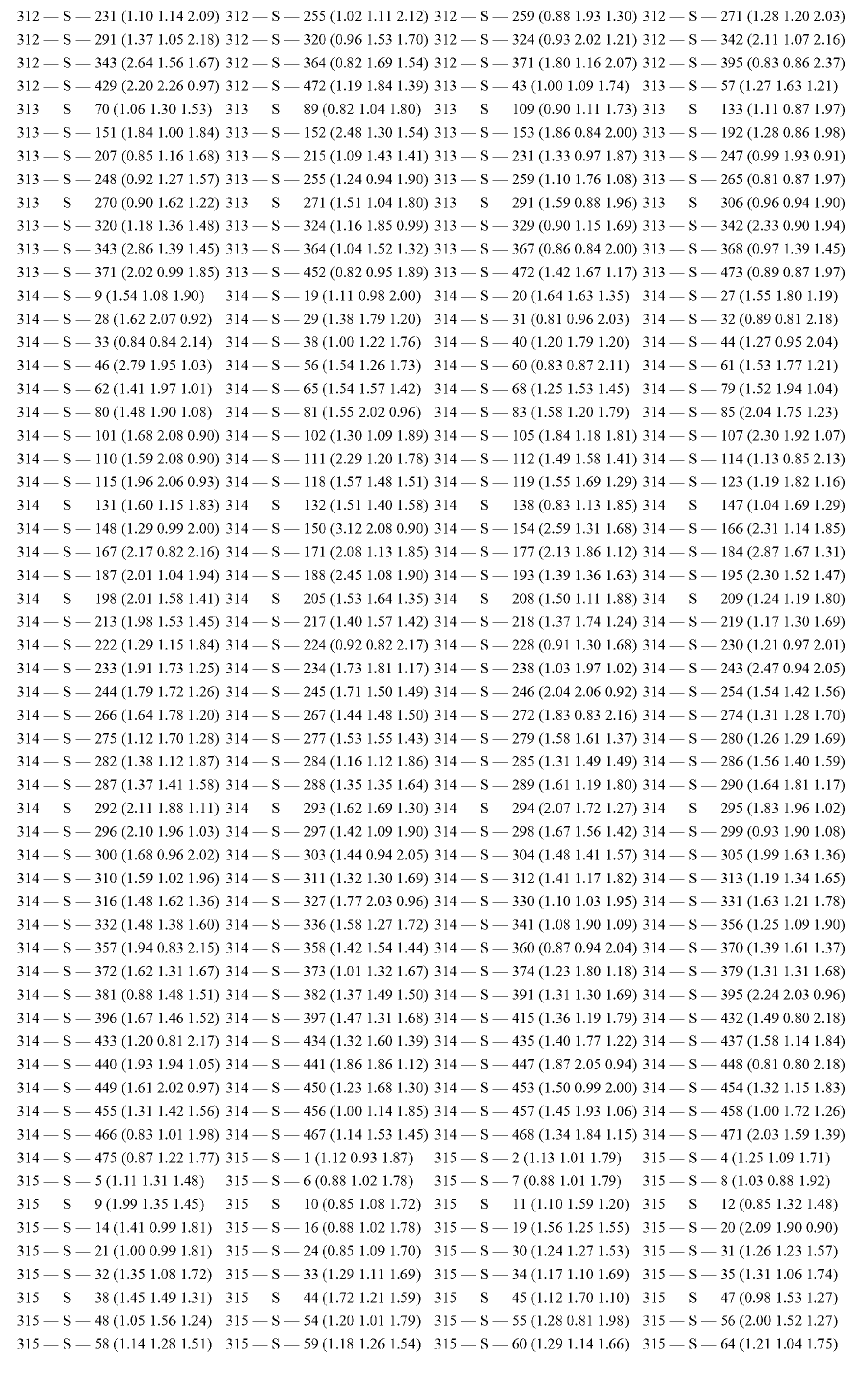

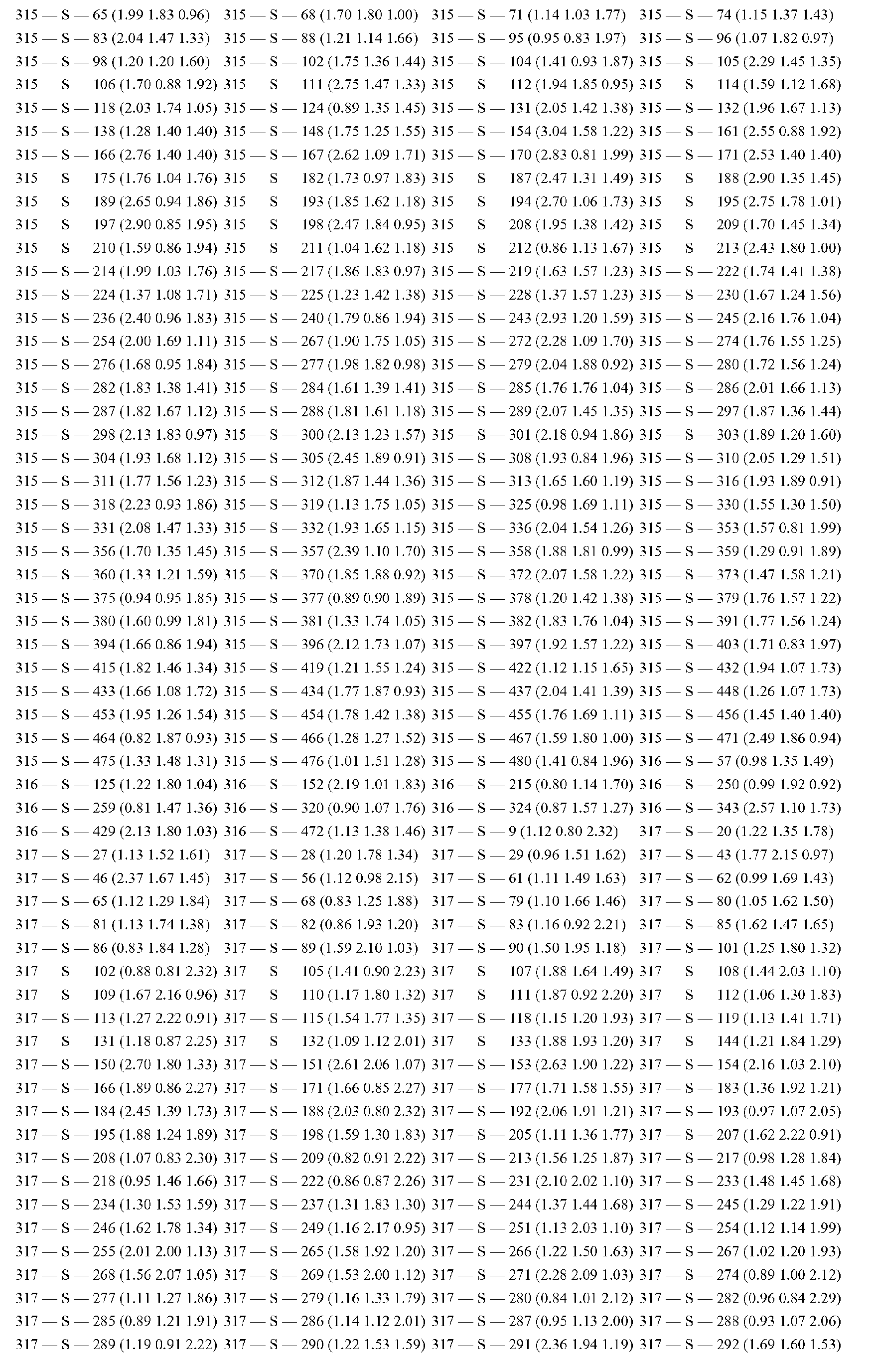

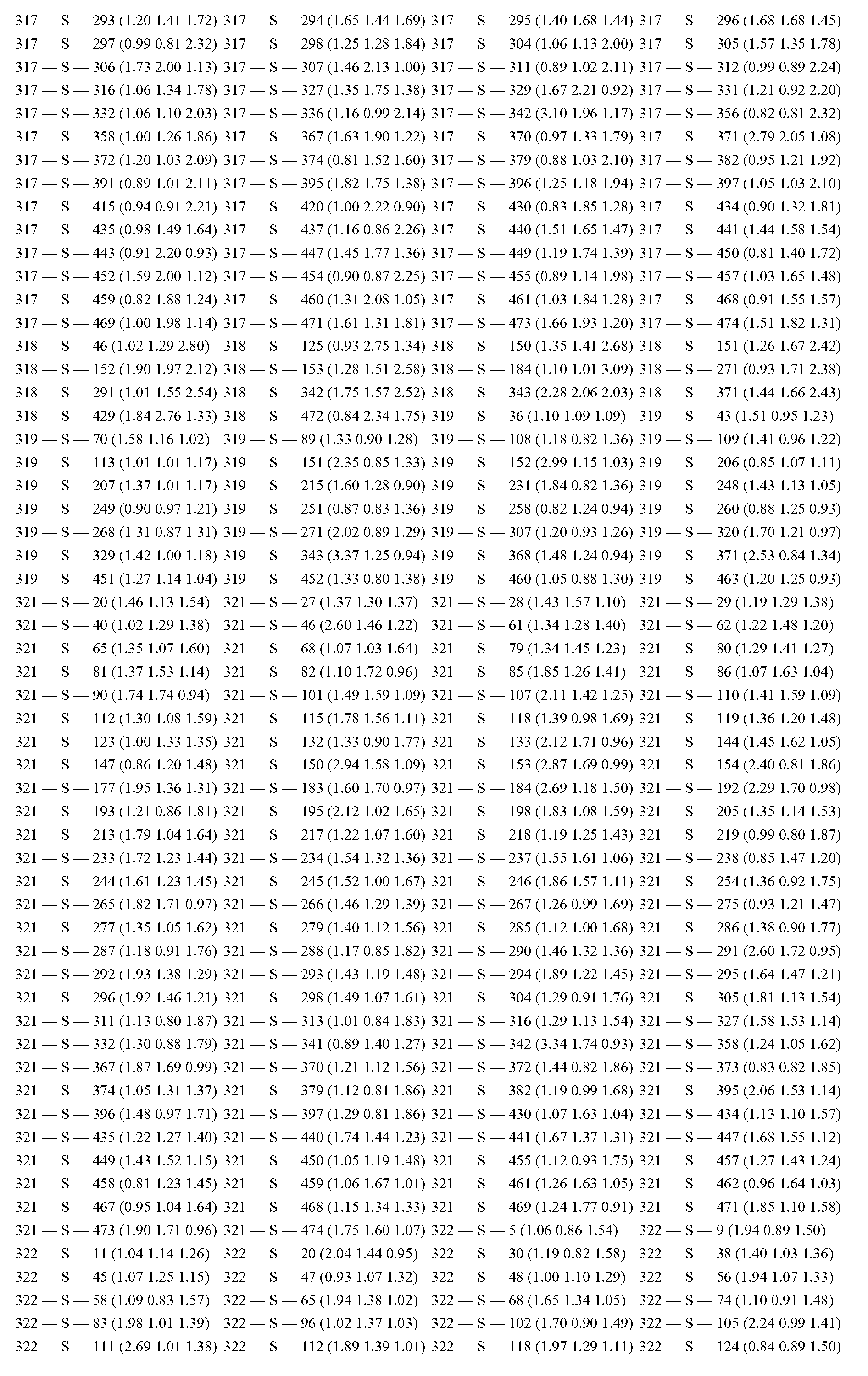

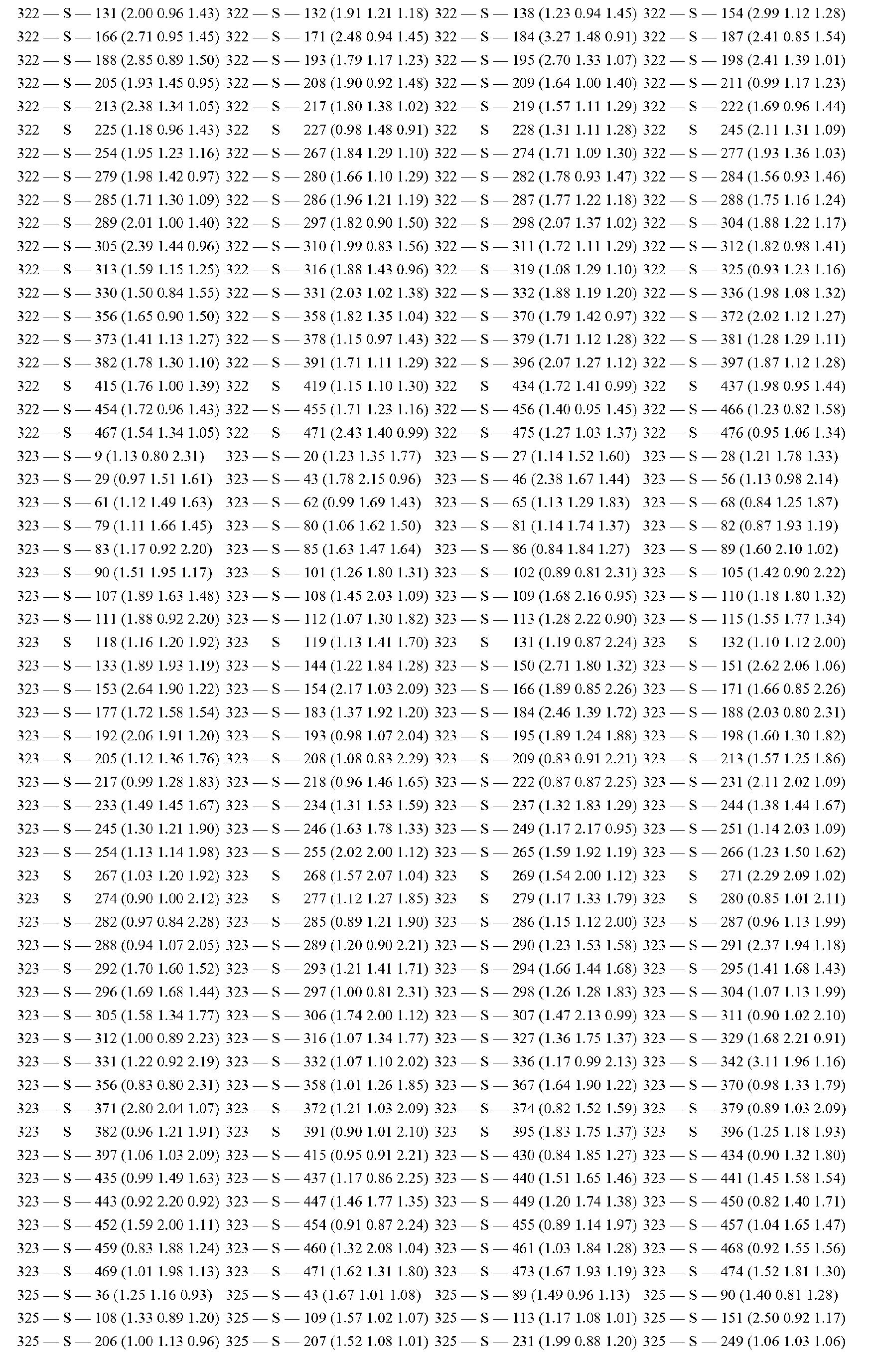

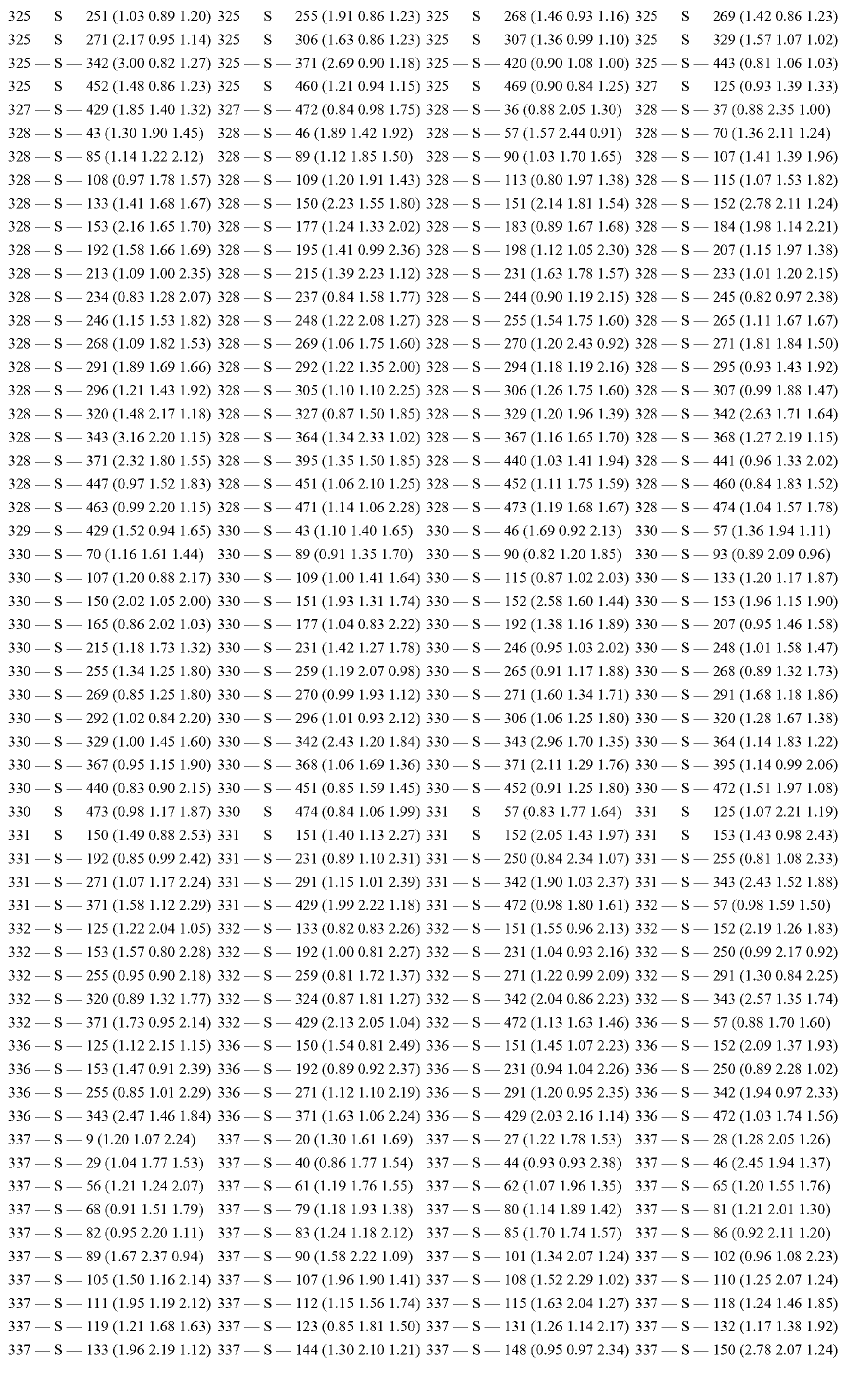

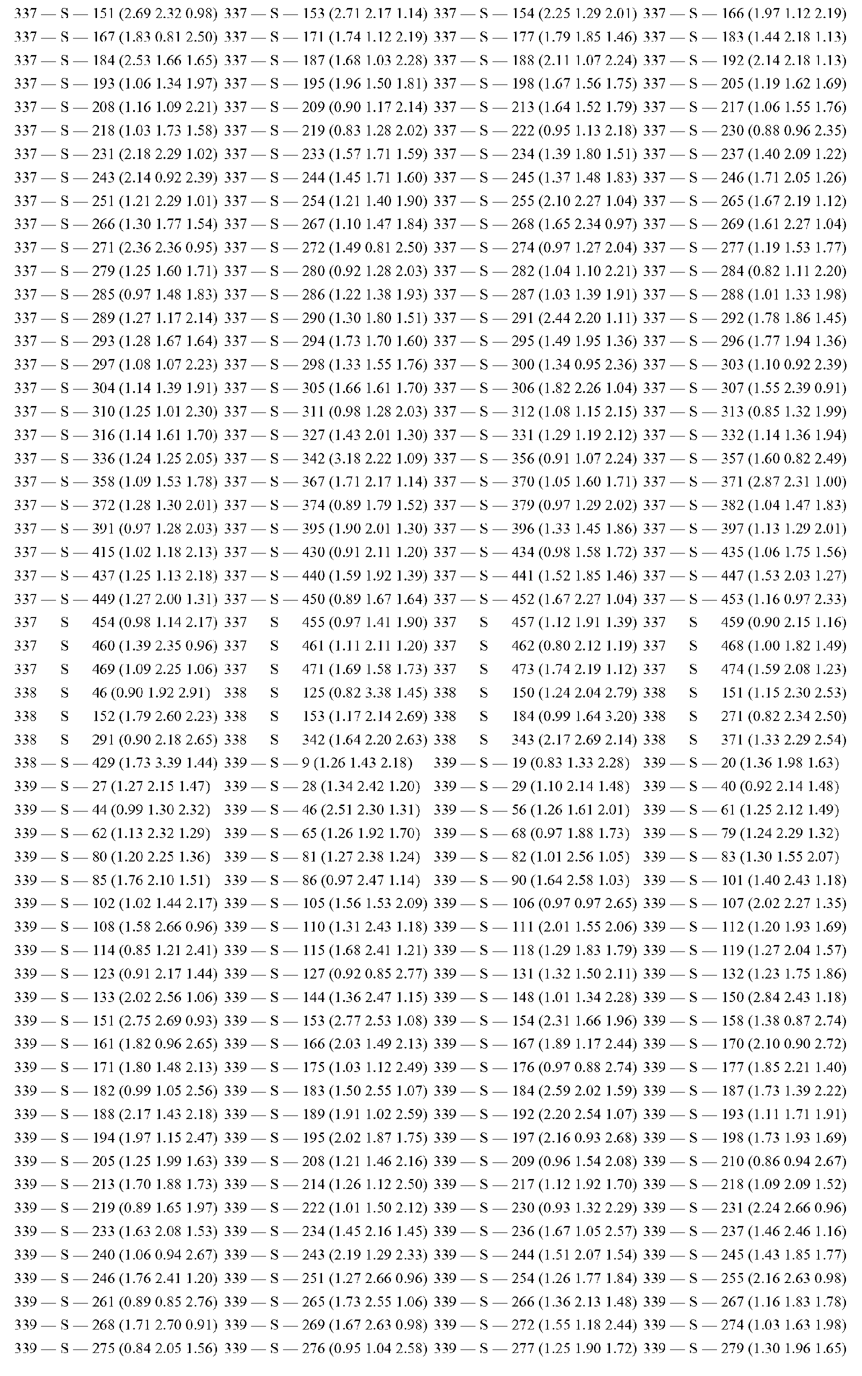

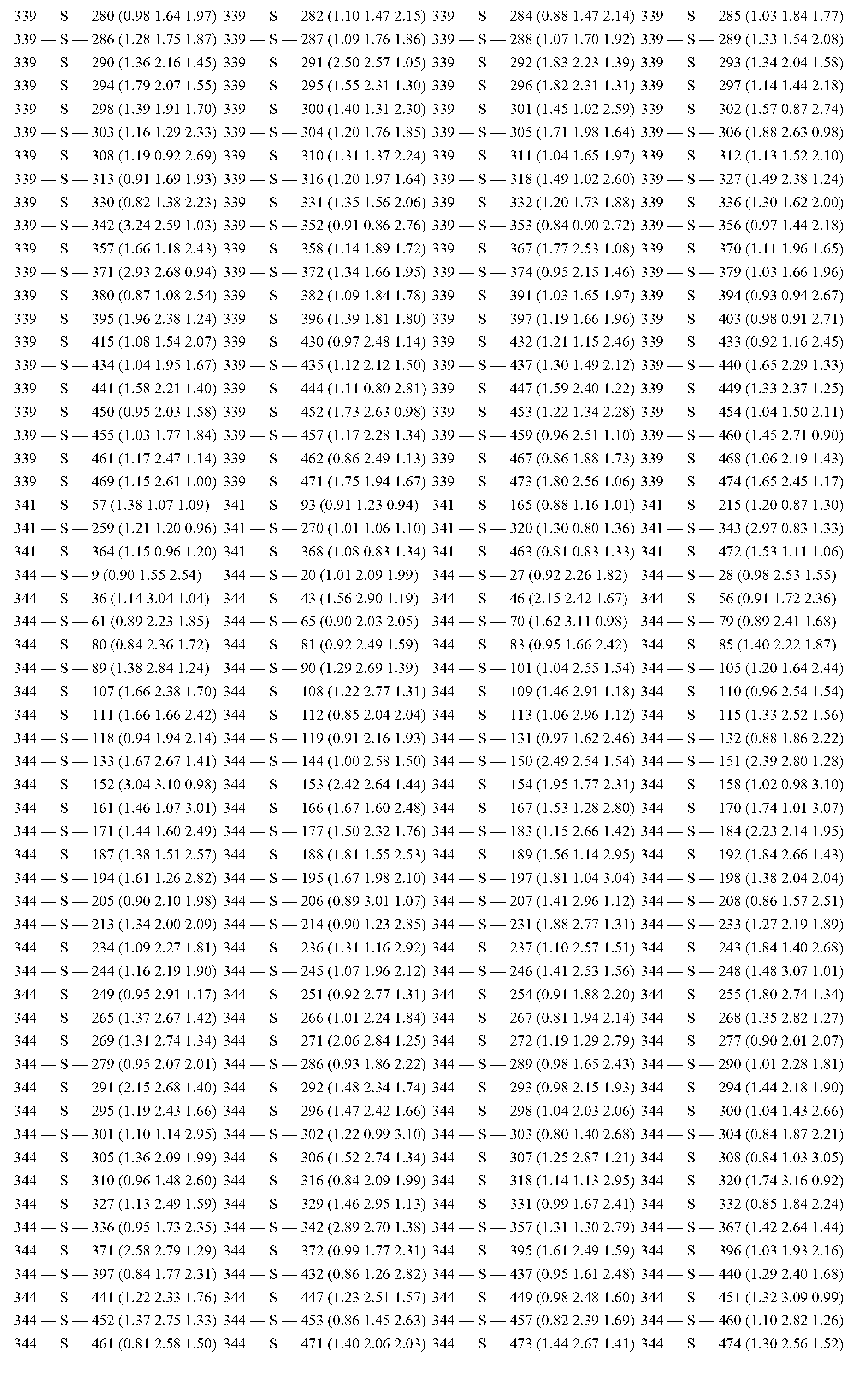

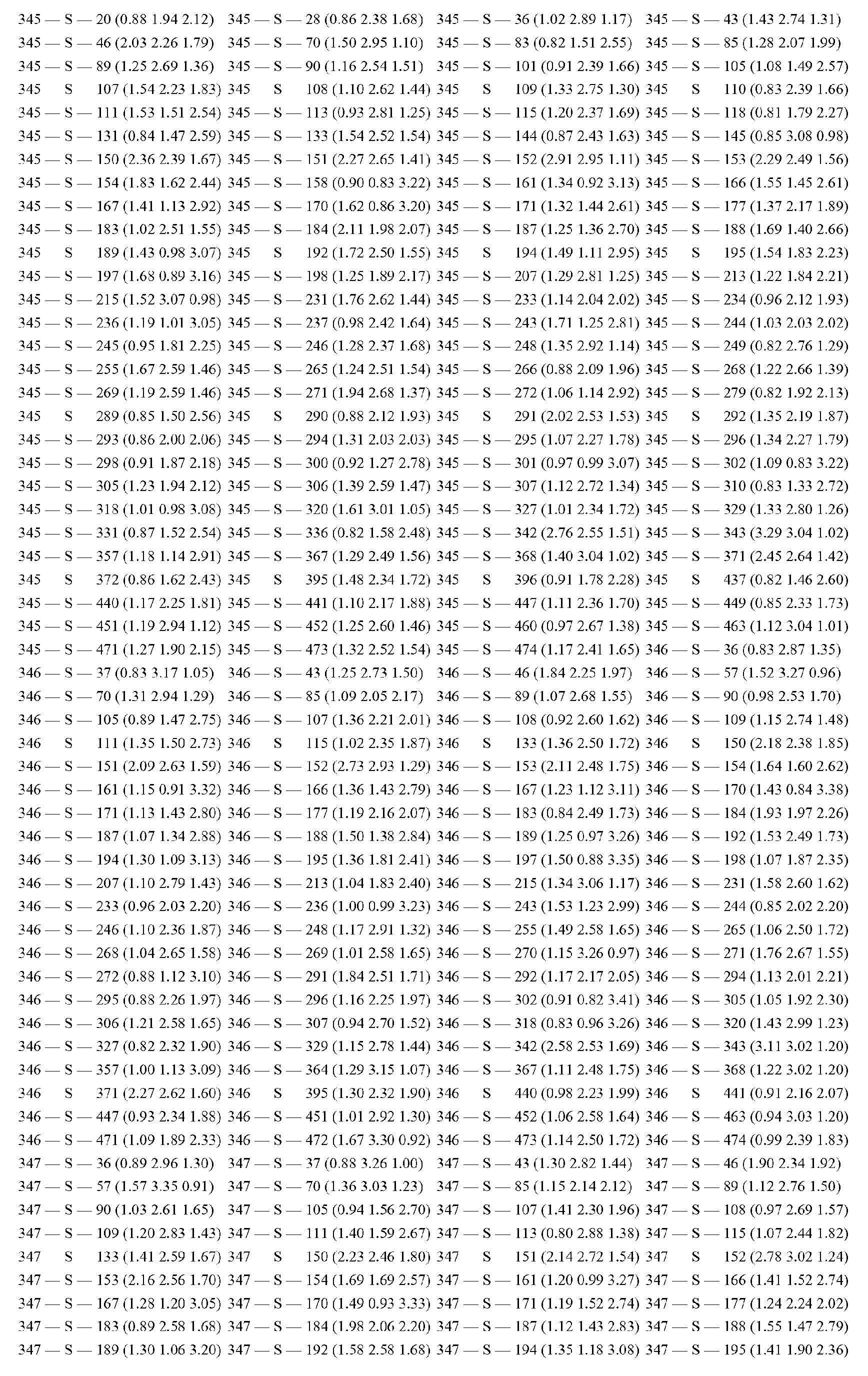

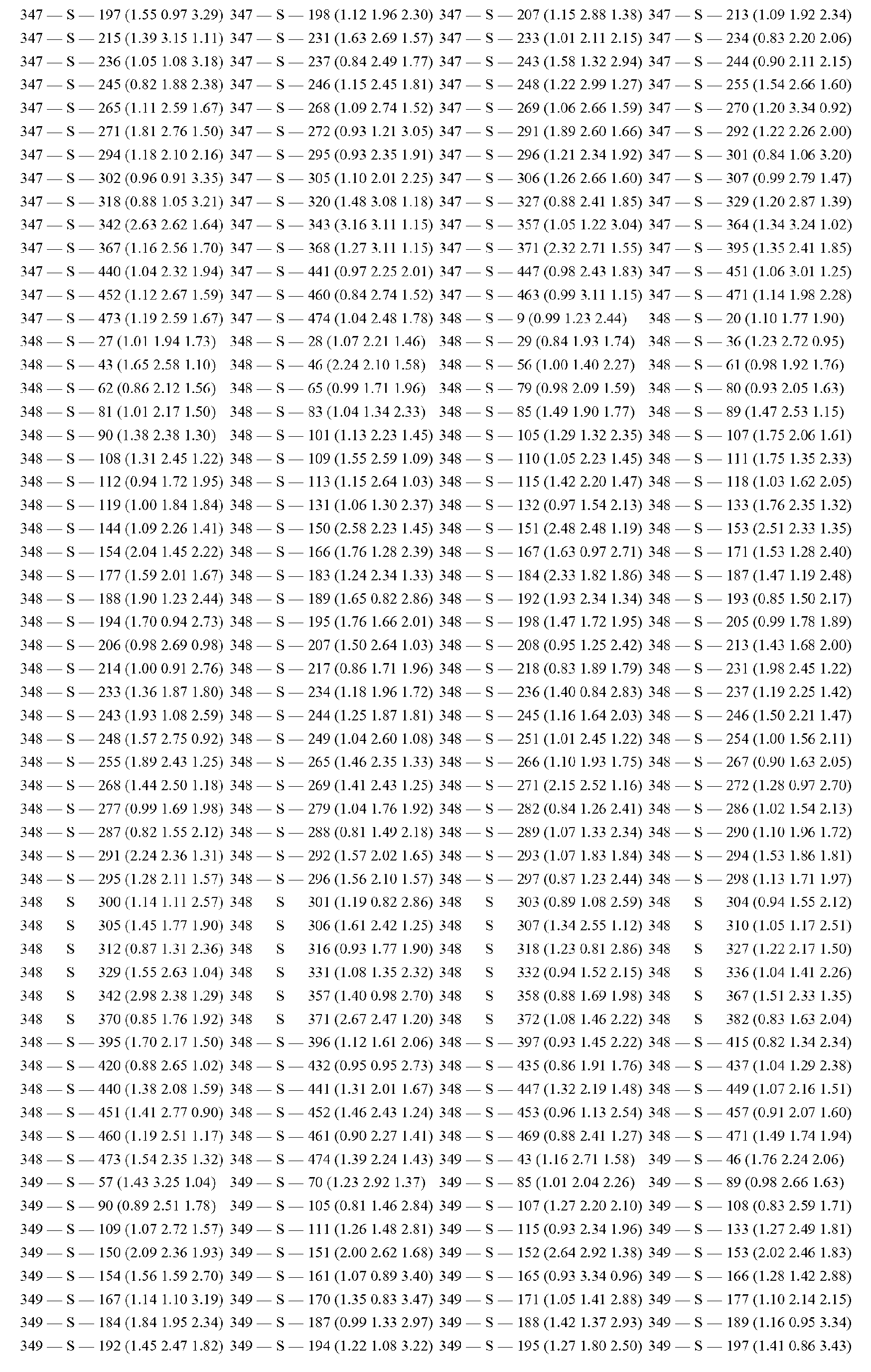

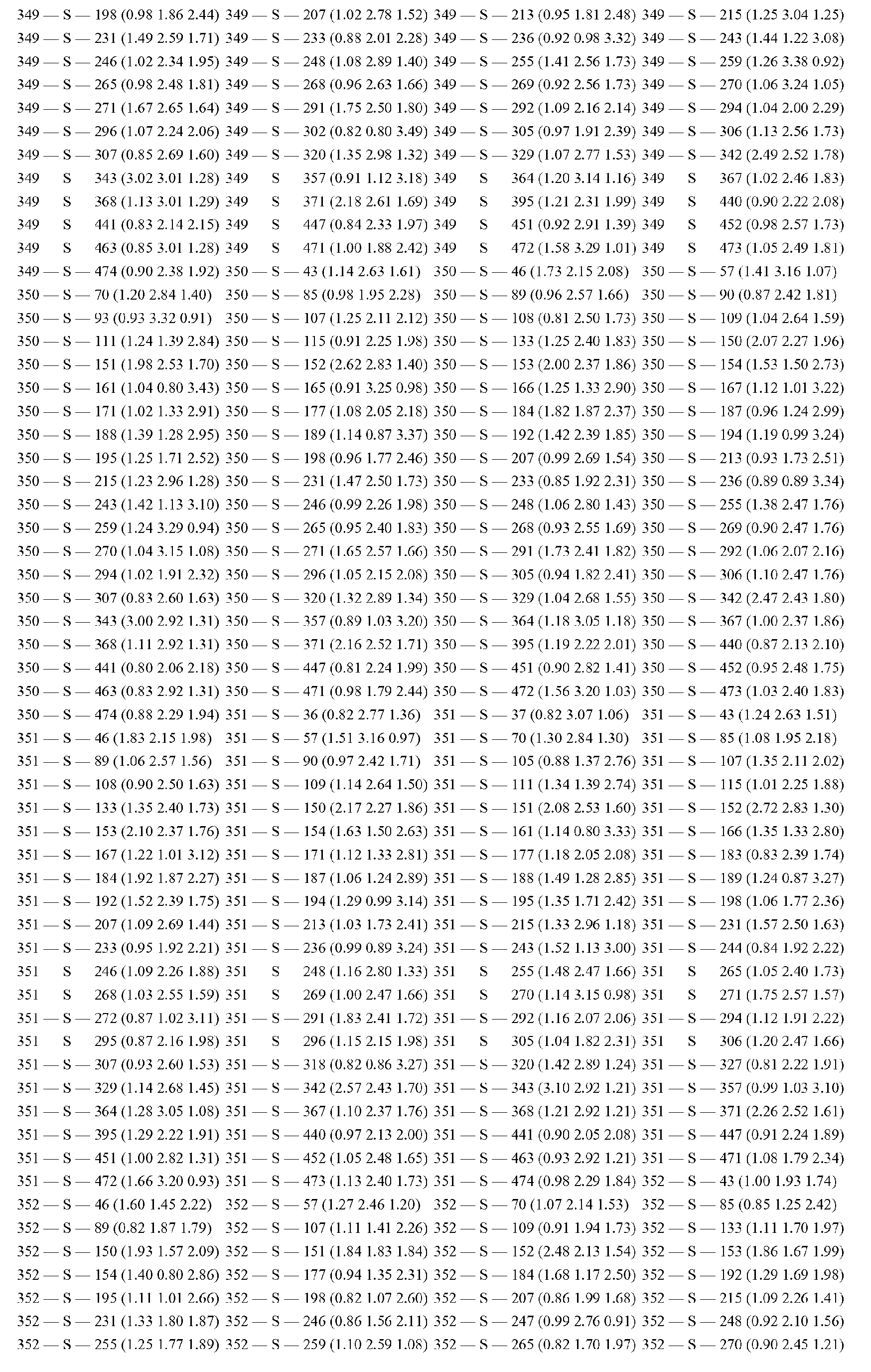

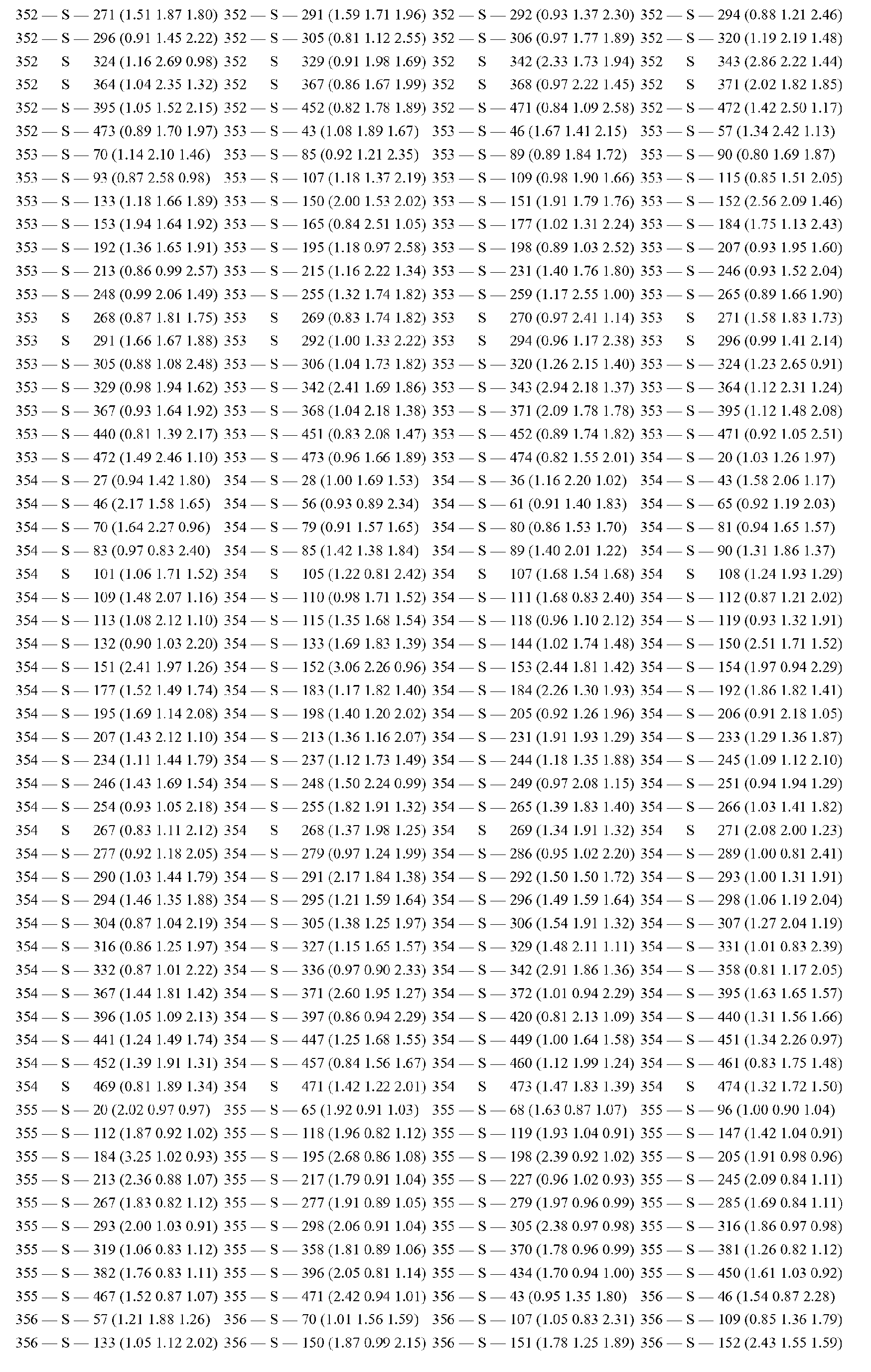

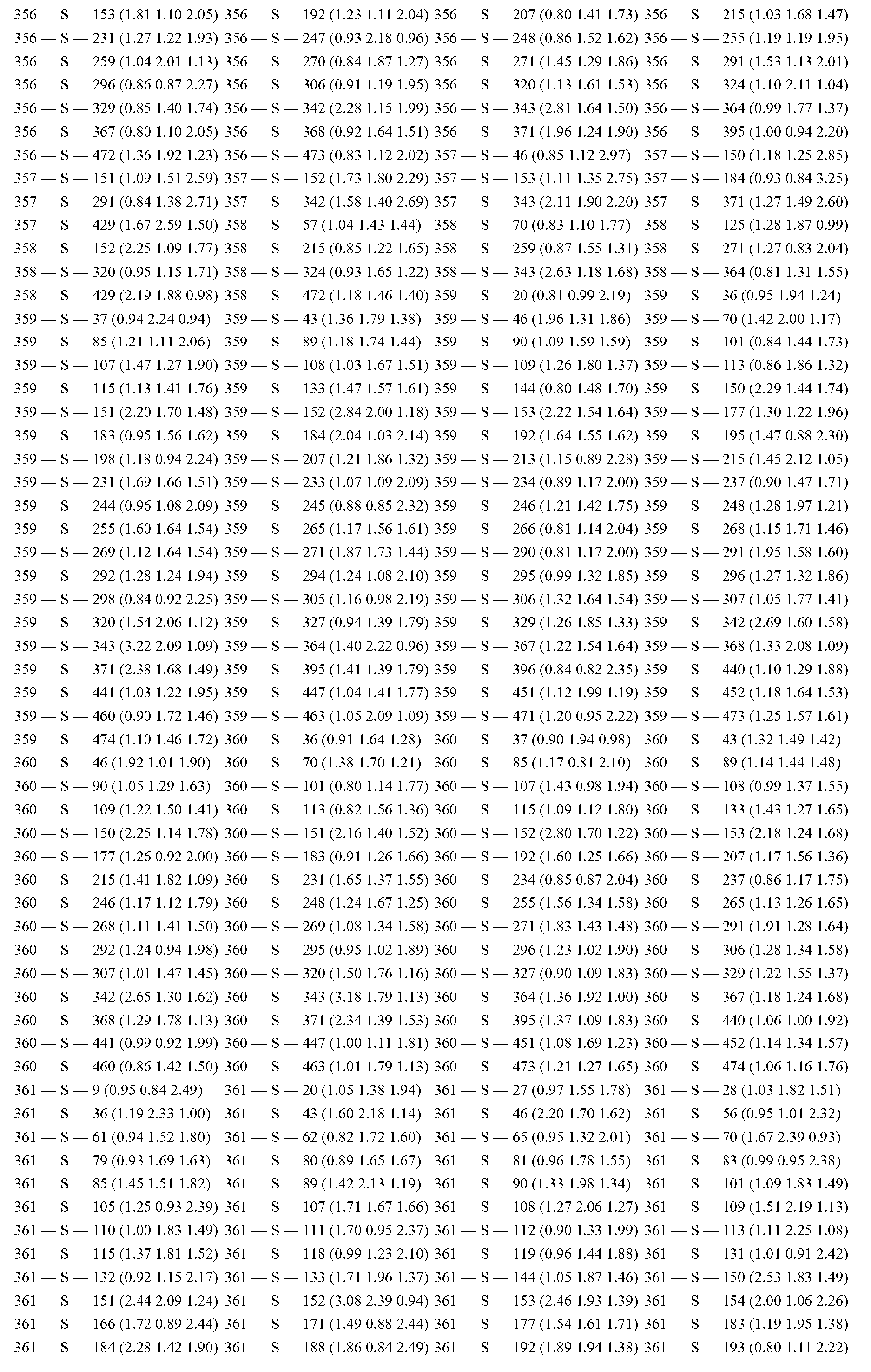

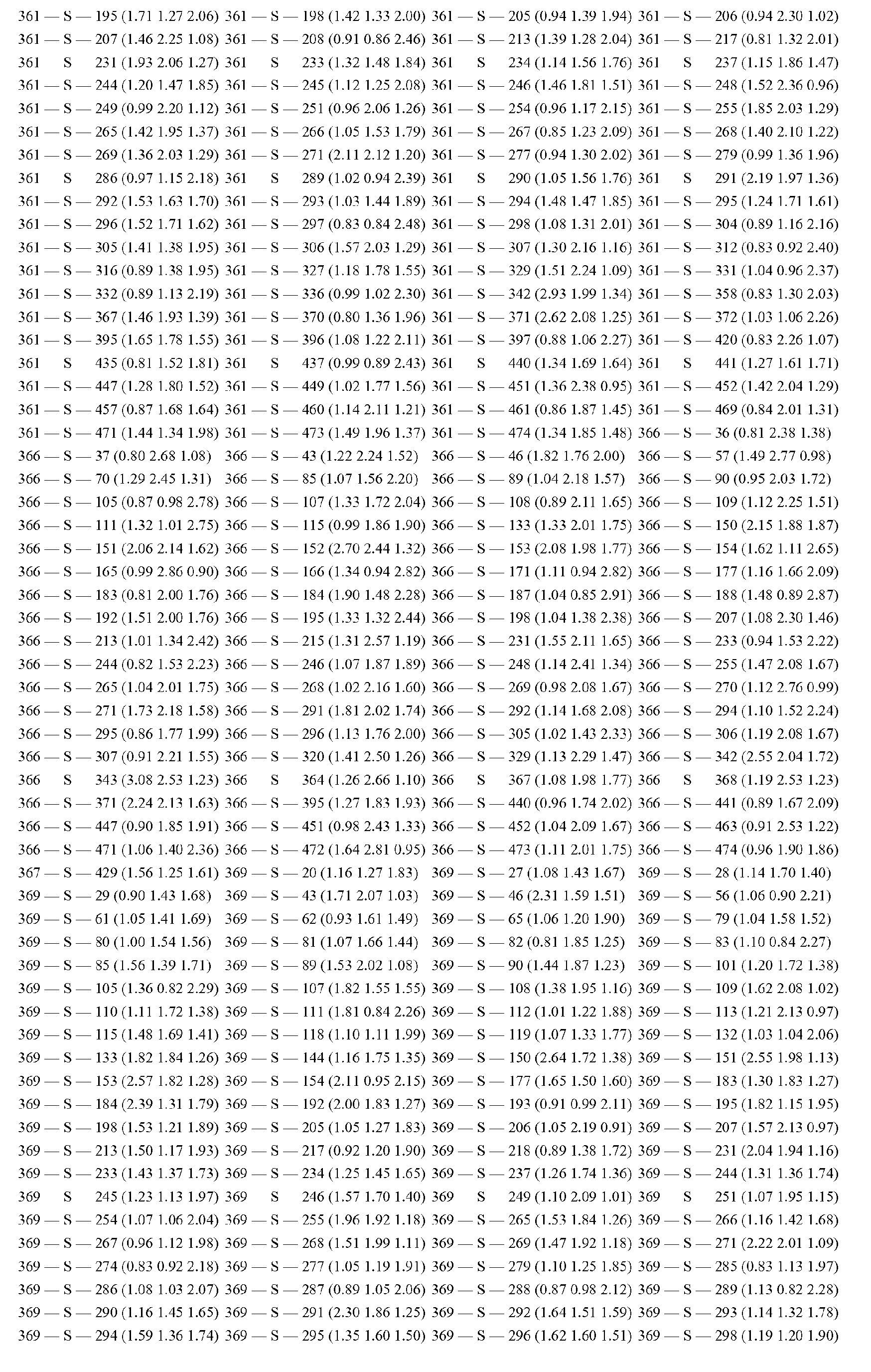

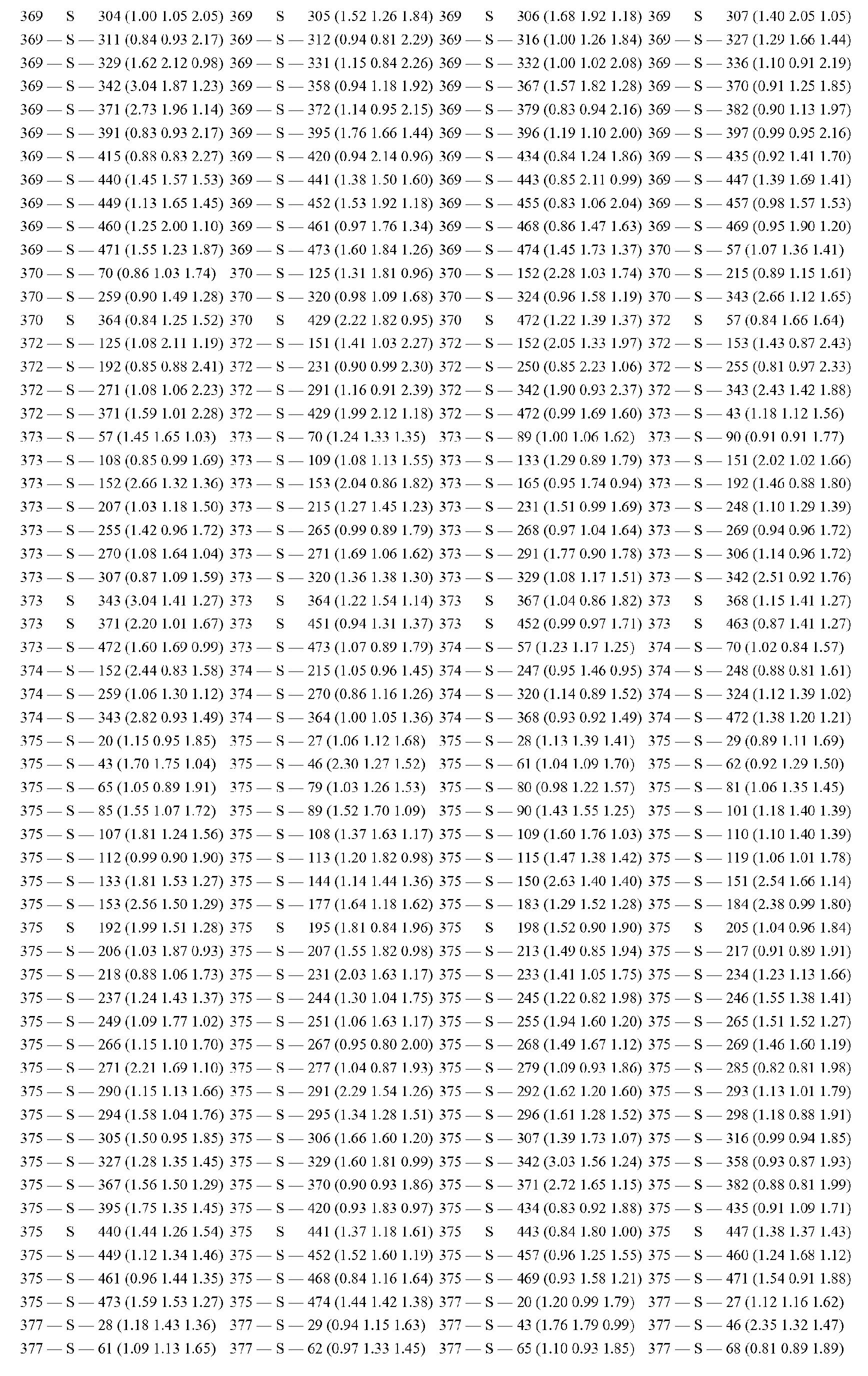

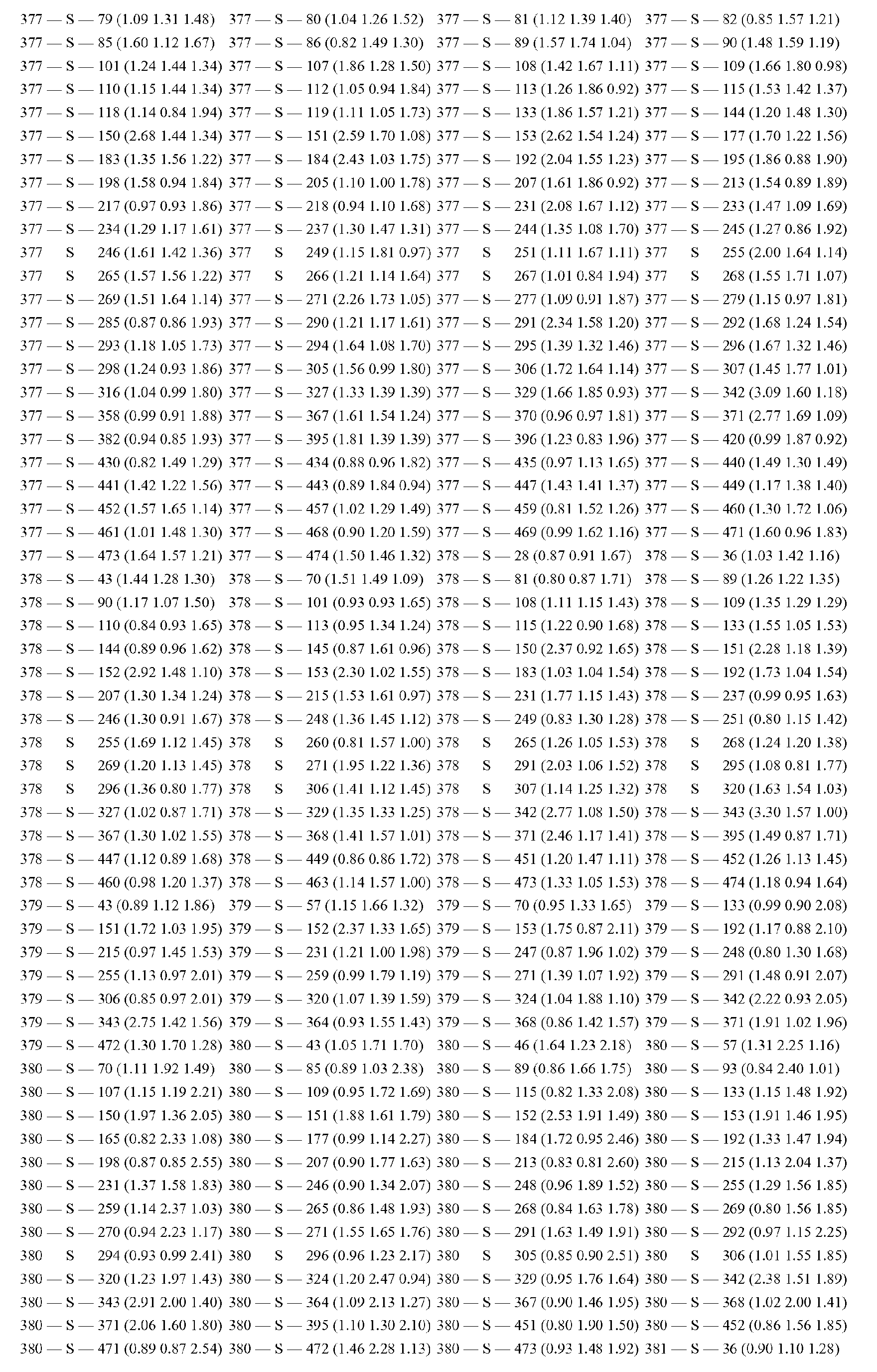

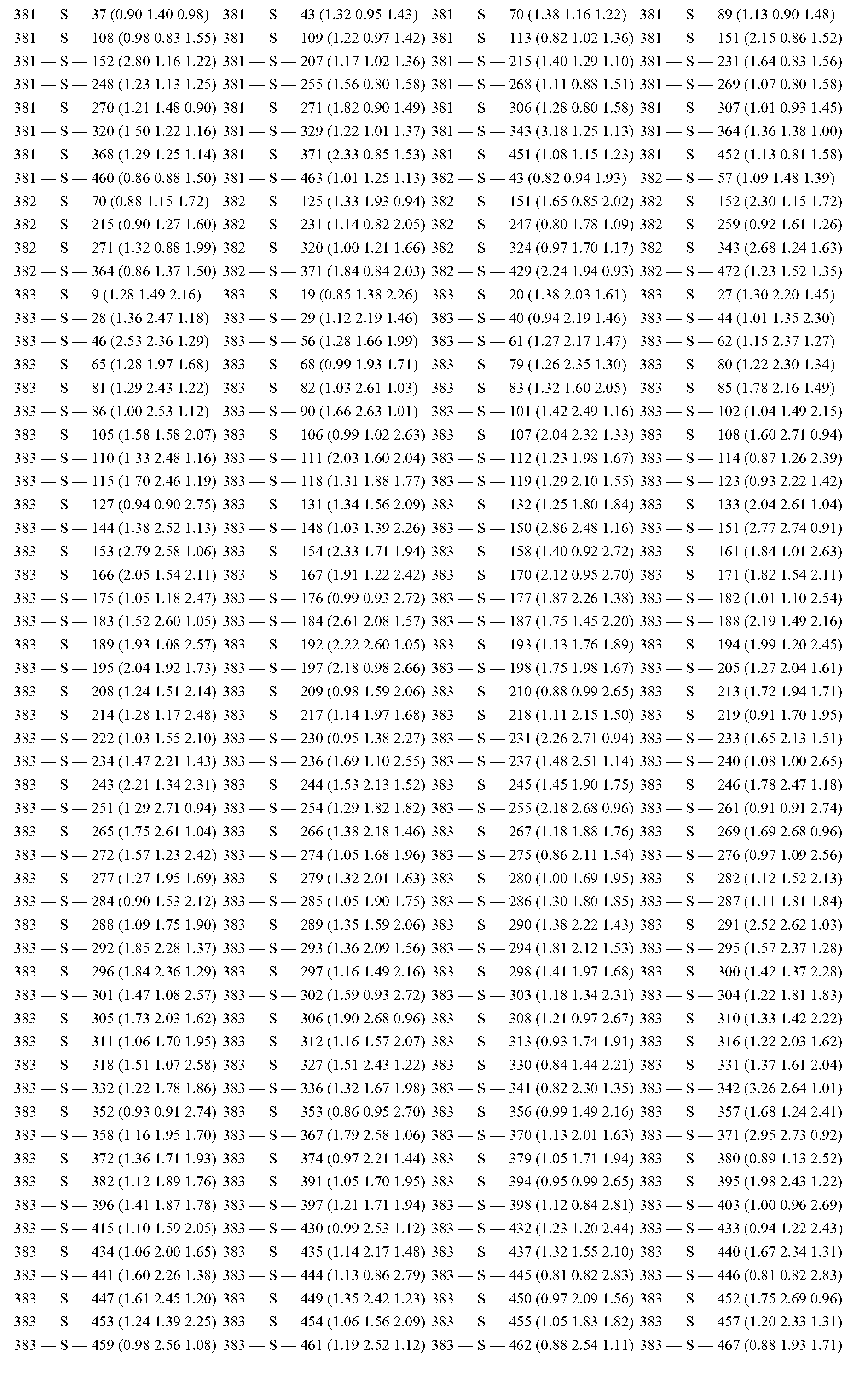

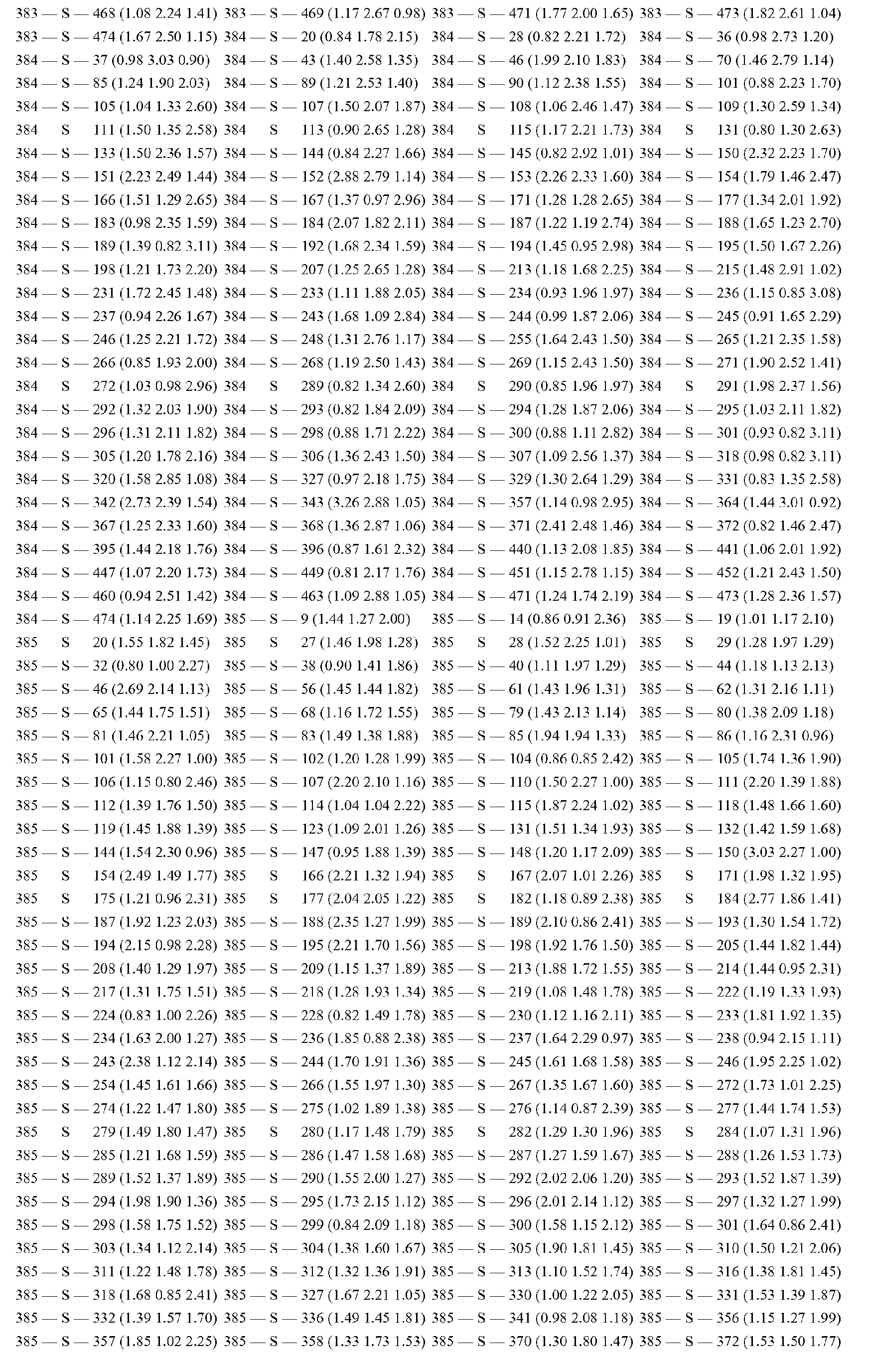

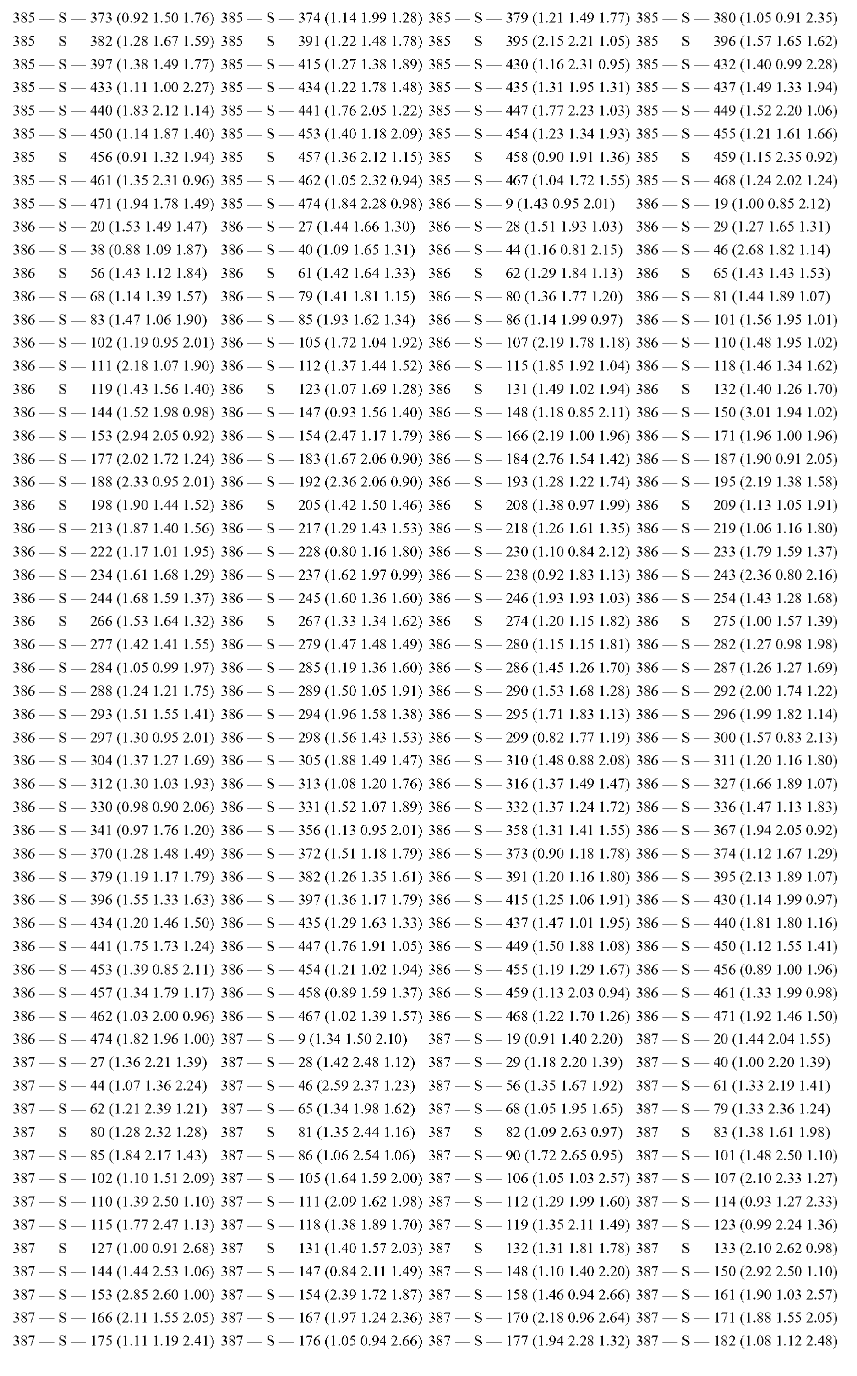

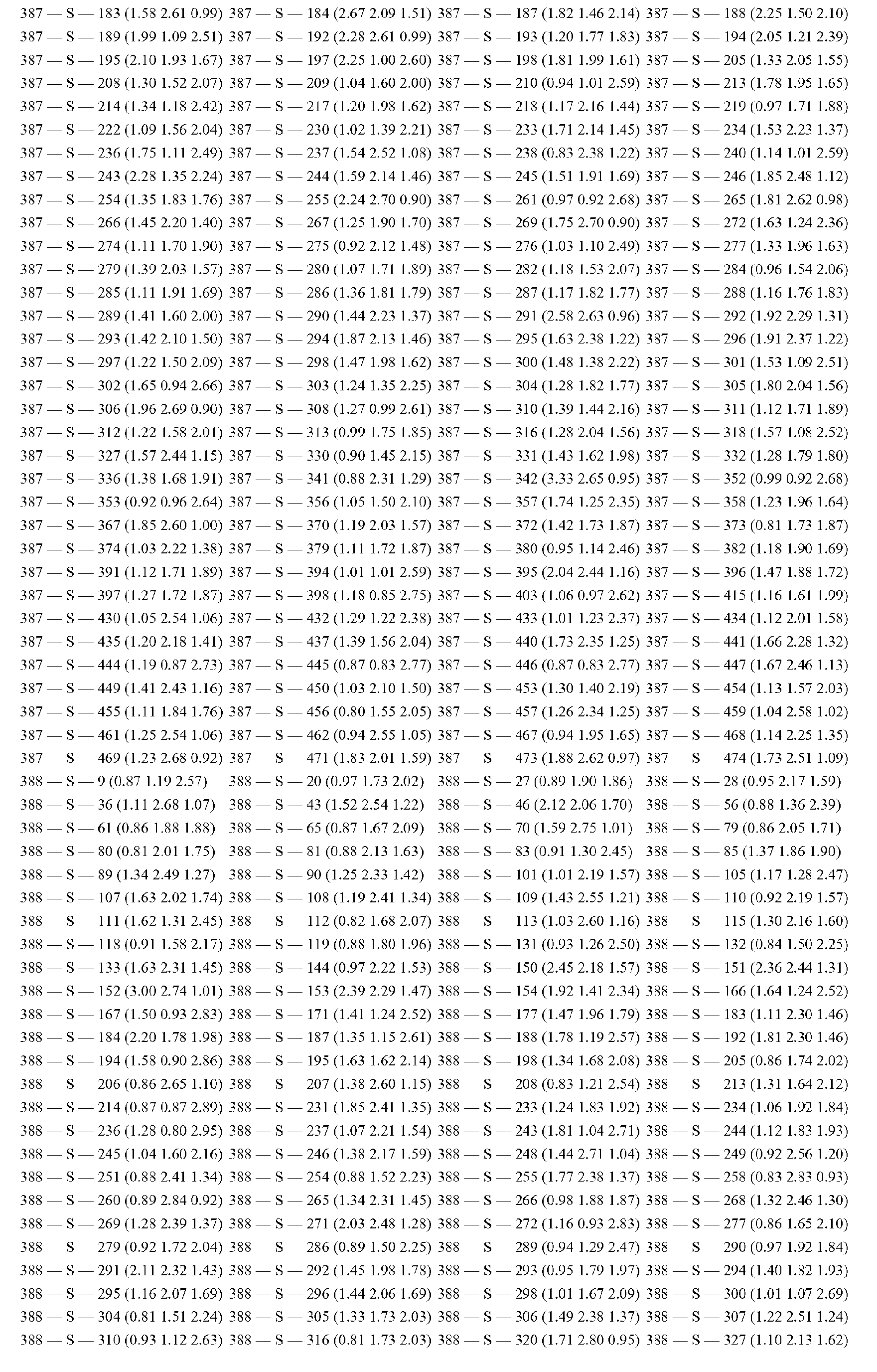

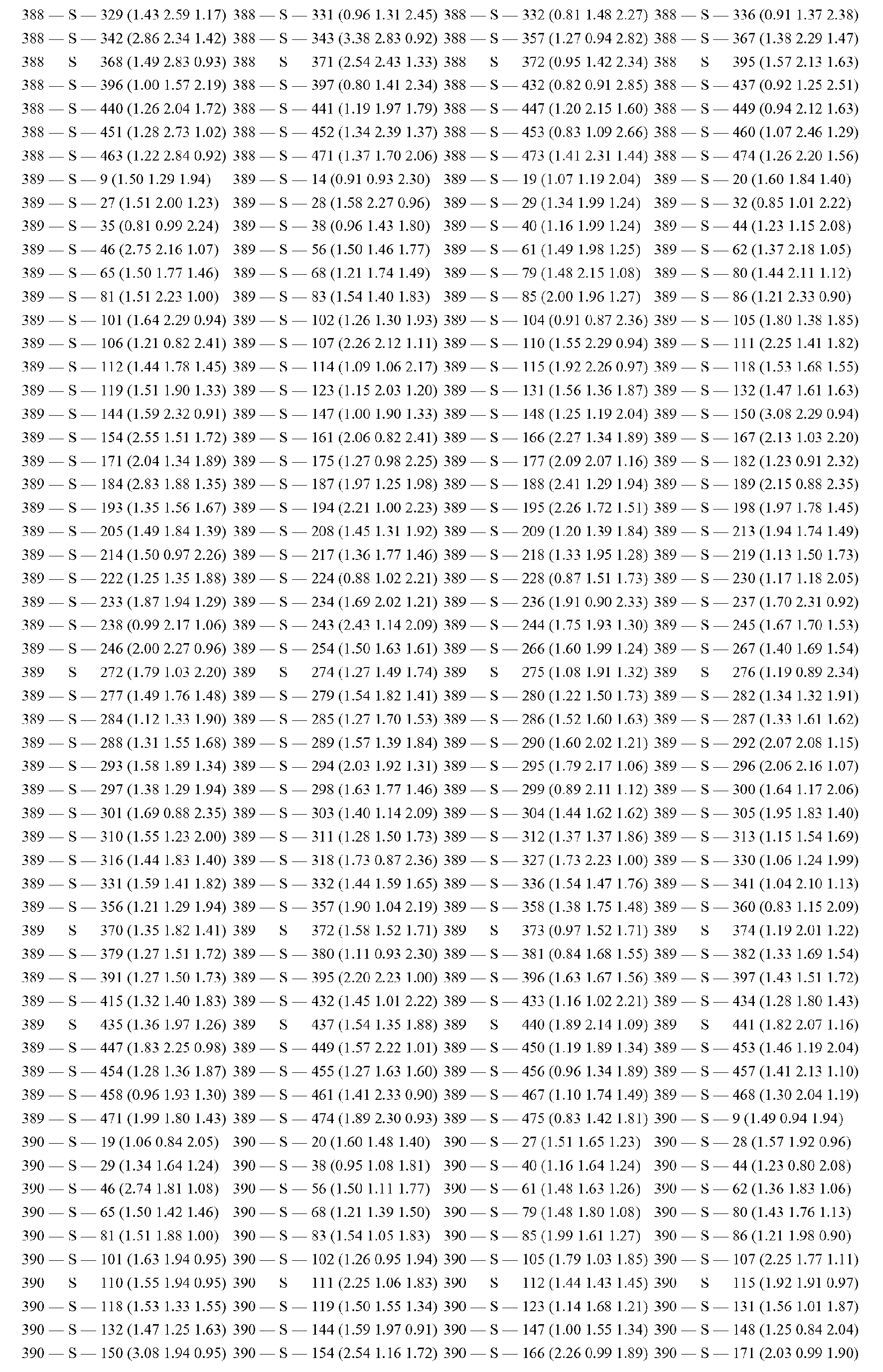

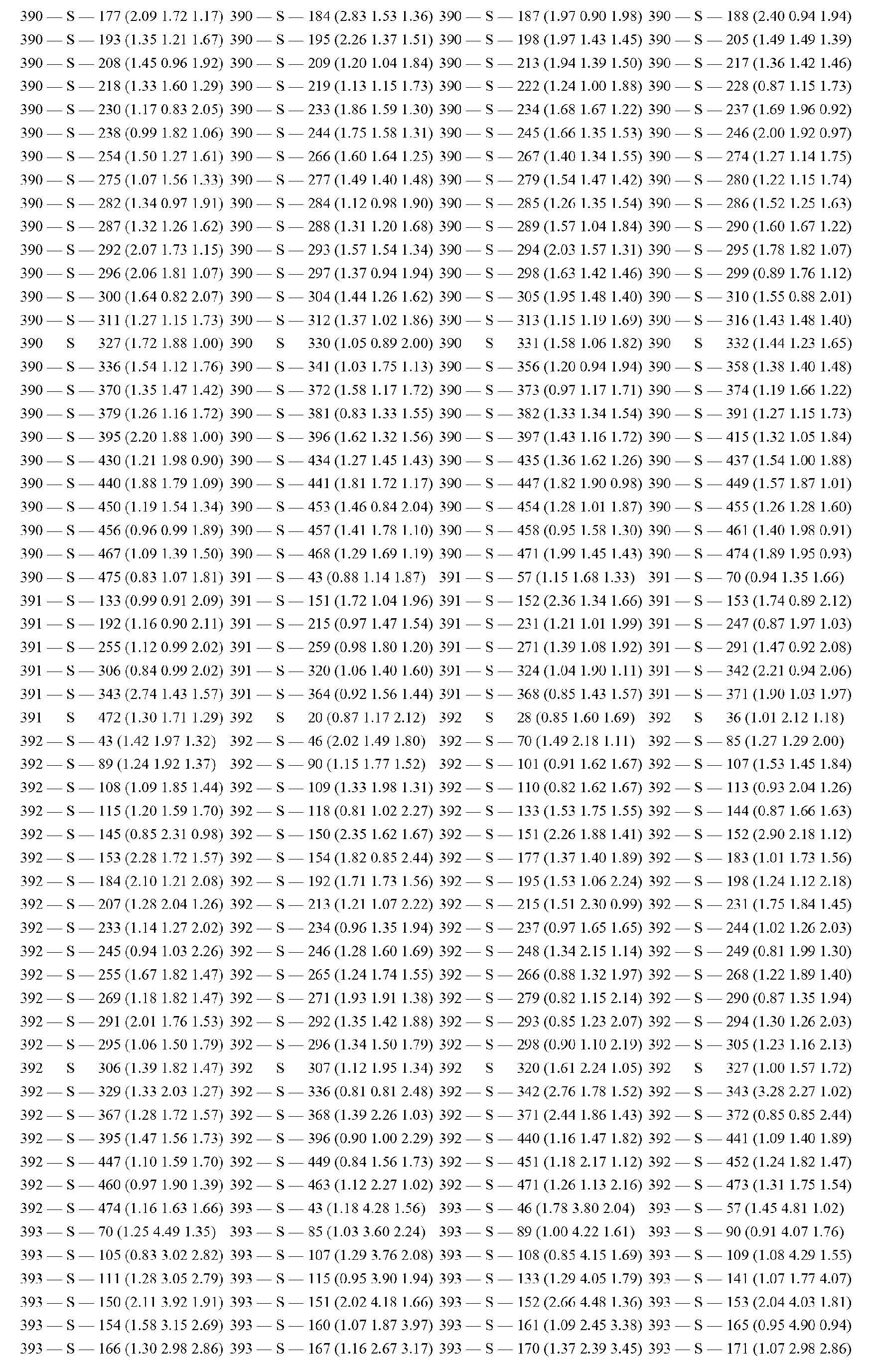

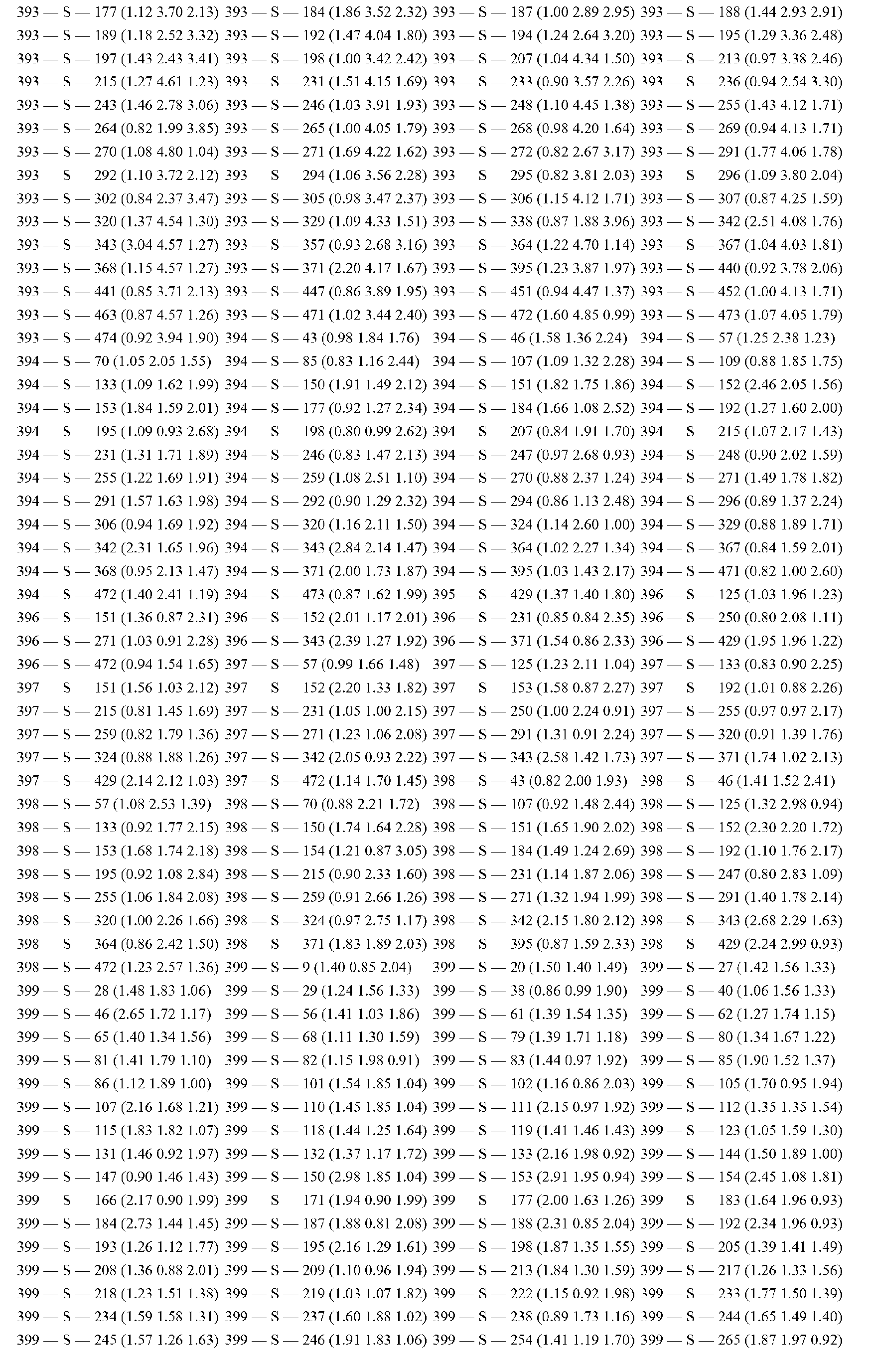

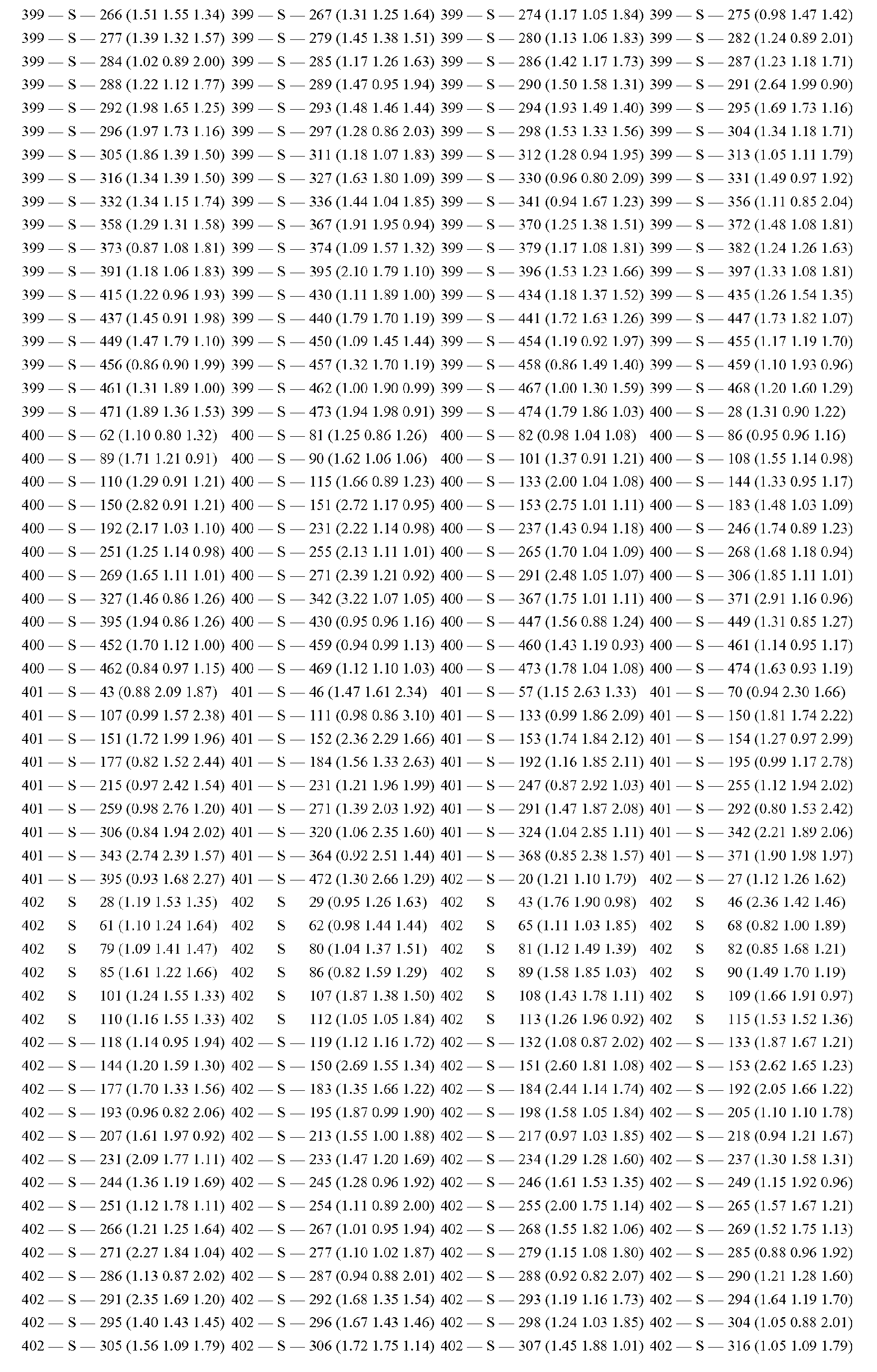

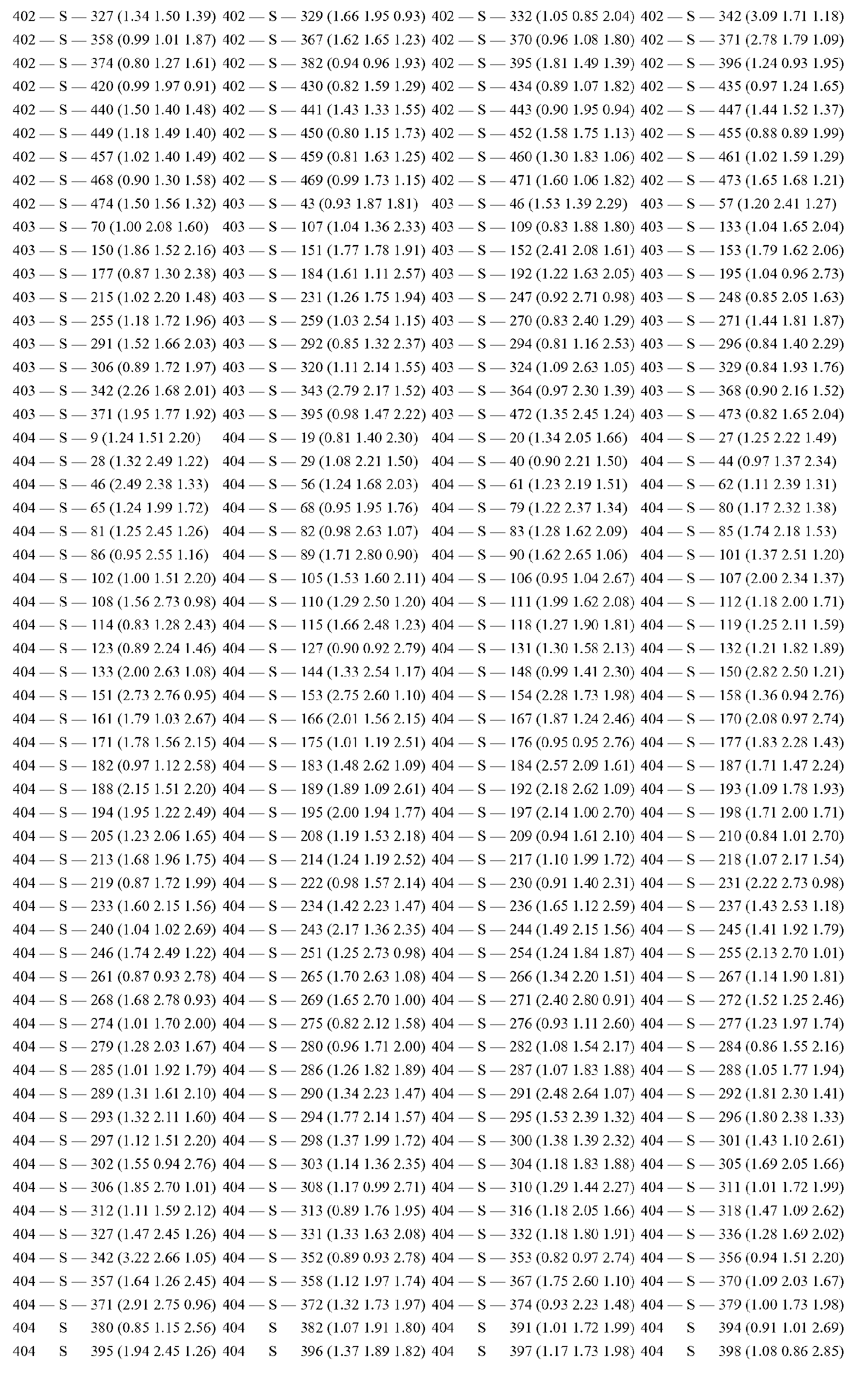

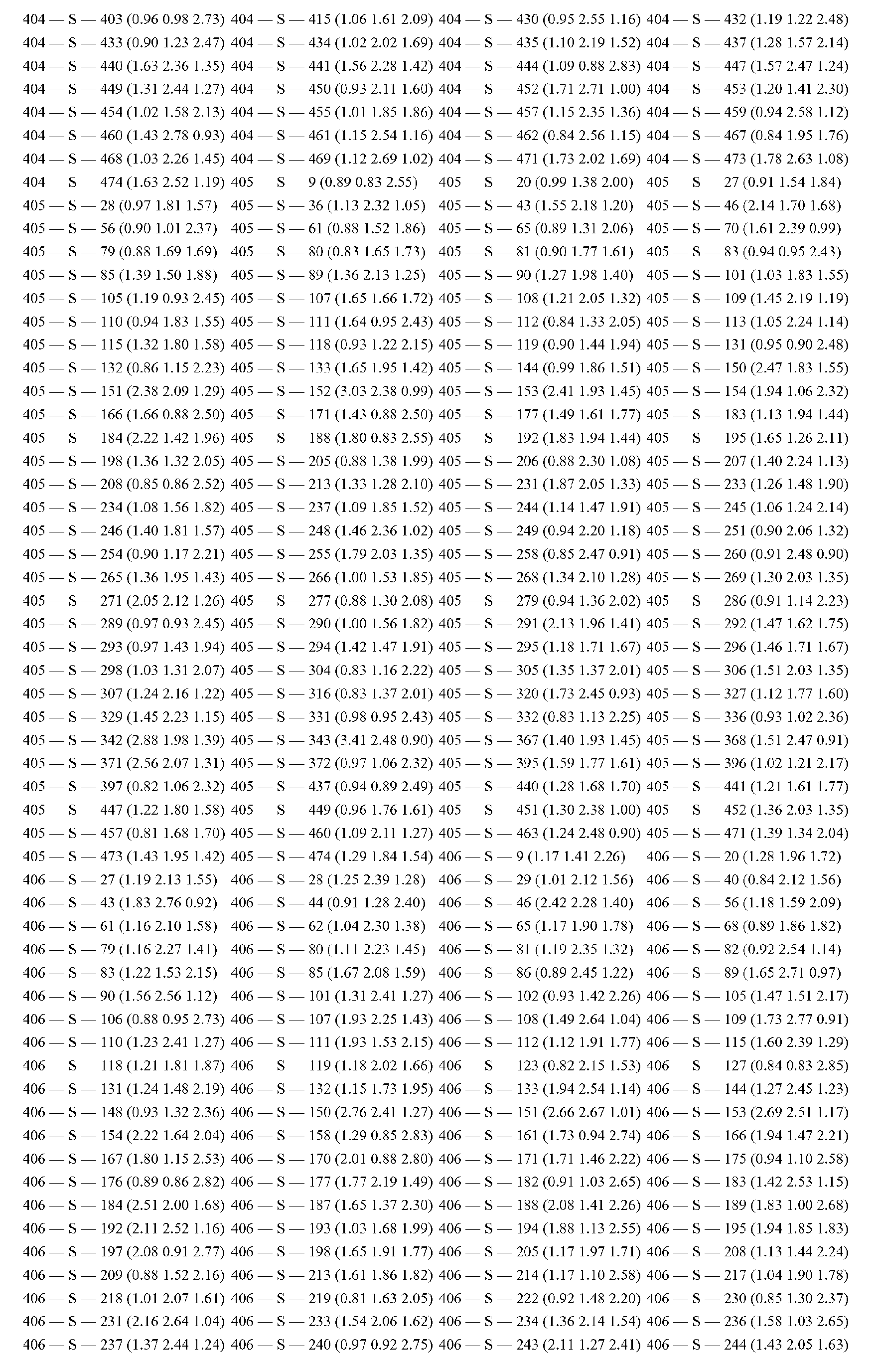

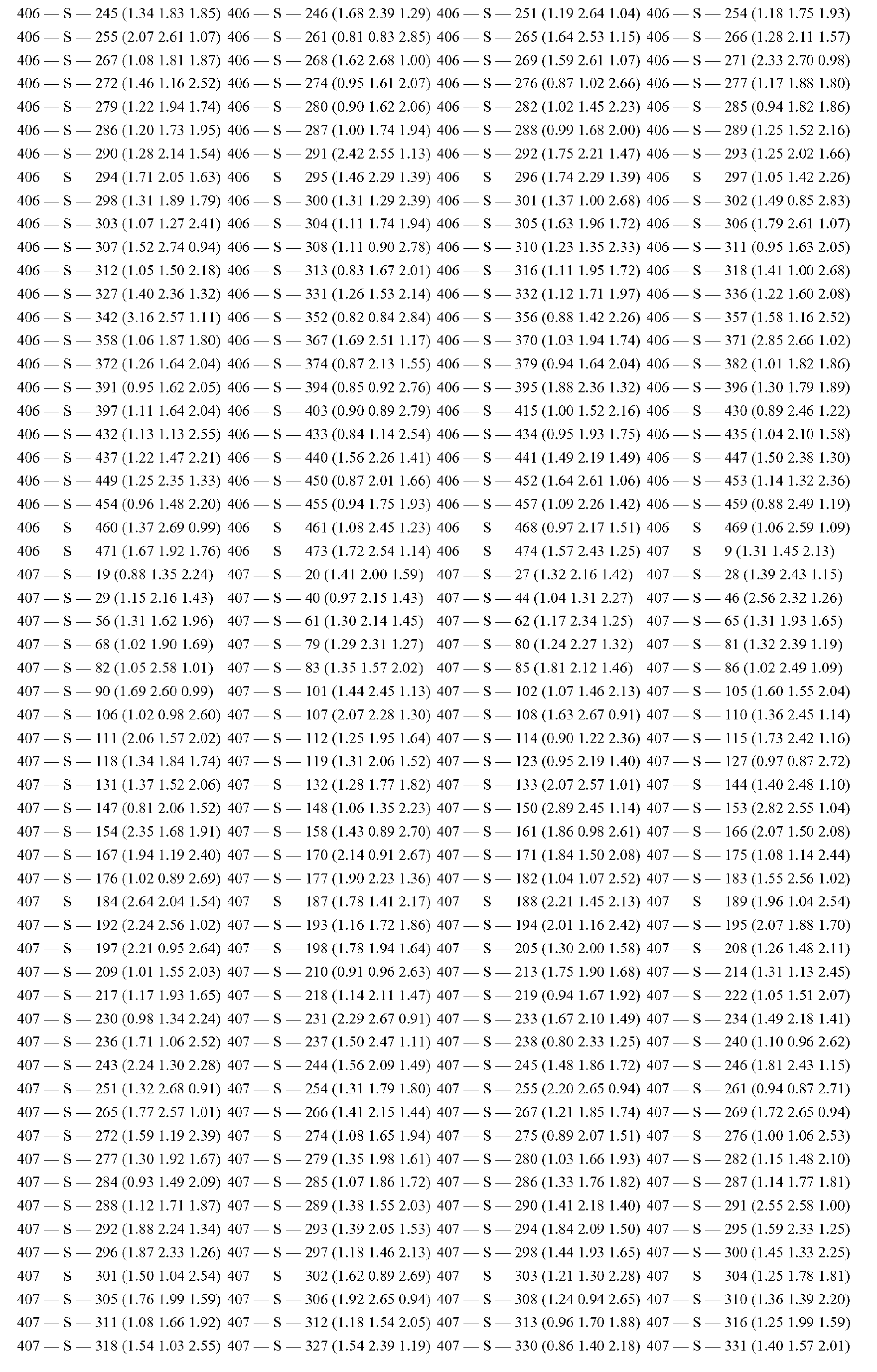

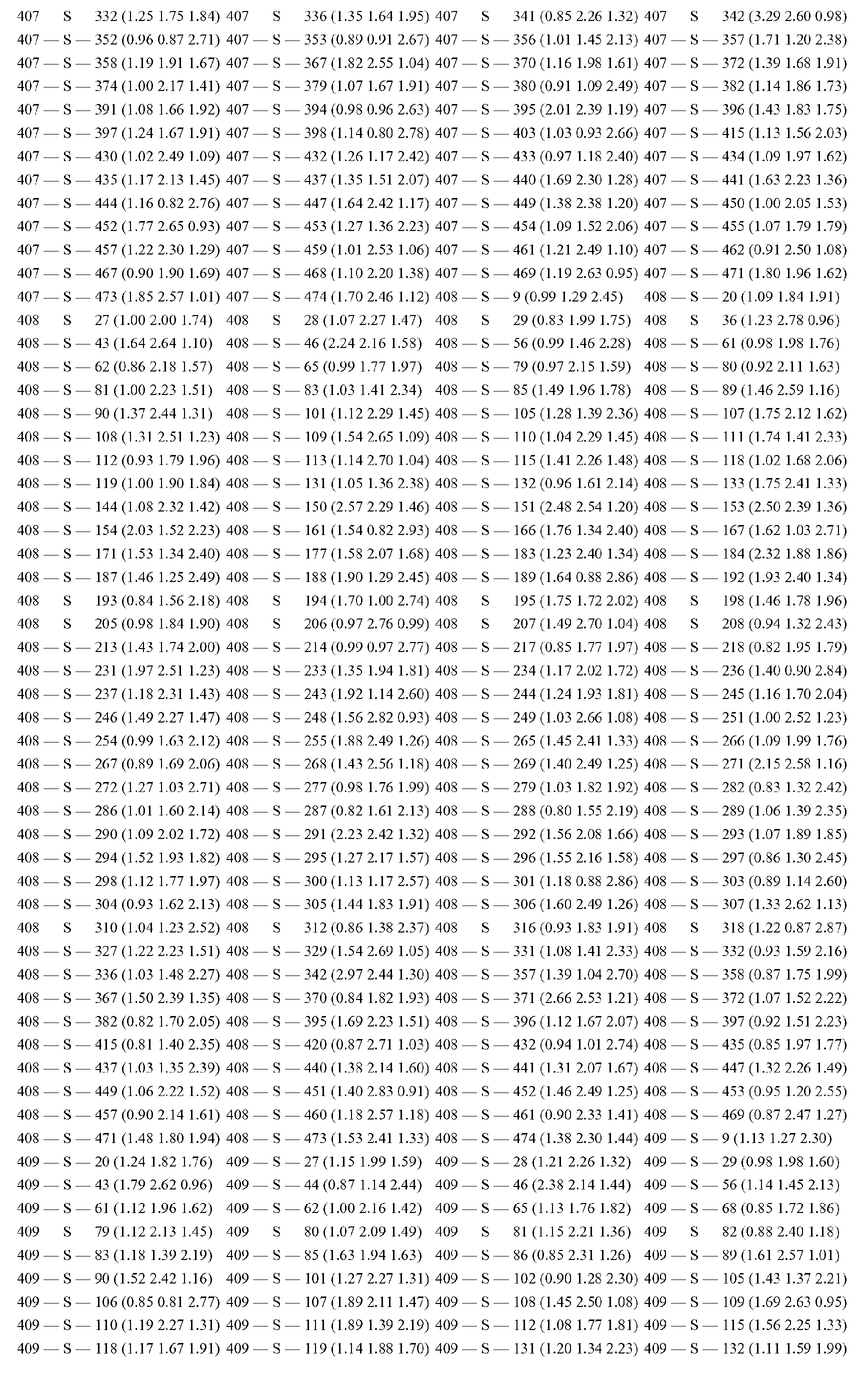

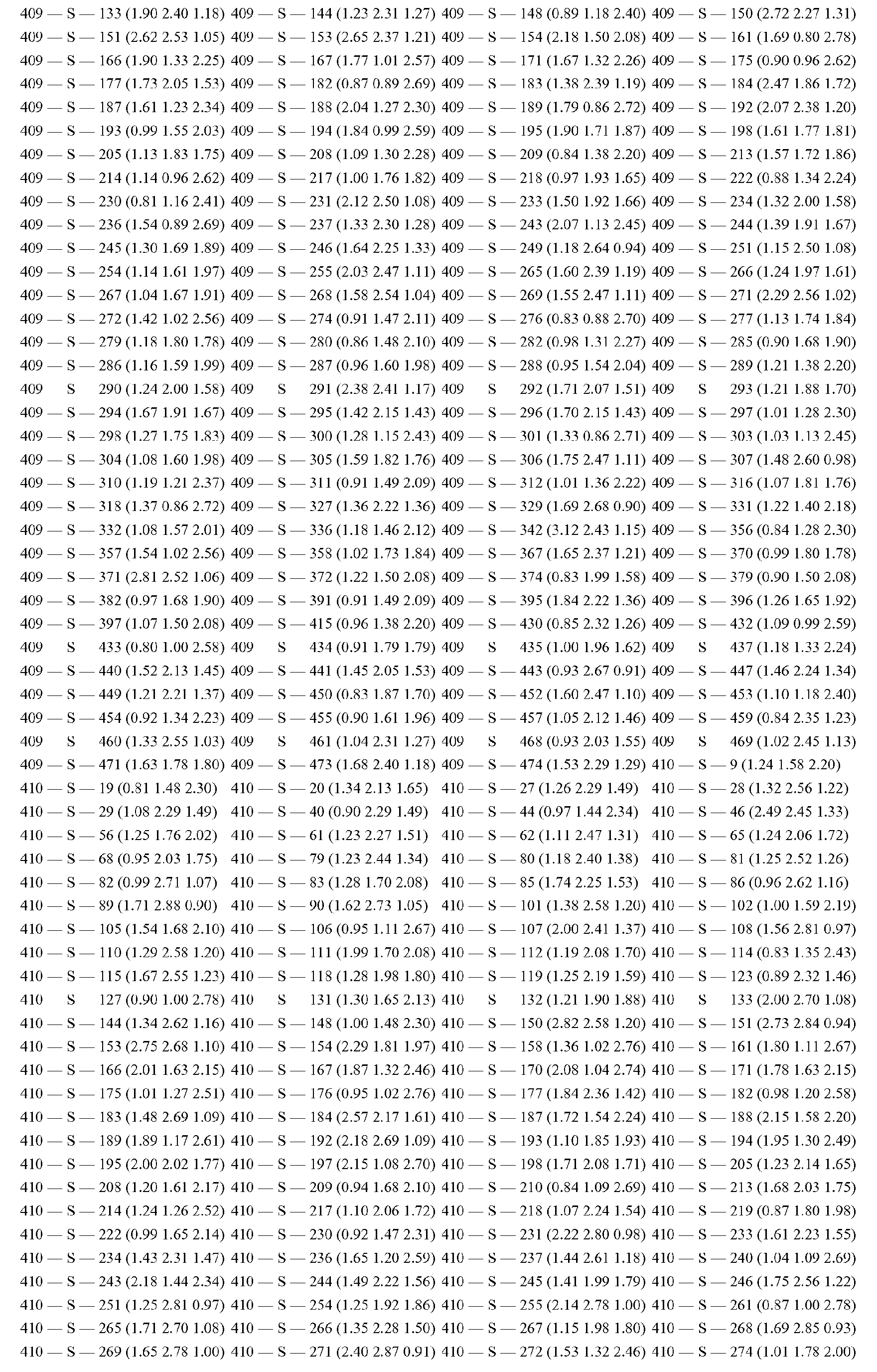

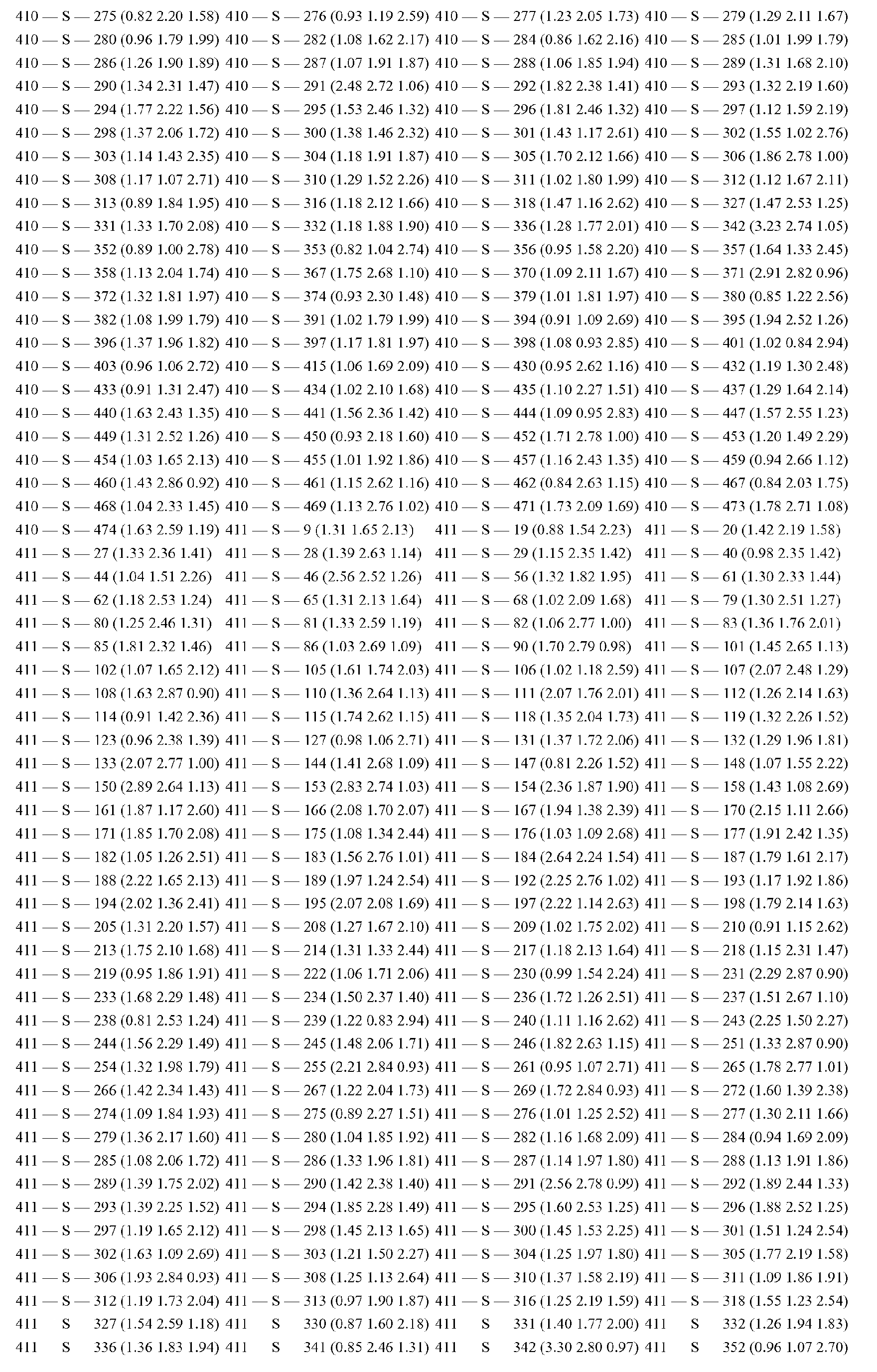

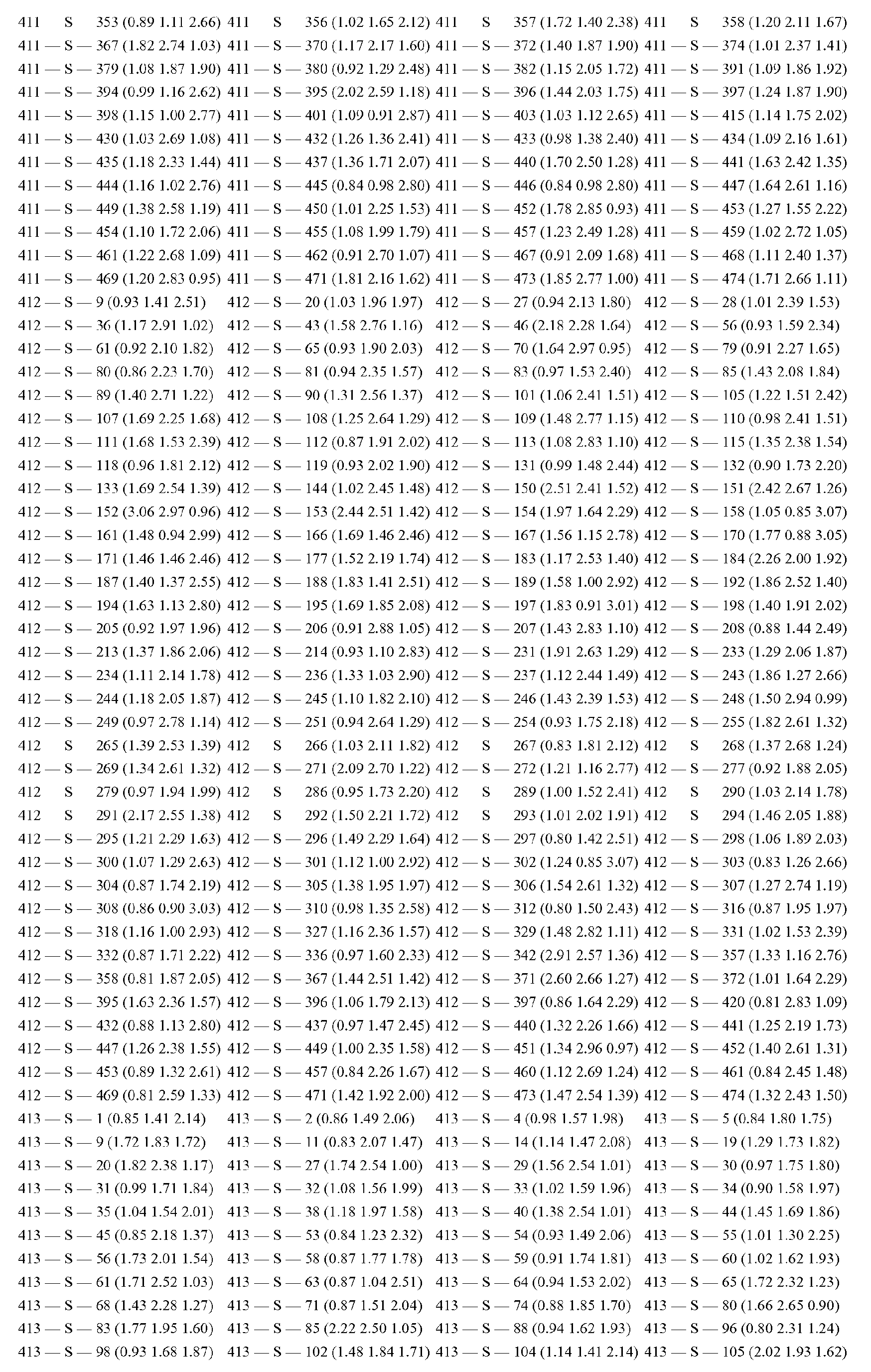

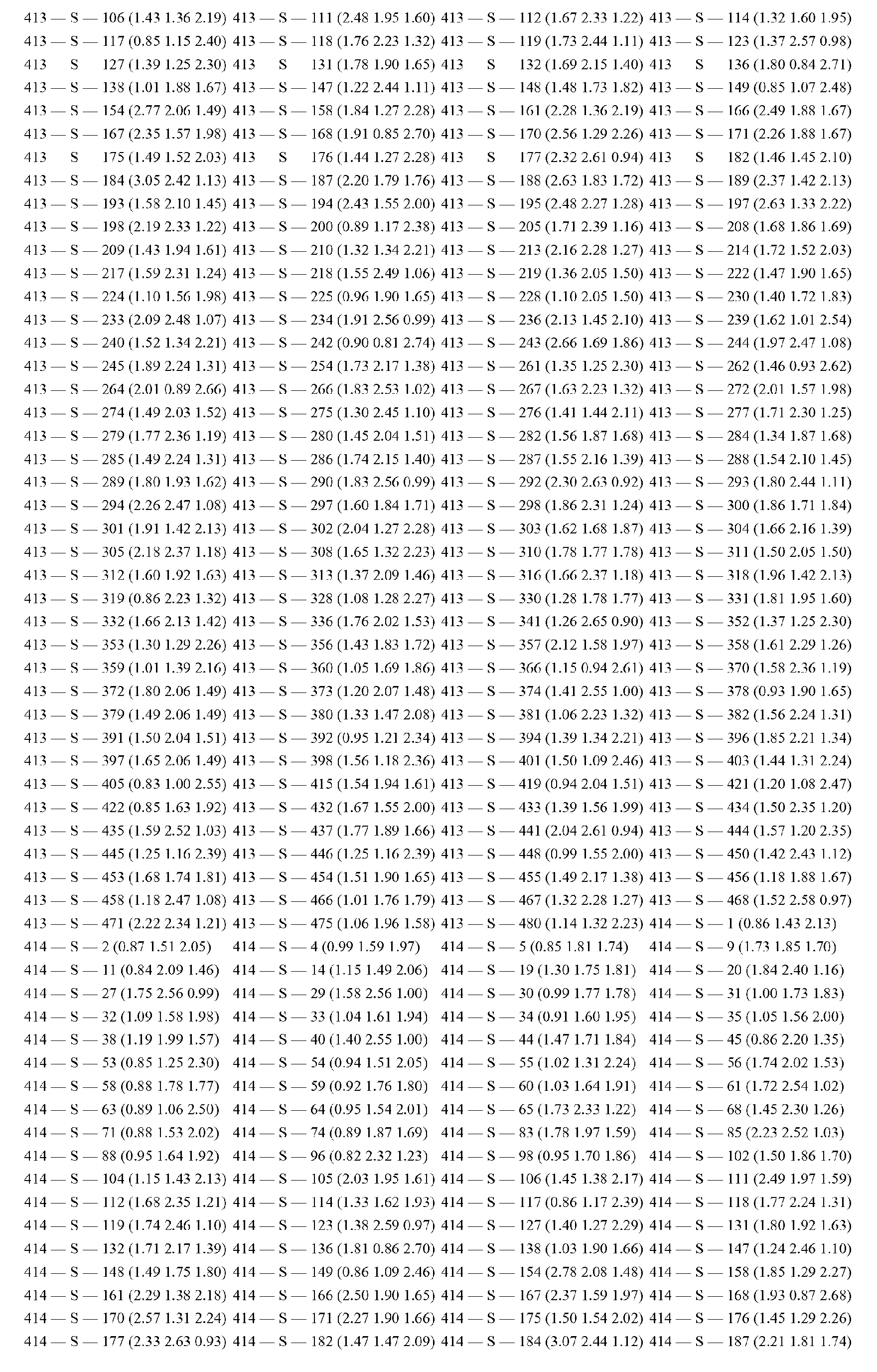

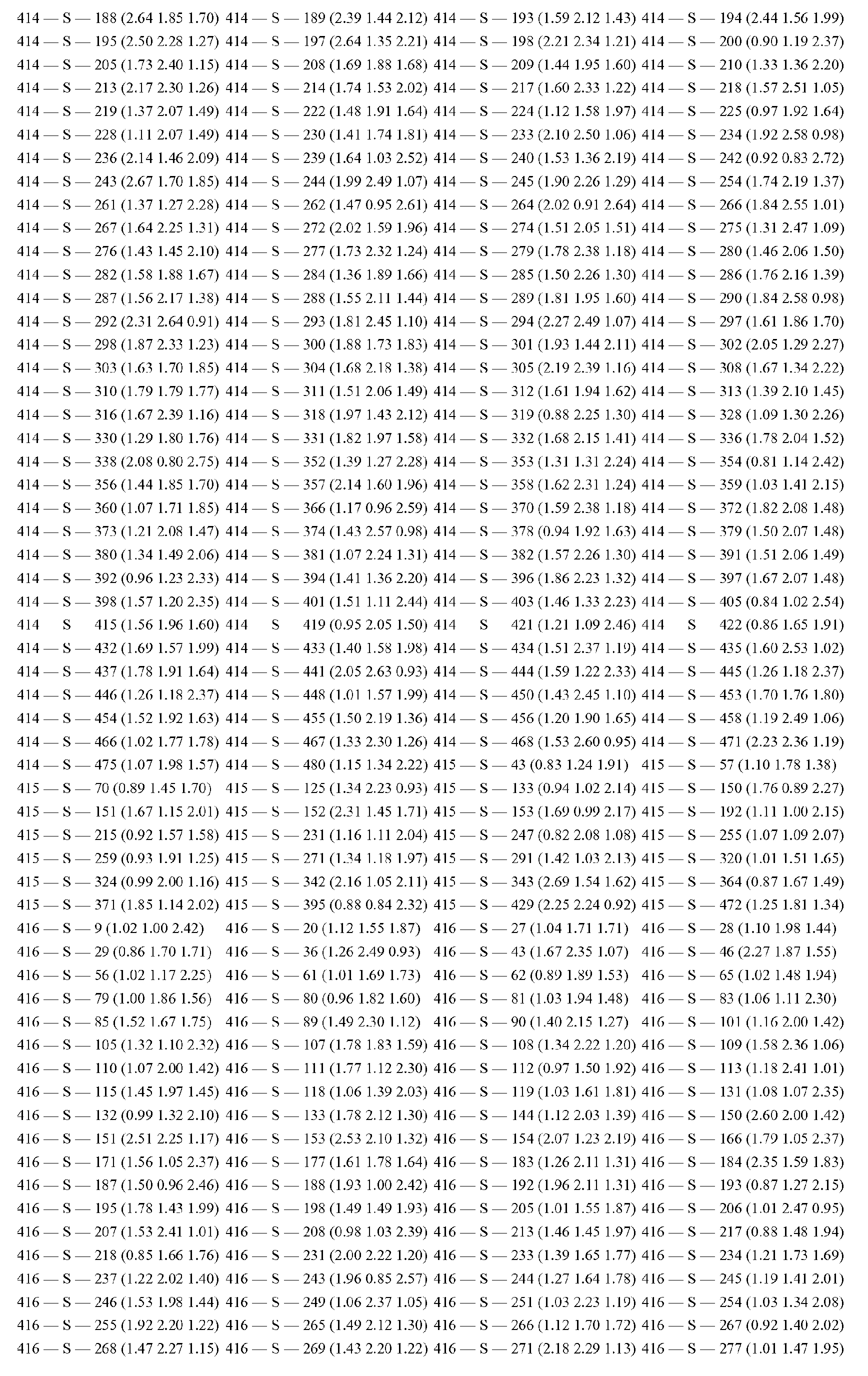

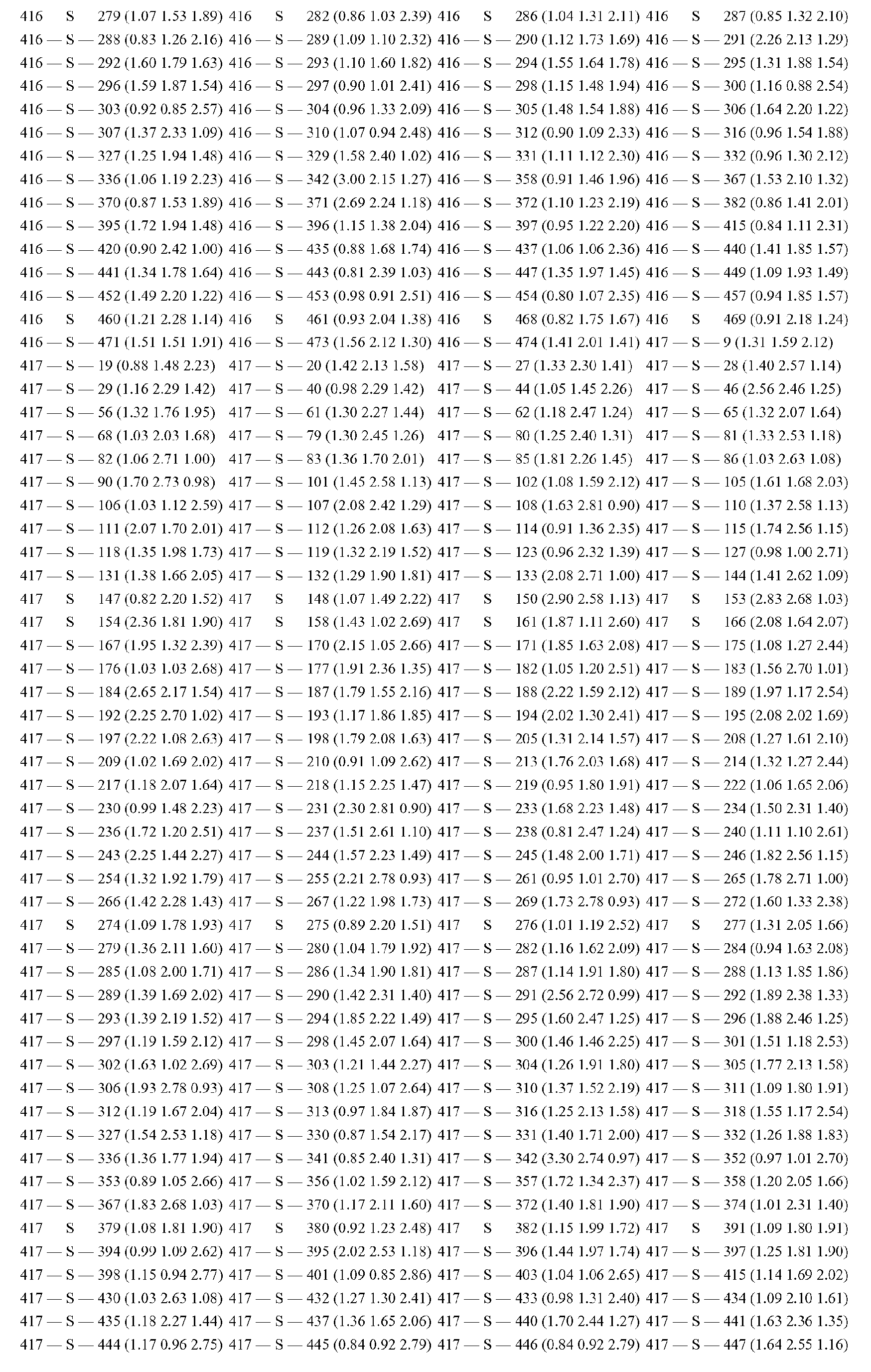

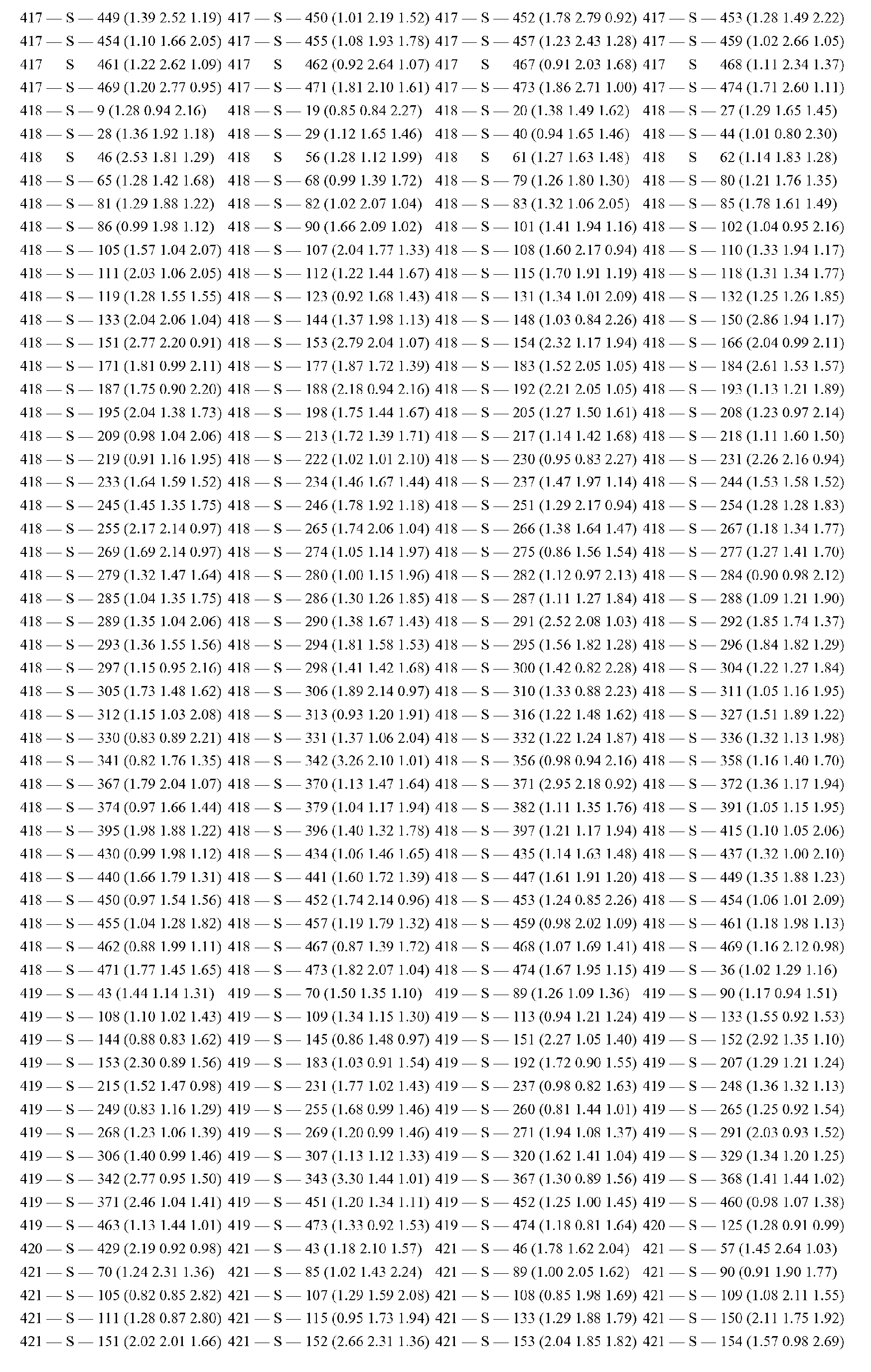

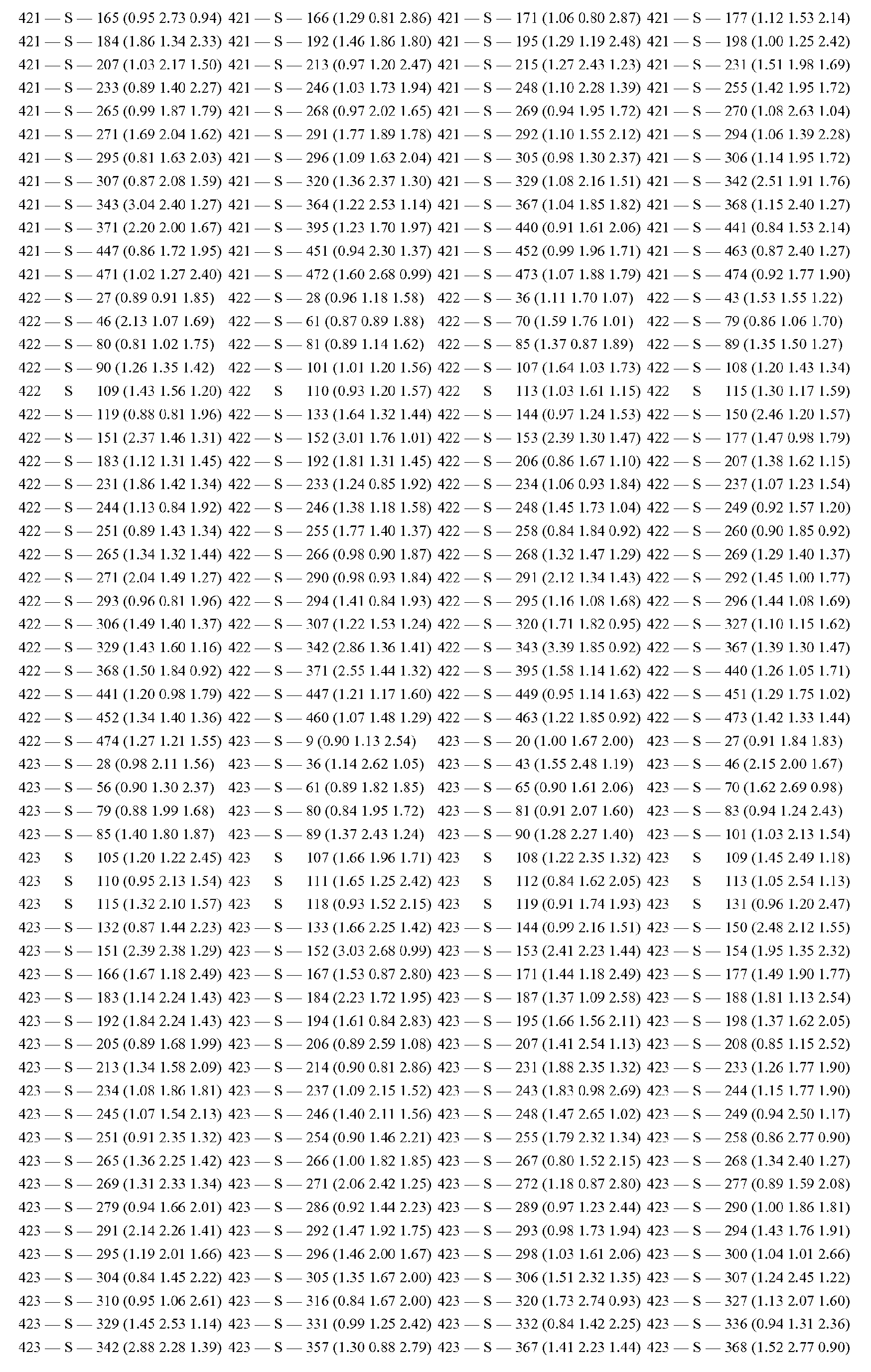

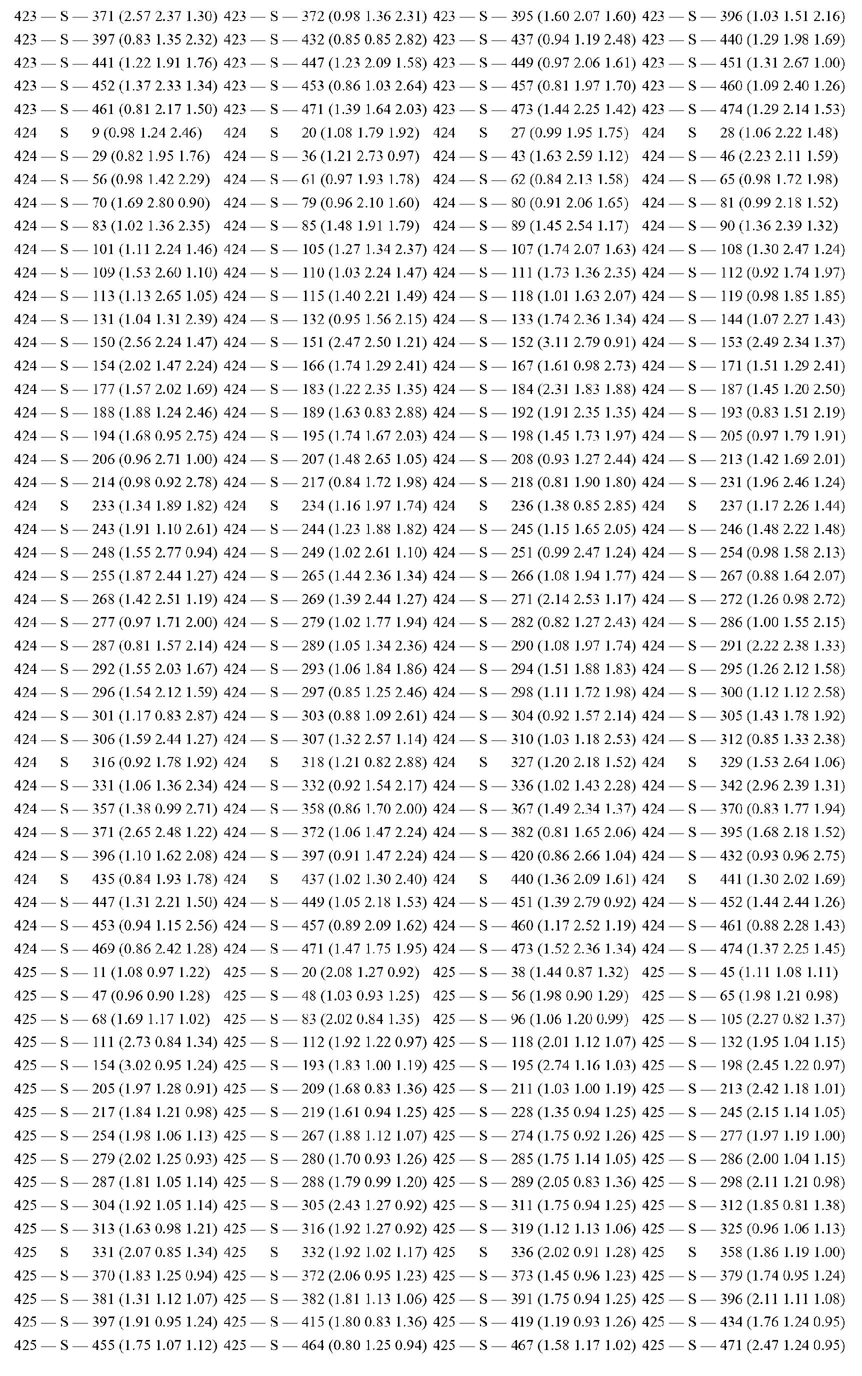

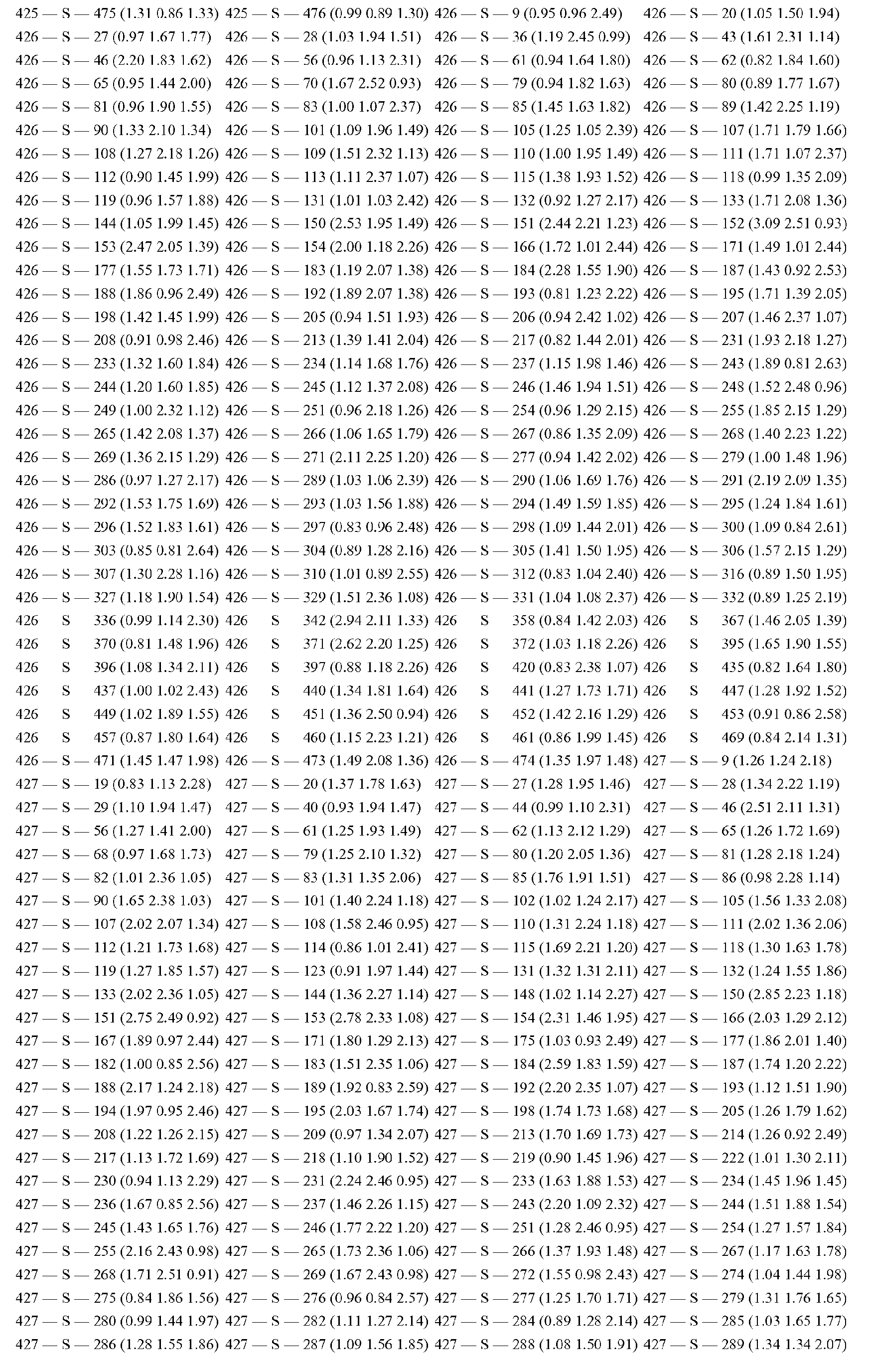

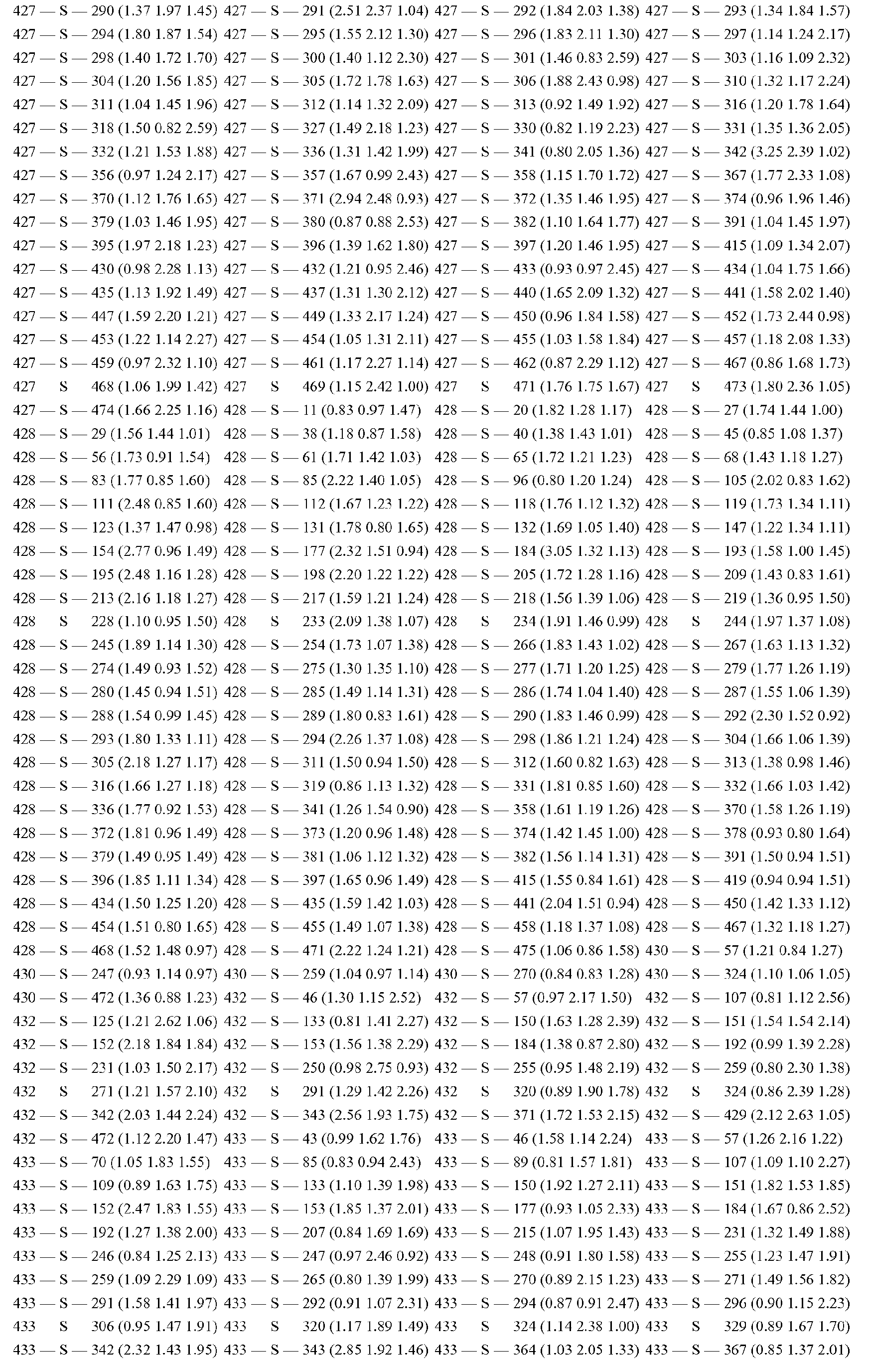

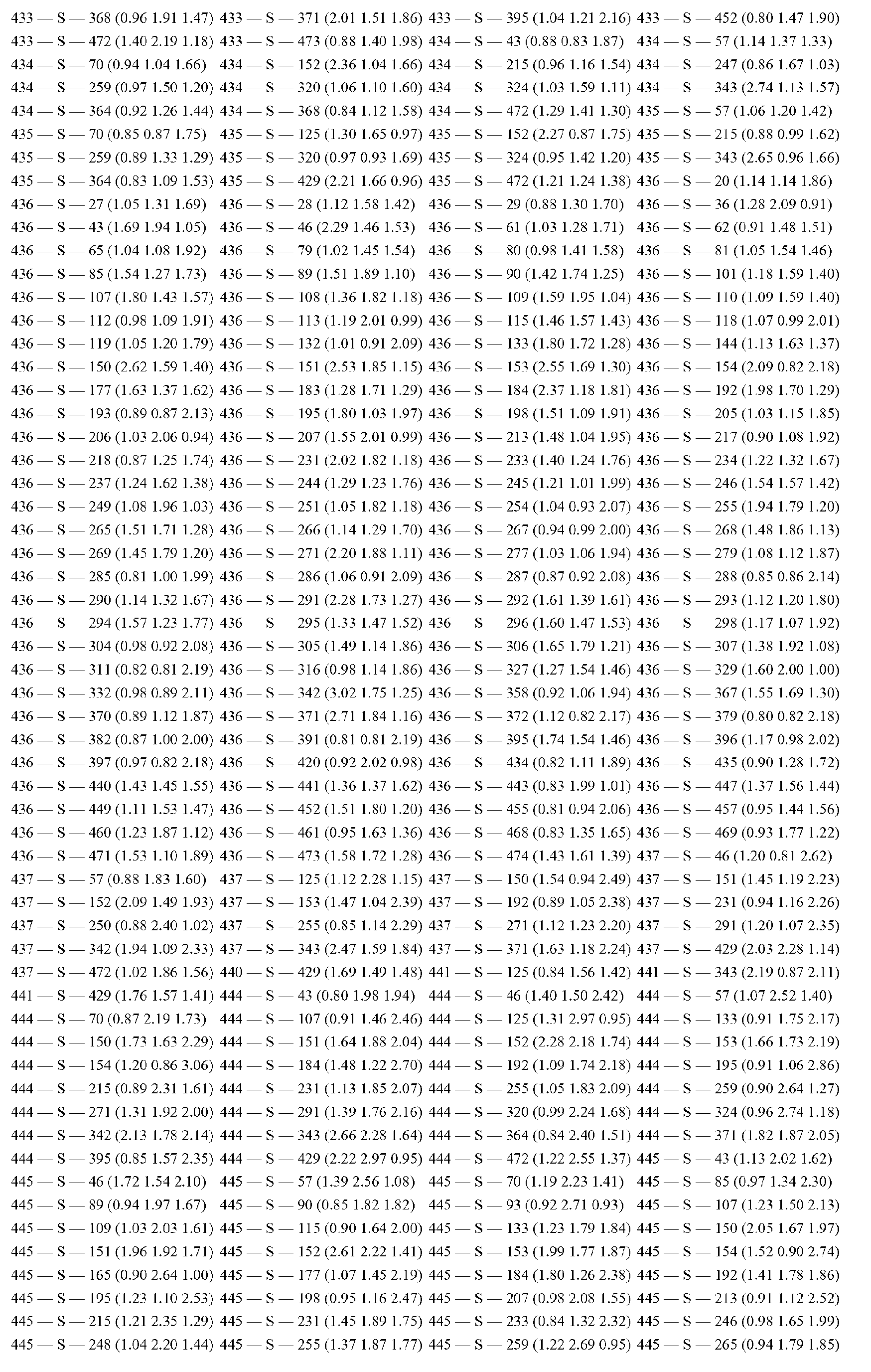

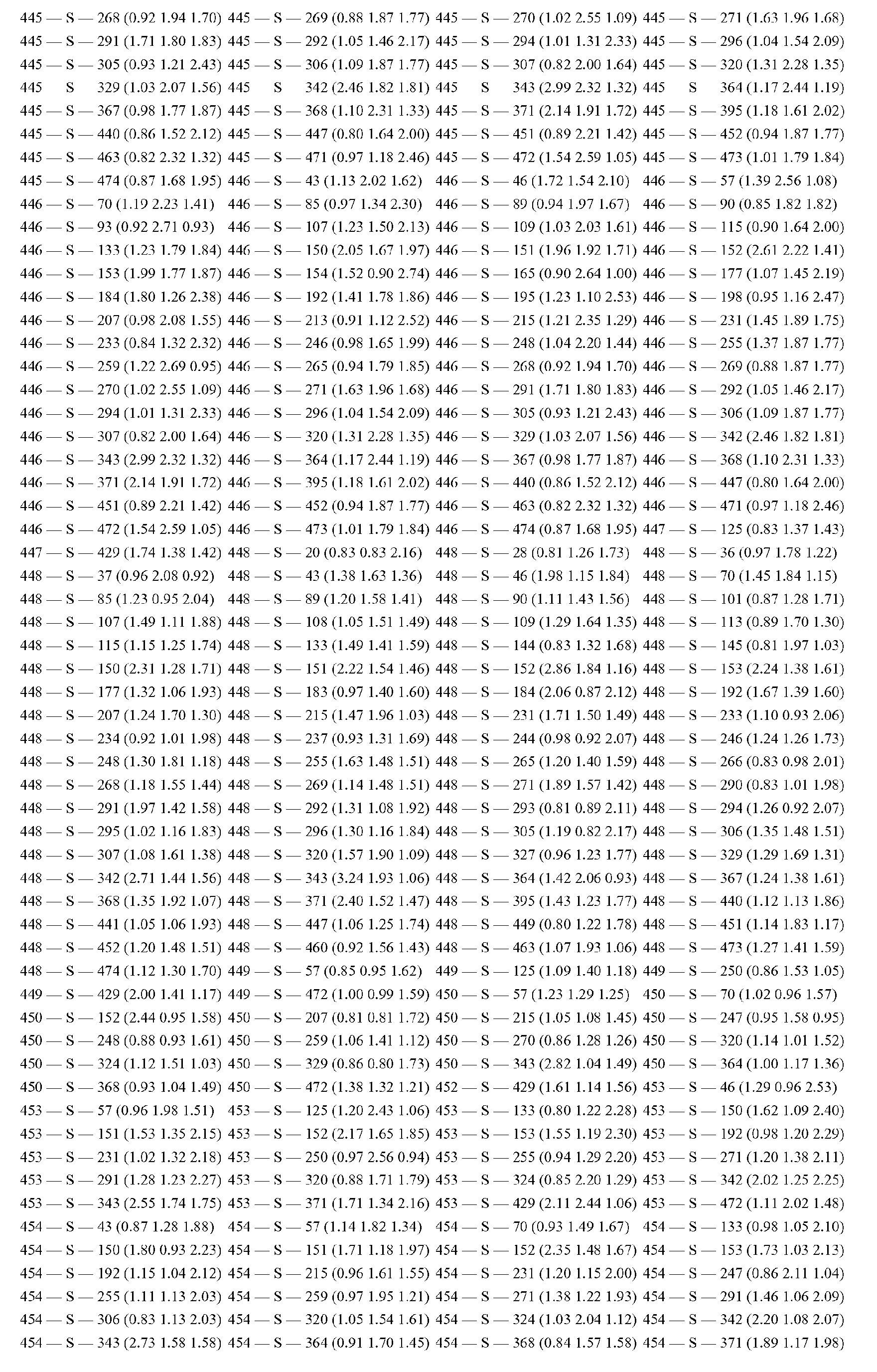

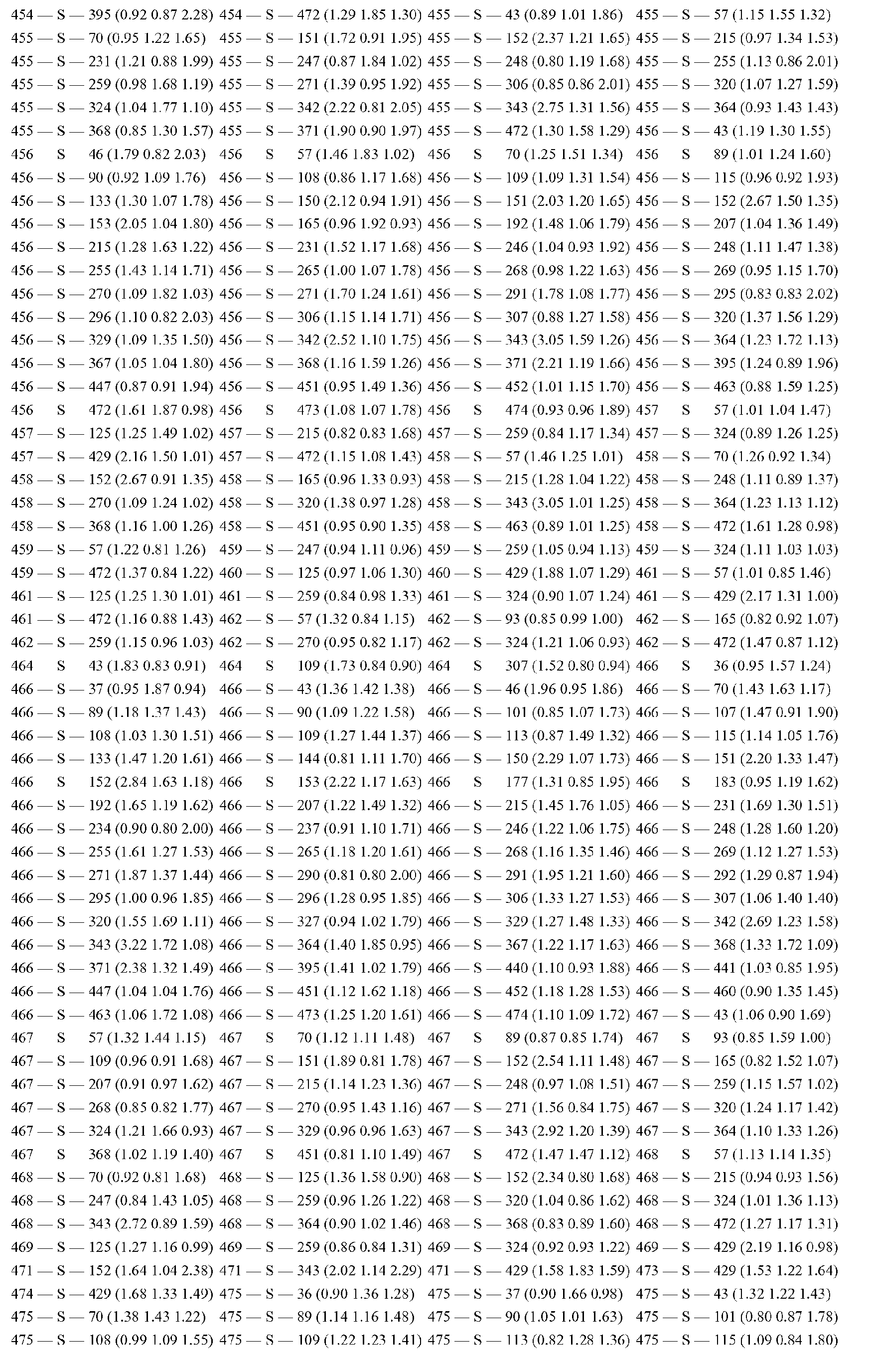

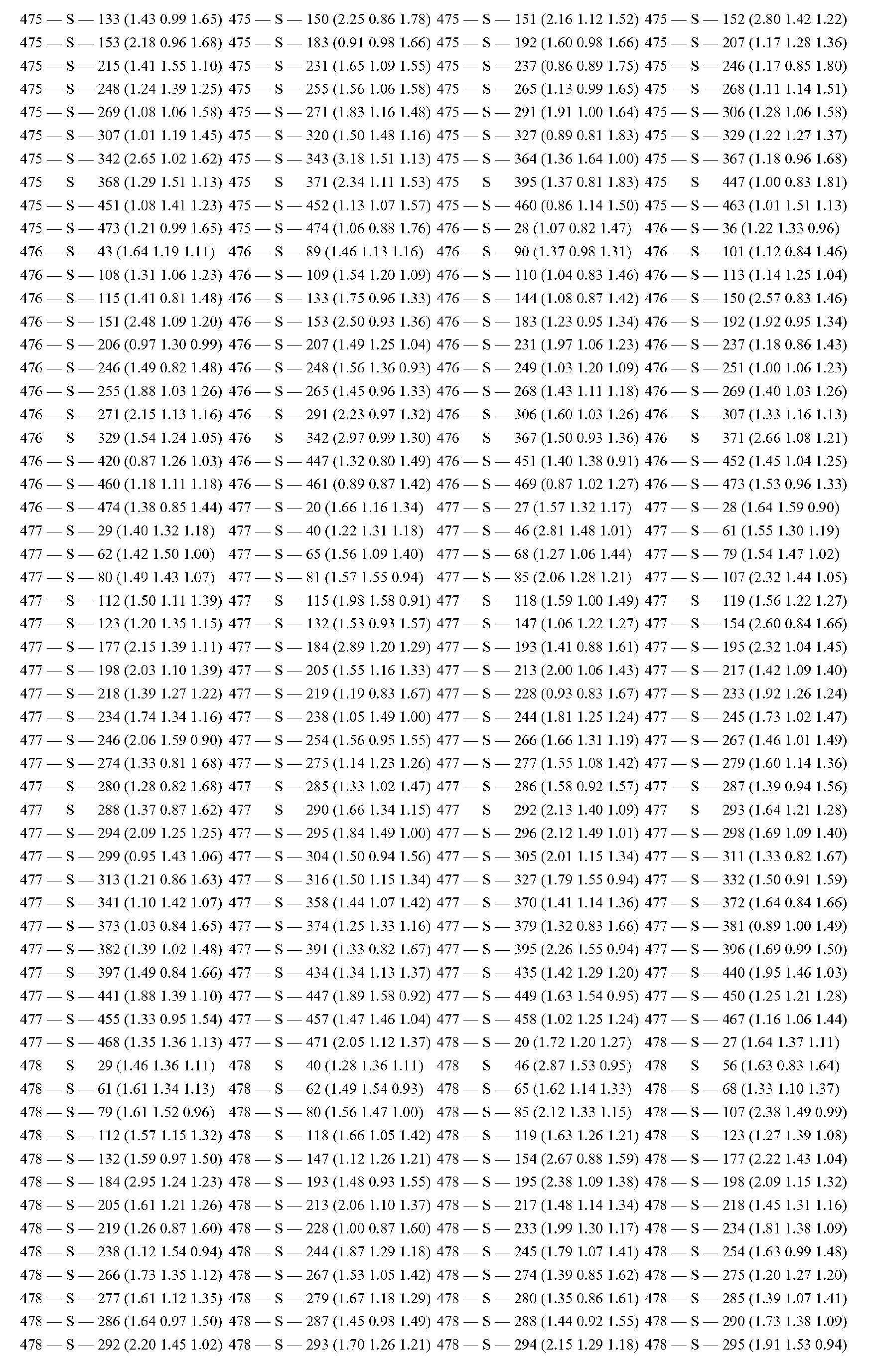

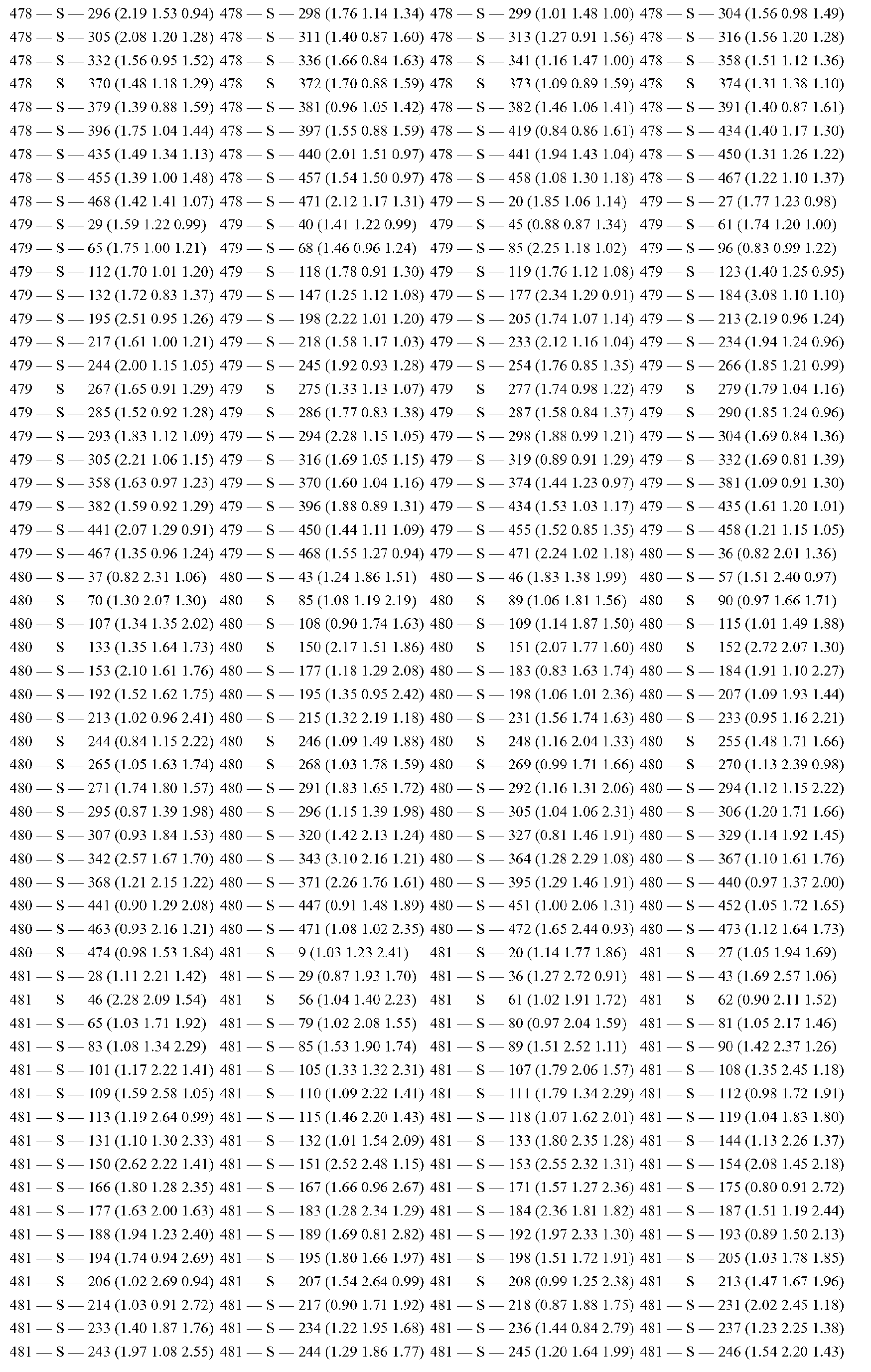

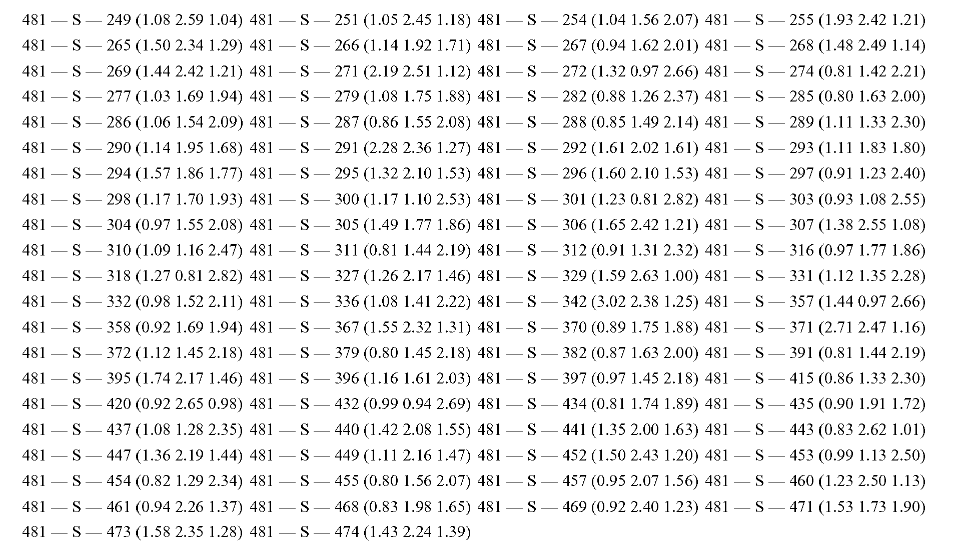

In einer Ausführungsform werden an die chemisch substituierbaren Positionen der so erhaltenen organischen Moleküle weitere Reste R angefügt, insbesondere um die Löslichkeit der Emitter zu steigern und/oder die Polymerisierbarkeit zu ermöglichen ohne dabei die elektronischen Eigenschaften des Moleküls signifikant zu verändern, sodass auch bei Verwendung von R ein Emitter vorliegt, wobei gilt:
jedes R ist bei jedem Auftreten unabhängig voneinander ausgewählt aus der Gruppe bestehend aus H, Deuterium, Phenyl, Naphthyl, F, Cl, Br, I, N(R²)₂, -CN, -NC, -SCN, -CF₃,-NO₂, -OH, C(=O)OH, C(=O)OR , C(=O)N(R³)₂, C(=O)SR³, C(=S)SR³, Si(R⁴)₃, B(OR⁵)₂, B(N(R⁶)₂)₂, C(=O)R³, P(=O)(R⁷)₂, As(=O)(R⁷)₂, P(=S)(R⁷)₂, As(=S)(R⁷)₂, S(=O)R³, S=NR³, S(=O)NR³, S(=O)₂NR³, S(=O)₂R³, O-S(=O)₂R³, SF₅, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R⁹ substituiert sein kann, wobei eine oder mehrere benachbarte CH₂-Gruppen durch-R⁹C=CR⁹-, -C≡C-, bzw. eine benachbarte CH₂-Gruppe durch -Si(R⁴)₂-, -Ge(R⁴)₂-, -Sn(R⁴)₂,-C(=O)-C(=S)-, -C(=Se)-, -C=N-, -C(=O)O-, -C(=O)N(R³)-, -P(=O)(R⁷)-, -As(=O)(R⁷)-,-P(=S)(R⁷)-, -As(=S)(R⁷)-, -S(=O)-, -S(=O)₂-, -NR²-, -O-, oder -S- ersetzt sein können und wobei ein oder mehrere H-Atome durch Deuterium, F, Cl, Br, I, CN, CF₃ oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R⁹ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R⁹ substituiert sein kann, oder eine Kombination dieser Systeme; dabei können zwei oder mehrere dieser Substituenten R auch miteinander ein monozyklisches aliphatisches Ringsystem mit insgesamt fünf oder sechs Ringgliedern bilden.

R² ist bei jedem Auftreten unabhängig voneinander ausgewählt aus der Gruppe bestehend aus H, Deuterium, Phenyl, Naphthyl, CF₃, C(=O)OR³ , C(=O)N(R²)₂, Si(R⁴)₃, C(=O)R³, P(=O)(R⁷)₂, As(=O)(R⁷)₂ P(=S)(R⁷)₂, As(=S)(R⁷)₂, S(=O)R³, S(=O)₂R³, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R⁹ substituiert sein kann, wobei eine oder mehrere benachbarte CH₂-Gruppen durch -R⁹C=CR⁹-, -C≡C-, bzw. eine benachbarte CH₂-Gruppe durch -Si(R⁴)₂-,-Ge(R⁴)₂-, -Sn(R⁴)₂, -C(=O)-, -C(=S)-, -C(=Se)-, -C=N-, -C(=O)O-, -C(=O)N(R³)-, -P(=O)(R⁷)-, - As(=O)(R⁷)-, -P(=S)(R⁷)-, -As(=S)(R⁷)-, -S(=O)-, -S(=O)₂-, -NR²-, -O-, oder -S-ersetzt sein können und wobei ein oder mehrere H-Atome durch Deuterium, F, Cl, Br, I, CN, CF₃ oder NO₂ ersetzt sein können, oder ein aromatisches oder heteroaromatisches Ringsystem mit 5 bis 60 aromatischen Ringatomen, das jeweils durch einen oder mehrere Reste R⁹ substituiert sein kann, oder eine Aryloxy- oder Heteroaryloxygruppe mit 5 bis 60 aromatischen Ringatomen, die durch einen oder mehrere Reste R⁹ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R⁹ substituiert sein kann, oder eine Kombination dieser Systeme; dabei können zwei oder mehrere dieser Substituenten R² auch miteinander ein mono- oder polycyclisches, aliphatisches, aromatisches und/oder benzoannelliertes Ringsystem bilden.

R³ ist bei jedem Auftreten unabhängig voneinander ausgewählt aus der Gruppe bestehend aus H, Deuterium, Phenyl, Naphthyl, CF₃ oder ein aliphatischer, aromatischer und/oder heteroaromatischer Kohlenwasserstoffrest mit 1 bis 20 C-Atomen, in dem auch ein oder mehrere H-Atome durch F oder CF₃ ersetzt sein können; dabei können zwei oder mehrere Substituenten R³ auch miteinander ein mono- oder polycyclisches, aliphatisches Ringsystem bilden.

R⁴ ist bei jedem Auftreten unabhängig voneinander ausgewählt aus der Gruppe bestehend aus H, Deuterium, Phenyl, Naphthyl, N(R²)₂, CN, CF₃, OH, C(=O)OR³, C(=O)N(R³)₂, C(=O)R³, P(=O)(R⁷)₂, As(=O)(R⁷)₂, P(=S)(R⁷)₂, As(=S)(R⁷)₂, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R⁹ substituiert sein kann, wobei eine oder mehrere benachbarte CH₂-Gruppen durch-R⁹C=CR⁹-, -C≡C-, bzw. eine benachbarte CH₂-Gruppe durch -Si(R⁴)₂-, -Ge(R⁴)₂-, -Sn(R⁴)₂,-C(=O)-, -C(=S)-, -C(=Se)-, -C=N-, -C(=O)O-, -C(=O)N(R³)-, -P(=O)(R⁷)-, -As(=O)(R⁷)-,-P(=S)(R⁷)-, -As(=S)(R⁷)-, -S(=O)-, -S(=O)₂-, -NR²-, -O-, oder -S- ersetzt sein können und wobei ein oder mehrere H-Atome durch Deuterium, F, CI, Br, I, CN, CF₃ oder NO₂ ersetzt sein können, oder ein aromatisches oder heteroaromatisches Ringsystem mit 5 bis 60 aromatischen Ringatomen, das jeweils durch einen oder mehrere Reste R⁸ substituiert sein kann, oder eine Aryloxy- oder Heteroaryloxygruppe mit 5 bis 60 aromatischen Ringatomen, die durch einen oder mehrere Reste R⁹ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R⁹ substituiert sein kann, oder eine Kombination dieser Systeme; dabei können zwei oder mehrere dieser Substituenten R⁴ auch miteinander ein mono- oder polycyclisches, aliphatisches, aromatisches und/oder benzoannelliertes Ringsystem bilden.

R⁵ ist bei jedem Auftreten unabhängig voneinander ausgewählt aus der Gruppe bestehend aus Phenyl, Naphthyl, CF₃, C(=O)R³, P(=O)(R⁷)₂, As(=O)(R⁷)₂, eine lineare Alkyl-, Alkoxy-oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R⁹ substituiert sein kann, wobei eine oder mehrere benachbarte CH₂-Gruppen durch-R⁹C=CR⁹-, -C≡C-, bzw. eine benachbarte CH₂-Gruppe durch -Si(R⁴)₂-, -Ge(R⁴)₂-, -Sn(R⁴)₂,-C(=O)-, -C(=S)-, -C(=Se)-, -C=N-, -C(=O)O-, -C(=O)N(R³)-, -P(=O)(R⁷)-, -As(=O)(R⁷)-,-P(=S)(R⁷)-, -As(=S)(R⁷)-, -S(=O)-, -S(=O)₂-, -NR²-, -O-, oder -S- ersetzt sein können und wobei ein oder mehrere H-Atome durch Deuterium, F, Cl, Br, I, CN, CF₃ oder NO₂ ersetzt sein können, oder ein aromatisches oder heteroaromatisches Ringsystem mit 5 bis 60 aromatischen Ringatomen, das jeweils durch einen oder mehrere Reste R⁹ substituiert sein kann, oder eine Aryloxy- oder Heteroaryloxygruppe mit 5 bis 60 aromatischen Ringatomen, die durch einen oder mehrere Reste R⁹ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R⁹ substituiert sein kann, oder eine Kombination dieser Systeme; dabei können zwei oder mehrere dieser Substituenten R⁵ auch miteinander ein mono- oder polycyclisches, aliphatisches, aromatisches und/oder benzoannelliertes Ringsystem bilden.

R⁶ ist bei jedem Auftreten unabhängig voneinander ausgewählt aus der Gruppe bestehend aus Phenyl, Naphthyl, CF₃, Si(R⁴)₃, C(=O)R³, P(=O)(R⁷)₂, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R⁹ substituiert sein kann, wobei eine oder mehrere benachbarte CH₂-Gruppen durch-R⁹C=CR⁹-, -C≡C-, bzw. eine benachbarte CH₂-Gruppe durch-Si(R⁴)₂-, -Ge(R⁴)₂-, -Sn(R⁴)₂,-C(=O)-, -C(=S)-, -C(=Se)-, -C=N-, -C(=O)O-, -C(=O)N(R³)-, -P(=O)(R⁷)-, -As(=O)(R⁷)-,-P(=S)(R⁷)-, -As(=S)(R⁷)-, -S(=O)-, -S(=O)₂-, -NR²-, -O-, oder -S- ersetzt sein können und wobei ein oder mehrere H-Atome durch Deuterium, F, Cl, Br, I, CN, CF₃ oder NO₂ ersetzt sein können, oder ein aromatisches oder heteroaromatisches Ringsystem mit 5 bis 60 aromatischen Ringatomen, das jeweils durch einen oder mehrere Reste R⁹ substituiert sein kann, oder eine Aryloxy- oder Heteroaryloxygruppe mit 5 bis 60 aromatischen Ringatomen, die durch einen oder mehrere Reste R⁹ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R⁹ substituiert sein kann, oder eine Kombination dieser Systeme; dabei können zwei oder mehrere dieser Substituenten R⁶ auch miteinander ein mono- oder polycyclisches, aliphatisches, aromatisches und/oder benzoannelliertes Ringsystem bilden.

R⁷ ist bei jedem Auftreten unabhängig voneinander ausgewählt aus der Gruppe bestehend aus Phenyl, Naphthyl, N(R²)₂, CN, CF₃, C(=O)OR³, C(=O)N(R³)₂, Si(R⁴)₃, C(=O)R³, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R⁹ substituiert sein kann, wobei eine oder mehrere benachbarte CH₂-Gruppen durch -R⁹C=CR⁹-, -C≡C-, bzw. eine benachbarte CH₂-Gruppe durch -Si(R⁴)₂-, -Ge(R⁴)₂-, -Sn(R⁴)₂, -C(=O)-, -C(=S)-, -C(=Se)-, -C=N-, -C(=O)O-,-C(=O)N(R³)-, -P(=O)(R⁷)-, -As(=O)(R⁷)-, -P(=S)(R⁷)-, -As(=S)(R⁷)-, -S(=O)-, -S(=O)₂-, -NR²-,-O-, oder -S- ersetzt sein können und wobei ein oder mehrere H-Atome durch Deuterium, F, Cl, Br, I, CN, CF₃ oder NO₂ ersetzt sein können, oder ein aromatisches oder heteroaromatisches Ringsystem mit 5 bis 60 aromatischen Ringatomen, das jeweils durch einen oder mehrere Reste R⁹ substituiert sein kann, oder eine Aryloxy- oder Heteroaryloxygruppe mit 5 bis 60 aromatischen Ringatomen, die durch einen oder mehrere Reste R⁹ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R³ substituiert sein kann, oder eine Kombination dieser Systeme; dabei können zwei oder mehrere dieser Substituenten R⁷ auch miteinander ein mono- oder polycyclisches, aliphatisches, aromatisches und/oder benzoannelliertes Ringsystem bilden.

R⁸ ist bei jedem Auftreten unabhängig voneinander ausgewählt aus der Gruppe bestehend aus H, Deuterium, Phenyl, Naphthyl, F, CF₃ oder ein aliphatischer, aromatischer und/oder heteroaromatischer Kohlenwasserstoffrest mit 1 bis 20 C-Atomen, in dem auch ein oder mehrere H-Atome durch F oder CF₃ ersetzt sein können; dabei können zwei oder mehrere Substituenten R⁸ auch miteinander ein mono- oder polycyclisches, aliphatisches Ringsystem bilden.

R⁹ ist bei jedem Auftreten unabhängig voneinander ausgewählt aus der Gruppe bestehend aus H, Deuterium, Phenyl, Naphthyl, N(R²)₂, CN, CF₃, NO₂, OH, COOH, C(=O)OR³, C(=O)N(R³)₂, Si(R⁴)₃, B(OR⁵)₂, C(=O)R³, P(=O)(R⁷)₂, P(=S)(R⁷)₂, As(=O)(R⁷)₂, P(=S)(R⁷)₂, S(=O)R³, S(=O)₂R³, OSO₂R³, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R⁸ substituiert sein kann, wobei eine oder mehrere nicht benachbarte CH₂-Gruppen durch -R³C=CR³-, -C≡C-, bzw. eine benachbarte CH₂-Gruppe durch -Si(R⁴)₂-, -Ge(R⁴)₂-, -Sn(R⁴)₂, -C(=O)-, -C(=S)-, -C(=Se)-,-C=N-, -C(=O)O-, -C(=O)N(R³)-, -P(=O)(R⁷)-, -As(=O)(R⁷)-, -P(=S)(R⁷)-, -As(=S)(R⁷)-, -S(=O)-, -S(=O)₂-, -NR²-, -O-, oder -S- ersetzt sein können und wobei ein oder mehrere H-Atome durch Deuterium, F, Cl, Br, I, CN, CF₃ oder NO₂ ersetzt sein können, oder ein aromatisches oder heteroaromatisches Ringsystem mit 5 bis 60 aromatischen Ringatomen, das jeweils durch einen oder mehrere Reste R⁸ substituiert sein kann, oder eine Aryloxy- oder Heteroaryloxygruppe mit 5 bis 60 aromatischen Ringatomen, die durch einen oder mehrere Reste R³ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R⁸ substituiert sein kann, oder eine Kombination dieser Systeme; dabei können zwei oder mehrere dieser Substituenten R⁹ auch miteinander ein mono- oder polycyclisches, aliphatisches, aromatisches und/oder benzoannelliertes Ringsystem.

Polymerisierbare Reste sind solche Reste, die polymerisierbare funktionelle Einheiten tragen, die mit sich selbst homopolymerisiert oder mit anderen Monomeren copolymiersiert werden können. Somit können die erfindungsgemäßen Moleküle als Polymer mit folgenden Wiederholungseinheiten der Formeln 3 und 4 erhalten werden, die als Polymere in der lichtemittierenden Schicht des optoelektronischen Bauelements Verwendung finden können.

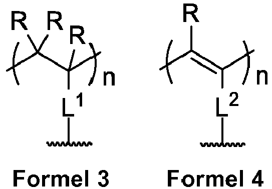

In Formel 3 und 4 stehen L1 und L2 für gleiche oder verschiedene Linkergruppen, die 0 bis 20, bevorzugt 1 bis 15, stärker bevorzugt 2 bis 10 Kohlenstoffatome aufweist, und wobei die gewellte Linie die Position kennzeichnet, über die die Linkergruppe an das organische Molekül der Formel 1 angebunden ist. In einer bevorzugten Ausführungsform weist die Linkergruppe L1 und/oder L2 eine Form -X-L3- auf, wobei X für O oder S steht und L3 für eine Linkergruppe ausgewählt aus der Gruppe aus einer substituierten und unsubstituierten Alkylengruppe (linear, verzweigt oder cyclisch) und einer substituierten und unsubstituierten Arylengruppe, bevorzugt eine substituierte oder unsubstituierte Alkylengruppe mit 1 bis 10 Kohlenstoffatomen oder eine substituierte oder unsubstituierte Phenylengruppe, wobei auch Kombinationen möglich sind. In einer weiteren Ausführungsform weist die Linkergruppe L1 und/oder L2 eine Form -C(=O)O- auf. R ist bei jedem Auftreten unabhängig voneinander ausgewählt aus der Gruppe bestehend aus H, Deuterium, Phenyl, Naphthyl, F, CF₃ oder ein aliphatischer, aromatischer und/oder heteroaromatischer Kohlenwasserstoffrest mit 1 bis 20 C-Atomen, in dem auch ein oder mehrere H-Atome durch F oder CF₃ ersetzt sein können; dabei können zwei oder mehrere Substituenten R auch miteinander ein mono- oder polycyclisches, aliphatisches Ringsystem bilden, außerdem kann R sein: eine lineare Alkoxy-oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy-oder Thioalkoxygruppe mit 3 bis 40 C-Atomen.

Vorteilhafte Ausführungsformen der Wiederholungseinheiten sind Strukturen der Formeln 5 bis 10:

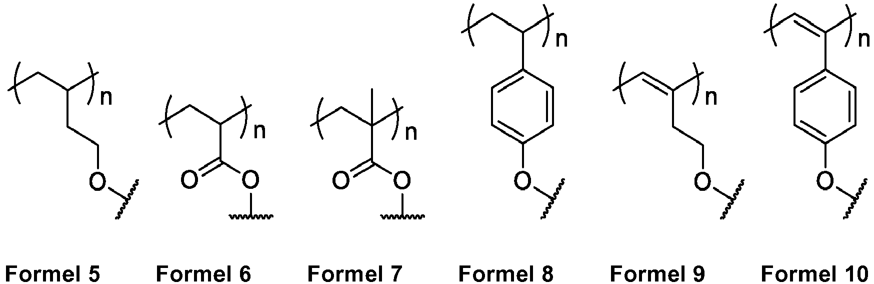

Zur Herstellung der Polymere, die die bevorzugten Wiederholungseinheiten gemäß Formel 5 bis 10 aufweisen, werden die polymerisierbaren funktionellen Einheiten über eine Linkergruppe der Formeln 11 bis 16, die eine Hydroxyleinheit aufweisen, an ein organisches Molekül der Formel 1 angebunden und die daraus resultierenden Verbindungen mit sich selbst homopolymerisiert oder mit anderen geeigneten Monomeren copolymerisiert.

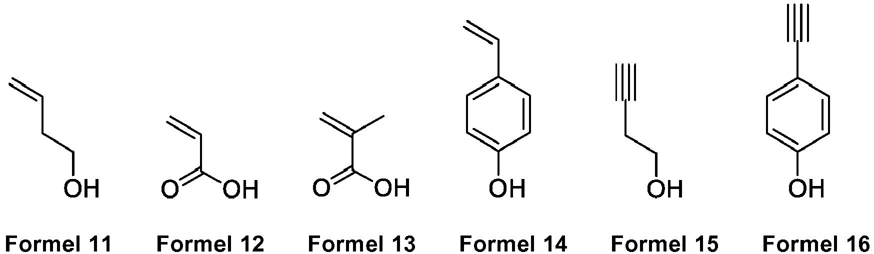

Polymere, die eine Einheit gemäß Formel 3 oder Formel 4 aufweisen, können dabei entweder ausschließlich Wiederholungseinheiten mit einer Struktur der allgemeinen Formel 3 oder 4, oder Wiederholungseinheiten mit einer anderen Struktur aufweisen. Beispiele von Wiederholungseinheiten mit anderen Strukturen weisen Einheiten auf, die sich aus entsprechenden Monomeren ergeben, die typischerweise in Copolymerisationen eingesetzt oder verwendet werden. Beispiele für derartige Wiederholungseinheiten, die sich aus Monomeren ergeben, sind Wiederholungseinheiten, die ungesättigte Einheiten wie Ethylen oder Styrol aufweisen.

Eine Ausführungsform der Erfindung betrifft organischen Moleküle, welche
- einen ΔE(S₁-T₁)-Wert zwischen dem untersten angeregten Singulett (S₁)- und dem darunter liegenden Triplett (T₁)-Zustand von kleiner als 0,2 eV, bevorzugt kleiner als 0,1 eV aufweisen und/oder
- eine Emissionslebensdauer von höchstens 50 µs aufweisen.

Eine weitere Ausführungsform der Erfindung betrifft die Verwendung eines Separators S in Form einer neutralen chemischen Einheit zur Bereitstellung eins erfindungsgemäßen organischen Moleküls, wobei mindestens eine erste chemische Einheit AF1, aufweisend ein konjugiertes System, insbesondere mindestens sechs in Konjugation stehende π-Elektronen und mindestens eine zweite chemische Einheit AF2, aufweisend ein konjugiertes System, insbesondere mindestens sechs in Konjugation stehende π-Elektronen, kovalent über den Separator S derart verknüpft wird, dass der Separator S eine direkte elektronische Wechselwirkung über konjugierte Bindungen zwischen dem konjugierten System der ersten chemischen Einheit AF1 und dem konjugierten System der zweiten chemischen Einheit AF2 unterbindet.

Die Erfindung betrifft in einem Aspekt die Verwendung eines erfindungsgemäßen organischen Moleküls als lumineszierender Emitter und/oder als Hostmaterial und/oder als Elektronentransportmaterial und/oder als Lochinjektionsmaterial und/oder als Lochblockiermaterial in einem optoelektronischen Bauelement, das insbesondere durch ein Vakuumverdampfungsverfahren oder aus Lösung hergestellt wird, wobei das optoelektronische Bauelement insbesondere ausgewählt ist aus der Gruppe bestehend aus:
- organischen lichtemittierenden Dioden (OLEDs),
- lichtemittierenden elektrochemischen Zellen,
- OLED-Sensoren, insbesondere in nicht hermetisch nach außen abgeschirmten Gas- und Dampf-Sensoren,
- organischen Dioden,
- organischen Solarzellen,
- organischen Transistoren,
- organischen Feldeffekttransistoren,
- organischen Lasern und
- Down-Konversions-Elementen.

Der Anteil des erfindungsgemäßen organischen Moleküls am lumineszierenden Emitter und/oder Hostmaterial und/oder Elektronentransportmaterial und/oder Lochinjektionsmaterial und/oder Lochblockiermaterial beträgt in einer Ausführungsform 1 % bis 99 % (Gew%), insbesondere beträgt der Anteil am Emitter in optischen Licht emittierenden Bauelementen, insbesondere in OLEDs, zwischen 5 % und 80 %.

Die Erfindung betrifft in einem weiteren Aspekt optoelektronische Bauelemente, aufweisend ein erfindungsgemäßes organisches Molekül, wobei das optoelektronische Bauelement insbesondere ausgeformt ist als ein Bauelement ausgewählt aus der Gruppe bestehend aus organischer lichtemittierender Diode (OLED), Licht-emittierender elektrochemischer Zelle, OLED-Sensor, insbesondere in nicht hermetisch nach außen abgeschirmten Gas- und Dampf-Sensoren, organischer Diode, organischer Solarzelle, organischem Transistor, organischem Feldeffekttransistor, organischem Laser und Down-Konversion-Element.

Eine Ausführungsform betrifft das erfindungsgemäße optoelektronische Bauelement aufweisend ein Substrat, eine Anode und eine Kathode, wobei die Anode und die Kathode auf das Substrat aufgebracht sind, und mindestens eine lichtemittierende Schicht, welche zwischen Anode und Kathode angeordnet ist und die ein erfindungsgemäßes organisches Molekül enthält.

In einer weiteren Ausführungsform des Bauelements wird das organische Molekül als Emissionsmaterial in einer Emissionsschicht eingesetzt, wobei sie in Kombination mit mindestens einem Hostmaterial oder insbesondere als Reinschicht eingesetzt werden kann. In einer Ausführungsform beträgt dabei der Anteil des organischen Moleküls als Emissionsmaterial in einer Emissionsschicht in optischen Licht-emittierenden Bauelementen, insbesondere in OLEDs, zwischen 5 % und 80 % (Gew%).

In einer weiteren Ausführungsform des erfindungsgemäßen Bauelements ist die ein erfindungsgemäßes organisches Molekül aufweisende lichtemittierende Schicht auf ein Substrat aufgebracht.

In einer Ausführungsform betrifft die Erfindung ein optoelektronisches Bauelement, bei dem die lichtemittierende Schicht ausschließlich ein erfindungsgemäßes organisches Molekül in 100 % Konzentration aufweist, wobei die Anode und die Kathode auf das Substrat aufgebracht ist, und die lichtemittierende Schicht zwischen Anode und Kathode aufgebracht ist.

In einer weiteren Ausführungsform weist das optoelektronische Bauelement neben dem erfindungsgemäßen organischen Molekül mindestens ein Hostmaterial auf, wobei insbesondere der angeregte Singulettzustand (S₁) und/oder der angeregte Triplettzustand (T₁) des mindestens einen Hostmaterials höher ist als der angeregte Singulettzustand (S₁) und/oder der angeregte Triplettzustand (T₁) des organischen Moleküls, und wobei die Anode und die Kathode auf das Substrat aufgebracht ist, und die lichtemittierende Schicht zwischen Anode und Kathode aufgebracht ist.

In einer weiteren Ausführungsform weist das optoelektronische Bauelement ein Substrat, eine Anode, eine Kathode und mindestens je eine löcherinjizierende und eine elektroneninjizierende Schicht und mindestens eine lichtemittierende Schicht auf, wobei die mindestens eine lichtemittierende Schicht ein erfindungsgemäßes organisches Molekül und ein Hostmaterial aufweist, dessen Triplett (T₁)- und Singulett (S₁)-Energieniveaus energetisch höher liegen als die Triplett (T₁)- und Singulett (S₁)-Energieniveaus des organischen Moleküls, und wobei die Anode und die Kathode auf das Substrat aufgebracht ist, und die löcher- und elektroneninjizierende Schicht zwischen Anode und Kathode aufgebracht ist und die lichtemittierende Schicht zwischen löcher- und elektroneninjizierende Schicht aufgebracht ist.

In einer weiteren Ausführungsform weist das optoelektronische Bauelement ein Substrat, eine Anode, eine Kathode und mindestens je eine löcherinjizierende und eine elektroneninjizierende Schicht, und mindestens je eine löchertransportierende und eine elektronentransportierende Schicht, und mindestens eine lichtemittierende Schicht auf, wobei die mindestens eine lichtemittierende Schicht ein erfindungsgemäßes organisches Molekül sowie ein Hostmaterial aufweist, dessen Triplett (T₁)- und Singulett (S₁)-Energieniveaus energetisch höher liegen als die Triplett (T₁)- und Singulett (S₁)-Energieniveaus des organischen Moleküls, und wobei die Anode und die Kathode auf das Substrat aufgebracht ist, und die löcher- und elektroneninjizierende Schicht zwischen Anode und Kathode aufgebracht ist, und die löcher- und elektronentransportierende Schicht zwischen löcher- und elektroneninjizierende Schicht aufgebracht ist, und die lichtemittierende Schicht zwischen löcher- und elektronentransportierende Schicht aufgebracht ist.

In einer weiteren Ausführungsform weist das optoelektronische Bauelement mindestens ein Hostmaterial aus einem Material gemäß Formel 1 auf.

In einer weiteren Ausführungsform des optoelektronischen Bauelements beinhaltet die lichtemittierende Schicht fluoreszente oder phosphoreszente Materialien, welche eine Struktur der Formel 1 aufweisen.

In einer weiteren Ausführungsform des optoelektronischen Bauelements bilden ein organisches Molekül gemäß Formel 1 und ein funktionelles Material, beispielsweise in Form eines weiteren Emitter-Materials, eines Host-Materials, oder eines weiteren organisches Moleküls, welches zur Bildung eines Exciplex mit dem Molekül gemäß Formel 1 befähigt ist, einen Exciplex. Funktionelle Materialien sind beispielsweise Hostmaterialien wie MCP, Elektronentransportmaterialien wie TPBI und Lochtransportmaterialien wie NPD oder MTDATA. Exciplexe sind Addukte aus elektronisch angeregten Molekülen und solchen im elektronischen Grundzustand, die zur Lichtemission fähig sind.

In einer weiteren Ausführungsform des optoelektronischen Bauelements ist die Emission durch thermisch aktivierte verzögerte Fluoreszenz (TADF) charakterisiert.

In einer weiteren Ausführungsform des optoelektronischen Bauelements werden organische Moleküle gemäß Formel 1 als Ladungstransportschicht verwendet.

Die Erfindung betrifft in einem Aspekt ein lichtemittierendes Material, aufweisend ein erfindungsgemäßes organisches Molekül und ein Hostmaterial, wobei die Triplett (T₁)- und Singulett (S₁)-Energieniveaus des Hostmaterials energetisch höher liegen als die Triplett (T₁)- und Singulett (S₁)-Energieniveaus des organischen Moleküls, und wobei das organische Molekül Fluoreszenz oder thermisch aktivierte verzögerte Fluoreszenz (TADF) emittiert, und einen AE(SᵣT₁)-Wert zwischen dem untersten angeregten Singulett (S₁)- und dem darunter liegenden Triplett (T₁)-Zustand von kleiner als 0,2 eV, bevorzugt kleiner als 0,1 eV aufweist.

Ein Aspekt der Erfindung betrifft ein Verfahren zur Herstellung eines optoelektronischen Bauelements aufweisend ein erfindungsgemäßes organisches Molekül. In einer Ausführungsform ist das Verfahren gekennzeichnet durch die Verarbeitung des organischen Moleküls mittels eines Vakuumverdampfungsverfahrens oder aus einer Lösung.

In einer bevorzugten Ausführungsform ist das Verfahren gekennzeichnet durch das Aufbringen des organischen Moleküls auf einen Träger, wobei das Aufbringen insbesondere nass-chemisch, mittels kolloidaler Suspension oder mittels Sublimation erfolgt.

In einer weiteren bevorzugten Ausführungsform des Verfahrens wird mindestens eine Schicht
- mit einem Sublimationsverfahren beschichtet
- mit einem OVPD (Organic Vapor Phase Deposition) Verfahren beschichtet
- mit Hilfe einer Trägergassublimation beschichtet oder
- aus Lösung oder mit einem beliebigen Druckverfahren hergestellt.

Ein Aspekt der Erfindung betrifft ein Verfahren zur Veränderung der Emissions- und/oder Absorptionseigenschaften eines elektronischen Bauelements, wobei ein erfindungsgemäßes organisches Molekül in ein Matrixmaterial zur Leitung von Elektronen oder Löchern in einem optoelektronischen Bauelement eingebracht wird.

Die Erfindung betrifft zudem in einem weiteren Aspekt die Verwendung eines erfindungsgemäßen Moleküls zur Umwandlung von UV-Strahlung oder von blauem Licht in sichtbares Licht, insbesondere in grünes, gelbes oder rotes Licht (Down-Konversion), insbesondere in einem optoelektronischen Bauelement der hier beschriebenen Art.

In einem weiteren Aspekt betrifft die Erfindung eine Anwendung, in der mindestens ein Material mit einer Struktur gemäß Formel 1, durch äußere energetische Anregung zum Leuchten angeregt wird. Die äußere Anregung kann elektronisch oder optisch oder radioaktiv sein.

### Figuren

Es zeigen
- Figur 1:: Schematische Darstellung des Aufbaus einer organischen lichtemittierenden Diode (OLED).
- Figur 2:: Schematische Darstellung des Energieniveaudiagrammes (relative Energie in eV) eines erfindungsgemäßen Emitters (Lichtemission resultiert aus dem Übergang LUMO AF1 hin zu HOMO AF2).

## Patentansprüche

1. Organisches Molekül, aufweisend eine Struktur der Formel 1; 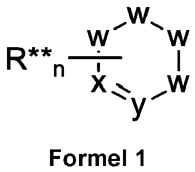 wobei in Formel 1 gilt:
w sind unabhängig voneinander ausgewählt aus sp2-hybridisiertem Kohlenstoff, sp3-hybridisiertem Kohlenstoff, sp2-hybridisiertem Silizium, sp3-hybridisiertem Silizium, sp2-hybridisiertem Stickstoff, sp3-hybridisiertem Stickstoff, oder eine direkte Bindung, wobei in einem erfindungsgemäßen Molekül jeweils nur ein w eine direkte Bindung sein kann und wobei das zyklische Gerüst aus w, x, y maximal 3 Atome aufweist, die nicht gleich Kohlenstoff sind;
x ist ausgewählt aus sp2-hybridisierten Kohlenstoff, sp2-hybridisierten Silizium oder sp2-hybridisierten Stickstoff;
y ist ausgewählt aus sp2-hybridisierten Kohlenstoff, sp2-hybridisierten Silizium oder sp2-hybridisierten Stickstoff;
n kann alle ganzzahligen Werte von 2 bis q annehmen, wobei q der Gesamtzahl der substituierbaren H-Atome des zyklischen Gerüsts aus w, x, y entspricht;
R** ist entweder ein Rest R* oder eine chemische Einheit AF, wobei in Formel 1 mindestens zwei R** gleich AF sein müssen, wobei mindestens zwei unterschiedliche chemische Einheiten AF1 und AF2 enthalten sind, wobei die Einheiten AF ausgewählt sind aus den in Tabelle 3 aufgeführten Strukturen;
R* ist bei jedem Auftreten unabhängig voneinander ausgewählt aus der Gruppe bestehend aus H, Deuterium, Phenyl, Naphthyl, N(R²)₂, -CN, -NC, -SCN, -CF₃, -NO₂, C(=O)OH, C(=O)OR³, C(=O)N(R³)₂, C(=O)SR³, C(=S)SR³, Si(R⁴)₃, B(OR⁵)₂, B(N(R⁶)₂)₂, C(=O)R³, P(=O)(R⁷)₂, As(=O)(R⁷)₂, P(=S)(R⁷)₂, As(=S)(R⁷)₂, S(=O)R³, S=NR³, S(=O)NR³, S(=O)₂NR³, S(=O)₂R³, O-S(=O)₂R³, SF₅, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R⁹ substituiert sein kann, wobei eine oder mehrere benachbarte CH₂-Gruppen durch -R⁹C=CR⁹-, -C≡C-, bzw. eine benachbarte CH₂-Gruppe durch -Si(R⁴)₂-, -Ge(R⁴)₂-, -Sn(R⁴)₂, -C(=O)-, -C(=S)-, -C(=Se)-,-C=N-, -C(=O)O-, -C(=O)N(R³)-, -P(=O)(R⁷)-, -As(=O)(R⁷)-, -P(=S)(R⁷), -As(=S)(R⁷)-, -S(=O)-, -S(=O)₂-, -NR²-, -O-, oder -S- ersetzt sein können und wobei ein oder mehrere H-Atome durch Deuterium, F, Cl, Br, I, CN, CF₃ oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R⁹ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R⁹ substituiert sein kann, oder eine Kombination dieser Systeme; dabei können zwei oder mehrere dieser Substituenten R* auch miteinander ein monozyklisches aliphatisches Ringsystem mit insgesamt fünf oder sechs Ringgliedern bilden;
R² ist bei jedem Auftreten unabhängig voneinander ausgewählt aus der Gruppe bestehend aus H, Deuterium, Phenyl, Naphthyl, CF₃, C(=O)OR³, C(=O)N(R²)₂, Si(R⁴)₃, C(=O)R³, P(=O)(R⁷)₂, As(=O)(R⁷)₂ P(=S)(R⁷)₂, As(=S)(R⁷)₂, S(=O)R³ S(=O)₂R³, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R⁹ substituiert sein kann, wobei eine oder mehrere benachbarte CH₂-Gruppen durch -R⁹C=CR⁹-, -C≡C-, bzw. eine benachbarte CH₂-Gruppe durch -Si(R⁴)₂-,-Ge(R⁴)₂-, -Sn(R⁴)₂, -C(=O)-, -C(=S)-, -C(=Se)-, -C=N-, -C(=O)O-, -C(=O)N(R³)-, -P(=O)(R⁷)-,-As(=O)(R⁷)-, -P(=S)(R⁷)-, -As(=S)(R⁷)-, -S(=O)-, -S(=O)₂-, -NR²-, -O-, oder -S- ersetzt sein können und wobei ein oder mehrere H-Atome durch Deuterium, F, CI, Br, I, CN, CF₃ oder NO₂ ersetzt sein können, oder ein aromatisches oder heteroaromatisches Ringsystem mit 5 bis 60 aromatischen Ringatomen, das jeweils durch einen oder mehrere Reste R⁹ substituiert sein kann, oder eine Aryloxy- oder Heteroaryloxygruppe mit 5 bis 60 aromatischen Ringatomen, die durch einen oder mehrere Reste R⁹ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R⁹ substituiert sein kann, oder eine Kombination dieser Systeme; dabei können zwei oder mehrere dieser Substituenten R² auch miteinander ein mono- oder polycyclisches, aliphatisches, aromatisches und/oder benzoannelliertes Ringsystem bilden;
R³ ist bei jedem Auftreten unabhängig voneinander ausgewählt aus der Gruppe bestehend aus H, Deuterium, Phenyl, Naphthyl, CF₃ oder ein aliphatischer, aromatischer und/oder heteroaromatischer Kohlenwasserstoffrest mit 1 bis 20 C-Atomen, in dem auch ein oder mehrere H-Atome durch F oder CF₃ ersetzt sein können; dabei können zwei oder mehrere Substituenten R³ auch miteinander ein mono- oder polycyclisches, aliphatisches Ringsystem bilden;
R⁴ ist bei jedem Auftreten unabhängig voneinander ausgewählt aus der Gruppe bestehend aus H, Deuterium, Phenyl, Naphthyl, N(R²)₂, CN, CF₃, OH, C(=O)OR³, C(=O)N(R³)₂, C(=O)R³, P(=O)(R⁷)₂, As(=O)(R⁷)₂, P(=S)(R⁷)₂, As(=S)(R⁷)₂, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R⁹ substituiert sein kann, wobei eine oder mehrere benachbarte CH₂-Gruppen durch-R⁹C=CR⁹-, -C≡C-, bzw. eine benachbarte CH₂-Gruppe durch -Si(R⁴)₂-, -Ge(R⁴)₂-, -Sn(R⁴)₂,-C(=O)-, -C(=S)-, -C(=Se)-, -C=N-, -C(=O)O-, -C(=O)N(R³)-, -P(=O)(R⁷)-, -As(=O)(R⁷)-,-P(=S)(R⁷), -As(=S)(R⁷)-, -S(=O)-, -S(=O)₂-, -NR²-, -O-, oder -S- ersetzt sein können und wobei ein oder mehrere H-Atome durch Deuterium, F, CI, Br, I, CN, CF₃ oder NO₂ ersetzt sein können, oder ein aromatisches oder heteroaromatisches Ringsystem mit 5 bis 60 aromatischen Ringatomen, das jeweils durch einen oder mehrere Reste R⁸ substituiert sein kann, oder eine Aryloxy- oder Heteroaryloxygruppe mit 5 bis 60 aromatischen Ringatomen, die durch einen oder mehrere Reste R⁹ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R⁹ substituiert sein kann, oder eine Kombination dieser Systeme; dabei können zwei oder mehrere dieser Substituenten R⁴ auch miteinander ein mono- oder polycyclisches, aliphatisches, aromatisches und/oder benzoannelliertes Ringsystem bilden;
R⁵ ist bei jedem Auftreten unabhängig voneinander ausgewählt aus der Gruppe bestehend aus Phenyl, Naphthyl, CF₃, C(=O)R³, P(=O)(R⁷)₂, As(=O)(R⁷)₂, eine lineare Alkyl-, Alkoxy-oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy-oder Thioalkoxygruppe mit 3 bis 40 C-Atomen, die jeweils mit einem oder mehreren Resten R⁹ substituiert sein kann, wobei eine oder mehrere benachbarte CH₂-Gruppen durch-R⁹C=CR⁹-, -C≡C-, bzw. eine benachbarte CH₂-Gruppe durch -Si(R⁴)₂-, -Ge(R⁴)₂-, -Sn(R⁴)₂,-C(=O)-, -C(=S)-, -C(=Se)-, -C=N-, -C(=O)O-, -C(=O)N(R³)-, -P(=O)(R⁷)-, -As(=O)(R⁷)-, - P(=S)(R⁷)-, -As(=S)(R⁷)-, -S(=O)-, -S(=O)₂-, -NR²-, -O-, oder -S- ersetzt sein können und wobei ein oder mehrere H-Atome durch Deuterium, F, Cl, Br, I, CN, CF₃ oder NO₂ ersetzt sein können, oder ein aromatisches oder heteroaromatisches Ringsystem mit 5 bis 60 aromatischen Ringatomen, das jeweils durch einen oder mehrere Reste R⁹ substituiert sein kann, oder eine Aryloxy- oder Heteroaryloxygruppe mit 5 bis 60 aromatischen Ringatomen, die durch einen oder mehrere Reste R⁹ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R⁹ substituiert sein kann, oder eine Kombination dieser Systeme; dabei können zwei oder mehrere dieser Substituenten R⁵ auch miteinander ein mono- oder polycyclisches, aliphatisches, aromatisches und/oder benzoannelliertes Ringsystem bilden;
R⁶ ist bei jedem Auftreten unabhängig voneinander ausgewählt aus der Gruppe bestehend aus Phenyl, Naphthyl, CF₃, Si(R⁴)₃, C(=O)R³, P(=O)(R⁷)₂, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R⁹ substituiert sein kann, wobei eine oder mehrere benachbarte CH₂-Gruppen durch-R⁹C=CR⁹-, -C≡C-, bzw. eine benachbarte CH₂-Gruppe durch-Si(R⁴)₂-, -Ge(R⁴)₂-, -Sn(R⁴)₂,-C(=O)-, -C(=S)-, -C(=Se)-, -C=N-, -C(=O)O-, -C(=O)N(R³)-, -P(=O)(R⁷)-, -As(=O)(R⁷)-,-P(=S)(R⁷), -As(=S)(R⁷)-, -S(=O)-, -S(=O)₂-, -NR²-, -O-, oder -S- ersetzt sein können und wobei ein oder mehrere H-Atome durch Deuterium, F, Cl, Br, I, CN, CF₃ oder NO₂ ersetzt sein können, oder ein aromatisches oder heteroaromatisches Ringsystem mit 5 bis 60 aromatischen Ringatomen, das jeweils durch einen oder mehrere Reste R⁹ substituiert sein kann, oder eine Aryloxy- oder Heteroaryloxygruppe mit 5 bis 60 aromatischen Ringatomen, die durch einen oder mehrere Reste R* substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R⁹ substituiert sein kann, oder eine Kombination dieser Systeme; dabei können zwei oder mehrere dieser Substituenten R⁶ auch miteinander ein mono- oder polycyclisches, aliphatisches, aromatisches und/oder benzoannelliertes Ringsystem bilden;
R⁷ ist bei jedem Auftreten unabhängig voneinander ausgewählt aus der Gruppe bestehend aus, Phenyl, Naphthyl, N(R²)₂, CN, CF₃, C(=O)OR³, C(=O)N(R³)₂, Si(R⁴)₃, C(=O)R³, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R⁹ substituiert sein kann, wobei eine oder mehrere benachbarte CH₂-Gruppen durch -R⁹C=CR⁹-, -C≡C-, bzw. eine benachbarte CH₂-Gruppe durch -Si(R⁴)₂-, -Ge(R⁴)₂-, -Sn(R⁴)₂, -C(=O)-, -C(=S)-, -C(=Se)-, -C=N-, -C(=O)O-,-C(=O)N(R³)-, -P(=O)(R⁷)-, -As(=O)(R⁷)-, -P(=S)(R⁷), -As(=S)(R⁷)-, -S(=O)-, -S(=O)₂-, -NR²-,-O-, oder -S- ersetzt sein können und wobei ein oder mehrere H-Atome durch Deuterium, F, CI, Br, I, CN, CF₃ oder NO₂ ersetzt sein können, oder ein aromatisches oder heteroaromatisches Ringsystem mit 5 bis 60 aromatischen Ringatomen, das jeweils durch einen oder mehrere Reste R⁹ substituiert sein kann, oder eine Aryloxy- oder Heteroaryloxygruppe mit 5 bis 60 aromatischen Ringatomen, die durch einen oder mehrere Reste R⁹ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R³ substituiert sein kann, oder eine Kombination dieser Systeme; dabei können zwei oder mehrere dieser Substituenten R⁷ auch miteinander ein mono- oder polycyclisches, aliphatisches, aromatisches und/oder benzoannelliertes Ringsystem bilden;
R⁸ ist bei jedem Auftreten unabhängig voneinander ausgewählt aus der Gruppe bestehend aus H, Deuterium, Phenyl, Naphthyl, F, CF₃ oder ein aliphatischer, aromatischer und/oder heteroaromatischer Kohlenwasserstoffrest mit 1 bis 20 C-Atomen, in dem auch ein oder mehrere H-Atome durch F oder CF₃ ersetzt sein können; dabei können zwei oder mehrere Substituenten R⁸ auch miteinander ein mono- oder polycyclisches, aliphatisches Ringsystem bilden;
R⁹ ist bei jedem Auftreten unabhängig voneinander ausgewählt aus der Gruppe bestehend aus H, Deuterium, Phenyl, Naphthyl, N(R²)₂, CN, CF₃, NO₂, OH, COOH, C(=O)OR³, C(=O)N(R³₎2, Si(R⁴)₃, B(OR⁵)₂, C(=O)R³, P(=O)(R⁷)₂, P(=S)(R⁷)₂, As(=O)(R⁷)₂, P(=S)(R⁷)₂, S(=O)R³, S(=O)₂R³, OSO₂R³, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R⁸ substituiert sein kann, wobei eine oder mehrere nicht benachbarte CH₂-Gruppen durch -R³C=CR³-, -C≡C-, bzw. eine benachbarte CH₂-Gruppe durch -Si(R⁴)₂-, -Ge(R⁴)₂-, -Sn(R⁴)₂, -C(=O)-, -C(=S)-, -C(=Se)-,-C=N-, -C(=O)O-, -C(=O)N(R³)-, -P(=O)(R⁷)-, -As(=O)(R⁷)-, -P(=S)(R⁷), -As(=S)(R⁷)-, -S(=O)-, -S(=O)₂-, -NR²-, -O-, oder -S- ersetzt sein können und wobei ein oder mehrere H-Atome durch Deuterium, F, CI, Br, I, CN, CF₃ oder NO₂ ersetzt sein können, oder ein aromatisches oder heteroaromatisches Ringsystem mit 5 bis 60 aromatischen Ringatomen, das jeweils durch einen oder mehrere Reste R⁸ substituiert sein kann, oder eine Aryloxy- oder Heteroaryloxygruppe mit 5 bis 60 aromatischen Ringatomen, die durch einen oder mehrere Reste R³ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R⁸ substituiert sein kann, oder eine Kombination dieser Systeme; dabei können zwei oder mehrere dieser Substituenten R⁹ auch miteinander ein mono- oder polycyclisches, aliphatisches, aromatisches und/oder benzoannelliertes Ringsystem bilden;
wobei insbesondere
- die Differenz der Energie des HOMO der zweiten chemischen Einheit AF2 und der Energie des HOMO der ersten chemischen Einheit AF1 > 0,8 eV ist (Δ HOMO = HOMO(AF2)-HOMO(AF1) > 0,8 eV);
- die Differenz der Energie des LUMO der zweiten chemischen Einheit AF2 und der Energie des LUMO der ersten chemischen Einheit AF1 > 0,8 eV ist (Δ LUMO = LUMO(AF2)-LUMO(AF1) > 0,8 eV); und/oder
- die Differenz der Energie des LUMO der ersten chemischen Einheit AF1 und der Energie des HOMO der zweiten chemischen Einheit AF2 > 0,9 eV ist (Δ Gap = LUMO (AF1)-HOMO(AF2)> 0,9 eV).

2. Organisches Molekül nach Anspruch 1, wobei das organische Molekül eine Struktur der Formel 2a oder 2b aufweist; 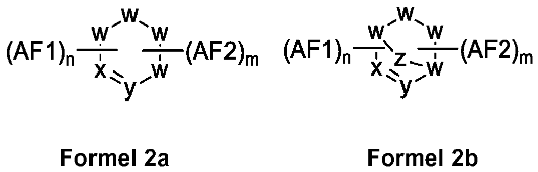 wobei die bei Formel 1 angegebenen Definitionen für w, x, y gelten;
wobei gilt: n ist 1 bis q-1, m ist 1 bis q-1, wobei q die Gesamtzahl der substituierbaren H-Atome des zyklischen Gerüsts aus w, x, y ist;
und wobei jedes substituierbare H-Atom der erfindungsgemäßen Moleküle optional durch einen Rest R* substituiert ist;
und wobei z ein sp3-hybridisiertes Kohlenstoffatom (-CR¹⁰₂-), sp3-hybridisiertes Stickstoffatom (-NR'⁰-), sp3-hybridisiertes Sauerstoffatom oder sp3-hybridisiertes Schwefelatom sein kann;
und wobei im Falle z = (-NR¹⁰-) oder z = (-CR¹⁰₂-) gilt: R¹⁰ ist bei jedem Auftreten unabhängig voneinander ausgewählt aus der Gruppe bestehend aus H, Deuterium, Phenyl, Naphthyl, F, CF₃ oder ein aliphatischer, aromatischer und/oder heteroaromatischer Kohlenwasserstoffrest mit 1 bis 20 C-Atomen, in dem auch ein oder mehrere H-Atome durch F oder CF₃ ersetzt sein können; dabei können zwei oder mehrere Substituenten R auch miteinander ein mono- oder polycyclisches, aliphatisches Ringsystem bilden, außerdem kann R sein: eine lineare Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen.

3. Organisches Molekül nach Anspruch 1 oder 2, wobei das Molekül ausgewählt ist aus den Strukturen 2-1 bis 2-82, wobei die in Anspruch 1 und/oder Anspruch 2 angegebenen Definitionen gelten und die Moleküle 2-1 bis 2-45 optional eine Brücke z aufweisen, wobei z wie in Anspruch 2 definiert ist;

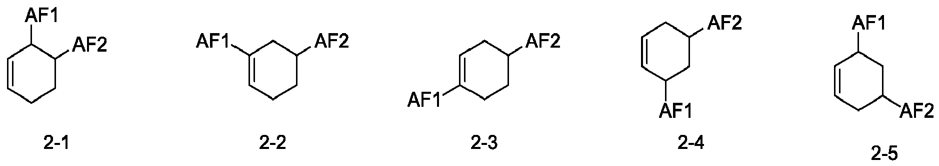

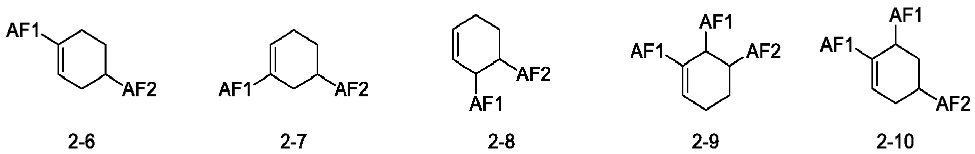

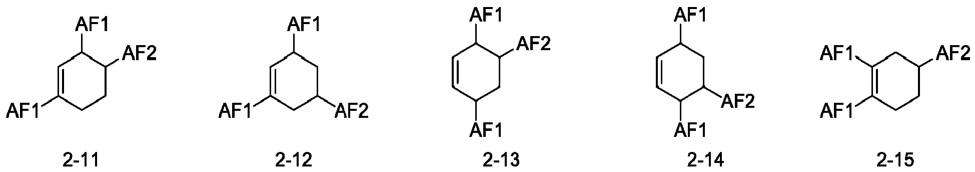

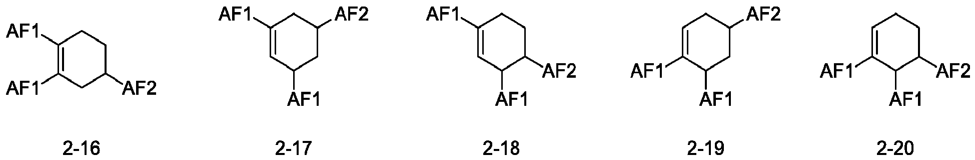

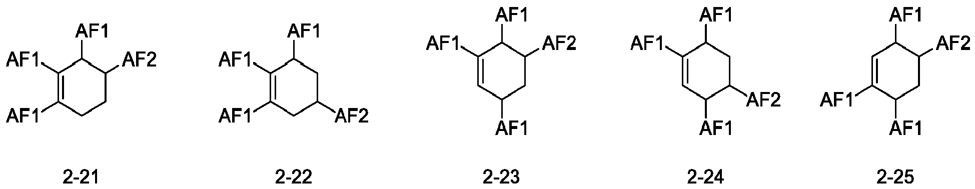

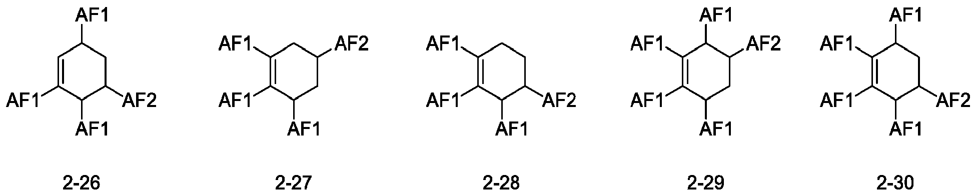

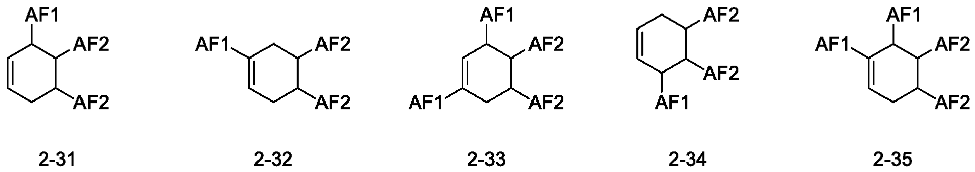

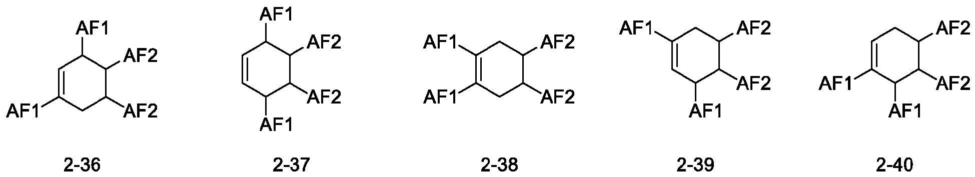

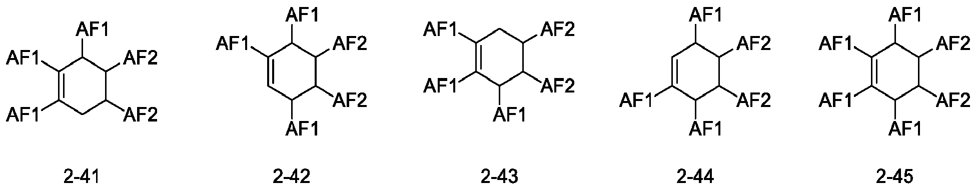

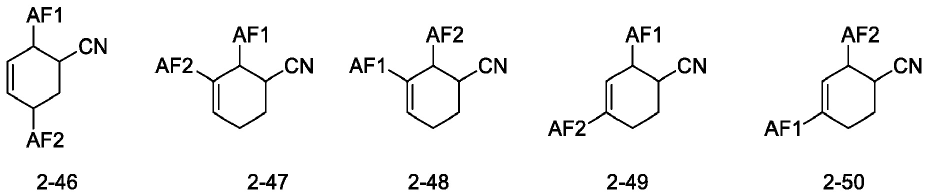

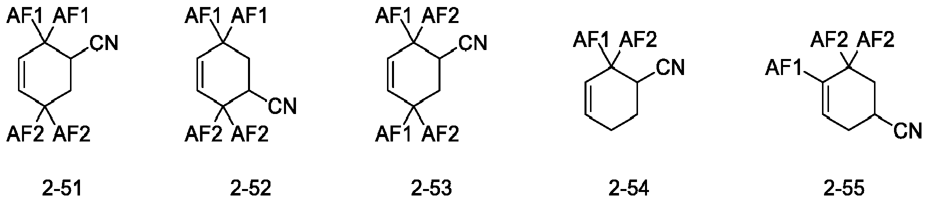

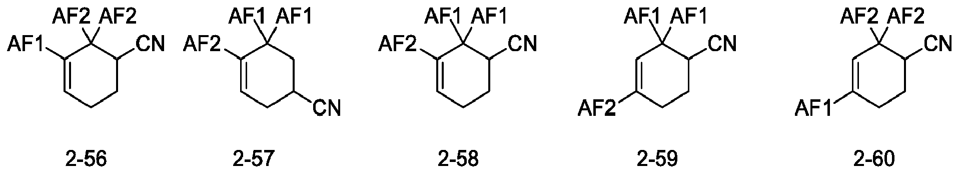

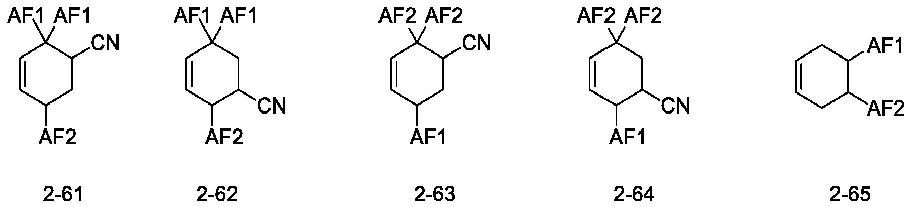

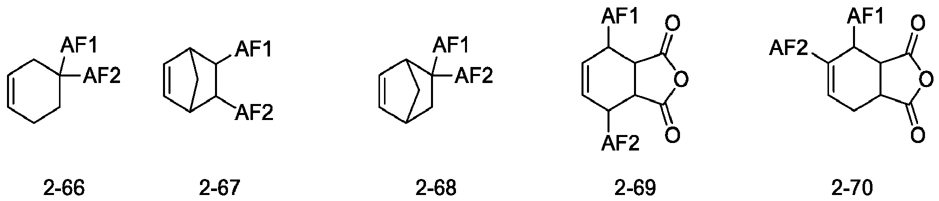

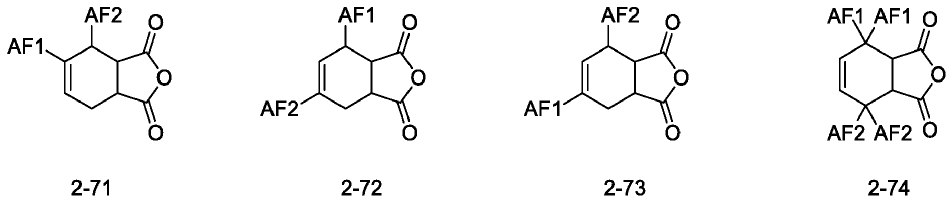

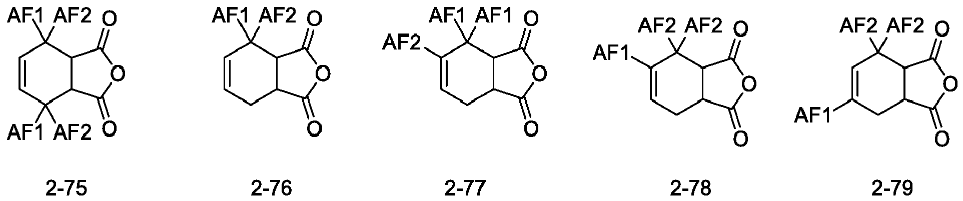

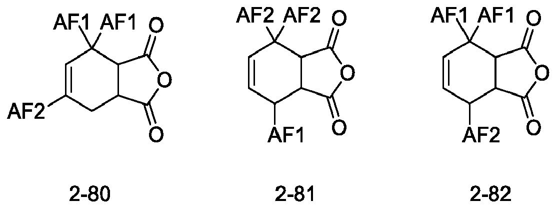

4. Organisches Molekül nach Anspruch 1 bis 2, wobei das organische Molekül einen Separator S aufweist, der ausgewählt ist aus den folgenden Verbindungen, wobei jedes substituierbare H-Atom der Separator-Einheit durch mindestens eine chemische Einheit AF1 und mindestens eine Einheit AF2 ersetzt ist und wobei optional jedes weitere substituierbare H-Atom des resultierenden Moleküls durch einen Rest R* ersetzt ist:

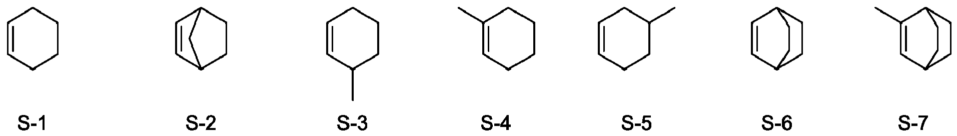

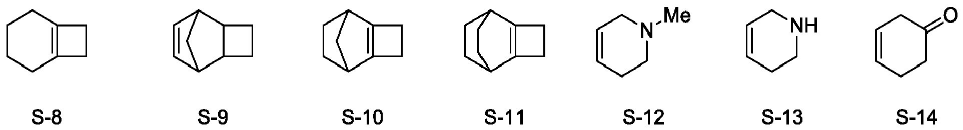

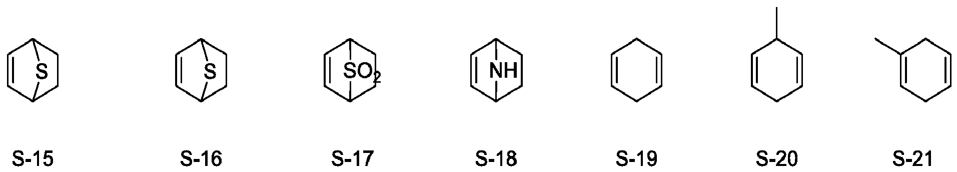

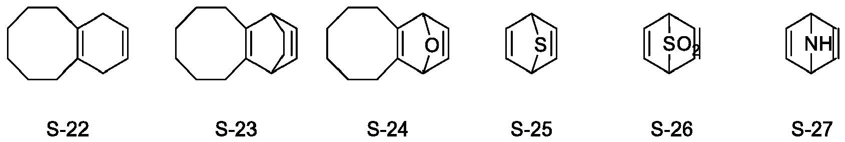

5. Organisches Molekül nach Anspruch 1 bis 4, wobei das organische Molekül an mindestens einer chemisch substituierbaren Position mindestens einen weiteren Rest R, insbesondere zur Steigerung der Löslichkeit und/oder der Polymerisierbarkeit, aufweist, wobei gilt:
R ist bei jedem Auftreten unabhängig voneinander ausgewählt aus der Gruppe bestehend aus H, Deuterium, Phenyl, Naphthyl, F, CI, Br, I, N(R²)₂, -CN, -NC, -SCN,-CF₃, -NO₂, -OH, C(=O)OH, C(=O)OR³, C(=O)N(R³)₂, C(=O)SR³ , C(=S)SR³, Si(R⁴)₃, B(OR⁵)₂, B(N(R⁶)₂)₂, C(=O)R³, P(=O)(R⁷)₂, As(=O)(R⁷)₂, P(=S)(R⁷)₂, As(=S)(R⁷)₂, S(=O)R³, S=NR³, S(=O)NR³, S(=O)₂NR³, S(=O)₂R³, O-S(=O)₂R³, SF₅, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R⁹ substituiert sein kann, wobei eine oder mehrere benachbarte CH₂-Gruppen durch -R⁹C=CR⁹-, -C≡C-, bzw. eine benachbarte CH₂-Gruppe durch -Si(R⁴)₂-, -Ge(R⁴)₂-, -Sn(R⁴)₂, -C(=O)-, -C(=S)-,-C(=Se)-, -C=N-, -C(=O)O-, -C(=O)N(R³)-, -P(=O)(R⁷)-, -As(=O)(R⁷)₂-, -P(=S)(R⁷)₂,-As(=S)(R⁷)₂-, -S(=O)-, -S(=O)₂-, -NR²-, -O-, oder -S- ersetzt sein können und wobei ein oder mehrere H-Atome durch Deuterium, F, Cl, Br, I, CN, CF₃ oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R⁹ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R⁹ substituiert sein kann, oder eine Kombination dieser Systeme; dabei können zwei oder mehrere dieser Substituenten R auch miteinander ein monozyklisches aliphatisches Ringsystem mit insgesamt fünf oder sechs Ringgliedern bilden; wobei R² bis R⁹ definiert sind wie in Anspruch 1;
und wobei für polymerisierbare Reste gilt:
polymerisierbare Reste sind solche Reste, die polymerisierbare funktionelle Einheiten tragen, die mit sich selbst homopolymerisiert oder mit anderen Monomeren copolymiersiert werden können, wodurch das organische Molekül als Polymer mit Wiederholungseinheiten nach den Formeln 3 und/oder 4 erhalten werden 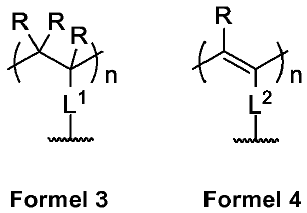 mit
gewellte Linie = Position, über die die Linkergruppe L¹ oder L² an das organische Molekül gebunden ist;
L¹ und L² = gleiche oder verschiedene Linkergruppen, aufweisend zwischen 0 bis 20,
bevorzugt 1 bis 15, besonders bevorzugt 2 bis 10 Kohlenstoffatome; oder aufweisend eine Form -C(=O)O;
und wobei besonders bevorzugt
L¹ und L² = -X-L³ mit
X = O oder S;
L³ = eine weitere Linkergruppe ausgewählt aus der Gruppe aus einersubstituierten und unsubstituierten Alkylengruppe (linear, verzweigt oder cyclisch) und einer substituierten und unsubstituierten Arylengruppe, bevorzugt eine substituierte oder
unsubstituierte Alkylengruppe mit 1 bis 10 Kohlenstoffatomen oder eine substituierte oder unsubstituierte Phenylengruppe, wobei auch Kombinationen möglich sind.

6. Verwendung eines organischen Moleküls nach Anspruch 1 bis 5 als lumineszierender Emitter und/oder als Hostmaterial und/oder als Elektronentransportmaterial und/oder als Lochinjektionsmaterial und/oder als Lochblockiermaterial in einem optoelektronischen Bauelement.

7. Verwendung nach Anspruch 6, wobei das optoelektronische Bauelement ausgewählt ist aus der Gruppe bestehend aus:
• organischen lichtemittierenden Dioden (OLEDs),
• lichtemittierenden elektrochemischen Zellen,
• OLED-Sensoren, insbesondere in nicht hermetisch nach außen abgeschirmten Gas- und Dampf-Sensoren,
• organischen Dioden,
• organischen Solarzellen,
• organischen Transistoren,
• organischen lichtemittierenden Dioden,
• organischen Feldeffekttransistoren,
• organischen Lasern und
• Down-Konversions-Elementen.

8. Verwendung nach Anspruch 6 oder 7, wobei der Anteil des organischen Moleküls am Emitter 1 % bis 99 % beträgt.

9. Verwendung nach Anspruch 6, 7 oder 8, wobei die Konzentration des organischen Moleküls als Emitter in optischen Licht emittierenden Bauelementen, insbesondere in OLEDs, zwischen 5 % und 80 % beträgt.

10. Optoelektronisches Bauelement, aufweisend ein organisches Molekül nach Anspruch 1 bis 5, insbesondere ausgeformt als ein Bauelement ausgewählt aus der Gruppe bestehend aus organischer lichtemittierender Diode (OLED), lichtemittierender elektrochemischer Zelle, OLED-Sensor, insbesondere in nicht hermetisch nach außen abgeschirmten Gas- und Dampf-Sensoren, organischer Diode, organischer Solarzelle, organischem Transistor, organischem Feldeffekttransistor, organischem Laser und Down-Konversion-Element.

11. Optoelektronisches Bauelement nach Anspruch 10, aufweisend ein Substrat, eine Anode und eine Kathode, wobei die Anode und die Kathode auf das Substrat aufgebracht sind, und mindestens eine lichtemittierende Schicht, welche zwischen Anode und Kathode angeordnet ist und die ein organisches Molekül nach Anspruch 1 bis 5 enthält.

12. Optoelektronisches Bauelement nach Anspruch 11, in dem die lichtemittierende Schicht mindestens ein Hostmaterial aufweist, wobei insbesondere der angeregte Singulettzustand (S₁) und/oder der angeregte Triplettzustand (T₁) des mindestens einen Hostmaterials höher ist als der angeregte Singulettzustand (S₁) und/oder der angeregte Triplettzustand (T₁) des organischen Moleküls nach Anspruch 1 bis 5.

13. Optoelektronisches Bauelement nach Anspruch 10 bis 12, wobei mindestens ein Hostmaterial aus einem Material nach Anspruch 1 bis 5 besteht.

14. Optoelektronisches Bauelement nach Anspruch 10 bis 13, wobei die lichtemittierende Schicht ein fluoreszentes oder phosphoreszentes Material aufweist und wobei ein Material der lichtemittierenden Schicht ein organisches Moleküle nach Anspruch 1 bis 5 ist.

15. Optoelektronisches Bauelement nach Anspruch 14, in dem ein organischen Moleküls gemäß Anspruch 1 bis 5 und ein funktionelles Material einen Exciplex bilden.

16. Optoelektronisches Bauelement nach Anspruch 14 oder 15, wobei die Lichtemission durch thermisch aktivierte verzögerte Fluoreszenz (TADF) charakterisiert ist.

17. Optoelektronisches Bauelement nach Anspruch 10 oder 11, in dem ein organisches Molekül nach Anspruch 1 bis 5 als Ladungstransportschicht verwendet wird.

18. Verfahren zur Herstellung eines optoelektronischen Bauelements, wobei ein organisches Molekül nach Anspruch 1 bis 5 verwendet wird.

19. Verfahren nach Anspruch 18, wobei die Verarbeitung des organischen Moleküls mittels eines Vakuumverdampfungsverfahrens oder aus einer Lösung erfolgt.

20. Verwendung eines Separators S in Form einer neutralen chemischen Einheit zur Bereitstellung eins organischen Moleküls nach Anspruch 1 bis 5, wobei mindestens eine erste chemische Einheit AF1, aufweisend ein konjugiertes System, insbesondere mindestens sechs in Konjugation stehende π-Elektronen und mindestens eine zweite chemische Einheit AF2, aufweisend ein konjugiertes System, insbesondere mindestens sechs in Konjugation stehende π-Elektronen, kovalent über den Separator S derart verknüpft wird, dass der Separator S eine direkte elektronische Wechselwirkung über konjugierte Bindungen zwischen dem konjugierten System der ersten chemischen Einheit AF1 und dem konjugierten System der zweiten chemischen Einheit AF2 unterbindet.

21. Verfahren zur Herstellung eines organischen Moleküls nach Anspruch 1 bis 6, wobei die Herstellung mittels einer Diels-Alder-Reaktion erfolgt, insbesondere Herstellung eines organischen Moleküls nach Formel 2a oder 2b gemäß Schema 1 oder Schema 2
